# EUROPEAN PATENT SPECIFICATION

(11) **EP 4 308 244 B1**
(45) Date of publication and mention of the grant of the patent: **17.06.2026**
(21) Application number: 22714428.4
(22) Date of filing: 14.03.2022
(51) Int. Cl.: A61P 35/02, A61P 37/06, C07D 471/04, C07D 519/00, A61K 31/5025

(54) **HETEROCYCLIC DERIVATIVES AS JANUS KINASE INHIBITORS**
HETEROCYCLISCHE DERIVATE ALS JANUS-KINASEHEMMER
DÉRIVÉS HÉTÉROCYCLIQUES COMME INHIBITEURS DE JANUS KINASE

(30) Priority: 15.03.2021 EP 21162515; 23.12.2021 EP 21217278
(43) Date of publication of application: 24.01.2024
(73) Proprietor: Chiesi Farmaceutici S.p.A., 43122 Parma (IT)
(72) Inventor: ACCETTA, Alessandro, 43122 Parma (IT); RANCATI, Fabio, 43122 Parma (IT); RIZZI, Andrea, 43122 Parma (IT); CUZZOLIN, Alberto, 43122 Parma (IT); MESIC, Milan, 43122 Parma (IT); ZIHER, Dinko, 43122 Parma (IT); ELENKOV, Ivaylo, 43122 Parma (IT); BUTKOVIC, Kristina, 43122 Parma (IT)
(74) Representative: Chiesi Farmaceutici S.p.A.
(86) International application number: PCT/EP2022/056551
(87) International publication number: WO 2022/194781

(56) References cited:
- WO-A1-2008/052734
- WO-A1-2012/069202
- HOWELL MICHAEL D. ET AL: "Targeting the Janus Kinase Family in Autoimmune Skin Diseases", FRONTIERS IN IMMUNOLOGY, vol. 10, 1 January 2019 (2019-01-01), pages 2342, XP055863286, Retrieved from the Internet <URL:https://www.ncbi.nlm.nih.gov/pmc/articles/PMC6794457/pdf/fimmu-10-02342.pdf> DOI: 10.3389/fimmu.2019.02342

## Description

### FIELD OF THE INVENTION

The present invention relates to chemical compounds that are derivatives useful as JAK inhibitors, such as JAK 1, useful for the treatment of various inflammatory disease including asthma, COPD and other respiratory diseases.

### BACKGROUND OF THE INVENTION

The JAK family consists of non-receptor tyrosine protein kinases and has four main members, JAK1, JAK2, JAK3, and TYK2. More than 50 cytokines and growth factors bind to type I and II receptors noncovalently associated with different combinations of JAK kinases. The signalling triggered by the ligands consists in tyrosine phosphorylation of receptors by JAK and recruitment of one or more STATs proteins. Tyrosine-phosphorylated STATs dimerize and are then transported into the nucleus through the nuclear membrane to regulate specific genes. JAKs have seven homology domains (the JAK homology domain, JH). Starting from the carboxyl terminus, JH1 is the first JH, known as the kinase domain, and is composed of approximately 250 amino acid residues. JH1 encodes a kinase protein that constitutes the kinase structure domain that phosphorylates a substrate; JH2 is a pseudokinase domain which regulates the activity of the kinase domain. JAK3 is expressed in the bone marrow and lymphatic system, as well as endothelial cells and vascular smooth muscle cells; other members are expressed in almost all tissues (Hu X et al., Signal Transduct Target Ther. 2021, 26;6(1):402). Many cellular processes are downstream JAK/STAT signalling: hematopoiesis, immune balance, tissue repair, inflammation, apoptosis, and adipogenesis. Different biological responses are regulated by specific pairing of JAK isoforms. JAK1/JAK3 combination mediates IL-2, -4, -7, -9, -15, and -21 signalling that is relevant for growth/maturation of lymphoid cells, differentiation/homeostasis of T-cells/NK cells, B-cell class switching and other inflammatory processes. Combinations of JAK1/TYK2-JAK1/JAK2, regulate the signal associated with the innate immune response, such as IL-6 and the type I interferons, involved into naïve T cell differentiation, T cell homeostasis, granulopoiesis and other inflammatory processes. (Howell MD et al., Front. Immunol. 2019, 10, 2342). JAK2 frequently associates with itself (JAK2/ JAK2) controlling the signalling of various cytokines and growth factors, such as IL-3, IL-5, granulocyte macrophage colony-stimulating factor (GM-CSF), erythropoietin (EPO), and thrombopoietin (TPO) (Hodge et al., Clin Exp Rheumatol 2016; 34(2):318-28).

Genetically modified mouse models and human diseases prove the importance of JAK/STAT pathways in immune fitness. In particular, overexpression or mutations involving some JAK isoforms as well as aberrant JAK/STAT signalling drive malignancies of hematopoietic and lymphoid tissues as well as inflammatory disorders. Currently, several Food and Drug Administration (FDA)- and/or EU- approved JAK inhibitors are in clinical use. Two (ruxolitinib and fedratinib) small molecules are in use for hematologic disorders as myelofibrosis and polycythemia vera; six JAK inhibitors (tofacitinib, baricitinib, ruxololitinib, filgotinib, upadicitinib and delgocitinib in Japan) result in use for immune-mediated disorders as rheumatoid arthritis, polyarticular juvenile idiopathic arthritis, atopic dermatitis, ulcerative colitis and acute graft-versus-host disease. Moreover, some of these drugs as well as others are currently under phase II and III of clinical trials for indications that span from autoimmune diseases (lupus, vitiligo, etc.), inflammatory bowel disease to Non-Hodgkin lymphoma and COVID-19 (Hu X. et al., Sig Transduct Target Ther 2021, 6: 402).

The small molecules targeting JAK/STAT represent an attractive option also for the therapy of fibrotic disorders. In fact, inflammatory cytokines (IL-4, IL-3, IL-6, IL-11, IL-31, etc) and growth factors (FGF, VEGF, etc.) involved in the fibrotic processes activate JAK/STAT pathway. Ruxolitinib tested in a bleomycin-induced fibrosis mouse model ameliorated the fibrotic lesions in lung, and reduced levels of fibrotic molecular markers (Zhang, Y et al., Ann. Rheum. Dis. 2017, 76, 1467-1475) while tofacitinib acted as a preventive agent in experimental dermal and pulmonary fibrosis (Wang, W et al., Scleroderma Relat. Disord. 2020, 5, 40-50). In patients, some case reports were studied. A single-case report corroborated the efficacy and safety of tofacitinib in combination with nintedanib in the management of an aggressive interstitial lung disease with poor prognosis (Conca, W et al., Front. Pharmacol. 2020, 11, 5857619). Baricitinib was demonstrated to be a safe immune modulator that reduces the biomarkers' levels of lung fibrosis and inflammation in RA patients, including a subgroup with interstitial lung disease (D'Alessandro M et al., Int. Immunopharmacol. 2020, 86, 106748).

In COVID-19, there are some JAK inhibitors undergoing clinical trials, and they are tofacitinib, baricitinib, and ruxolitinib. Baricitinib and ruxolitinib were associated with a reduced risk of mortality. They reduced the use of invasive mechanical ventilation and had a borderline impact on the admission rate of the intensive care unit and the incidence of acute respiratory distress syndrome (ARDS). (Wijaya, I. et al. Clin. Epidemiol. Glob. Health 2021, 11, 100755). Ruxolitinib also was tested in COVID-19 patients. and improved the clinical symptoms and chest computed tomography images (Cao Y. et al., J. Allergy Clin. Immunol. 2020 146, 137-146).

Asthma can be included in the plethora of immune-mediated diseases for which pathogenesis is characterized by an essential role of JAK/STAT signalling. Asthma is a chronic inflammatory disease of the airways due to a complex interplay between immune response, genetic susceptibility and nonspecific external stimuli like cold, allergens and exercise leading to hyperresponsiveness, remodelling of the airways, ultimately contributing to airflow limitation. Severe asthma affects 5% to 15% of the population with adult asthma (which is 300 million people worldwide) and represents a public health issue associated with increased mortality, increased hospitalizations, significant burden of symptoms, health care costs, and missed work and school (Steve NG et al., J Allergy Clin Immunol 2021;148:953-63). Severe asthma represents a subset of difficult-to-treat asthma and occurs in patients whose disease remains uncontrolled despite the use of high doses of inhaled corticosteroids (ICSs) combined with long-acting β-agonists or other controllers. To date, four types of biologics are licensed for severe asthma, i.e. omalizumab (anti-immunoglobulin E) antibody, mepolizumab and reslizumab (anti-interleukin [IL]-5antibody), benralizumab (anti-IL-5 receptor a antibody) and dupilumab (anti-IL-4 receptor alpha antibody). Despite their efficacy, many patients continue to experience exacerbations or uncontrolled disease, indicating a need for more novel therapies (Israel E, Reddel HK. N Engl J Med 2017; 377:965-76).

Recently, the better understanding of asthma pathobiology brought to a shift from a phenotypic classification system to the introduction of the "endotype" concept. According to the latter, classification is performed on the basis of pathophysiologic mechanisms and clinical biomarkers associated with a given patient (Wenzel SE et a., Am J Respir Crit Care Med 2021;203:809-21). There are two major endotypes in asthma: type 2 and non-type 2. The type 2 pathway is defined by activation of cytokines derived from TH2 cells and group 2 innate lymphoid cells (ILC2s); these include IL-4, IL-5, and IL-13 that cause airway inflammation by activating eosinophils, B cells, airway epithelial cells, and other cell types. Biomarkers of type 2 asthma include blood/sputum eosinophilia and elevated levels of fractional exhaled nitric oxide (FENO) and IgE. The type 2-low pathway is characterized by absence of type 2-high cytokines and biomarkers, and it manifests either increased levels of neutrophils in the airways or a paucigranulocytic profile, with normal levels of airway neutrophils and eosinophils. Type 2-low asthma is currently not well understood, and it likely encompasses multiple distinct endotypes. Potential mediators and/or biomarkers of T2 low endotypes under investigation include IL-6, IL-17A/F, IL-23, Type I interferons, CXCL10, TNF, alarmins (TSLP, IL-25, IL-33), IL-1β, IL-8, IFN-γ (Hinks TSC et al., ERJ 2021, 57 (1) 2000528).

Almost all the mediators mentioned above both for T2 and T2-low endotypes activate JAK/STAT pathway, here the rationale for the potential use of JAK inhibitors in both endotypes of severe asthma. Targeting simultaneously several cytokines by JAK inhibitors may offer advantage over the biologics (for no-responder patients) and standard therapies (for patients who remain uncontrolled) considering their administration on top of ICS.

Despite strong rationale of JAK inhibitors in asthma, safety concerns may arise by administration of systemic inhibitors or may limits administration into particular asthma subjects such as children. Considering that Asthma is a lung restricted disease, inhalatory route of administration for a JAK inhibitor may offers the advantage of therapeutic efficacy while limiting systemic exposure and correlated side effects. To date, some companies are developing inhaled JAK inhibitor for asthma treatment. Astrazeneca pipeline include AZD-0449 (completed Phase I clinical trial) and AZD-4604 (ongoing Phase I clinical trial); Theravance Biopharma is starting a new preclinical program on TD-8236 inhaled JAK inhibitor and Kinaset/Vectura is developing VR588 (ongoing Phase I clinical trial) as inhalatory compound. Many preclinical studies sponsored by the companies mentioned above demonstrated the efficacy of JAK inhibitors in the modulation of asthma. In the preclinical phase of drug development, JAK1/3 inhibitor R256 (now referred as AZD0449) orally given showed be effective in decreasing airway resistance, BAL eosinophilia, mucus production and if administered during sensitization, also TH2 cytokine responses (Ashino S et al., J Allergy Clin Immunol 2014;133:1162-74). iJak-381 from Genentech given as dry powder reduced BAL eosinophilia, CCL11, airway resistance, and Muc5AC in OVA-challenged mice. Moreover, it reduced BAL eosinophilia, neutrophilia, CCL11, and CXCL1 in a in mouse model of chronic exposure to AAH allergens (Dengler HS et al., Sci Transl Med 2018;10:eaao2151). Moreover, an oral JAK inhibitor as Tofacitinib, formulated for being administered as aerosol, reduced eosinophils count in a house dust mite mouse model of asthma (Younis US et al., AAPS PharmSci-Tech 2019;20:167).

Another respiratory disease that could benefit from lung restricted JAK inhibition is Chronic obstructive pulmonary disease (COPD), an inflammatory disease of the lung, most commonly resulting from cigarette smoke exposure, characterised by a largely irreversible and progressive airflow limitation. Despite inflammatory cytokines are drivers of chronic airway inflammation and some of them trigger JAK/STAT activation (IL-6, IFN-γ, IL-2, etc.), the role of this pathway in COPD pathogenesis is poorly characterized. Phosphorylated-STAT4+ cells (Di Stefano A et al., Eur Respir J. 2004 Jul; 24(1):78-85) were found to be increased in COPD compared to non-smokers healthy controls. In another study, phosphorylated-STAT3+ and phosphorylated-STAT1+ cells counts were higher in lung biopsies of COPD patients than non-smokers controls while it was not possible to reproduce previous data on phosphorylated-STAT4 molecule (Yew-Booth L et al., Eur Respir J 2015; 46(3):843-5). These data might also suggest a therapeutic use of JAK inhibitors also in COPD disease.

In view of the number of pathological responses which are mediated by JAK enzymes, there is a continuing need for inhibitors of JAK enzymes which can be useful in the treatment of many disorders and particularly respiratory diseases.

Thus, the finding of novel and potent JAK inhibitor suitable for local administration to the lungs for treatment of asthma and respiratory disease still remains an important need.

### SUMMARY OF THE INVENTION

Accordingly, it is one object of the present invention to provide compounds of formula (I)

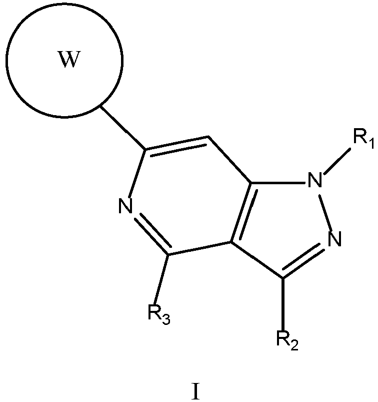

Wherein W, R₁, R₂, R₃ are as defined in the detailed description of the invention; or a pharmaceutically-acceptable salt thereof, that are useful as JAK kinase inhibitors.

It is another object of the present invention to provide pharmaceutical compositions comprising such compounds, for use in the prevention and/or treatment of respiratory diseases, and processes and intermediates useful for preparing such compounds.

In one aspect, the present invention provides a compound of formula (I) for use as a medicament. In one aspect the present invention provides the use of a compound of the invention for the manufacture of a medicament.

In a further aspect, the present invention provides a compound of the invention for use in the preparation of a medicament for the treatment of any disease associated with JAK enzyme mechanisms.

In a Particular aspect the compounds of the invention are used alone or combined with other active ingredients suitable to be administered for the prevention and/or treatment of a pulmonary disease including asthma, Chronic obstructive pulmonary disease (COPD), cystic fibrosis (CF), interstitial lung diseases and idiopathic pulmonary fibrosis (IPF), acute lung injury and acute respiratory distress syndrome (ARDS).

### DETAILED DESCRIPTION OF THE INVENTION

### Definitions

The term "Pharmaceutically acceptable salts" refers to derivatives of compounds of formula (I) wherein the parent compound is suitably modified by converting any of the free acid or basic group, if present, into the corresponding addition salt with any base or acid conventionally intended as being pharmaceutically acceptable.

Suitable examples of said salts may thus include mineral or organic acid addition salts of basic residues such as amino groups, as well as mineral or organic basic addition salts of acid residues such as carboxylic groups.

Cations of inorganic bases which can be suitably used to prepare salts of the invention comprise ions of alkali or alkaline earth metals such as potassium, sodium, calcium or magnesium. Those obtained by reacting the main compound, functioning as a base, with an inorganic or organic acid to form a salt comprise, for example, salts of hydrochloric, hydrobromic, sulfuric, phosphoric, methane sulfonic, camphor sulfonic, acetic, oxalic, maleic, fumaric, succinic and citric acids.

Many organic compounds can form complexes with solvents in which they are reacted or from which they are precipitated or crystallized. These complexes are known as "solvates" which are a further object of the invention. Polymorphs and crystalline forms of compounds of formula (I), or of pharmaceutically acceptable salts, or solvates thereof are a further object of the invention.

The term "Halogen" or "halogen atoms" includes fluorine, chlorine, bromine, and iodine atom ; meaning Fluoro, Chloro, Bromo, Iodo as substituent.

The term "(C₁-C₆)Alkyl" refers to straight-chained or branched alkyl groups wherein the number of carbon atoms is in the range 1 to 6. Particular alkyl groups are for example methyl, ethyl, n-propyl, isopropyl, t-butyl, 3-methylbutyl and the like.

The expressions "(C₁-C₆)Haloalkyl" refer to the above defined "(C₁-C₆)alkyl" groups wherein one or more hydrogen atoms are replaced by one or more halogen atoms, which can be the same or different from each other. Examples include halogenated, poly-halogenated and fully halogenated alkyl groups wherein all of the hydrogen atoms are replaced by halogen atoms, e.g. trifluoromethyl or difluoro methyl groups.

By way of analogy, the terms "(C₁-Cₓ) hydroxyalkyl" or "(C₁-Cₓ) aminoalkyl" refer to the above defined "(C₁-Cₓ) alkyl" groups wherein one or more hydrogen atoms are replaced by one or more hydroxy (OH) or amino group respectively.

The definition of aminoalkyl encompasses alkyl groups (i.e. "(C₁-C₆)alkyl" groups) substituted by one or more amino groups (-NR₄R₅). An example of aminoalkyl is a mono-aminoalkyl group such as R₄R₅N-(C₁-C₆)alkyl, or -(CH₂)ₘNR₄R₅. Wherein R₄ and R₅ and m are as defined in the detailed description of the invention.

With reference to the substituent R₄ and R₅ as above defined, it is here further explained that when either R₄ and R₅ are taken together with the nitrogen atom they are linked to form a 5 to 6 membered heterocyclic radical, at least one further ring carbon atom in the said heterocyclic radical may be replaced by at least one heteroatom or hetero-group (e.g. N, NH, S or O) or may bear an -oxo (=O) substituent group. The said heterocyclic radical might be further optionally substituted on the available points in the ring, namely on a carbon atom, or on an heteroatom or hetero-group available for substitution. Thus, Examples of said heterocycle radicals are 1-pyrrolidinyl, 1-piperidinyl, 1-piperazinyl, 4-morpholinyl, piperazin-4yl-2-one, 4-methylpiperazine-1-yl.

The term "(C₃-C₁₀)cycloalkyl" likewise "(C₃-C₆)cycloalkyl" refers to saturated cyclic hydrocarbon groups containing the indicated number of ring carbon atoms. Examples include cyclopropyl, cyclobutyl, cyclopentyl, cyclohexyl and cycloheptyl, and polycyclic ring systems such as adamantan-yl.

The expression "Aryl" refers to mono, bi- or tri-cyclic carbon ring systems which have 6 to 20, preferably from 6 to 15 ring atoms, wherein at least one ring is aromatic. The expression "heteroaryl" refers to mono-, bi- or tri-cyclic ring systems with 5 to 20, preferably from 5 to 15 ring atoms, in which at least one ring is aromatic and in which at least one ring atom is a heteroatom (e.g. N, S or O).

Examples of aryl or heteroaryl monocyclic ring systems include, for instance, phenyl, thienyl, pyrrolyl, pyrazolyl, imidazolyl, isoxazolyl, oxazolyl, isothiazolyl, thiazolyl, pyridinyl, pyrimidinyl, pyrazinyl, pyridazinyl, triazinyl, furanyl radicals and the like.

Examples of aryl or heteroaryl bicyclic ring systems include naphthalenyl, biphenylenyl, purinyl, pteridinyl, pyrazolopyrimidinyl, benzotriazolyl, benzoimidazoleyl, quinolinyl, isoquinolinyl, indolyl, isoindolyl, indazolyl, benzothiopheneyl, benzodioxinyl, dihydrobenzodioxinyl, indenyl, dihydro-indenyl, dihydrobenzo[1,4]dioxinyl, benzothiazole-2-yl, dihydrobenzodioxepinyl, benzooxazinyl, 1,2,3,4-tetrahydroisoquinoline-6-yl, 4,5,6,7-tetrahydrothiazolo[4,5-c]pyridine, 4,5,6,7-tetrahydrobenzo[d]thiazol-2-yl, 5,6,7,8-tetrahydro-1,7-naphthyridine, radicals and the like.

Examples of aryl or heteroaryl tricyclic ring systems include fluorenyl radicals as well as benzocondensed derivatives of the aforementioned heteroaryl bicyclic ring systems.

The derived expression "(C₃-C₁₀)heterocycloalkyl" likewise "(C₃-C₆)heterocycloalkyl" refers to saturated or partially unsaturated mono, bi- or tricycloalkyl groups of the indicated number of carbons, in which at least one ring carbon atom is replaced by at least one heteroatom (e.g. N, NH, S or O) and/or may bear an -oxo (=O) substituent group (e.g. C(=O), S(=O)₂). Said heterocycloalkyl (i.e. heterocyclic radical or group) is further optionally substituted on the available points in the ring, namely on a carbon atom, or on an heteroatom available for substitution. Examples of heterocycloalkyl are represented by: oxetanyl, tetrahydro-furanyl, pyrrolidinyl, imidazolidinyl, thiazolidinyl, piperazinyl, piperidinyl, morpholinyl, thiomorpholinyl, dihydro- or tetrahydro-pyridinyl, tetrahydropyranyl, pyranyl, 2H- or 4H-pyranyl, dihydro- or tetrahydrofuranyl, dihydroisoxazolyl, 9yridine9ne-2-one-yl, dihydropyrrolyl, 5-oxopyrrolidin-3-yl, (1R,5S,6r)-3-oxabicyclo[3.1.0]hexan-6-yl, 1,1-dioxidothiomorpholino, octahydrocyclopenta[c]pyrrol-5-yl, 4,5,6,7-tetrahydropyrazolo[1,5-a]pyrazin-2-yl; 4,5,6,7-tetrahydrothiazolo[5,4-c]9yridine-2-yl radicals and the like.

The term "Aryl(C₁-C₆)alkyl" refers to an aryl ring linked to a straight-chained or branched alkyl group wherein the number of constituent carbon atoms is in the range from 1 to 6, e.g. phenylmethyl (i.e. benzyl), phenylethyl or phenylpropyl.

Likewise the term "Heteroaryl(C₁-C₆)alkyl" refers to an heteroaryl ring linked to a straight-chained or branched alkyl group wherein the number of constituent carbon atoms is in the range from 1 to 6, e.g. furanylmethyl.

The term "alkanoyl", refers to HC(O)- or to alkylcarbonyl groups (e.g. (C₁-C₆)alkylC(O)-) wherein the group "alkyl" has the meaning above defined. Examples include formyl, acetyl, propanoyl, butanoyl.

The term "(C₁-C₁₀) alkoxy" or "(C₁-C₁₀) alkoxyl", likewise "(C₁-C₆) alkoxy" or "(C₁-C₆) alkoxyl" etc., refers to a straight or branched hydrocarbon of the indicated number of carbons, linked to the rest of the molecule through an oxygen bridge. "(C₁-C₆)Alkylthio" refers to the above hydrocarbon linked through a sulfur bridge.

The derived expression "(C₁-C₆)haloalkoxy" or "(C₁-C₆)haloalkoxyl" refers to the above defined haloalkyl, linked through an oxygen bridge. Example of (C₁-C₆)haloalkoxy is difluoromethoxy, trifluoromethoxy.

Likewise derived expression "(C₃-C₆)heterocycloalkyl-(C₁-C₆)alkyl" and "(C₃-C₆)cycloalkyl-(C₁-C₆)alkyl" refer to the above defined heterocycloalkyl and cycloalkyl groups linked to the rest of the molecule via an alkyl group of the indicated number of carbons, for example piperidin-4-yl-methyl, cyclohexylethyl.

The derived expression "(C₁-C₆)alkoxy (C₁-C₆)alkyl" refers to the above defined alkoxy group linked to the rest of the molecule via an alkyl group of the indicated number of carbons, for example methoxymethyl.

Likewise "(C₁-C₆)haloalkoxy(C₁-C₆)alkyl" refers to the above defined (C₁-C₆)haloalkoxy" group linked to the rest of the molecule via an alkyl group of the indicated number of carbons, for example difluoromethoxypropyl.

Likewise "(C₁-C₆)alkoxycarbonyl" refers to the above defined alkoxy group linked to the rest of the molecule via an carbonyl group.

And "(C₁-C₆)alkoxycarbonyl-(C₁-C₆)alkyl" refers to the above defined alkoxy group linked to the rest of the molecule via an carbonyl group further enchained with an alkyl group of the indicated number of carbons, for example methoxycarbonylmethyl.

And "(C₁-C₆)alkoxycarbonyl-(C₁-C₆)alkylthio consequently refer to enchained groups like methoxycarbonylmethylthio.

An oxo moiety is represented by (O) as an alternative to the other common representation, e.g. (=O). Thus, in terms of general formula, the carbonyl group is herein preferably represented as -C(O)- as an alternative to the other common representations such as -CO-, -(CO)- or -C(=O)-. In general the bracketed group is a lateral group, not included into the chain, and brackets are used, when deemed useful, to help disambiguating linear chemical formulas; e.g. the sulfonyl group -SO₂- might be also represented as-S(O)₂- to disambiguate e.g. with respect to the sulfinic group -S(O)O-.

When a numerical index the statement (value) "p is zero" or "p is 0" means that the substituent or group bearing the index p (e.g. Ip) is absent, that is to say no substituent, other than H when needed, is present. Likewise when the index is attached to a bridging divalent group (e.g. (CH₂)n) the statement "n in each occurrence is zero..." or "n is 0" means that the bridging group is absent, that is to say it is a bond.

Whenever basic amino or quaternary ammonium groups are present in the compounds of formula (I), physiological acceptable anions, selected among chloride, bromide, iodide, trifluoroacetate, formate, sulfate, phosphate, methanesulfonate, nitrate, maleate, acetate, citrate, fumarate, tartrate, oxalate, succinate, benzoate, p-toluenesulfonate, pamoate and naphthalene disulfonate may be present. Likewise, in the presence of acidic groups such as COOH groups, corresponding physiological cation salts may be present as well, for instance including alkaline or alkaline earth metal ions.

Compounds of formula (I) when they contain one or more stereogenic center, may exist as optical stereoisomers.

Where the compounds of the invention have at least one stereogenic center, they may accordingly exist as enantiomers. Where the compounds of the invention possess two or more stereogenic centers, they may additionally exist as diastereoisomers. It is to be understood that all such single enantiomers, diastereoisomers and mixtures thereof in any proportion are encompassed within the scope of the present invention. The absolute configuration (R) or (S) for carbon bearing a stereogenic center is assigned on the basis of Cahn-Ingold-Prelog nomenclature rules based on groups' priorities.

"Single stereoisomer", "single diastereoisomer" or "single enantiomer", when reported near the chemical name of a compound indicate that the isomer was isolated as single diastereoisomer or enantiomer (e.g via chiral chromatography) but the absolute configuration at the relevant stereogenic center was not determined/assigned.

Atropisomers result from hindered rotation about single bonds where the steric strain barrier to rotation is high enough to allow for the isolation of the conformers (Bringmann G et al, Angew. Chemie Int. Ed. 44 (34), 5384-5427, 2005. doi:10.1002/anie.200462661).

Oki defined atropisomers as conformers that interconvert with a half-life of more than 1000 seconds at a given temperature (Oki M, Topics in Stereochemistry 14, 1-82, 1983).

Atropisomers differ from other chiral compounds in that in many cases they can be equilibrated thermally whereas in the other forms of chirality isomerization is usually only possible chemically.

Separation of atropisomers is possible by chiral resolution methods such as selective crystallization. In an atropo-enantioselective or atroposelective synthesis one atropisomer is formed at the expense of the other. Atroposelective synthesis may be carried out by use of chiral auxiliaries like a Corey Bakshi Shibata (CBS) catalyst, an asymmetric catalyst derived from proline, or by approaches based on thermodynamic equilibration when an isomerization reaction favors one atropisomer over the other.

Racemic forms of compounds of formula (I) as well as the individual atropisomers (substantially free of its corresponding enantiomer) and stereoisomer-enriched atropisomer mixtures are included in the scope of the present invention.

The invention further concerns the corresponding deuterated derivatives of compounds of formula (I). In the context of the present invention, deuterated derivative means that at least one position occupied by a hydrogen atom is occupied by deuterium in an amount above its natural abundance. Preferably, the percent of deuterium at that position is at least 90%, more preferably at least 95%, even more preferably 99%.

All preferred groups or embodiments described above and here below for compounds of formula (I) may be combined among each other and apply as well *mutatis mutandis.*

As above mentioned, the present invention provides compounds of general formula (I), acting as JAK inhibitors, to processes for the preparation thereof, pharmaceutical compositions comprising them either alone or in combination with one or more active ingredient, in admixture with one or more pharmaceutically acceptable carriers.

In a first aspect the present invention provides a class of compounds of formula (I) 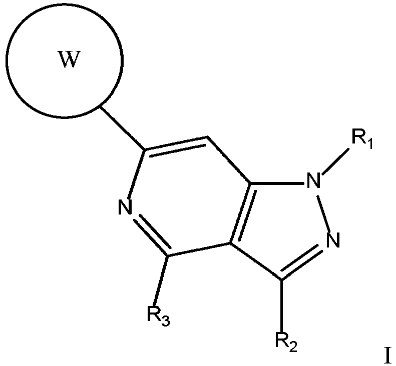 Wherein,
W is a heteroaryl selected from pyrazolo[1,5-a]pyrimidin-3-yl, imidazo[1,2-b]pyridazin-3-yl and (3-oxo-3,4-dihydropyrazin-2-yl)amino;
R₁ is selected in the group of pyridinyl, or phenyl optionally substituted by one or more group, preferably 2 or 3 groups, independently selected from
halogen, preferably Cl and F,
-OH,
-CN,
-NO₂,
-(CH₂)ₘNR₄R₅, which is preferably -NH₂
(C₁-C₆)alkyl,
(C₁-C₆)hydroxyalkyl,
(C₁-C₆)alkoxy, preferably methoxy,
(C₁-C₆)alkylthio-,
(C₁-C₆)haloalkyl,
(C₁-C₆)haloalkoxy, preferably difluoromethoxy,
and R₁, particularly preferably when it is phenyl, it is substituted by at least one further group of formula K 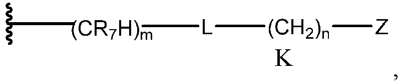 in para position with respect to the point of attachment of R₁ with the rest of the molecule;
**L** is absent or is a divalent group selected from O, S, S(O)₂, (CO), C(O)O, O(O)C, C(O)N(R₆), N(R₆)C(O), NHCONH, N(R₆)S(O)₂, S(O)₂N(R₆);
**Z** is selected from the group consisting of -OH, -CN, -NO₂, (C₁-C₆)alkyl, (C₁-C₆)hydroxyalkyl, (C₁-C₆)haloalkyl, (C₁-C₆)alkoxy, (C₁-C₆)alkoxy (C₁-C₆)alkyl, (C₁-C₆)alkoxycarbonyl, -(CH₂)ₘNR₄R₅, -C(O)NH(R₆), (C₃-C₈)cycloalkyl, aryl, heteroaryl and (C₃-C₆)heterocycloalkyl; wherein said
(C₃-C₈)cycloalkyl, aryl, heteroaryl and (C₃-C₆)heterocycloalkyl are further optionally substituted by one or more substituents selected from the group consisting of
(C₁-C₁₀)alkyl, alkanoyl, (C₁-C₆)alkoxycarbonyl, oxo, -C(O)NH(R₆), (C₁-C₆)alkoxy(C₁-C₆)alkyl;
**R₃** is H, and
**R₂ is** selected independently from the group consisting of H, (C₁-C₆)alkyl, preferably methyl, and a group of formula J 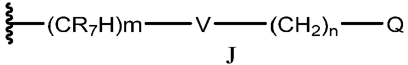
wherein
**V** is absent or is a divalent group selected from O, S, S(O)₂, C(O), C(O)O, O(O)C, C(O)N(R₆), N(R₆)C(O); N(R₆)-(CH₂)ₘ-N(R₆),-N(R₆)-;
**Q** is selected from the group consisting of -CN, -OH, (C₁-C₆)alkyl, (C₁-C₆)hydroxyalkyl, (C₁-C₆)haloalkyl, (C₁-C₆)alkoxy, (C₁-C₆)alkoxycarbonyl, hydroxycarbonyl, -(CH₂)ₘNR₄R₅, -C(O)NR₄R₅, -N(R₆)C(O)R₆, -CH(CN)NR₄R₅, (C₃-C₈)cycloalkyl, aryl, heteroaryl and (C₃-C₆)heterocycloalkyl; wherein said (C₃-C₈)cycloalkyl, aryl, heteroaryl and (C₃-C₆)heterocycloalkyl are further optionally substituted by one or more substituents selected from the group consisting of -OH, oxo, (C₁-C₁₀)alkyl, (C₁-C₁₀)alkyl-S(O)₂-O-, alkanoyl, (C₁-C₆)hydroxyalkyl, (C₁-C₆)alkoxy(C₁-C₆)alkyl, (C₁-C₆)alkoxycarbonyl, (C₁-C₆)alkoxycarbonyl-NH-, -N(R₆)(CH₂)ₘC(O)NR₄R₅, -NR₄R₅, (C₃-C₆)heterocycloalkyl;
wherein **n and m are in each occurrence independently** 0 or an integer selected from 1, 2, 3 and 4;
**R₄ and R₅,** the same or different, are selected from the group consisting of -H,
(C₁-C₆)alkyl,
(C₁-C₆)haloalkyl,
(C₁-C₆)hydroxyalkyl,
alkanoyl, (C₁-C₆)alkoxycarbonyl, and
(C₃-C₆)heterocycloalkyl;
R₆ is in each occurrence independently selected from the group consisting of H, (C₁-C₆)alkyl, (C₁-C₆)hydroxyalkyl, and alkanoyl,
R₇ is in each occurrence independently selected from the group consisting of H, (C₁-C₆)alkyl, -NR₄R₅
or a pharmaceutically acceptable salt or solvate thereof.

Preferably compounds according to the invention are 1H-pyrazolo[4,3-c]pyridine, 1H-pyrazolo[4,3-b]pyridine or 1H-pyrazolo[3,4-b]pyridine derivatives.

In a preferred embodiment the present invention provides compounds of formula (I) further having R₃ which is H, R₂ which is a group J and R₁ which is a substituted phenyl, as represented in formula (Ia)

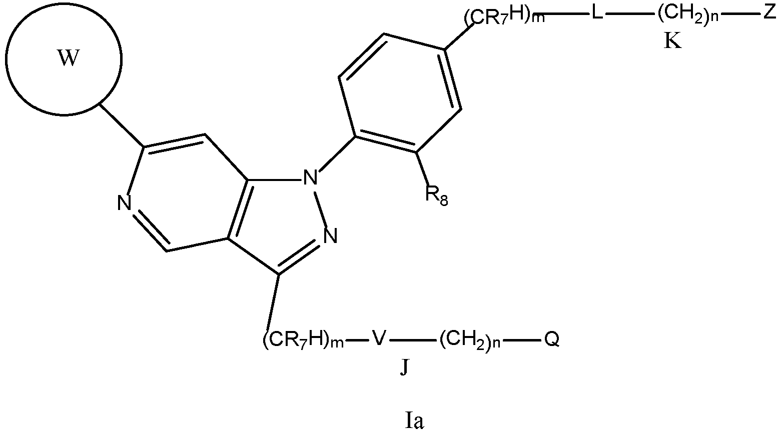

Wherein
R₈ is selected in the group consisting of
(C₁-C₆)alkoxy,
(C₁-C₆)haloalkoxy;
**L** is N(R₆)S(O)₂, S(O)₂N(R₆);
**Z** is selected from the group consisting of (C₁-C₆)alkyl, , (C₁-C₆)haloalkyl, (C₁-C₆)alkoxy, (C₁-C₆)alkoxycarbonyl, -(CH₂)ₘNR₄R₅, -C(O)NH(R₆),
(C₃-C₈)cycloalkyl, aryl, heteroaryl and (C₃-C₆)heterocycloalkyl said C₃-C₈)cycloalkyl, aryl, heteroaryl and (C₃-C₆)heterocycloalkyl being optionally substituted by one or more substituents selected from the group consisting of
(C₁-C₁₀)alkyl, alkanoyl, (C₁-C₆)alkoxycarbonyl, oxo, -C(O)NH(R₆), (C₁-C₆)alkoxy(C₁-C₆)alkyl;
**V** is absent or is selected from divalent groups consisting of -N(R₆)-,
**Q** is selected from the group consisting of (C₁-C₆)alkyl, -(CH₂)ₘNR₄R₅ and
(C₃-C₆)heterocycloalkyl, said (C₃-C₆)heterocycloalkyl being optionally substituted by one or more substituents selected from the group consisting of (C₁-C₁₀)alkyl;
wherein **n and m are in each occurrence independently** 0 or an integer from 1 to 4;
**R₄ and R_{5,}** the same or different, are selected from the group consisting of
-H,
(C₁-C₆)alkyl,
R₆ is in each occurrence independently selected from the group consisting of H, (C₁-C₆)alkyl, (C₁-C₆)hydroxyalkyl,
R₇ is in each occurrence independently selected from the group consisting of H, (C₁-C₆)alkyl,
or pharmaceutically acceptable salts and solvates thereof.

Particularly preferred in this last embodiment are compounds of formula (Ia) wherein
R₈ is (C₁-C₆)alkoxy, preferably methoxy;
**L** is -N(R₆)S(O)₂-;
**Z** is selected from the group consisting of
(C₁-C₆)alkyl, preferebly selected from methyl, ethyl and propyl; and
(C₃-C₈)cycloalkyl, preferably cyclopropyl;
**V** is absent or is -N(R₆)-;
**Q** is selected from the group consisting of,
-CN,
(C₁-C₆)alkyl, preferably methyl,
-(CH₂)ₘNR₄R₅, preferably dimethylaminoethyl and
(C₃-C₆)heterocycloalkyl, preferably selected from piperidin-1-yl and morpholin-yl;
wherein **n is 0 and m is 0 or 1 in group K; n is 0, 1 or 2 and m is 0 or 1 in group J;**
**R₄ and R_{5,}** the same or different, are selected from the group consisting of
-H,
(C₁-C₆)alkyl, preferably methyl;
R₆ is H,
R₇ is H,
or pharmaceutically acceptable salts and solvates thereof.

| **Example** | **Chemical Name** |
|---|---|
| 1 | N-(3-methoxy-4-(3-methyl-6-(pyrazolo[1,5-a]pyrimidin-3-yl)-1H-pyrazolo[4,3-c]pyridin-1-yl)phenyl)methanesulfonamide |
| 23 | N-(3-methoxy-4-(3-(methylamino)-6-(pyrazolo[1,5-a]pyrimidin-3-yl)-1H-pyrazolo[4,3-c]pyridin-1-yl)phenyl)methanesulfonamide |
| 24 | N-(3-methoxy-4-(3-((2-morpholinoethyl)amino)-6-(pyrazolo[1,5-a]pyrimidin-3-yl)-1H-pyrazolo[4,3-c]pyridin-1-yl)phenyl)methanesulfonamide |
| 31 | N-(4-(3-((2-(dimethylamino)ethyl)amino)-6-(pyrazolo[1,5-a]pyrimidin-3-yl)-1H-pyrazolo[4,3-c]pyridin-1-yl)-3-methoxyphenyl)methanesulfonamide |
| 32 | N-(4-(3-((2-(dimethylamino)ethyl)amino)-6-(pyrazolo[1,5-a]pyrimidin-3-yl)-1H-pyrazolo[4,3-c]pyridin-1-yl)-3-methoxyphenyl)cyclopropanesulfonamide |
| 34 | N-(3-methoxy-4-(3-((2-morpholinoethyl)amino)-6-(pyrazolo[1,5-a]pyrimidin-3-yl)-1H-pyrazolo[4,3-c]pyridin-1-yl)phenyl)cyclopropanesulfonamide |
| 36 | N-(3-methoxy-4-(3-((2-(piperidin-1-yl)ethyl)amino)-6-(pyrazolo[1,5-a]pyrimidin-3-yl)-1H-pyrazolo[4,3-c]pyridin-1-yl)phenyl)cyclopropanesulfonamide |
| 55 | N-(3-methoxy-4-(3-(methylamino)-6-(pyrazolo[1,5-a]pyrimidin-3-yl)-1H-pyrazolo[4,3-c]pyridin-1-yl)benzyl)methanesulfonamide |
| 60 | N-(4-(3-(cyanomethyl)-6-(pyrazolo[1,5-a]pyrimidin-3-yl)-1H-pyrazolo[4,3-c]pyridin-1-yl)-3-methoxybenzyl)methanesulfonamide |
| 85 | N-(3-methoxy-4-(3-methyl-6-((3-oxo-3,4-dihydropyrazin-2-yl)amino)-1H-pyrazolo[4,3-c]pyridin-1-yl)phenyl)methanesulfonamide |
| C31 (comparative) | N-(3-(3-((2-(dimethylamino)ethyl)amino)-6-(pyrazolo[1,5-a]pyrimidin-3-yl)-1H-pyrazolo[4,3-c]pyridin-1-yl)-4-methoxyphenyl)methanesulfonamide |

It is evident that compounds of the invention in this last preferred embodiments show an increase in potency (by at least 1 log or even up to 2 log on JAK1) when compared with example C31 having sulfonamidic meta substitution instead of para substitution in R1 when it is a Phenyl. In terms of inhibitory concentration the preferred compounds showed values lower than 50 nM at least on JAK1, preferably less than 5 nM.

The said preferred compounds showed balanced profile for inhalatory route of administration

Another particularly preferred embodiment is directed to compounds of formula (I)

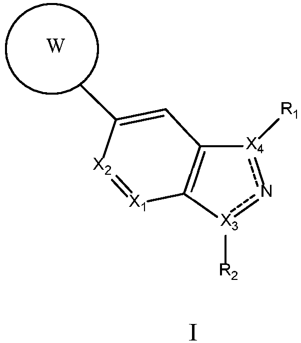

Wherein, X₁ and X₂ are alternatively N or CH; and
X₃ and X₄ are alternatively N or CH, and the two dashed lines indicate that a double bond is accordingly alternatively between X₃=N or between N=X₄,
W is a heteroaryl selected from pyrazolo[1,5-a]pyrimidin-3-yl, imidazo[1,2-b]pyridazin-3-yl
R₁ is selected in the group piperidinyl, phenyl or benzyl optionally substituted by one or more group selected from cyanomethylcarbonyl, difluoromethoxy, Cl and F.
R₂ is methyl or selected from hydroxycarbonylmethyl, metoxycarbonylmethyl, dimethylaminocarbonylmethyl, hydroxymethyl;
or pharmaceutically acceptable salts and solvates thereof.

According to specific embodiments, the present invention provides the compounds of examples 1a-10a (according to the last preferred embodiment described hereabove) and further compounds of examples 1-92, as listed in the table below, or pharmaceutical acceptable salts and solvates thereof.

| **Example** | **Chemical Name** |
|---|---|
| 1a | (1-(5-chloro-2-(difluoromethoxy)phenyl)-6-(pyrazolo[1,5-a]pyrimidin-3-yl)-1H-pyrazolo[4,3-b]pyridin-3-yl)methanol |
| 2a | 2-(3-(5-chloro-2-(difluoromethoxy)phenyl)-5-(pyrazolo[1,5-a]pyrimidin-3-yl)-1H-pyrazolo[3,4-b]pyridin-1-yl)-N,N-dimethylacetamide |
| 3a | methyl 1-(5-chloro-2-(difluoromethoxy)phenyl)-6-(pyrazolo[1,5-a]pyrimidin-3-yl)-1H-pyrazolo[4,3-b]pyridine-3-carboxylate |
| 4a | 2-(1-(5-chloro-2-(difluoromethoxy)phenyl)-6-(pyrazolo[1,5-a]pyrimidin-3-yl)-1H-pyrazolo[4,3-b]pyridin-3-yl)-N,N-dimethylacetamide |
| 5a | 2-(1-(5-chloro-2-(difluoromethoxy)phenyl)-6-(pyrazolo[1,5-a]pyrimidin-3-yl)-1H-pyrazolo[4,3-b]pyridin-3-yl)acetic acid |
| 6a | 1-(5-chloro-2-(difluoromethoxy)phenyl)-6-( imidazo[1,2-b]pyridazin-3-yl)-3-methyl-1H-pyrazolo[4,3-c]pyridine |
| 7a | 1-(5-chloro-2-(difluoromethoxy)phenyl)-3-methyl-6-(pyrazolo[1,5-a]pyrimidin-3-yl)-1H-pyrazolo[4,3-c]pyridine |
| 8a | 1-(4-chloro-2-fluorobenzyl)-6-( imidazo[1,2-b]pyridazin-3-yl )-3-methyl-1H-pyrazolo[4,3-b]pyridine |
| 9a | 1-(5-chloro-2-(difluoromethoxy)phenyl)-3-methyl-6-(pyrazolo[1,5-a]pyrimidin-3-yl)-1H-pyrazolo[4,3-b]pyridine |
| 10a | 3-(3-(3-methyl-6-(pyrazolo[1,5-a]pyrimidin-3-yl)-1H-pyrazolo[4,3-b]pyridin-1-yl)piperidin-1-yl)-3-oxopropanenitrile |
| 1 | N-(3-methoxy-4-(3-methyl-6-(pyrazolo[1,5-a]pyrimidin-3-yl)-1H-pyrazolo[4,3-c]pyridin-1-yl)phenyl)methanesulfonamide |
| 2 | N-(3-methoxy-4-(3-methyl-6-(pyrazolo[1,5-a]pyrimidin-3-yl)-1H-pyrazolo[4,3-c]pyridin-1-yl)phenyl)-1-(tetrahydrofuran-2-yl)methanesulfonamide |
| 3 | N-(3-methoxy-4-(3-methyl-6-(pyrazolo[1,5-a]pyrimidin-3-yl)-1H-pyrazolo[4,3-c]pyridin-1-yl)phenyl)tetrahydro-2H-pyran-4-sulfonamide |
| 4 | N-(3-methoxy-4-(3-methyl-6-(pyrazolo[1,5-a]pyrimidin-3-yl)-1H-pyrazolo[4,3-c]pyridin-1-yl)phenyl)propane-1-sulfonamide |
| 5 | 1-cyclopropyl-N-(3-methoxy-4-(3-methyl-6-(pyrazolo[1,5-a]pyrimidin-3-yl)-1H-pyrazolo[4,3-c]pyridin-1-yl)phenyl)methanesulfonamide |
| 6 | N-(3-methoxy-4-(3-methyl-6-(pyrazolo[1,5-a]pyrimidin-3-yl)-1H-pyrazolo[4,3-c]pyridin-1-yl)phenyl)cyclopropanesulfonamide |
| 7 | N-(3-(difluoromethoxy)-4-(3-methyl-6-(pyrazolo[1,5-a]pyrimidin-3-yl)-1H-pyrazolo[4,3-c]pyridin-1-yl)phenyl)methanesulfonamide |
| 8 | N-(3-methoxy-4-(3-methyl-6-(pyrazolo[1,5-a]pyrimidin-3-yl)-1H-pyrazolo[4,3-c]pyridin-1-yl)phenyl)oxetane-3-sulfonamide |
| 9 | N-(3-methoxy-4-(3-methyl-6-(pyrazolo[1,5-a]pyrimidin-3-yl)-1H-pyrazolo[4,3-c]pyridin-1-yl)phenyl)tetrahydrofuran-3-sulfonamide |
| 10 | N-(3-methoxy-4-(3-methyl-6-(pyrazolo[1,5-a]pyrimidin-3-yl)-1H-pyrazolo[4,3-c]pyridin-1-yl)phenyl)-1-methyl-1H-pyrazole-4-sulfonamide |
| 11 | N-(5-methoxy-6-(3-methyl-6-(pyrazolo[1,5-a]pyrimidin-3-yl)-1H-pyrazolo[4,3-c]pyridin-1-yl)pyridin-3-yl)methanesulfonamide |
| 12 | N-(2-fluoro-5-methoxy-4-(3-methyl-6-(pyrazolo[1,5-a]pyrimidin-3-yl)-1H-pyrazolo[4,3-c]pyridin-1-yl)phenyl)methanesulfonamide |
| 13 | 3-(N-(3-methoxy-4-(3-methyl-6-(pyrazolo[1,5-a]pyrimidin-3-yl)-1H-pyrazolo[4,3-c]pyridin-1-yl)phenyl)sulfamoyl)propanamide |
| 14 | 2-(N-(3-methoxy-4-(3-methyl-6-(pyrazolo[1,5-a]pyrimidin-3-yl)-1H-pyrazolo[4,3-c]pyridin-1-yl)phenyl)sulfamoyl)acetamide |
| 15 | 3-methoxy-4-(3-methyl-6-(pyrazolo[1,5-a]pyrimidin-3-yl)-1H-pyrazolo[4,3-c]pyridin-1-yl)benzonitrile |
| 16 | 4-methoxy-N-methyl-5-(3-methyl-6-(pyrazolo[1,5-a]pyrimidin-3-yl)-1H-pyrazolo[4,3-c]pyridin-1-yl)pyridin-2-amine |
| 17 | 3-methoxy-4-(3-methyl-6-(pyrazolo[1,5-a]pyrimidin-3-yl)-1H-pyrazolo[4,3-c]pyridin-1-yl)aniline |
| 18 | 1-(3-methoxy-4-(3-methyl-6-(pyrazolo[1,5-a]pyrimidin-3-yl)-1H-pyrazolo[4,3-c]pyridin-1-yl)phenyl)-3-methylurea |
| 19 | 3-methoxy-N-methyl-4-(3-methyl-6-(pyrazolo[1,5-a]pyrimidin-3-yl)-1H-pyrazolo[4,3-c]pyridin-1-yl)benzamide |
| 20 | 3-methoxy-4-(3-methyl-6-(pyrazolo[1,5-a]pyrimidin-3-yl)-1H-pyrazolo[4,3-c]pyridin-1-yl)-N-phenylbenzamide |
| 21 | tert-butyl (3-((1-(2-methoxy-4-(methylsulfonamido)phenyl)-6-(pyrazolo[1,5-a]pyrimidin-3-yl)-1H-pyrazolo[4,3-c]pyridin-3-yl)amino)propyl)carbamate |
| 22 | N-(4-(3-((3-(dimethylamino)propyl)amino)-6-(pyrazolo[1,5-a]pyrimidin-3-yl)-1H-pyrazolo[4,3-c]pyridin-1-yl)-3-methoxyphenyl)methanesulfonamide |
| 23 | N-(3-methoxy-4-(3-(methylamino)-6-(pyrazolo[1,5-a]pyrimidin-3-yl)-1H-pyrazolo[4,3-c]pyridin-1-yl)phenyl)methanesulfonamide |
| 24 | N-(3-methoxy-4-(3-((2-morpholinoethyl)amino)-6-(pyrazolo[1,5-a]pyrimidin-3-yl)-1H-pyrazolo[4,3-c]pyridin-1-yl)phenyl)methanesulfonamide |
| 25 | N-(3-(difluoromethoxy)-4-(3-((2-morpholinoethyl)amino)-6-(pyrazolo[1,5-a]pyrimidin-3-yl)-1H-pyrazolo[4,3-c]pyridin-1-yl)phenyl)cyclopropanesulfonamide |
| 26 | N-(4-(3-((2-hydroxyethyl)amino)-6-(pyrazolo[1,5-a]pyrimidin-3-yl)-1H-pyrazolo[4,3-c]pyridin-1-yl)-3-methoxyphenyl)methanesulfonamide |
| 27 | N-(3-methoxy-4-(3-(((1-(2-methoxyethyl)piperidin-4-yl)methyl)amino)-6-(pyrazolo[1,5-a]pyrimidin-3-yl)-1H-pyrazolo[4,3-c]pyridin-1-yl)phenyl)methanesulfonamide |
| 28 | N-(3 -methoxy-4-(3 -(((4-methylmorpholin-2-yl)methyl)amino)-6-(pyrazolo[1,5-a]pyrimidin-3-yl)-1H-pyrazolo[4,3-c]pyridin-1-yl)phenyl)methanesulfonamide |
| 29 | N-(6-(3-((2-(dimethylamino)ethyl)amino)-6-(pyrazolo[1,5-a]pyrimidin-3-yl)-1H-pyrazolo[4,3-c]pyridin-1-yl)-5-methoxypyridin-3-yl)cyclopropanesulfonamide |
| 30 | N-(4-(3-((2-(3-oxa-8-azabicyclo[3.2.1]octan-8-yl)ethyl)amino)-6-(pyrazolo[1,5-a]pyrimidin-3-yl)-1H-pyrazolo[4,3-c]pyridin-1-yl)-3-methoxyphenyl)methanesulfonamide |
| 31 | N-(4-(3-((2-(dimethylamino)ethyl)amino)-6-(pyrazolo[1,5-a]pyrimidin-3-yl)-1H-pyrazolo[4,3-c]pyridin-1-yl)-3-methoxyphenyl)methanesulfonamide |
| 32 | N-(4-(3-((2-(dimethylamino)ethyl)amino)-6-(pyrazolo[1,5-a]pyrimidin-3-yl)-1H-pyrazolo[4,3-c]pyridin-1-yl)-3-methoxyphenyl)cyclopropanesulfonamide |
| 33 | N-(3-methoxy-4-(3-((3-morpholinopropyl)amino)-6-(pyrazolo[1,5-a]pyrimidin-3-yl)-1H-pyrazolo[4,3-c]pyridin-1-yl)phenyl)cyclopropanesulfonamide |
| 34 | N-(3-methoxy-4-(3-((2-morpholinoethyl)amino)-6-(pyrazolo[1,5-a]pyrimidin-3-yl)-1H-pyrazolo[4,3-c]pyridin-1-yl)phenyl)cyclopropanesulfonamide |
| 35 | N-(3-methoxy-4-(3-(methylamino)-6-(pyrazolo[1,5-a]pyrimidin-3-yl)-1H-pyrazolo[4,3-c]pyridin-1-yl)phenyl)cyclopropanesulfonamide |
| 36 | N-(3-methoxy-4-(3-((2-(piperidin-1-yl)ethyl)amino)-6-(pyrazolo[1,5-a]pyrimidin-3-yl)-1H-pyrazolo[4,3-c]pyridin-1-yl)phenyl)cyclopropanesulfonamide |
| 37 | N-(3-methoxy-4-(3-((2-(piperidin-1-yl)ethyl)amino)-6-(pyrazolo[1,5-a]pyrimidin-3-yl)-1H-pyrazolo[4,3-c]pyridin-1-yl)phenyl)methanesulfonamide |
| 38 | N-(3-methoxy-4-(3-((2-(4-methylpiperazin-1-yl)ethyl)amino)-6-(pyrazolo[1,5-a]pyrimidin-3-yl)-1H-pyrazolo[4,3-c]pyridin-1-yl)phenyl)methanesulfonamide |
| 39 | N-(4-(3-((2-((2-fluoroethyl)(methyl)amino)ethyl)amino)-6-(pyrazolo[1,5-a]pyrimidin-3-yl)-1H-pyrazolo[4,3-c]pyridin-1-yl)-3-methoxyphenyl)methanesulfonamide |
| 40 | N-(3-methoxy-4-(3-(methyl(2-morpholinoethyl)amino)-6-(pyrazolo[1,5-a]pyrimidin-3-yl)-1H-pyrazolo[4,3-c]pyridin-1-yl)phenyl)methanesulfonamide |
| 41 | N-(1-(2-methoxy-4-(methylsulfonamido)phenyl)-6-(pyrazolo[1,5-a]pyrimidin-3-yl)-1H-pyrazolo[4,3-c]pyridin-3-yl)-1-methylpiperidine-4-carboxamide |
| 42 | N-(1-(2-methoxy-4-(methylsulfonamido)phenyl)-6-(pyrazolo[1,5-a]pyrimidin-3-yl)-1H-pyrazolo[4,3-c]pyridin-3-yl)-1-methylpyrrolidine-3-carboxamide |
| 43 | N-(3-(dimethylamino)propyl)-1-(2-methoxy-4-(methylsulfonamido)phenyl)-6-(pyrazolo[1,5-a]pyrimidin-3-yl)-1H-pyrazolo[4,3-c]pyridine-3-carboxamide |
| 44 | N-(2-(dimethylamino)ethyl)-1-(2-methoxy-4-(methylsulfonamido)phenyl)-6-(pyrazolo[1,5-a]pyrimidin-3-yl)-1H-pyrazolo[4,3-c]pyridine-3-carboxamide |
| 45 | 1-(2-methoxy-4-(methylsulfonamido)phenyl)-N-(2-morpholinoethyl)-6-(pyrazolo[1,5-a]pyrimidin-3-yl)-1H-pyrazolo[4,3-c]pyridine-3-carboxamide |
| 46 | N-(3-(dimethylamino)propyl)-1-(2-methoxy-4-(propylsulfonamido)phenyl)-6-(pyrazolo[1,5-a]pyrimidin-3-yl)-1H-pyrazolo[4,3-c]pyridine-3-carboxamide |
| 47 | 1-(2-methoxy-4-(propylsulfonamido)phenyl)-N-(3-morpholinopropyl)-6-(pyrazolo[1,5-a]pyrimidin-3-yl)-1H-pyrazolo[4,3-c]pyridine-3-carboxamide |
| 48 | N-(5-methoxy-6-(3-methyl-6-(pyrazolo[1,5-a]pyrimidin-3-yl)-1H-pyrazolo[4,3-c]pyridin-1-yl)pyridin-3-yl)benzamide |
| 49 | 3-methoxy-N-methyl-4-(3-methyl-6-(pyrazolo[1,5-a]pyrimidin-3-yl)-1H-pyrazolo[4,3-c]pyridin-1-yl)benzenesulfonamide |
| 50 | 3-methoxy-N-methyl-4-(3-((2-morpholinoethyl)amino)-6-(pyrazolo[1,5-a]pyrimidin-3-yl)-1H-pyrazolo[4,3-c]pyridin-1-yl)benzenesulfonamide |
| 51 | 3-methoxy-N-methyl-4-(3-(methylamino)-6-(pyrazolo[1,5-a]pyrimidin-3-yl)-1H-pyrazolo[4,3-c]pyridin-1-yl)benzenesulfonamide |
| 52 | 4-(3-((3-(dimethylamino)propyl)amino)-6-(pyrazolo[1,5-a]pyrimidin-3-yl)-1H-pyrazolo[4,3-c]pyridin-1-yl)-3-methoxy-N-methylbenzenesulfonamide |
| 53 | N-(3-methoxy-4-(3-((2-morpholinoethyl)amino)-6-(pyrazolo[1,5-a]pyrimidin-3-yl)-1H-pyrazolo[4,3-c]pyridin-1-yl)benzyl)methanesulfonamide |
| 54 | N-(3-methoxy-4-(3-((2-(piperidin-1-yl)ethyl)amino)-6-(pyrazolo[1,5-a]pyrimidin-3-yl)-1H-pyrazolo[4,3-c]pyridin-1-yl)benzyl)methanesulfonamide |
| 55 | N-(3-methoxy-4-(3-(methylamino)-6-(pyrazolo[1,5-a]pyrimidin-3-yl)-1H-pyrazolo[4,3-c]pyridin-1-yl)benzyl)methanesulfonamide |
| 56 | N-(4-(3-((2-(dimethylamino)ethyl)amino)-6-(pyrazolo[1,5-a]pyrimidin-3-yl)-1H-pyrazolo[4,3-c]pyridin-1-yl)-3-methoxybenzyl)methanesulfonamide |
| 57 | N-(2-chloro-5-methoxy-4-(3-(methylamino)-6-(pyrazolo[1,5-a]pyrimidin-3-yl)-1H-pyrazolo[4,3-c]pyridin-1-yl)benzyl)methanesulfonamide |
| 58 | N-(2-chloro-5-methoxy-4-(3-((2-morpholinoethyl)amino)-6-(pyrazolo[1,5-a]pyrimidin-3-yl)-1H-pyrazolo[4,3-c]pyridin-1-yl)benzyl)methanesulfonamide |
| 59 | N-(3-(dimethylamino)propyl)-1-(2-methoxy-4-(methylsulfonamidomethyl)phenyl)-6-(pyrazolo[1,5-a]pyrimidin-3-yl)-1H-pyrazolo[4,3-c]pyridine-3-carboxamide |
| 60 | N-(4-(3-(cyanomethyl)-6-(pyrazolo[1,5-a]pyrimidin-3-yl)-1H-pyrazolo[4,3-c]pyridin-1-yl)-3-methoxybenzyl)methanesulfonamide |
| 61 | N-(3-(difluoromethoxy)-4-(3-methyl-6-(pyrazolo[1,5-a]pyrimidin-3-yl)-1H-pyrazolo[4,3-c]pyridin-1-yl)benzyl)methanesulfonamide |
| 62 | N-(1-(3-methoxy-4-(3-methyl-6-(pyrazolo[1,5-a]pyrimidin-3-yl)-1H-pyrazolo[4,3-c]pyridin-1-yl)phenyl)ethyl)methanesulfonamide |
| 63 | N-(3-methoxy-4-(3-methyl-6-(pyrazolo[1,5-a]pyrimidin-3-yl)-1H-pyrazolo[4,3-c]pyridin-1-yl)benzyl)methanesulfonamide |
| 64 | N-(3-methoxy-4-(3-methyl-6-(pyrazolo[1,5-a]pyrimidin-3-yl)-1H-pyrazolo[4,3-c]pyridin-1-yl)benzyl)cyclopropanesulfonamide |
| 65 | N-(3-methoxy-4-(3-methyl-6-(pyrazolo[1,5-a]pyrimidin-3-yl)-1H-pyrazolo[4,3-c]pyridin-1-yl)benzyl)-N-methylmethanesulfonamide |
| 66 | (3-methoxy-4-(3-methyl-6-(pyrazolo[1,5-a]pyrimidin-3-yl)-1H-pyrazolo[4,3-c]pyridin-1-yl)phenyl)methanol |
| 67 | (3-methoxy-4-(3-methyl-6-(pyrazolo[1,5-a]pyrimidin-3-yl)-1H-pyrazolo[4,3-c]pyridin-1-yl)phenyl)methanamine |
| 68 | 1-cyano-N-(3-methoxy-4-(3-methyl-6-(pyrazolo[1,5-a]pyrimidin-3-yl)-1H-pyrazolo[4,3-c]pyridin-1-yl)benzyl)methanesulfonamide |
| 69 | methyl (3-methoxy-4-(3-methyl-6-(pyrazolo[1,5-a]pyrimidin-3-yl)-1H-pyrazolo[4,3-c]pyridin-1-yl)benzyl)carbamate |
| 70 | N-(3-methoxy-4-(3-methyl-6-(pyrazolo[1,5-a]pyrimidin-3-yl)-1H-pyrazolo[4,3-c]pyridin-1-yl)benzyl)acetamide |
| 71 | 1-(3-methoxy-4-(3-methyl-6-(pyrazolo[1,5-a]pyrimidin-3-yl)-1H-pyrazolo[4,3-c]pyridin-1-yl)benzyl)-3-methylurea |
| 72 | 1-(3-methoxy-4-(3-methyl-6-(pyrazolo[1,5-a]pyrimidin-3-yl)-1H-pyrazolo[4,3-c]pyridin-1-yl)benzyl)-3-phenylurea |
| 73 | 2,2-difluoro-N-(3-methoxy-4-(3-methyl-6-(pyrazolo[1,5-a]pyrimidin-3-yl)-1H-pyrazolo[4,3-c]pyridin-1-yl)phenyl)ethane-1-sulfonamide |
| 74 | N-(3-methoxy-4-(3-methyl-6-(pyrazolo[1,5-a]pyrimidin-3-yl)-1H-pyrazolo[4,3-c]pyridin-1-yl)phenyl)benzamide |
| 75 | N-(4-(3-((2-(dimethylamino)ethyl)amino)-6-(pyrazolo[1,5-a]pyrimidin-3-yl)-1H-pyrazolo[4,3-c]pyridin-1-yl)-3-methoxyphenyl)benzamide |
| 76 | N-(4-(6-(imidazo[1,2-b]pyridazin-3-yl)-3-((2-morpholinoethyl)amino)-1H-pyrazolo[4,3-c]pyridin-1-yl)-3-methoxyphenyl)methanesulfonamide |
| 77 | N-(4-(3-((2-(dimethylamino)ethyl)amino)-6-(imidazo[1,2-b]pyridazin-3-yl)-1H-pyrazolo[4,3-c]pyridin-1-yl)-3-methoxyphenyl)methanesulfonamide |
| 78 | N-(4-(3-(((1s,3s)-3-(dimethylamino)cyclobutyl)amino)-6-(pyrazolo[1,5-a]pyrimidin-3-yl)-1H-pyrazolo[4,3-c]pyridin-1-yl)-3-methoxyphenyl)cyclopropanesulfonamide |
| 79 | N-(4-(3-amino-6-(pyrazolo[1,5-a]pyrimidin-3-yl)-1H-pyrazolo[4,3-c]pyridin-1-yl)-3-methoxyphenyl)methanesulfonamide |
| 80 | N-(1-(2-methoxy-4-(methylsulfonamido)phenyl)-6-(pyrazolo[1,5-a]pyrimidin-3-yl)-1H-pyrazolo[4,3-c]pyridin-3-yl)-2-(methylamino)acetamide |
| 81 | N-(1-(2-methoxy-4-(methylsulfonamido)phenyl)-6-(pyrazolo[1,5-a]pyrimidin-3-yl)-1H-pyrazolo[4,3-c]pyridin-3-yl)-2-(4-methylpiperazin-1-yl)acetamide |
| 82 | N-(1-(2-methoxy-4-(propylsulfonamido)phenyl)-6-(pyrazolo[1,5-a]pyrimidin-3-yl)-1H-pyrazolo[4,3-c]pyridin-3-yl)-3-(4-methylpiperazin-1-yl)propanamide |
| 83 | N-(3-methoxy-4-(3-((methylamino)methyl)-6-(pyrazolo[1,5-a]pyrimidin-3-yl)-1H-pyrazolo[4,3-c]pyridin-1-yl)phenyl)propane-1-sulfonamide |
| 84 | N-(4-(3-(cyanomethyl)-6-(pyrazolo[1,5-a]pyrimidin-3-yl)-1H-pyrazolo[4,3-c]pyridin-1-yl)-3-methoxyphenyl)cyclopropanesulfonamide |
| 85 | N-(3-methoxy-4-(3-methyl-6-((3-oxo-3,4-dihydropyrazin-2-yl)amino)-1H-pyrazolo[4,3-c]pyridin-1-yl)phenyl)methanesulfonamide |
| 86 | N-(3-methoxy-4-(3-methyl-6-((3-oxo-3,4-dihydropyrazin-2-yl)amino)-1H-pyrazolo[4,3-c]pyridin-1-yl)phenyl)-1-methyl-1H-pyrazole-4-sulfonamide |
| 87 | N-(3-methoxy-4-(3-((2-morpholinoethyl)amino)-6-((3-oxo-3,4-dihydropyrazin-2-yl)amino)-1H-pyrazolo[4,3-c]pyridin-1-yl)phenyl)methanesulfonamide |
| 88 | N-(3-methoxy-4-(6-((3-oxo-3,4-dihydropyrazin-2-yl)amino)-3-((2-(piperidin-1-yl)ethyl)amino)-1H-pyrazolo[4,3-c]pyridin-1-yl)phenyl)propane-1-sulfonamide |
| 89 | N-(3-(difluoromethoxy)-4-(3-((2-morpholinoethyl)amino)-6-((3-oxo-3,4-dihydropyrazin-2-yl)amino)-1H-pyrazolo[4,3-c]pyridin-1-yl)phenyl)propane-1-sulfonamide |
| 90 | N-(4-(3-((2-(3-oxa-8-azabicyclo[3.2.1]octan-8-yl)ethyl)amino)-6-((3-oxo-3,4-dihydropyrazin-2-yl)amino)-1H-pyrazolo[4,3-c]pyridin-1-yl)-3-methoxyphenyl)methanesulfonamide |
| 91 | N-(3-methoxy-4-(3-(methylamino)-6-((3-oxo-3,4-dihydropyrazin-2-yl)amino)-1H-pyrazolo[4,3-c]pyridin-1-yl)phenyl)propane-1-sulfonamide |
| 92 | N-(3-methoxy-4-(3-((2-morpholinoethyl)amino)-6-((3-oxo-3,4-dihydropyrazin-2-yl)amino)-1H-pyrazolo[4,3-c]pyridin-1-yl)benzyl)methanesulfonamide |

The compounds of the invention, including all the compounds hereabove listed, can be prepared from readily available starting materials using general methods and procedures as described in the experimental part below or by using slightly modified processes readily available to those of ordinary skill in the art. Although a particular embodiment of the present invention may be shown or described herein, those skilled in the art will recognize that all embodiments or aspects of the present invention can be prepared using the methods described herein or by using other known methods, reagents and starting materials. When typical or preferred process conditions (i.e. reaction temperatures, times, mole ratios of reactants, solvents, pressures, etc.) are given, other process conditions can also be used unless otherwise stated. While the optimum reaction conditions may vary depending on the particular reactants or solvent used, such conditions can be readily determined by those skilled in the art by routine optimization procedures. The general schemes and detailed procedures are described in the PREPARATION OF INTERMEDIATES AND EXAMPLES sections below.

As herein described in details, the compounds of the invention are inhibitors of kinase activity, in particular. inhibiting JAK kinase activity for the treatment of JAK-dependent diseases.

In one aspect the invention provides compounds according to the invention , i.e. a compound of formula (I) or a pharmaceutical composition thereof, for use as a medicament, preferably for the prevention and /or treatment of respiratory and specifically pulmonary disease.

In a further aspect the invention provides the use of a compound (I), or a pharmaceutically acceptable salt thereof, in the manufacture of a medicament for the treatment of disorders associated with JAK mechanisms, particularly for the treatment of disorders such as respiratory and pulmonary diseases.

In particular the invention provides compounds of formula (I) for use in the prevention and /or treatment of pulmonary disease selected from the group consisting of asthma, chronic obstructive pulmonary disease COPD, idiopathic pulmonary fibrosis (IPF)acute lung injury and acute respiratory distress syndrome (ARDS).

Preferred is the use of the compounds of the invention for the prevention of the aforesaid disorders.

Equally preferred is the use of the compounds of the invention for the treatment of the aforesaid disorders.

Generally speaking, compounds which are JAK inhibitors may be useful in the treatment of many disorders associated with JAK enzyme mechanisms.

In one embodiment, the disorder that can be treated by the compound of the present invention is selected from the group consisting of asthma, chronic obstructive pulmonary disease (COPD) and interstitial lung disease such as idiopathic pulmonary fibrosis (IPF), acute lung injury and acute respiratory distress syndrome (ARDS).

In a further embodiment, the disorder is selected from asthma and chronic obstructive pulmonary disease (COPD).

As used in the present description , " effective amount" of a compound of formula (I) or a pharmaceutically acceptable salt thereof or other pharmaceutically-active agent, means an amount of the compound sufficient to treat the patient's condition but low enough to avoid serious side effects and it can nevertheless be routinely determined by the skilled artisan. The compounds of formula (I) or pharmaceutically acceptable salts thereof may be administered in effective amounts once or according to a dosing regimen wherein a number of doses are administered at varying intervals of time for a given period of time. Typical daily dosages may vary depending upon the particular route of administration chosen.

The invention also provides pharmaceutical compositions of compounds of formula (I) in admixture with one or more pharmaceutically acceptable carrier or excipient, for example those described in Remington's Pharmaceutical Sciences Handbook, XVII Ed., Mack Pub., N.Y., U.S.A.

The present invention is also directed to use of the compounds of the invention and their pharmaceutical compositions for various route of administration.

Administration of the compounds of the invention and their pharmaceutical compositions may be accomplished according to patient needs, for example, orally, nasally, parenterally (subcutaneously, intravenously, intramuscularly, intrasternally and by infusion), by inhalation, rectally, vaginally, topically, locally, transdermally, and by ocular administration.

Various solid oral dosage forms can be used for administering compounds of the invention including such solid forms as tablets, gelcaps, capsules, caplets, granules, lozenges and bulk powders. The compounds of the present invention can be administered alone or combined with various pharmaceutically acceptable carriers, diluents (such as sucrose, mannitol, lactose, starches) and known excipients, including suspending agents, solubilizers, buffering agents, binders, disintegrants, preservatives, colorants, flavorants, lubricants and the like. Time release capsules, tablets and gels are also advantageous.

Various liquid oral dosage forms can also be used for administering compounds of the invention, including aqueous and non-aqueous solutions, emulsions, suspensions, syrups, and elixirs. Such dosage forms can also contain suitable known inert diluents such as water and suitable known excipients such as preservatives, wetting agents, sweeteners, flavorants, as well as agents for emulsifying and/or suspending the compounds of the invention. The compounds of the present invention may be formulated as injectable composition, for example to be injected intravenously, in the form of an isotonic sterile solution. Other preparations are also possible.

Suppositories for rectal administration of the compounds of the invention can be prepared by mixing the compound with a suitable excipient such as cocoa butter, salicylates and polyethylene glycols.

Formulations for vaginal administration can be in the form of cream, gel, paste, foam, or spray formula containing, in addition to the active ingredient, such as suitable carriers, are also known.

For topical administration the pharmaceutical composition can be in the form of creams, ointments, liniments, lotions, emulsions, suspensions, gels, solutions, pastes, powders, sprays, and drops suitable for administration to the skin, eye, ear or nose. Topical administration may also involve transdermal administration via means such as transdermal patches.

For the treatment of the diseases of the respiratory tract, the compounds according to the invention, as above said, may be administered by inhalation.

Inhalable preparations include inhalable powders, propellant-containing metering aerosols or propellant-free inhalable formulations and may be administered through a suitable inhalation device which may be respectively selected from dry powder inhaler, pressurized metered dosed inhaler, or a nebulizer.

For administration as a dry powder, single- or multi-dose inhalers known from the prior art may be utilized. In that case the powder may be filled in gelatine, plastic or other capsules, cartridges or blister packs or in a reservoir.

A diluent or carrier, e.g. lactose or any other additive suitable for improving the respirable fraction may be added to the powdered compounds of the invention.

Inhalation aerosols containing propellant gas such as hydrofluoroalkanes may contain the compounds of the invention either in solution or in dispersed form. The propellant-driven formulations may also contain other ingredients such as co-solvents, stabilizers and optionally other excipients.

The propellant-free inhalable formulations comprising the compounds of the invention may be in the form of solutions or suspensions in an aqueous, alcoholic or hydroalcoholic medium and they may be delivered by jet or ultrasonic nebulizers known from the prior art or by soft-mist nebulizers such as Respimat^{®},a registered trademark of Boehringer Ingelheim Pharmaceuticals (Wachtel, H., Kattenbeck, S., Dunne, S. et al. Pulm Ther (2017) 3: 19.

The compounds of the invention, regardless of the route of administration, can be administered as the sole active agent or in combination (i.e. as co-therapeutic agents administered in fixed dose combination or in combined therapy of separately formulated active ingredients) with other pharmaceutical active ingredients.

The compounds of the invention can be administered as the sole active agent or in combination with other pharmaceutical active ingredients including those currently used in the treatment of respiratory disorders, and known to the skilled person, such as beta2-agonists, antimuscarinic agents, corticosteroids mitogen-activated kinases (P38 MAP kinases) inhibitors, nuclear factor kappa-B kinase subunit beta inhibitors (IKK2), human neutrophil elastase (HNE) inhibitors, phosphodiesterase 4 (PDE4) inhibitors, leukotriene modulators, non-steroidal anti-inflammatory agents (NSAIDs) and mucus regulators).

The invention is also directed to a kit comprising the pharmaceutical compositions of compounds of the invention alone or in combination with or in admixture with one or more pharmaceutically acceptable carriers and/or excipients and a device which may be a single- or multi-dose dry powder inhaler, a metered dose inhaler or a nebulizer.

The dosages of the compounds of the invention depend upon a variety of factors including the particular disease to be treated, the severity of the symptoms, the route of administration, the frequency of the dosage interval, the particular compound utilized, the efficacy, toxicology profile, and pharmacokinetic profile of the compound.

A pharmaceutical composition comprising a compound of the invention suitable to be administered by inhalation is in various respirable forms, such as inhalable powders (DPI), propellant-containing metering aerosols (PMDI) or propellant-free inhalable formulations (e.g. UDV).

The invention is also directed to a device comprising the pharmaceutical composition comprising a compound according to the invention, which may be a single- or multi-dose dry powder inhaler, a metered dose inhaler and a nebulizer particularly soft mist nebulizer.

The following examples illustrate the invention in more detail.

The features of the invention will become apparent in the course of the following descriptions of exemplary embodiments which are given for illustration of the invention and are not intended to be limiting thereof.

### PREPARATION OF INTERMEDIATES AND EXAMPLES 1a-10a

The following compounds of Example 1a-10a reported in table 1 below were prepared and characterized as follows:

**Table 1**

| **Example No.** | **Structure** | **Chemical Name** | **Biochemical Potency JAK 1 (pIC50)** | **Cell Based assay PBMC (IL-2 stimulated pSTAT5)** | **1H-NMR** | **LC-MS** |
|---|---|---|---|---|---|---|
| 1a | 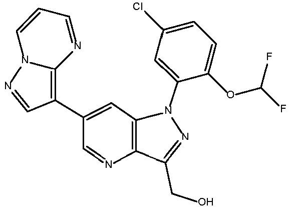 | (1-(5-chloro-2-(difluoromethoxy)p henyl)-6-(pyrazolo[1,5-a]pyrimidin-3-yl)-1H-pyrazolo[4,3-b]pyridin-3-yl)methanol | 8.1 | 7.3 | 1H-NMR (500 MHz, dmso-d6) δ: 9.47 (d, J = 1.8 Hz, 1H); 9.23 (dd, J = 7.1, 1.6 Hz, 1H); 9.00 (s, 1H); 8.72 (dd, J = 4.0, 1.7 Hz, 1H); 8.53 (d, J = 1.8 Hz, 1H); 7.81 (d, J = 2.5 Hz, 1H); 7.72 (dd, J = 8.8, 2.5 Hz, 1H); 7.59 (d, J = 8.8 Hz, 1H); 7.31 (t, J = 73 Hz, 1H); 7.18 (dd, J = 6.9, 4.0 Hz, 1H); 5.40 (t, J = 5.5 Hz, 1H); 4.92 (d, J = 5.3 Hz, 2H). | [MH]+ m/z 443.1/445. 1 |
| 2a | 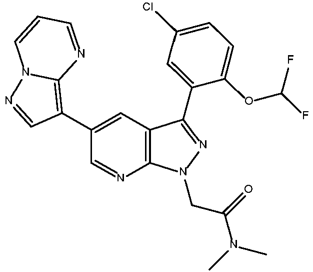 | 2-(3-(5-chloro-2-(difluoromethoxy)p henyl)-5-(pyrazolo[1,5-a]pyrimidin-3-yl)-1H-pyrazolo[3,4-b]pyridin-1-yl)-N,N-dimethylacetamide | 7.3 | 6.2 | 1H-NMR (500 MHz, dmso-d6) δ: 9.33 (d, J = 2.0 Hz, 1H); 9.21 (dd, J = 7.0, 1.5 Hz, 1H); 8.92 (d, J = 2.0 Hz, 1H); 8.87 (s, 1H); 8.68 (dd, J = 4.0, 1.5 Hz, 1H); 7.77 (d, J = 2.6 Hz, 1H); 7.65 (dd, J = 8.8, 2.6 Hz, 1H); 7.48 (d, J = 8.8 Hz, 1H); 7.35 (t, J = 73 Hz, 1H); 7.15 (dd, J = 6.9, 4.0 Hz, 1H); 5.54 (s, 2H); 3.17 (s, 3H); 2.86 (s, 3H). | [MH]+ m/z 497.9/499. 9 |
| 3a | 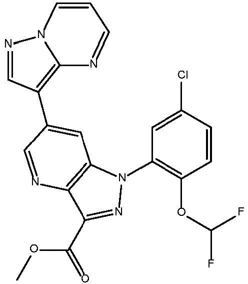 | methyl 1-(5-chloro-2-(difluoromethoxy)p henyl)-6-(pyrazolo[1,5-a]pyrimidin-3-yl)-1H-pyrazolo[4,3-b]pyridine-3-carboxylate | 8.1 | not yet available | 1H-NMR (500 MHz, dmso-d6) δ: 9.65 (d, J = 1.8 Hz, 1H); 9.24 (dd, J = 6.9, 1.6 Hz, 1H); 9.03 (s, 1H); 8.74 (dd, J = 4.0, 1.5 Hz, 1H); 8.59 (d, J = 4.0, 1.8 Hz, 1H); 7.99 (d, J = 2.6 Hz, 1H); 7.84 (dd, J = 8.8, 2.6 Hz, 1H); 7.64 (d, J = 8.8 Hz, 1H); 7.28 (t, J = 73 Hz, 1H); 7.20 (dd, J = 6.9, 4.0 Hz, 1H); 3.98 (s, 3H). | [MH]+ m/z 471.1/473. 1 |
| 4a | 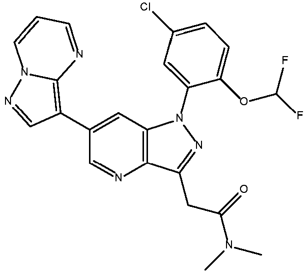 | 2-(1-(5-chloro-2-(difluoromethoxy)p henyl)-6-(pyrazolo[1,5-a]pyrimidin-3-yl)-1H-pyrazolo[4,3-b]pyridin-3-yl)-N,N-dimethylacetamide | 7.8 | 6.5 | 1H-NMR (500 MHz, dmso-d6) δ: 9.43 (d, J = 1.8 Hz, 1H); 9.23 (dd, J = 7.0, 1.7 Hz, 1H); 8.99 (s, 1H); 8.72 (dd, J = 4.0, 1.8Hz, 1H); 8.53 (d, J = 1.8 Hz, 1H); 7.78 (d, J = 2.6 Hz, 1H); 7.72 (dd, J = 8.9, 2.6 Hz, 1H); 7.58 (d, J = 8.9 Hz, 1H); 7.31 (t, J = 73 Hz, 1H); 7.18 (dd, J = 6.9, 4.0 Hz, 1H); 4.15 (s, 2H); 3.17 (s, 3H); 2.87 (s, 3H). | [MH]+ m/z 498.1/500. 1 |
| 5a | 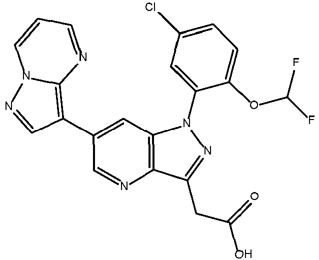 | 2-(1-(5-chloro-2-(difluoromethoxy)p henyl)-6-(pyrazolo[1,5-a]pyrimidin-3-yl)-1H-pyrazolo[4,3-b]pyridin-3-yl)acetic acid | 7.7 | 5.2 | 1H-NMR (500 MHz, dmso-d6) δ: 9.44 (d, J = 1.6 Hz, 1H); 9.23 (dd, J = 7.0, 1.7 Hz, 1H); 8.99 (s, 1H); 8.73 (dd, J = 4.0, 1.8Hz, 1H); 8.54 (d, J = 1.6 Hz, 1H); 7.79 (d, J = 2.6 Hz, 1H); 7.72 (dd, J = 8.9, 2.6 Hz, 1H); 7.58 (d, J = 8.9 Hz, 1H); 7.55 (bs, 1H); 7.32 (t, J = 73 Hz, 1H); 7.18 (dd, J = 6.9, 4.0 Hz, 1H); 7.11 (bs, 1H); 6.64 (bs, 1H); 4.02 (s, 2H). | [MH]+ m/z 471.6/473. 5 |
| 6a | 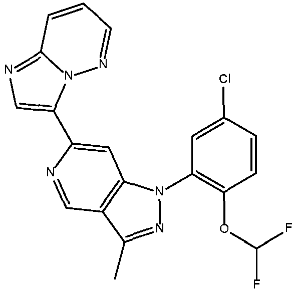 | 1-(5-chloro-2-(difluoromethoxy)p henyl)-6-( imidazo[1,2-b]pyridazin-3-yl)-3-methyl-1H-pyrazolo[4,3-c]pyridine | 8.4 | not yet available | 1H-NMR (600 MHz, dmso-d6) δ: 9.27 (d, J = 1.1 Hz, 1H); 8.70 (dd, J = 4.5, 1.7 Hz, 1H); 8.60 (d, J = 1.1 Hz, 1H); 8.58 (s, 1H); 8.29 (dd, J = 9.1, 1.7 Hz, 1H); 7.85 (d, J = 2.6 Hz, 1H); 7.74 (dd, J = 9.0, 2.6 Hz, 1H); 7.60 (d, J = 9.0, 1H); 7.36 (dd, J = 9.0, 4.3 Hz, 1H); 7.29 (t, J = 73 Hz, 1H); 2.69 (s, 3H). | [MH]+ m/z 427.0/429. 0 |
| 7a | 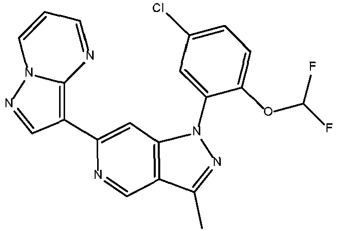 | 1-(5-chloro-2-(difluoromethoxy)p henyl)-3-methyl-6-(pyrazolo[1,5-a]pyrimidin-3-yl)-1H-pyrazolo[4,3-c]pyridine | 8.5 | 7.4 | 1H-NMR (600MHz, dmso-d6) δ: 9.22 (dd, J =6.9, 1.6 Hz, 1H); 9.19 (d, J = 1.1 Hz, 1H); 8.90 (s, 1H); 8.70 (dd, J = 4.0, 1.6 Hz, 1H); 8.34 (d, J = 1.1 Hz, 1H); 7.83 (d, J = 2.7 Hz, 1H); 7.73 (dd, J =8.9, 2.7 Hz, 1H); 7.59 (d, J = 8.9 Hz, 1H); 7.24 (t, J = 73 Hz, 1H); 7.16 (dd, J = 6.9, 4.0 Hz, 1H); 2.65 (s, 3H). | RT = 3.87, [MH]+ m/z 427.2/429, 2 |
| 8a | 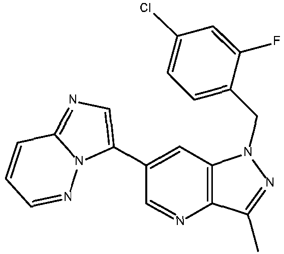 | 1-(4-chloro-2-fluorobenzyl)-6-( imidazo[1,2-b]pyridazin-3-yl )-3-methyl-1H-pyrazolo[4,3-b]pyridine | 6.7 | 6.1 | 1H-NMR (500 MHz, dmso-d6) δ: 9.19 (d, J = 1.1 Hz, 1H); 8.92 (d, J = 1.2 Hz, 1H); 8.72 (dd, J = 4.5, 1.0 Hz, 1H); 8.48 (s, 1H); 8.28 (dd, J = 9.0, 1.3 Hz, 1H); 7.47 (dd, J = 10.7, 1.2 Hz, 1H); 7.37 (dd, J = 9.1, 4.5 Hz, 1H); 7.27-7.32 (m, 2H); 5.69 (s, 2H); 2.54 (s, 3H). | [MH]+ m/z 393.2/395. 2 |
| 9a | 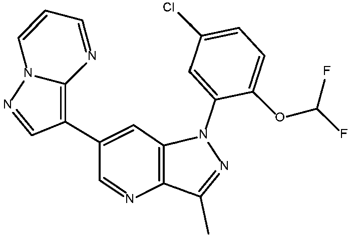 | 1-(5-chloro-2-(difluoromethoxy)p henyl)-3-methyl-6-(pyrazolo[1,5-a]pyrimidin-3-yl)-1H-pyrazolo[4,3-b]pyridine | 7.8 | 7.3 | 1H-NMR (600MHz, CDCl3) δ: 9.26 (d, J = 1.6 Hz, 1H); 8.76 (dd, J = 7.0, 1.6 Hz, 1H); 8.62 (dd, J = 4.0, 1.8 Hz, 1H); 7.58 (s, 1H); 8.45 (d, J = 1.6 Hz, 1H); 7.72 (d, J = 2.5 Hz, 1H); 7.46 (dd, J = 8.8, 2.4 Hz, 1H); 7.43 (d, J = 8.8 Hz, 1H); 6.95 (dd, J = 7.0, 4.0 Hz, 1H); 6.47 (t, J = 73 Hz, 1H); 2.79 (s, 3H). | [MH]+ m/z 427.5/429. 4 |
| 10a | 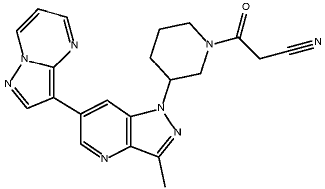 | 3-(3-(3-methyl-6-(pyrazolo[1,5-a]pyrimidin-3-yl)-1H-pyrazolo[4,3-b]pyridin-1-yl)piperidin-1-yl)-3-oxopropanenitrile | 6.0 | 5.2 | 1H- NMR (400 MHz, 353 K, dmso-d6) δ: 9.29 (d, J = 1.8 Hz, 1H); 9.12 (dd, J = 7.0, 1.6 Hz, 1H); 8.83 (s, 1H); 8.72 (dd, J = 4.1, 1.8 Hz, 1H); 8.62 (bs, 1H); 7.13 (dd, J = 7.1, 4.1 Hz, 1H); 4.70 (m, 1H); 3.87-4.06 (m, 3H); 3.01 (m, 3H); 2.56 (s, 3H); 2.21-2.23 (m, 2H); 1.88-1.97 (m, 1H); 1.64-1.78 (m, 1H). | [MH]+ m/z 401.6 |

The compound of Example 1a was prepared according to the following scheme:

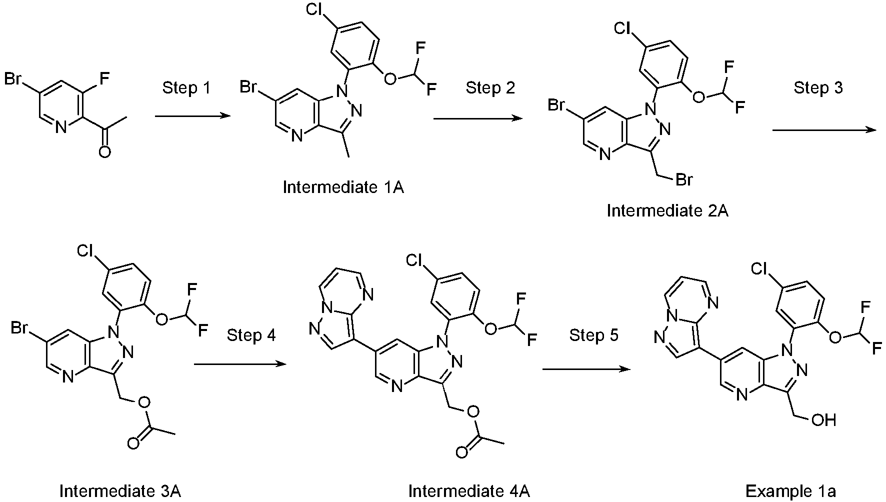

### Step 1

### Intermediate 1A: (6-bromo-1-[5-chloro-2-(difluoromethoxy)phenyl]-3-methyl-pyrazolo[4,3-b]pyridine)

A round-bottom flask was charged with 1-(5-bromo-3-fluoro-2-pyridyl)ethanone (2.00 g, 9.2 mmol), [5-chloro-2-(difluoromethoxy)phenyl]hydrazine hydrochloride (2.47 g, 10 mmol) and potassium carbonate (3.80 g, 28 mmol) in dimethylformammide (16 mL) and the reaction mixture stirred at 85 °C for 1.5 hours, then 120 °C for 4 h. After cooling to room temperature, the reaction mixture was diluted with ethyl acetate (80 mL), washed with aqueous saturated NaCl (3 x 30 mL) and the organic layer dried over Na₂SO₄. Solvent was partially removed under reduced pressure, and a solid was formed from the crude by standing at room temperature. The solid was filtered, washed with petrol ether and then dried to give the title product (1.797 g). ES⁺ m/z 388.0/390.0/392.0 [MH]⁺

### Step 2

### Intermediate 2A: (6-bromo-3-(bromomethyl)-1-[5-chloro-2-(difluoromethoxy)phenyl]pyrazolo[4,3-b]pyridine)

In a round-bottom flask under nitrogen were charged Intermediate 1A (200 mg, 0.51 mmol) and 1,2-dichloroethane (4.0 mL), then N-Bromosuccinimide (110 mg, 0.62 mmol) and AIBN (2,2'-Azobis(2-methylpropionitrile) (17 mg, 0.1 mmol) were added. The reaction mixture was heated at 80 °C for 2 h, then cooled to room temperature and quenched with water (10 mL). The resulting mixture was extracted with dichloromethane (3x5 mL), and the combined organics washed with aqueous saturated NaCl (10 mL) and dried over Na₂SO₄. After evaporation under reduced pressure, the crude was purified by SPE (solid phase extraction) on silica gel to give the title compound (134 mg). ES⁺ m/z 465.9/467.9/469.9/491.9 [MH]⁺

### Step 3

### Intermediate 3A: ([6-bromo-1-[5-chloro-2-(difluoromethoxy)phenyl]pyrazolo[4,3-b]pyridin-3-yl]methyl acetate)

A vial charged with Intermediate 2A (140 mg, 0.30 mmol), dimethylformamide (1.5 mL) and potassium acetate (103 mg, 1.0 mmol was heated at 60 °C for 1.5 hours. Reaction mixture was diluted with water (10 mL) and extracted with ethyl acetate (3x5 mL). Combined organics were washed with aqueous saturated NaCl (2x5 mL), dried over Na₂SO₄ and evaporated to dryness. The crude product was used in the next synthetic steps without further purification. ES⁺ m/z 446.0/448.0/450.0 [MH]⁺.

### Step 4

### Intermediate 4A: ([1-[5-chloro-2-(difluoromethoxy)phenyl]-6-pyrazolo[1,5-a]pyrimidin-3-yl-pyrazolo[4,3-b]pyridin-3-yl]methyl acetate)

Intermediate 3A (83 mg, 0.16 mmol) in THF (1 mL) and potassium phosphate tribasic solution (0.50 M, 0.65 mL, 0.33 mmol) in water were degassed with nitrogen for 10 min, then 3-(4,4,5,5-tetramethyl-1,3,2-dioxaborolan-2-yl)pyrazolo[1,5-a]pyrimidine (44 mg, 0.18 mmol) and XPhos-Pd-G3 ((2-Dicyclohexylphosphino-2',4',6'-triisopropyl-1,1'-biphenyl)[2-(2'-amino-1,1'-biphenyl)]palladium(II) methanesulfonate) (6.9 mg, 0.0082 mmol) were added. The reaction mixture was heated at 55 °C for 1.5 hours, cooled to room temperature and diluted with dichloromethane (10 mL) and water (10 mL).Aqueous layer was extracted with dichloromethane (4x5 mL), then combined organics washed with water (10 mL) and dried over Na₂SO₄. Solvent was removed under reduced pressure and crude residue purified by SPE (solid phase extraction) on silica gel to afford the title compound (66 mg). ES⁺ m/z 485.1.1/487.1 [MH]⁺.

### Step 5

### Example 1a: ([1-[5-chloro-2-(difluoromethoxy)phenyl]-6-pyrazolo[1,5-a]pyrimidin-3-yl-pyrazolo[4,3-b]pyridin-3-yl]methanol)

A round-bottom flask was charged with Intermediate 4A (95 %, 48 mg, 0.094 mmol) and methanol (5 mL), then K₂CO₃ (0.039 g, 0.28 mmol) was added and the mixture stirred at room temperature overnight. Solvent was evaporated and the crude residue purified by SPE (solid phase extraction) on silica gel to afford the title compound (30 mg). ES⁺ m/z 443.1/445.1 [MH]⁺.

The compounds of example 2a-10a were prepared in a similar manner to Example 1a, following the same synthetic sequence; modification of reaction conditions reactants or solvent used can be readily determined by those skilled in the art by routine optimization procedures.

### PHARMACOLOGICAL ACTIVITY OF THE COMPOUNDS OF THE INVENTION (1a-10a).

### Biochemical Potency JAK1 (Data displayed into the table 1 as pIC50)

The objective of this study was to assess the activity of novel JAK inhibitors measuring the capability of compounds to inhibit JAK1 kinase activity in a biochemical time-resolved fluorescence resonance energy transfer (TR-FRET) LANCE assay. In LANCE Ultra kinase assay in the presence of JAK1 kinase and ATP (corresponding to Km), the ULight peptide substrate (LANCE Ulight-JAK-1 (Tyr1023) Peptide, Perkin Elmer, TRF0121) was phosphorylated. It was then captured by a Eu-anti-phospho-substrate antibody (LANCE Eu-W1024 Anti-phosphotyrosine (PT66), Perkin Elmer, AD0069), which brought the Eu-chelate donor and ULight acceptor dyes into close proximity. Upon excitation at 320 nm, the Eu-chelate transfers its energy to the ULight dye, resulting in a fluorescent light emission at 665 nm. Inhibitors were tested at 11 consecutive 5-fold dilutions starting from 30 µM (30 µM - 3 pM) in duplicate. Calculation of IC50 data, curves and QC analysis were made using Excel tools and GraphPadPrism software. QC criteria parameters: Z' ≥ 0.5, Hill Slope range 0.5 to 5, S:B > 2.

In addition to enzymatic potency, the effects of the inhibitors against JAK1/JAK3 activity in a cellular assay was characterized against IL-2 induced phosphorylation of STAT5 level in human peripheral blood mononuclear cells (PBMCs).

### Cell Based assay PBMC (IL-2 stimulated pSTATS) (Data displayed into the table as pIC50)

PBMC have been isolated from human healthy volunteers. Cells were seeded in wells and treated with compounds and rh IL-2. After 30 min incubation cells were lysed and pSTAT5 determined by PathScan phospho-stat5 (Tyr694) ELISA (Cell signaling). Inhibitors were tested at 11 consecutive 5-fold dilutions starting from 30 µM (30 µM - 3 pM) in duplicate. Calculation of IC50 data, curves and QC analysis were made using Excel tools and GraphPadPrism software. QC criteria parameters: Z' ≥ 0.35, Hill Slope range 0.5 to 5, S:B > 2.

### NMR spectra

NMR spectra were recorded on a Bruker Avance III 600 (5 mm RT inverse probehead), Bruker DRX 500, Bruker Avance AV 400 (5 mm RT direct probehead) and Bruker DPX 300 spectrometers using standard Bruker pulse sequences. DMSO-d₆ or CDCl3 were used as solvents and TMS as the internal standard unless in the latter case where solvent residual peak was used. All experiments were recorded at 25 °C, unless stated differently.

LC-MS spectra were recorded on Acquity UPLC coupled with SQD mass spectrometer. **Chromatographic Columns:** Acquity UPLC BEH C18 (50mm x 2.1mm i.d., 1.7µm packing diameter), or Acquity UPLC BEH C18 (50mm x 2.1mm i.d., 1.7µm packing diameter), column temperature 40 °C. Mobile phase: A = = 0.1% v/v solution of formic acid in water, B = 0.1% v/v solution of formic acid in acetonitrile or A = 10 mM aqueous solution of NH4HCO3 (adjusted to pH 10 with ammonia) and B = Acetonitrile. Analytical samples were dissolved in mixture of water :acetonitrile (1:1). If necessery about 10 % of dmso was used in order to improve solubility.

### PREPARATION OF INTERMEDIATES AND EXAMPLES 1-92

Processes of preparation described below and reported in the following schemes should not be viewed as limiting the scope of the synthetic methods available for the preparation of the compounds of the invention.

Those skilled in the art will recognize that all embodiments or aspects of the present invention (including examples 1a to 10a) can be prepared using the methods described herein or easily adapted by using other known methods, reagents and starting materials.

In some cases a step is needed in order to mask or protect sensitive or reactive moieties, generally known protective groups (PG) could be employed, in accordance with general principles of chemistry (Protective group in organic syntheses, 3rd ed. T. W. Greene, P. G. M. Wuts).

Compounds of formula (**I**), here reported again for clarity, including all here above listed, can be usually prepared according to the procedures shown in the schemes below. Where a specific detail or step differs from the general schemes it has been detailed in the specific examples, and/or in additional schemes.

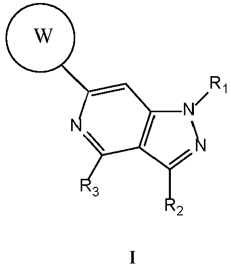

Compounds of formula (**I**) can be prepared according to *scheme 1.* Compound **IV** is an intermediate where general group r_{1,} r₂ , r₃ and w can be converted into R₁, R₂ R₃ and W respectively by means of procedures well known to those skilled in the art such as protective groups deprotection and/or functional group conversion that may involve more than one step. Said procedures can be applied to one or more of those groups (r_{1,} r₂ , r₃ and w) to allow the conversion of intermediate **IV** into compound of general formula **I** and they are detailed in the experimental section for specific examples. It is apparent that in the case such conversions are not needed (when r_{1,} r₂ , r₃ and w correspond to R₁, R₂ R₃ and W respectively), any general approach described below for the preparation of intermediate **IV** will provide a compound of general formula (**I).**

Compound of formula (**I)** (or intermediate **IV**) can be obtained by direct introduction of W through a metal/palladium catalyzed cross coupling reaction such as Suzuki coupling, Stille coupling, Buchwald-Hartwig or similar (Strategic application of named reactions in organic synthesis, L. Kurti, B. Czako, Ed. 2005) by reaction of intermediate **II** with intermediate **III.**

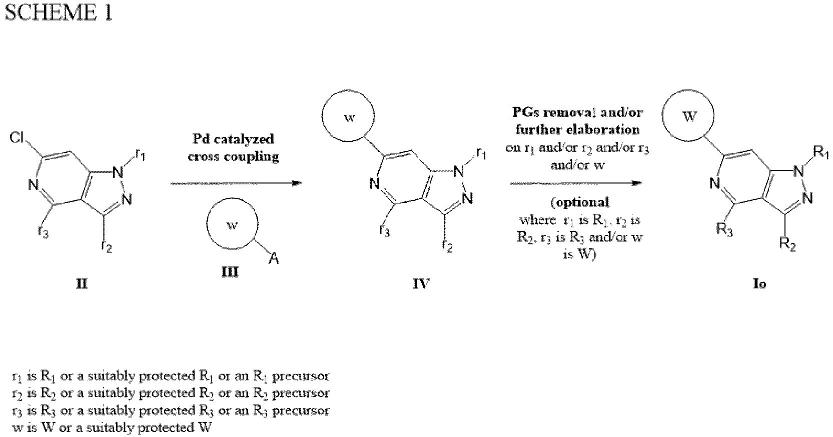

For example, a suitable palladium catalyzed cross coupling for introducing W, when it is an pyrazolo[1,5-a]pyrimidin-3-yl, is a Suzuki coupling. Suzuki coupling can be performed by reacting intermediate **II** with the corresponding boronic acid or boron pinacolate (intermediate **III,** where w is pyrazolo[1,5-a]pyrimidin-3-yl and A is dihydroxyboryl or 4,4,5,5-tetramethyl-1,3,2-dioxaborolanyl) in the presence of a Pd catalyst such as tetrakistriphenylphosphinepalladium(0), PdCl₂(dppf)₂, or a ligand-palladacycle precatalyst such as XPhos-Pd-G3 [(2-Dicyclohexylphosphino-2',4',6'-triisopropyl-1,1'-biphenyl)[2-(2'-amino-1,1'-biphenyl)]palladium(II) methanesulfonate], in an organic solvent such as 1,4-dioxane, THF, 1,2-dimethoxyethane, 2-propanol or DMF, with or without water, in the presence of an inorganic base such as an alkaline carbonate (for example Cs₂CO₃ or K₂CO₃) or an inorganic phosphate (for example K₃PO₄), under heating (typically in the range of 50-100°C) for few hours (typically 1 to 3h). Boronic acid and boronic pinacolate esters are generally commercially available or may be readily prepared by those skilled in the art starting from commercially available reagents. For synthetic convenience, primary/secondary amines and/or acidic NH (like NH from sulfonamides, amides, urea and carbamates) that may be present in intermediates of Suzuki coupling might need to be protected with suitable protective groups. A suitable protective group for protecting acidic NH (like NH from sulfonamides, amides, urea and carbamates) can be benzyl type protective groups such as DMB (2,4-dimethoxybenzyl), PMB group (para-methoxybenzyl). DMB groups can be easily removed by treating corresponding DMB protected intermediate **IV** in acidic conditions with an organic or an inorganic strong acid. For example, DMB can be deprotected by treating the intermediates with trifluoroacetic acid neat or in mixture with an organic solvent such as DCM, THF or similar, typically at room temperature for few hours (typically 1 to 3 h). A suitable protective group for protecting primary and secondary amines, eventually present in r₁/r₂/r₃ groups, can be carbamate type protective groups such as Boc (tert-butoxycarbonyl). Boc group can be easily removed by treating Boc protected intermediate **IV** in acidic conditions with an organic or an inorganic strong acid. For example, Boc group can be cleaved by treating the intermediates with trifluoroacetic acid neat or in mixture with an organic solvent such as DCM, DCE, THF or similar, typically at room temperature for few hours (typically 1 to 3 h).

A suitable palladium catalyzed cross coupling for introducing W when it is an imidazo[1,2-b]pyridazin-3-yl, is a Stille coupling that can be performed by reacting intermediate **II** with the corresponding stannane (intermediate **III,** where w is imidazo[1,2-b]pyridazin-3-yl and A is tributylstannyl or a trimethylstannyl) in the presence of an appropriate palladium catalyst (such as Pd(PPh₃)2Cl₂) in a polar organic solvent (for example DMF or 1,4-dioxane with or without additives (like base or lithium salt). Stannanes are generally commercially available or may be readily prepared by those skilled in the art starting from commercially available reagents.

A suitable palladium catalyzed cross coupling for introducing W, when it is an (3-oxo-3,4-dihydropyrazin-2-yl)amino, is a Buchwald-Hartwig coupling. For synthetic convenience the carbonyl group of (3-oxo-3,4-dihydropyrazin-2-yl)amino needs to be masked as an alkoxy group (such as methoxy group) and that is deprotectd at the end of the synthesis from intermediate **IV.** Intermediate **II** and intermediate **III** (where w is 3-methoxypyrazin-2-aminyl and A is H) can be reacted to give intermediate **IV** (where w is 3-methoxypyrazin-2-aminyl) in the presence of a suitable ligand palladacyle system such as XPhos-Pd-G3 (2-Dicyclohexylphosphino-2',4',6'-triisopropyl-1,1'-biphenyl)[2-(2'-amino-1,1'-biphenyl)]palladium(II) methanesulfonate) or RuPhos-Pd-G3 (2-Dicyclohexylphosphino-2',6'-diisopropoxy-1,1'-biphenyl)[2-(2'-amino-1,1'-biphenyl)]palladium(II) methanesulfonate) or in general a suitable Pd source (for example Pd₂(dba)₃ or Pd(OAc)₂) with a suitable biphenylphosphine ligand type (RuPhos, X-Phos, or similar), in the presence of a strong organic base such as sodium tert-butoxyde or an inorganic base such as Cs₂CO₃, in an organic solvent such 1,4-dioxane, THF or toluene, under heating at high temperature (typically 80-120°C), for few hours (typically 1-5 h). Methoxy group in intermediate **IV** can be demethylated to give compound of formula (**I)** (where W is (3-oxo-3,4-dihydropyrazin-2-yl)amino) by treatment with TMS-Cl (trimethylsilyl chloride) and sodium iodide in acetonitrile for 1 to 5 h at 60 - 100°C.

The above described process may provide at least one non limiting synthetic route for the preparation examples 1 to 62, examples 76 to 77, example 80, examples 83 to 92 and intermediate **IV,** where r₁, r₂, r₃ and/or w are independently precursors of R₁, R₂, R₃ and/or W.

Intermediate **II** (when r₁ is phenyl substituted with R₈ and K'), reported as intermediate **IIa** can be obtained according to scheme 2. K' and r₂ are meant to be groups that can be further elaborated to give respectively K and R₂ by means of procedures well known to those skilled in the art such as protective groups removal and/or functional group conversion that may involve more than one step.

Intermediates **IIa** can be obtained from intermediate **V** from transformation of ewg and rᵢ groups, stepwise or concurrently, by general accepted methods and in accordance with principles of chemistry.

Intermediate **V** can be obtained from an aromatic nucleophilic substitution of intermediate **VII** with intermediate **VI.** An aromatic nulceophilic subtitution can be performed by heating (typically 80 - 130°C) in a polar organic solvent (for example DMF, DMA or 1,4-dioxane) nulceophiles and aromatic electrophile (where Lg is generally is fluorine) in a presence of an inorganic base (such as K₂CO₃ or Cs₂CO₃) or an organic base (such as TEA, DBU or DIPEA).

In a more general approach, intermediate **V** may be prepared from intermediate **VI** and intermediate **VII** by means of N-arylation. An N-arylation can be performed by using copper catalyzed Ullmann type reaction. An Ullmann reaction between a NH heteroaryl and an aryl/heteroaryl halide (chloride, bromide or iodide) can be performed in the presence of a suitable copper(I) catalyst/promoter such as CuI, Cu₂O or CuTC (copper thiophene carboxylate), ligandless or with a suitable ligand such as N,N-dimethylglicine, proline or dimethylcyclohexane-1,2-diamine (DMCHA), in the presence of an inorganic base such as K₂CO₃ or Cs₂CO₃, by heating (typically 90-150°C) in a polar organic solvent such as DMSO, DMF or DMA, for few hours or longer (typically 3-12h).

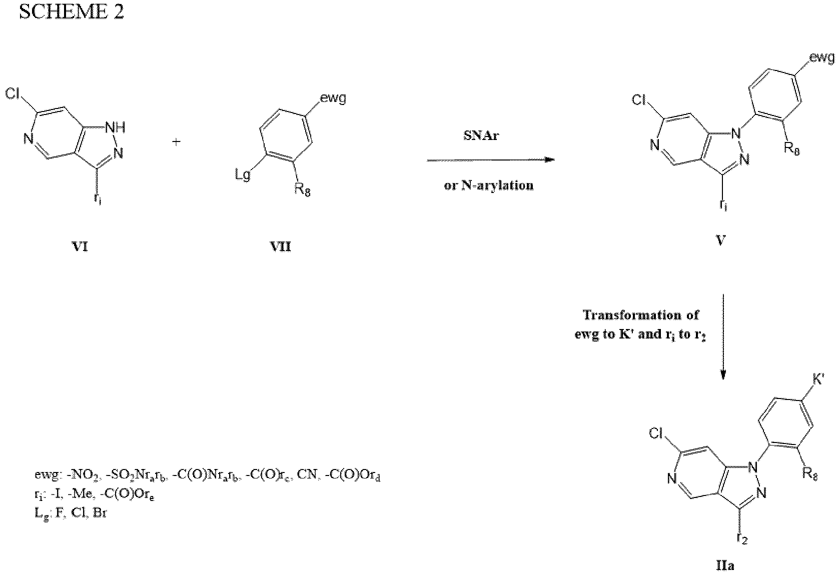

In the following schemes the most common methods that can be used to obtain specific intermediates **IIa**, are detailed. For sake of clarity they were labelled with an additional letter/number.

Intermediate **IIa-A,** according to scheme 3, can be prepared in a two step process from intermediate **Va.** First, the nitro group of intermediate **Va** can be reduced to the corresponding aniline for example by treating such intermediate with a metal powder such as iron or zinc, in the presence of an acid such as acetic acid or ammonium chloride, in an alcoholic solvent like ethanol or methanol with or without water at room temperature (or higher up to 80°C). An alternative approach for reducing nitro compounds can be performed by means of a catalytic hydrogenation using a platinum catalyst such as Pt/C (platinum on carbon) sulfided and in the presence of an hydrogen source as ammonium chloride by heating in an alcholic solvent such as ethanol at temperature up to 70°C.

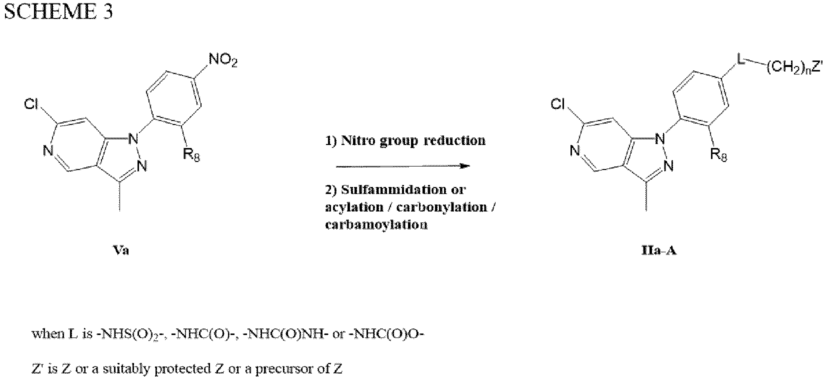

In the second step, the aniline can be transformed to L (when L is -NHS(O)₂-, - NHC(O)-, -NHC(O)NH- or -NHC(O)O-) by respectively a sulfammidation, or an ammidation, or a carbonylation or a carbamoylation. A sulfammidation reaction can be performed by reacting the aniline with a suitable sulfonyl chloride in an organic solvent like DCM, in the presence of an organic base like pyridine or TEA, at temperature typically from 0°C to room temperature. An acylation, a carbonylation or a carbamoylation can be performed by reacting aniline intermediate with the respective acyl chloride, chloroformate or carbamoyl chloride, and a base like pyridine or TEA in an organic solvent like DCM or THF. In some cases L group, when L is - NHS(O)₂, can be further elaborated to -NR₆S(O)₂ by introducing R₆ group by means of an alkylation reaction, for example by treating the corresponding sulfonamide with a suitable alkylating agents and base. In other cases, group Z' can be converted to Z by specific functional group elaboration. For example, if Z' is an ester it can be transformed in an amide in a two step process that involve ester hydrolysis and ammidation.

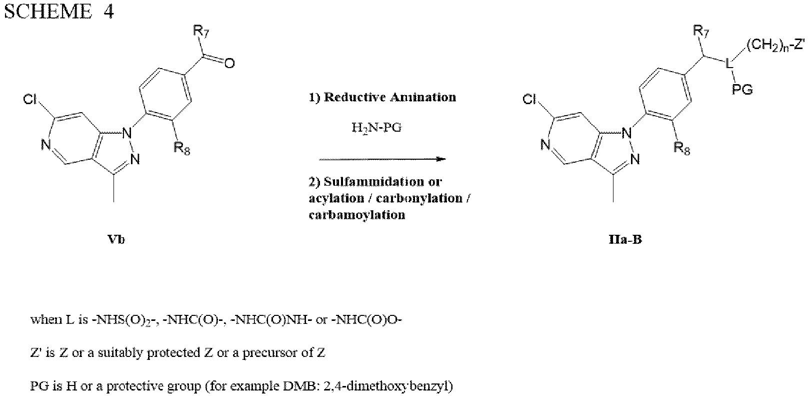

Intermediate **IIa-B,** according to scheme 4, can be prepared in a two step process from intermediate **Vb.** In the first step, intermediate **Vb** can be reacted with a source of ammonia H₂N-PG like p-methoxybenzylamine in an organic solvent as DCM or THF, in the presence of a carboxylic acid such as acetic acid, to form the corresponding immine/enammine that can be reduced by the addition of a suitable borohydryde as STAB (sodium triacetoxyborohydride) or NaBH₃CN. In some cases, typically when R₇ is not H, the formation of immine/enammine can be promoted by a Lewis acid instead of carboxylic acid in the presence of an organic base as TEA or DIPEA. The sulfammidation or acylation / cabonylation or carbamoylation can be performed by using similar conditions to those already described in scheme 3.

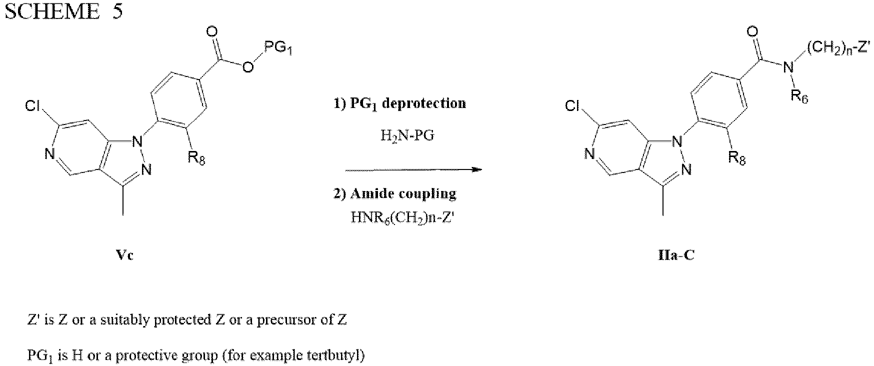

In an alternative approach reported in scheme 5, intermediates of formula **IIa-C** may be obtained from intermediate **Ve** by a two step process. In the first step, PG₁ ester (PG₁ is tert-butyl) can be deprotected by acidic treatment using for example TFA or concentrated HCl in an organic solvent like DCM or 1,4-dioxane. In the second step the acid can be reacted with an amine of formula HNR₆(CH₂)n-Z' by means of amide coupling reaction. An amide coupling can be performed by reacting the amine and the acid in an organic solvent like DMF, DCM, or THF, in the presence of a coupling agent like HATU((1-[Bis(dimethylamino)methylene]-1H-1,2,3-triazolo[4,5-b]pyridinium 3-oxide hexafluorophosphate), HBTU (O-(Benzotriazol-1-yl)-N,N,N',N'-tetramethyluronium hexafluorophosphate) or COMU ((1-Cyano-2-ethoxy-2-oxoethylidenaminooxy)dimethylamino-morpholino-carbenium) and in the presence of an organic base like TEA, DIEA or pyridine.

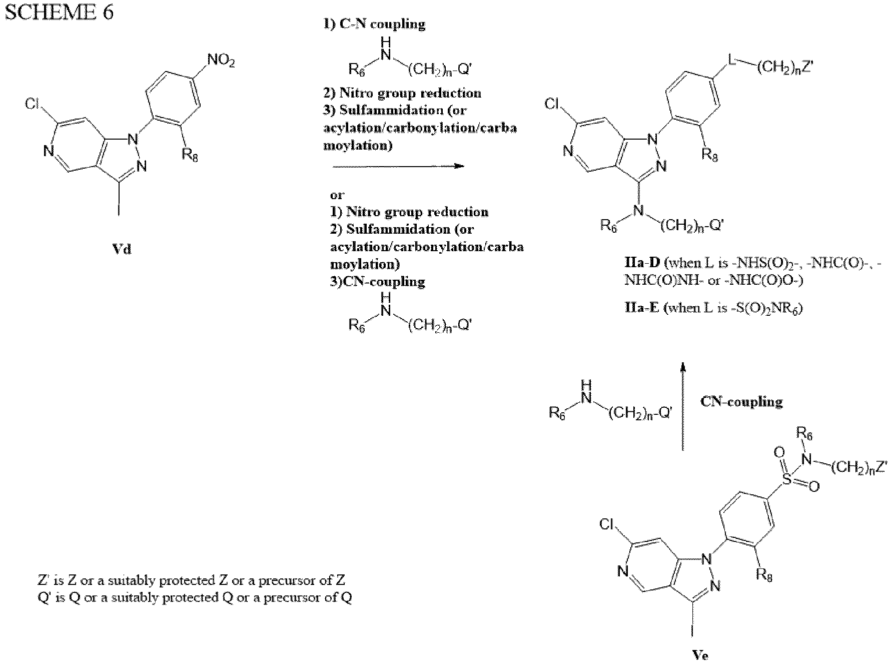

In a different approach reported in scheme 6, intermediates of formula **IIa-D** may be obtained from intermediate **Vd** in a three step process that involve 1) C-N coupling, 2) nitro group reduction and 3) sulfammidation (or acylation/carbonylation/carbamoylation). In a sligthly different approach, the order of event can be changed by performing first 1) nitro reduction, then 2) sulfammidation (or acylation/carbonylation/carbamoylation) and at lastest step 3) C-N coupling. A C-N coupling between intermediate **Vd** and HNR₆(CH₂)ₙ-Q' may be performed by heating (typically 60-100°C) the heteroaromatic iodide and the amine in an organic solvent like DMSO, in the presence of copper(I) catalyst/promoter such as CuI, Cu₂O or CuTC (copper thiophene carboxylate), ligandless or with a suitable ligand such as proline, N,N-dimethylglicine, dimethylcyclohexane-1,2-diamine (DMCHA), an inorganic base as K₂CO₃ or Cs₂CO₃. Nitro group reduction and sulfammidation (or acylation/carbonylation/carbamoylation) can be performed according conditions already described in scheme 3.

In a sligthly different approach, the group r₂ (-NR₆C(O)(CH₂)ₙ-Q') can be build using a stepwise approach that starts from replacing iodine with a protected source of ammonia (like 2,4-dimethylbenzylamine - DMB-NH₂) and tha is then deprotected to give the primary amino group that can be further elaborated by means of amide coupling or acylation to give -N(CO)R₆(CH₂)-Q'. In other cases, for example the group - NR₆C(O)(CH₂)ₙ-Q' can be further elaborated by transforming secondary amine of Q' in the respective tertiary amines. This transformation may be carried out by alkylation of the secondary amine with a suitable alkylating agent. Where, for synthetic convenience of previous step the secondary amine is Boc protected, the group needs to be cleaved by general accepted methods.

In an alternative approach, intermediate **IIa-E** can be synthesized from intermediate **Ve** by C-N coupling in the same way already described above for intermediate **Vd.**

In another approach, intermediate **Vd** can be used to make compounds intermediates of formula **IIa** having r₂ that is -CH₂CN by a two step process. In the first step, a 4-isoxazolyl group can be inserted as masked precursor of -CH₂CN by a Suzuki coupling with the corresponding boron pinacolate, followed by degradation with KF to the desired moiety.

In a different way depicted in scheme 7, intermediate of formula **IIa-F** can be preparared from intermediate **Vf** (when PG₁ is H) by a three steps process that involves 1) Amide coupling with amine HNR₆(CH₂)ₙ-Z, 2) Nitro group reduction and 3) Sulfammidation (or acylation/carbonylation/carbamoylation) using similar conditions to those already described in scheme 3 and scheme 5 for the same reaction type. Alternatively, intermediate **IIa-F** can be obtained from intermediate **Vf** (when PG₁ is methyl or ethyl) by a three steps process that involves 1) Nitro group reduction, 2) Sulfammidation (or acylation/carbonylation/carbamoylation) and 3) amidation with HNR₆(CH₂)ₙ-Z. An amidation can be performed by heating (typically 100-150°C) ester and amine in an organic solvent like THF or dioxane and in the presence of a Lewis acid such as DABAL-Me₃ (Bis(trimethylaluminum)-1,4-diazabicyclo[2.2.2]octane adduct) or AlMe₃.

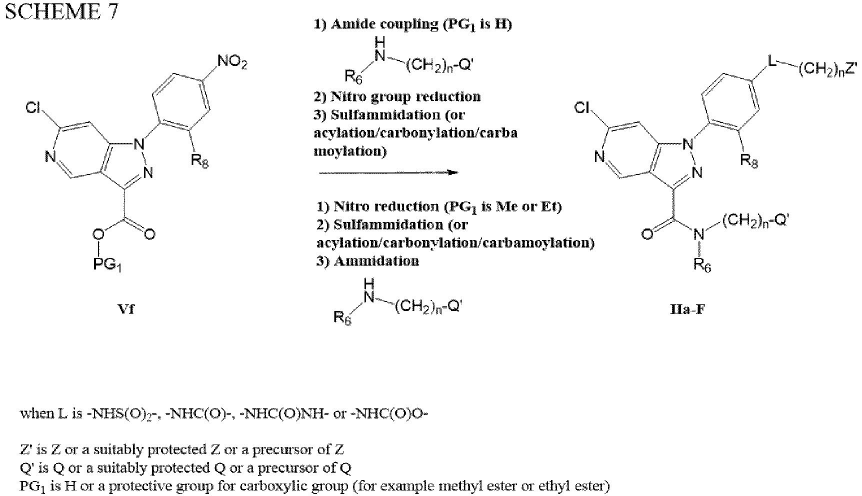

In another approach reported in scheme 8, intermediate **IIa-G** can be obtained from intermediate **Vg** by a four steps process that involves 1) Bromination of the methyl group, 2) nucleophilic substitution of the benzylic bromide with the amine HNR₆(CH₂)ₙ-Z', 3) nitro group reduction and 4) sulfammidation (or acylation/carbonylation/carbamoylation). Bromination can be selectively performed by reaction with NBS (N-bromosuccinimide), in the presence of a radical initiator like AIBN (azobisisobutyronitrile) and in a suitable inert organic solvent like trifluorotoluene or tetrachloromethane at temperature around 80-90°C to give the corresponding benzylic bromide that can be reacted with an excess of the amine (up to 30 equivalents) in THF or DMF.

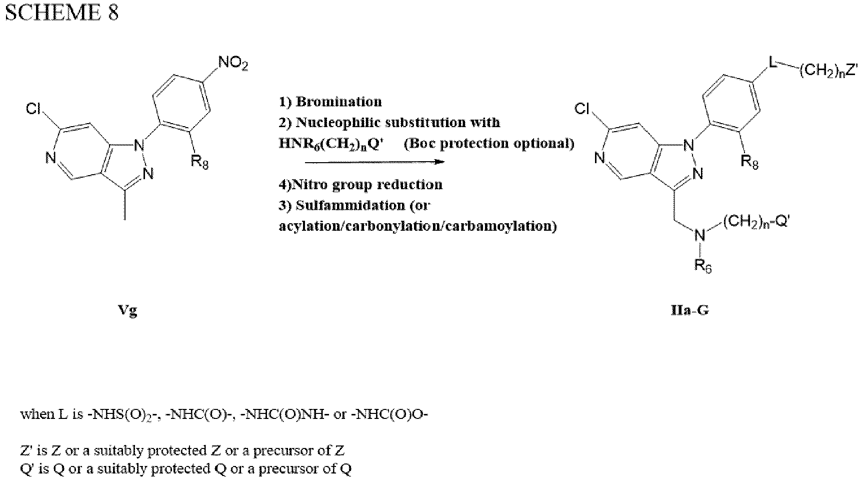

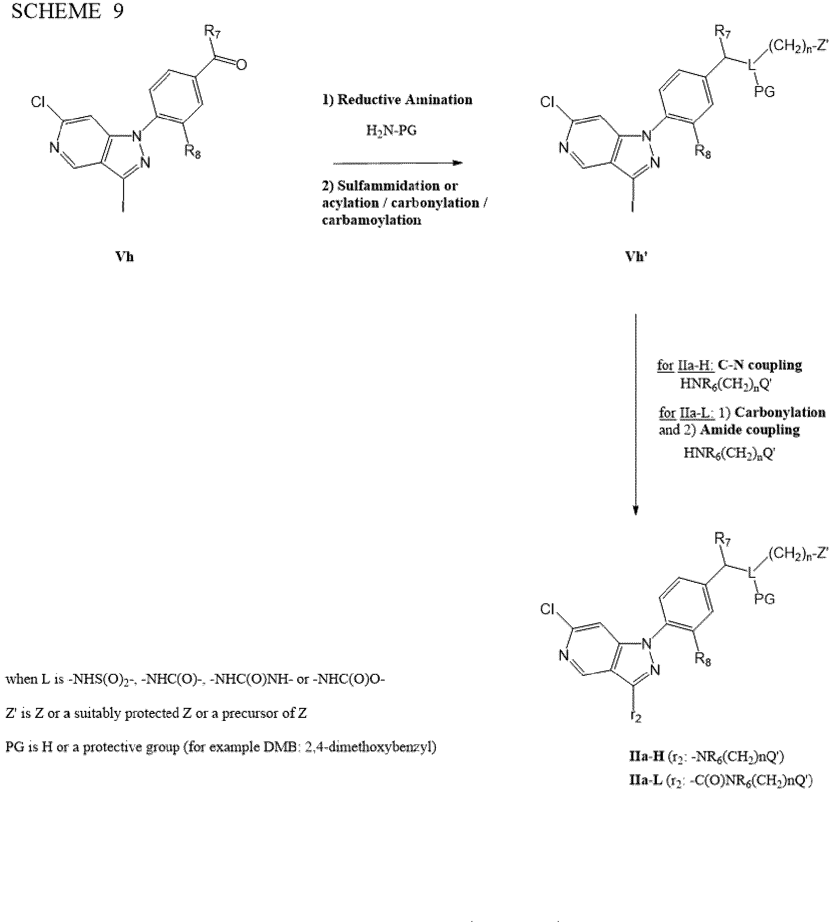

In another approach reported in scheme 9, intermediate **IIa-H** can be made starting from intermediate **Vh'** by C-N coupling with the corresponding amine similarly to what described in scheme 6. Intermediate **Vh'** can be obtained from intermediate **Vh** using a similar method to what described into scheme 4.

In an alternative way reported in scheme 9, intermediate **IIa-L** can be obtained from intermediate **Vh'** by a two step process that involves 1) carbonylation of the heteroaryl iodide to give a carboxylic acid derivative and followed by 2) an amide coupling with the amine HNR₆(CH₂)ₙ-Z'. The carbonylation reaction can be performed by reacting the iodide in the presence of a CO donor system such as formic acid / DCC(diciclohexyl carbodiimmide) or formic acid/acetic anhydride, in the presence of a palladium (II) source / ligand system such as Pd(OAc)₂ / Xanphos or Pd(PPh₃)₂Cl₂ by heating (typically 50-80°C) in an organic solvent as THF or 1,4-dioxane. The subsequent amide coupling can be performed as previously described in scheme 5.

In a similar approach, all synthetic pathways reported in the schemes above (from scheme 2 to scheme 9) can also be applied to make intermediate of formula **II** when r₁ is a phenyl further substituted, or when r₁ is a pyridine.

In another approach, compound of formula **(I)** can be obtained by further elaboration of specific functional groups present in r₁, r₂, r₃ of intermediate **IV** (prepared according to scheme 1), by means of the functional group transformation reported in table 1 thus providing at least one non limiting synthetic route for the preparation of examples reported into the table.

**Table 1**

| Starting Materials | Products | Reaction type / steps | Examples |
|---|---|---|---|
| r₁/R₁ : 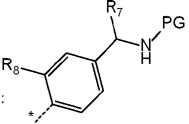 PG: 2,4-dimethoxybenzyl | R₁ : 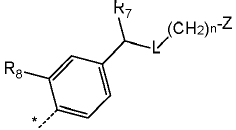 when L is -NHS(O)₂-, -NHC(O)-, -NHC(O)NH- or -NHC(O)O- | 1) Sulfammidation or acylation / carbonylation / carbamoylation | examples 63 to 64, 69 to 70, 75 |
| | | 2) Deprotection | |
| r₁/R₁ : 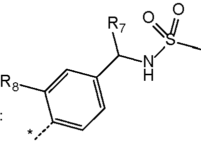 | R₁: 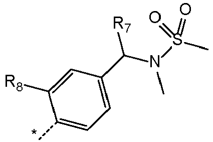 | Alkylation with Mel | example 65 |
| r₁/R₁ : 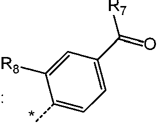 | R₁: 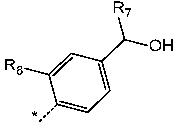 | Reduction with NaBH₄ | example 66 |
| r₁/R₁ : 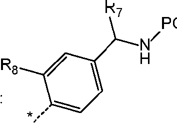 PG: 2,4-dimethoxybenzyl | R₁: 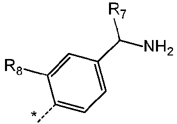 | Deprotection | example 67 |
| r₁/R₁ : 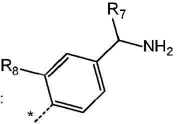 | R₁: 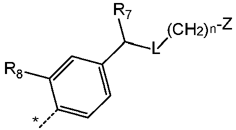 when L is -NHS(O)₂-, -NHC(O)-, -NHC(O)NH- or -NHC(O)O- | Sulfammidation or acylation / carbonylation / carbamoylation | example 68, 71 to 74 |
| r₂/R₂ : 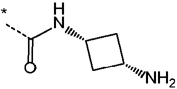 | R₂: 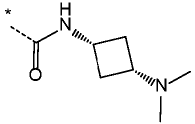 | Eschweiler Clarke (with HCOH/HCOOH) | example 78 |
| r₂/R₂: 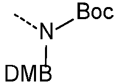 | R₂: 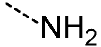 | Deprotection | example 79 |
| r₂/R₂: 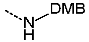 | R₂: 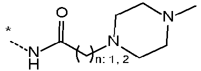 | 1) Acylation with chloroacetyl chloride (n= 1) or chloropropanoyl (n=2) | example 81 |
| | | 2) Nucleophilic substitution with methylpiperazine | |
| | | 3) Deprotection | |

Starting intermediates reported in all above schemes, unless their process for preparation have been detailed here and/or into experimental sections, are commercially available or may be readily prepared by those skilled in the art starting from commercially available reagents using common accepted methods.

### General Experimental details

Chemical Names of the compounds were generated with Structure To Name Enterprise 10.0 Cambridge Software or latest.

Purification by 'chromatography' or 'flash chromatography' refers to purification using a Biotage SP1, or Interchim puriFlash purification system, or equivalent MPLC system using a pre-packed polypropylene column containing stationary phase (cartridge). Where products were purified using an Si cartridge, this refers to an Interchim pre-packed polypropylene column (or equivalent) containing unbounded activated silica with spherical particles with average size of 15 µm or Isolute^{®} pre-packed polypropylene column (or equivalent) containing unbounded activated silica with irregular particles with average size of 50 µm. Fractions containing the required product (identified by TLC and/or LCMS analysis) were pooled and concentrated in vacuo. Where an SCX-2 cartridge was used, 'SCX-2 cartridge' refers to a Bond Elut^{®} pre-packed polypropylene column (or equivalent) containing a non-end-capped propylsulphonic acid functionalised silica strong cation exchange sorbent.

### NMR Methods

NMR spectra were obtained on a Bruker Avance III 600 (5 mm RT inverse probe head), Bruker DRX 500, Bruker Avance AV 400 (5 mm RT direct probehead) or Bruker DPX 300 spectrometers using standard Bruker pulse sequences. DMSO-d6 or CDCl₃ were used as solvents and tetramethylsilane as the internal standard unless in the latter case where solvent residual peak was used. All experiments were recorded at 298 K, unless stated differently. Chemical shift are reported as δ values in ppm relative to tetramethylsilane. Coupling constants (J values) are given in hertz (Hz) and multiplicities are reported using the following abbreviation: s=singlet, d=doublet, t=triplet, q=quartet, m=multiplet, br=broad, nd=not determined.

### LCMS Methods

### Method 1

Acquity UPLC coupled with SQD mass spectrometer; Column: Acquity BEH C18 (50mm x 2.1mm i.d., 1.7µm), mobile phase A: 0.1% (v/v) formic acid in water, mobile phase B: 0.1% (v/v) formic acid in acetonitrile;

| Gradient - Time | Flow (mL/min) | A% | B% |
|---|---|---|---|
| 0.00 | 0.9 | 97 | 3 |
| 1.50 | 0.9 | 3 | 97 |
| 1.90 | 0.9 | 3 | 97 |
| 2.00 | 0.05 | 97 | 3 |

Column temperature: 40 °C; UV detection: from 210 nm to 350 nm; MS conditions: Ionisation Mode: alternate-scan Positive and Negative Electrospray (ES+/ES-), Scan Range: 100 to 1000 AMU.

### Method 2

Acquity UPLC coupled with SQD mass spectrometer; Column: Acquity BEH C18 (50mm x 2.1mm i.d., 1.7µm), mobile phase A: 10 mM aqueous solution of ammonium bicarbonate (adjusted to pH 10 with ammonia), mobile phase B: acetonitrile;

| Gradient - Time | Flow (mL/min) | A % | B% |
|---|---|---|---|
| 0.00 | 0.9 | 97 | 3 |
| 1.50 | 0.9 | 3 | 97 |
| 1.90 | 0.9 | 3 | 97 |
| 2.00 | 0.05 | 97 | 3 |

Column temperature: 40 °C; UV detection: from 210 nm to 350 nm; MS conditions: Ionisation Mode: alternate-scan Positive and Negative Electrospray (ES+/ES-), Scan Range: 100 to 1000 AMU.

### Method 3

Acquity UPLC coupled with SQD mass spectrometer; Column: Acquity BEH C18 (50mm x 2.1mm i.d., 1.7µm), mobile phase A: 0.1% (v/v) formic acid in water, mobile phase B: 0.1% (v/v) formic acid in acetonitrile

| Gradient - Time | Flow (mL/min) | A % | B% |
|---|---|---|---|
| 0.00 | 0.9 | 97 | 3 |
| 3.20 | 0.9 | 3 | 97 |
| 3.90 | 0.9 | 3 | 97 |
| 4.00 | 0.05 | 97 | 3 |

Column temperature: 40°C; UV detection: from 210 nm to 350 nm; MS conditions: Ionisation Mode: alternate-scan Positive and Negative Electrospray (ES+/ES-), Scan Range: 100 to 1500 AMU.

### Method 4

Acquity UPLC coupled with SQD mass spectrometer; Column: Acquity BEH C18 (50mm x 2.1mm i.d., 1.7µm), mobile phase A: 10 mM aqueous solution of ammonium bicarbonate (adjusted to pH 10 with ammonia), mobile phase B: acetonitrile;

| Gradient - Time | Flow (mL/min) | A % | B% |
|---|---|---|---|
| 0.00 | 0.9 | 97 | 3 |
| 3.20 | 0.9 | 3 | 97 |
| 3.90 | 0.9 | 3 | 97 |
| 4.00 | 0.05 | 97 | 3 |

Column temperature: 40 °C; UV detection: from 210 nm to 350 nm; MS conditions: Ionisation Mode: alternate-scan Positive and Negative Electrospray (ES+/ES-), Scan Range: 100 to 1500 AMU.

### Method 5

Acquity UPLC coupled with SQD mass spectrometer; Column: Acquity BEH C18 (50mm x 2.1mm i.d., 1.7µm), mobile phase A: 0.1% (v/v) formic acid in water, mobile phase B: 0.1% (v/v) formic acid in acetonitrile;

| Gradient - Time | Flow (mL/min) | A % | B% |
|---|---|---|---|
| 0.00 | 0.6 | 97 | 3 |
| 0.50 | 0.6 | 97 | 3 |
| 7.00 | 0.6 | 3 | 97 |
| 7.50 | 0.05 | 97 | 3 |

Column temperature: 40 °C; UV detection: from 210 nm to 350 nm; MS conditions: Ionisation Mode: alternate-scan Positive and Negative Electrospray (ES+/ES-), Scan Range: 100 to 1500 AMU.

### Method 6

Acquity UPLC coupled with SQD mass spectrometer; Column: Acquity BEH C18 (50mm x 2.1mm i.d., 1.7µm), mobile phase A: 10 mM aqueous solution of ammonium bicarbonate (adjusted to pH 10 with ammonia), mobile phase B: acetonitrile;

| Gradient - Time | Flow (mL/min) | A % | B% |
|---|---|---|---|
| 0.00 | 0.6 | 97 | 3 |
| 0.50 | 0.6 | 97 | 3 |
| 7.00 | 0.6 | 3 | 97 |
| 7.50 | 0.05 | 97 | 3 |

Column temperature: 40 °C; UV detection: from 210 nm to 350 nm; MS conditions: Ionisation Mode: alternate-scan Positive and Negative Electrospray (ES+/ES-), Scan Range: 100 to 1500 AMU.

### Method 7

AGILENT LC 1260 Infinity with SFC and Agilent 6540 UHD Accurate-Mass Q-TOF LC/MS; Column: Acquity BEH C18 (100mm x 2.1mm i.d., 1.7µm), mobile phase A: 0.1% (v/v) formic acid in water, mobile phase B: 0.1% (v/v) formic acid in acetonitrile;

| Gradient - Time | Flow (mL/min) | A % | B% |
|---|---|---|---|
| 0.00 | 0.5 | 97 | 3 |
| 8.00 | 0.5 | 0 | 100 |
| 10.00 | 0.5 | 97 | 3 |
| 12.00 | 0.05 | 97 | 3 |

Column temperature: 40 °C; UV detection: from 210 nm to 350 nm; MS conditions: Ionisation Mode: alternate-scan Positive and Negative Electrospray (ES+/ES-), Scan Range: 100 to 1500 AMU.

### Method 8

AGILENT LC 1260 Infinity with SFC and Agilent 6540 UHD Accurate-Mass Q-TOF LC/MS; Column: Acquity UPLC BEH C18 (100mm x 2.1mm i.d., 1.7µm), mobile phase A: 0.05 % (v/v) aqueous ammonia, mobile phase B: acetonitrile;

| Gradient - Time | Flow (mL/min) | A % | B% |
|---|---|---|---|
| 0.00 | 0.5 | 97 | 3 |
| 8.00 | 0.5 | 0 | 100 |
| 10.00 | 0.5 | 97 | 3 |
| 12.00 | 0.05 | 97 | 3 |

Column temperature: 40 °C; UV detection: from 210 nm to 350 nm; MS conditions: Ionisation Mode: alternate-scan Positive and Negative Electrospray (ES+/ES-), Scan Range: 100 to 1000 AMU.

In the procedures that follow, some of the starting materials are identified through an "Intermediate" or "Example" number with indications on step name. This is provided merely for assistance to the skilled chemist. When reference is made to the use of a "similar" or "analogous" procedure, as will be appreciated by those skilled in the art, such a procedure may involve minor variations, for example reaction temperature, reagent/solvent amount, reaction time, work-up conditions or chromatographic purification conditions.

The stereochemistry of the compounds in the Examples, where indicated, has been assigned on the assumption that absolute configuration at resolved stereogenic centres of starting materials is maintained throughout any subsequent reaction conditions.

Unless otherwise stated, where absolute configuration (R) or (S) is reported in the compound name, ee% has to be considered equal or greater than 90%.

All solvents and commercial reagents were used as received. Where the preparation of starting materials is not described, these are commercially available, known in the literature, or readily obtainable by those skilled in the art using standard procedures.

### Abbreviations

AIBN = Azobisisobutyronitrile; BoC₂O = Di-tert-butyl dicarbonate, DABAL-Me₃ = Bis(trimethylaluminum)-1,4-diazabicyclo[2.2.2]octane adduct; DBU = 1,8-Diazabicyclo[5.4.0]undec-7-ene; DCC = Dicyclohexylcarbodiimine; DCE = 1,2-Dichloroethane; DCM = Dichloromethane; DIPEA = N,N-Diisopropylethylamine; DMF = N,N-Dimethylformamide; DMSO = Dimethylsulfoxide; EEDQ = 2-Ethoxy-1-ethoxycarbonyl-1,2-dihydroquinoline; EtOAc = Ethyl acetate; HATU = (1-[Bis(dimethylamino)methylene]-1H-1,2,3-triazolo[4,5-b]pyridinium 3-oxid hexafluorophosphate), HBTU = (2-(1H-benzotriazol-1-yl)-1,1,3,3-tetramethyluronium hexafluorophosphate; LCMS = Liquid chromatography-mass spectrometry; LiHMDS = Lithium bis(trimethylsilyl)amide; MW = microwave; NBS = N-Bromosuccinimide; 1H-NMR = Proton nuclear magnetic resonance; RM = Reaction mixture; Rt = Retention time; RT = Room temperature; TEA = Triethylamine; TFA - Trifluoroacetic acid; THF = Tetrahydrofuran; Xphos-Pd-G3 - (2-Dicyclohexylphosphino-2',4',6'-triisopropyl-1,1'-biphenyl)[2-(2'-amino-1,1'-biphenyl)]palladium(II) methanesulfonate

### PREPARATION OF INTERMEDIATES

### Intermediate 1

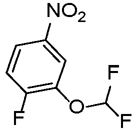

### 2-(Difluoromethoxy)-1-fluoro-4-nitrobenzene (Intermediate 1)

A solution of 2-fluoro-5-nitro-phenol (5.0 g, 32 mmol) and KOH (36 g, 637 mmol) in acetonitrile/water (1:1, 320 mL) was cooled to 0°C. Bromodifluorometyl diethylphosphonate (11 mL, 64 mmol) was added and RM stirred for 1 h. RM was diluted with water (150 mL) and extracted with EtOAc (2x150 mL). The organic layer was washed with aqueous saturated NaCl, then solvent removed under reduced pressure and the residue purified by bulb-to-bulb distillation (61°C/0.086mbar) to give the title compound.

LCMS (Method 2): Rt = 1.02 min
¹H-NMR (300 MHz, DMSO-*d₆*) δ(ppm): 8.29-8.20 (m, 2H), 7.73 (t, J=9.4 Hz, 1H), 7.44 (t, J=72.7 Hz, 1H)

### Intermediate 2

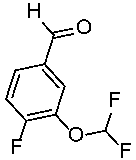

### 3-(Difluoromethoxy)-4-fluorobenzaldehyde (Intermediate 2)

4-Fluoro-3-hydroxy-benzaldehyde (500 mg, 3.57 mmol), sodium chlorodifluoroacetate (1251 mg, 8.21 mmol) and Cs₂CO₃ (1.63 g, 5.00 mmol) were suspended in DMF (8 mL) and stirred at 100 °C for 2 h. RM was cooled to RT. Water (80 mL) was added and product extracted with EtOAc (2x50 mL). Combined organic layers were washed with aqueous saturated NaHCO₃ (3x20 mL), water (20 mL) and aqueous saturated NaCl (20 mL), dried over Na₂SO₄, and evaporated to dryness. The crude material was purified by flash chromatography on a Si cartridge by eluting with 0-30% EtOAc in cyclohexane affording the title compound (226 mg).

LCMS (Method 2): Rt = 0.97 min
¹H-NMR (500 MHz, *CDCl₃*) δ(ppm): 9.95 (s, 1H), 7.77-7.81 (m, 2H), 7.34-7.38 (m, 1H), 6.63 (t, J = 70.6 Hz, 1H)

### Intermediate 3

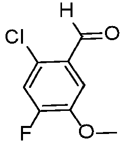

### 2-Chloro-4-fluoro-5-methoxybenzaldehyde (Intermediate 3)

1-Bromo-2-chloro-4-fluoro-5-methoxy-benzene (1.0 g, 4.2 mmol) was dissolved in THF (10 mL) and cooled to -5 °C, then under nitrogen blanket i-PrMgCl.LiCl (1.3 M in THF, 6.42 mL, 8.4 mmol) was added dropwise at ~0 °C. RM was stirred at 0 °C for 1 h, then DMF (1.62 mL, 21 mmol) added and RM stirred at RT for further 45 minutes.. RM was quenched with aqueous saturated NH₄Cl (30 mL) and extracted with EtOAc (30 mL). Organic layer was washed with aqueous saturated NaCl (20 mL) and evaporated under reduced pressure to give the title compound (800 mg) that was used in the next steps without further purifications.

LCMS (Method 2): Rt = 1.07 min
¹H-NMR (300 MHz, *DMSO-d₆*) δ(ppm): 10.24 (s, 1H), 7.68 (d, J = 10.9 Hz, 1H), 7.54 (d, J = 8.7 Hz, 1H)

### Intermediate 4

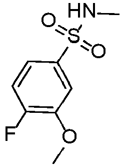

### 4-Fluoro-3-methoxy-N-methylbenzenesulfonamide (Intermediate 4)

4-Fluoro-3-methoxy-benzenesulfonyl chloride (1.5, 6.7 mmol) was added to a solution of methylamine in THF (2.0 M, 17 mL, 33mmol). RM was stirred at RT for 16 h then diluted with EtOAc (30 mL) and washed with water (2x20 mL). Organic layer was dried over Na₂SO₄ and solvents removed under reduced pressure affording the title product (1.08 g) that was used in the next steps without further purifications.

LCMS (Method 2): Rt = 0.80 min, ES⁻ 218.1 [M-H]⁻

### Intermediate 5

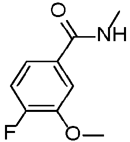

### 4-Fluoro-3-methoxy-N-methylbenzamide (Intermediate 5)

To a DMF (3 mL) solution of 4-fluoro-3-methoxy-benzoic acid (500 mg, 2.94 mmol), methylamine hydrochloride (595 mg, 8.82 mmol) and DIPEA (2.56 mL, 14.7 mmol) were added, followed by HATU (1.23 g, 3.23 mmol). After 2h a second equivalent of HATU was added and RM stirred overnight. RM was quenched with aqueous saturated NaHCO₃ (15 mL) and extracted with EtOAc (2x10 mL). Combined organic layers were washed with aqueous saturated NaHCO₃ (3x10 mL), 5% (w/w) aqueous LiCl (10 mL) and aqueous saturated NaCl (10 mL). After drying over Na₂SO₄, the solvent was removed *in vacuo* and the residue dissolved in DCM (20 mL), followed by washings with 0.1 N HCl (7x10 mL) and aqueous saturated NaCl (10 mL). Organic layer was dried over Na₂SO₄ and concentrated *in vacuo* to afford the title product (439 mg)

LCMS (Method 1): Rt = 0.67 min, ES⁺ *m*/*z* 184.1/185.2 [M+H]⁺

### Intermediate 6

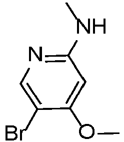

### 5-bromo-4-methoxy-N-methylpyridin-2-amine (Intermediate 6)

A vial charged with 5-bromo-4-methoxy-pyridin-2-amine (300 mg, 1.48 mmol) and THF (7 mL) was cooled to 0°C and then NaH (60 % dispersion in mineral oil, 83 mg, 2.07 mmol) added. RM was stirred at RT for 30 min and then cooled to 0°C before dropwise addition of methyl iodide (92 µL, 1.48 mmol). RM was stirred at RT for additional 1 h, quenched with aqueous saturated NaCl (10 mL) and extracted with EtOAc (20 mL). Organic layers were dried over Na₂SO₄, solvent evaporated to dryness and residue purified by flash chromatography on a Si cartridge by eluting with 0-30% EtOAc in cyclohexane to afford the title product (104.7 mg).

LCMS (Method 2): Rt = 0.79 min, ES⁻ *m*/*z* 216.9/218.9 [M+H]⁺

### Intermediate 7

### Step 1

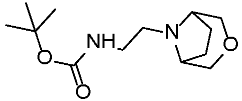

### tert-Butyl (2-(3-oxa-8-azabicyclo[3.2.1] octan-8-yl)ethyl)carbamate (Intermediate 7-1)

*tert*-Butyl *N*-(2-bromoethyl)carbamate (599 mg, 2.7 mmol) and 3-oxa-8-azabicyclo[3.2.1]octane hydrochloride (400 mg, 2.7 mmol) were suspended in DMF (10.0 mL), then potassium carbonate (739 mg, 5.3 mmol) added and RM stirred at 65°C for 4 days. RM was allowed to cool to RT, poured in ice-cold water and extracted with DCM (4 x 10 mL Combined organic layers were dried over Na₂SO₄, evaporated to dryness, and the resulting crude material chromatographed on a Si cartridge by eluting with 0-30% DCM/MeOH/NH₄OH (90:9:0.5) in DCM to afford the desired product (249 mg).

¹H-NMR (500 MHz, *CDCl₃*) δ(ppm): 5.16 (bs, 1H), 3.64 (d, J = 10.4 Hz, 2H), 3.49 (d, J = 10.4 Hz, 2H), 3.16 (m, 2H), 2.98 (m, 2H), 2.34 (m, 2H), 1.85 (s, 4H), 1.44 (s, 9H).

### Step 2

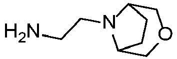

### 2-(3-Oxa-8-azabicyclo[3.2.1]octan-8-yl)ethan-1-amine (Intermediate 7)

Intermediate 7-1 (249 mg, 0.61 mmol) was dissolved in a mixture of DCM (2 mL)/TFA (0.91 mL, 12 mmol) and stirred at 20 °C for 16 h. RM was loaded onto an SCX column, washed with methanol and eluted with 2M methanolic ammonia. Relevant fractions were pooled and evaporated to give the title product (126.6 mg).

¹H-NMR (500 MHz, *CDCl₃*) δ: 3.68 (d, J = 9.9 Hz, 2H), 3.51 (d, J = 9.9 Hz, 2H), 3.00 (bs, 2H), 2.73 (t, J = 5.5 Hz, 2H), 2.33 (t, J = 5.5 Hz, 2H), 1.87 (s, 4H)

### Intermediate 8

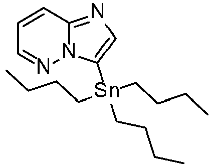

### 3-(Tributylstannyl)imidazo[1,2-b]pyridazine (Intermediate 8)

A solution of 3-bromoimidazo[1,2-*b*]pyridazine (400 mg, 2.0 mmol) in THF (5 mL) was cooled to 0 °C then i-PrMgCl LiCl complex (1.3 M solution in THF, 4.7 mL, 6.1 mmol) added dropwise over 5 minutes and RM stirred at 0°C for 15 minutes. Tri-n-butyltin chloride (685 µL, 2.5 mmol) was added dropwise and RM further stirred at RT for 30 minutes. RM was cooled in an ice bath and quenched with water (5 mL), added of aqueous saturated NH₄Cl (10 mL) and extracted with EtOAc (15 mL). Organic layer was washed with aqueous saturated NaCl (10 mL), dried over MgSO₄ and evaporated under reduced pressure. The residue that was purified by chromatography on neutral Al₂O₃ by elution with mixtures of cyclohexane and EtOAc to afford the title product (285 mg).

LCMS (Method 2): Rt = 1.82 min, ES⁻ *m*/*z* (most abundant isotope) 410.0 [M+H]⁺

### Intermediate 9a

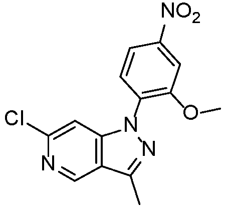

### 6-Chloro-1-(2-methoxy-4-nitrophenyl)-3-methyl-1H-pyrazolo[4,3-c]pyridine (Intermediate 9a)

A mixture of 6-chloro-3-methyl-1H-pyrazolo[4,3-c]pyridine (1.00 g, 5.97 mmol), 1-fluoro-2-methoxy-4-nitro-benzene (1.02 g, 5.97 mmol), potassium carbonate (2.47 g, 17.9 mmol) and DMF (10.0 mL) was stirred at 80 °C for 45 min. RM was cooled to RT, quenched with water (50 mL) and the formed precipitate filtered, washed with water (2 x 35 mL) and dried to afford the title product (2.28 g).

LCMS (Method 2): Rt = 0.99 min
¹H-NMR (300 MHz, *CDCl₃*) δ(ppm) 8.81 (d, J=0.8 Hz, 1H), 8.03-7.96 (m, 2H), 7.67 (d, J=8.5 Hz, 1H), 7.12 (d, J=0.8 Hz, 1H), 3.97 (s, 3H), 2.69 (s, 3H)

### Preparation of intermediates 9b to 9e

The following intermediates were prepared in a similar manner to Intermediate 9a by replacing *1-fluoro-2-methoxy-4-nitro-benzene* with the indicated starting materials. When minor modifications on base, solvent,temperature and/or reaction time were made, they were detailed below in brackets.

| Intermediate | Structure | Starting material | LCMS |
|---|---|---|---|
| Intermediate 9b | 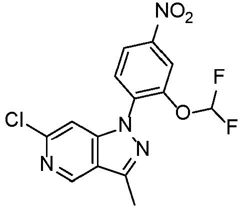 | Intermediate 1 (Reaction temperature 65 °C, reaction time 2.5 h) | (Method 2) Rt = 1.14 min, ES⁺ *m*/*z* 355.1/357.1 [MH]⁺ |
| Intermediate 9c | 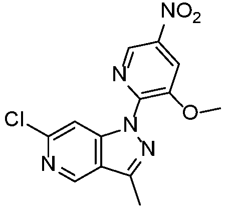 | 2-chloro-3-methoxy-5-nitro-pyridine (Reaction temperature 85 °C, reaction time 2.5 h) | (Method 2):Rt = 0.99 min, ES⁺ *m*/*z* 319.8/321.7 [M+H]⁺ |
| Intermediate 9d | 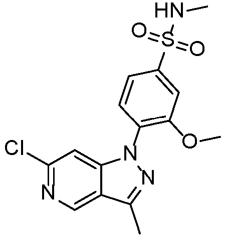 | Intermediate 4 (Reaction temperature 90 °C, reaction time overnight) | (Method 2) Rt = 0.92, ES⁺ *m*/*z* 367.1/369.1 [M+H]⁺ |
| Intermediate 9e | 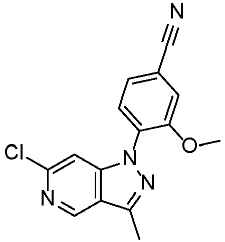 | 4-fluoro-3-methoxy-benzonitrile (Reaction temperature 70 °C, reaction time overnight) | (Method 1) Rt = 1.08 min, ES⁺ *m*/*z* 299.1/301.1 [M+H]⁺ |
| Intermediate 9f | 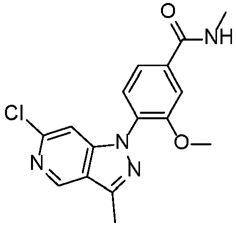 | Intermediate 5 (reaction temperature 180 °C, reaction time overnight) | (Method 1) Rt = 0.83 min, ES⁺ *m*/*z* 331.1/333.1 [M+H]⁺ |
| Intermediate 9g | 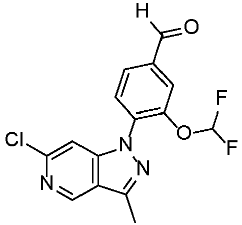 | Intermediate 2 (reaction temperature 105 °C, reaction time 2 h) | (Method 1) Rt = 1.05 min, ES⁺ *m*/*z* 337.7/339.6 [M+H]⁺ |
| Intermediate 9h | 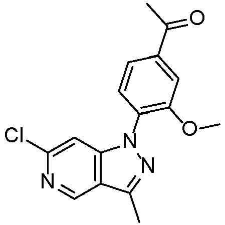 | 1-(4-fluoro-3-methoxyphenyl)ethan-1-one (120 °C for 30 min, MW irradiation and 150 °C for 30 minMW irradiation) | (Method 1): Rt = 1.04 min, ES⁺ *m*/*z* 316.2/318.2 [M+H]⁺ |
| Intermediate 9i | 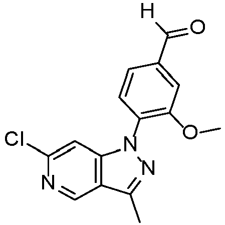 | 1-(4-fluoro-3-methoxyphenyl)ethan-1-one (Reaction temperature 105 °C for reaction time 1.5 h) | (Method 2) Rt = 1.01 min, ES⁺ *m*/*z* 302.1/304.1 [M+H]⁺ |

### Intermediate 9j

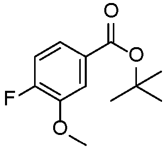

### Step 1

### tert-Butyl 4-fluoro-3-methoxybenzoate (Intermediate 9i-1)

A flask was charged with DCM (100 mL), MgSO₄ (5 g, 42.0 mmol) and conc. 98%(w/w) H₂SO₄ (0.6mL, 11 mmol) was stirred at RT for 15 min, then 4-Fluoro-3-methoxy-benzoic acid (1.78 g, 11 mmol) added, followed by t-BuOH (5 mL, 52 mmol). RM was stirred at RT for 20 hours. A further equivalent of H₂SO₄ and MgSO₄, followed by further 5mL of *t*-BuOH were added. RM was stirred at RT overnight, then slowly poured onto ice and extracted with DCM (3x50 mL). Combined organic layers were washed with aqueous saturated NaHCO₃ (3x60 mL) and aqueous saturated NaCl (80 mL), dried over Na₂SO₄, filtered and concentrated *in vacuo* to afford the title product (1.77 g).

LCMS (Method 1): Rt = 1.31 min
¹H-NMR (300 mHz, CDCl3) δ(ppm): 7.52-7.61 (m, 2H), 7.06 (dd, J = 10.8, 7.8 Hz, 1H), 3.91 (s, 3H), 1.56 (s, 9H)

### Step 2

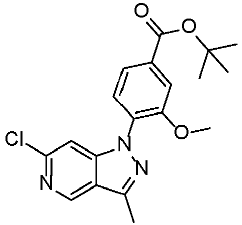

### tert-Butyl 4-(6-chloro-3-methyl-1H-pyrazolo[4,3-c]pyridin-1-yl)-3-methoxybenzoate (Intermediate 9i-2)

A vial charged with 6-chloro-3-methyl-1H-pyrazolo[4,3-c]pyridine (250 mg, 1.37 mmol), intermediate 9j-1 (310 mg, 1.37 mmol), K₂CO₃ (284 mg, 2.06 mmol) and DMF (2.00 mL) was stirred at 125°C overnight. After cooling to RT, iodomethane (1.11 g, 7.82 mmol) was added and RM stirred at RT for 2.5 h. RM was quenched with water (100 mL) and extracted with EtOAc (3x20 mL). Combined organic layers were washed with water (6x10 mL) and aqueous saturated NaCl (20 mL), dried over Na₂SO₄ and filtered to give the title product that was used in the next steps without further purifications.

LCMS (Method 1): Rt = 1.40 min, ES⁺ *m*/*z* 374.3/376.2 [M+H]⁺

### Step 3

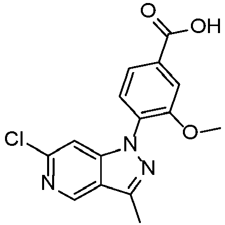

### 4-(6-Chloro-3-methyl-1H-pyrazolo[4,3-c]pyridin-1-yl)-3-methoxybenzoic acid (Intermediate 9j-3)

TFA (20 mL, 246 mmol) was added dropwise to an ice cold solution of intermediate 9j-2 (1.00 g, 2.67 mmol) in DCM (20 mL) and RM was stirred at RT overnight. RM was evapotated under reduced pressure, the residue treated with aqueous saturated NaHCO₃ (20 mL) and washed with EtOAc (2x40 mL). The aqueous layer was brougth to pH ~ 3-4.5 using aqueous conc. HCl 36% w/w and the formed precipitate filtered, triturated with MeOH and toluene to give the title product (1.05 g).

LCMS (Method 1): Rt = 0.91 min, ES⁺ *m*/*z* 318.1/320.1 [M+H]⁺

### Step 4

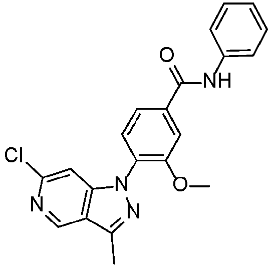

### 4-(6-Chloro-3-methyl-1H-pyrazolo[4,3-c]pyridin-1-yl)-3-methoxy-N-phenylbenzamide (Intermediate 9i)

A flask was charged with intermediate 9j-3 (120 mg, 0.30 mmol), HATU (126 mg, 0.33 mmol), DIPEA (110 µL, 0.6 mmol) and DMF (5 mL), then aniline (30 µL, 0.33 mmol) added and RM stirred at RT for 1 h. RM was quenched with water (5 mL) and extracted with EtOAc (3 x 10mL). Combined organic layers were washed with aqueous saturated NaHCO₃ (10 x 5mL) and aqueous saturated NaCl (10 mL), dried over Na₂SO₄ and concentrated *in vacuo.* Residue was purified by flash chromatography on a Si cartridge by eluting with 0-50% DCM/MeOH/NH₄OH (90:9:1.5) in DCM to give title product (82 mg).

LCMS (Method 1): Rt = 1.14 min, ES⁺ *m*/*z* 393.1/395.1 [M+H]⁺

### Intermediate 10a

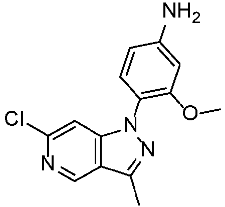

### 4-(6-Chloro-3-methyl-1H-pyrazolo[4,3-c]pyridin-1-yl)-3-methoxyaniline (Intermediate 10a)

A three-neck-round-bottom-flask, equipped with a magnetic stir bar, a thermometer and a reflux condenser, was charged with intermediate 9a (2.3 g, 7.2 mmol) and acetic acid (15 mL). The suspension was heated to 80 °C, then iron powder (3.4 g, 62 mmol) was added portion-wise. The mixture was stirred at 80 °C for 30 min. The RM was cooled to RT,t precipitate filtered and washed with acetic acid.. The mother liquor was partitioned between water (2x100 mL) and EtOAc(20 mL),the organic layer washed with aqueous saturated NaHCO₃ and aqueous saturated NaCl, dried over Na₂SO₄ and concentrated *in vacuo* to yield title product (1.5 g).

LCMS (Method 2): RT=0.86 min, ES⁻ *m*/*z* 289.2/291.0 [M+H]⁺

### Intermediate 10b

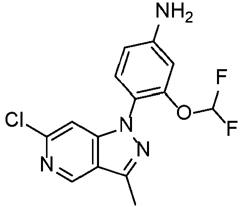

### 4-(6-chloro-3-methyl-pyrazolo[4.3-c]pyridin-1-yl)-3 (difluoromethoxy)aniline (Intermediate 10b)

The title product was prepared in a similar manner to intermediate 10a starting from intermediate 9b.

LCMS (Method 2): Rt = 0.95 min, ES⁺ *m*/*z* 325.1/327.0 [M+H]⁺

### Intermediate 10c

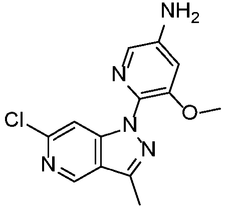

### 6-(6-chloro-3-methyl-1H-pyrazolo[4,3-c]pyridin-1-yl)-5-methoxypyridin-3-amine (Intermediate 10c)

To a solution of intermediate 9c (144 mg, 0.45 mmol) in EtOH (4.1 mL), ammonium formate (170 mg, 2.7 mmol) and Pt/C 3% on activated carbon sulfided (wet 50 %, 35 mg, 0.090 mmol) were added. RM was stirred at 85 °C for 3 h. RM was cooled to RT and diluted with DCM, filtered through a pad of Celite^{®} and evaporated to give the product (151.9 mg) that was used *as is* in the next steps.

LCMS (Method 2): Rt = 0.70 min, ES⁺ *m*/*z* 290.0/291.9 [M+H]⁺

### Intermediate 10d

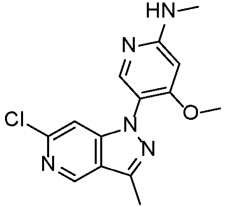

### 5-(6-Chloro-3-methyl-1H-pyrazolo[4,3-c]pyridin-1-yl)-4-methoxy-N-methylpyridin-2-amine (Intermediate 10d)

A vial was charged under argon with 6-chloro-3-methyl-1H-pyrazolo[4,3-c]pyridine (80.8 mg, 0.482 mmol), intermediate 6 (105 mg, 0.482 mmol), Cs₂CO₃ (471 mg, 1.45 mmol), and N,N-Dimethylglycine (49.7 mg, 0.482 mmol). DMSO (2 mL) was added, followed by CuI (46 mg, 0.241 mmol) and RM stirred at 120°C overnight. After cooling to RT, RM was diluted with water and extracted with EtOAc. Organic layer was separated, washed with aqueous saturated NaCl, dried over Na₂SO₄ and concentrated. The residue was purified by flash chromatography on a Si cartridge by eluting with 0-100% MeOH/DCM (1:10) in DCM to give the title compound (43.7 mg).

LCMS (Method 2): Rt = 0.80 min, ES⁺ *m*/*z* 303.8/305.8 [M+H]⁺

### Intermediate 11a

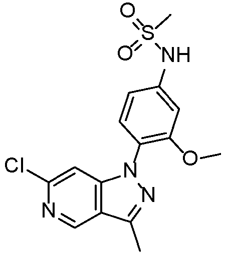

### N-(4-(6-chloro-3-methyl-1H-pyrazolo[4,3-c]pyridin-1-yl)-3-methoxyphenyl)methanesulfonamide (Intermediate 11a)

A two-neck-round-bottom flask, equipped with a thermometer and a magnetic stir bar, was charged with intermediate 10a (1.0 g, 3.5 mmol) and DCM (15 mL). Methanesulfonyl chloride (375 µL, 4.8 mmol) and pyridine (822 mg, 10 mmol) were added and RM stirred at RT for 20 min. RM was diluted with water (20 mL) and extracted with DCM (2x15 mL), then combined organic layers were washed with aqueous 2M citric acid (2x15 mL), with aqueous saturated NaCl (15 mL each) and dried over Na₂SO₄. The solvent was removed under reduced pressure, the residue suspended in toluene and evaporated under reduced pressure. The crude material was purified by flash chromatography on a Si cartridge by eluting with 0-30% DCM/MeOH/NH₄OH (90:9:1.5) in DCM to give the title product (840.6 mg).

LCMS (Method 1): Rt = 0.90 min, ES⁺ *m*/*z* 367.1/369.1 [M+H]⁺

### Preparation of Intermediates 11b to 11k

The following intermediates were prepared in a similar manner to intermediate 11a by replacing intermediate 10a and/or methanesulfonyl chloride with the indicated starting materials.

| Intermediate | Structure | Starting materials | LCMS |
|---|---|---|---|
| Intermediate 11b | 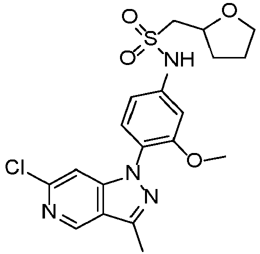 | Intermediate 10a and tetrahydrofuran-2-ylmethanesulfon yl chloride | (Method 2) Rt = 0.58 min, ES⁺ *m*/*z* 437.1/439. 0 [M+H]⁺ |
| Intermediate 11c | 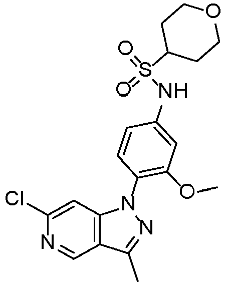 | Intermediate 10a and tetrahydropyran-4-sulfonyl chloride | (Method 2) Rt = 0.57 min, ES⁺ *m*/*z* 437.1/439. 0 [M+H]⁺ |
| Intermediate 11d | 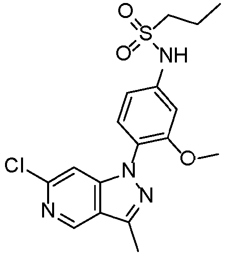 | Intermediate 10a and 1-propansulfonyl chloride | (Method 2) Rt = 0.61 min, ES⁺ *m*/*z* 395.1/397. 1 [M+H]⁺ |
| Intermediate 11e | 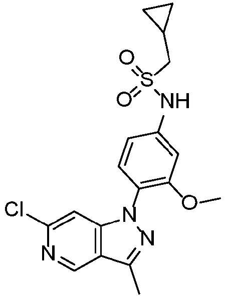 | Intermediate 10a and cyclopropylmeth anesulfonyl chloride | (Method 2)Rt = 0.76 min, ES⁺ *m*/*z* 407.1/409. 1 [M+H]⁺ |
| Intermediate 11f | 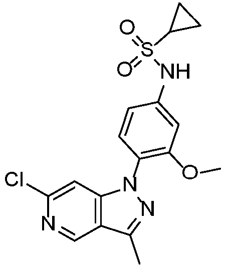 | Intermediate 10a and cyclopropanesul fonyl chloride | (Method 3)Rt = 1.69 min, ES⁺ *m*/*z* 393.2/395. 1 [M+H]⁺ |
| Intermediate 11g | 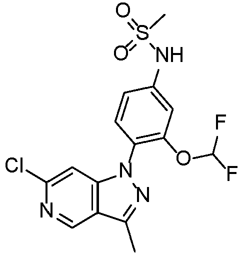 | Intermediate 10b and methanesulfonyl chloride | (Method 1) Rt = 0.52 min, ES⁺ *m*/*z* 403.1/405. 1 [M+H]⁺ |
| Intermediate 11h | 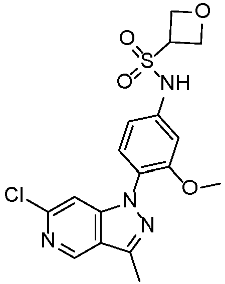 | Intermediate 10a and oxetane-3-sulfonyl chloride | (Method 2) Rt = 0.47 min, ES⁺ *m*/*z* 409.0/411. 0 [M+H]⁺ |
| Intermediate 11i | 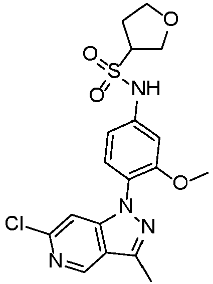 | Intermediate 10a and tetrahydrofuran-3-sulfonyl chloride | (Method 2) Rt = 0.52 min, ES⁺ *m*/*z* 423.2/425. 1 [M+H]⁺ |
| Intermediate 11j | 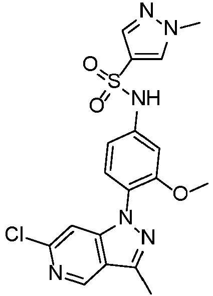 | Intermediate 10a and 1-methylpyrazole-4-sulfonyl chloride | (Method 1) Rt = 0.92 min, ES⁺ *m*/*z* 433.1/435. 1 [M+H]⁺ |
| Intermediate 11k | 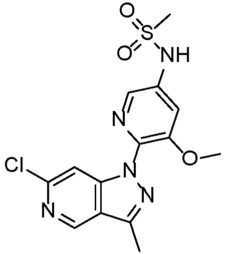 | Intermediate 10c and methanesulfonyl chloride | (Method 1) Rt = 0.80 min, ES⁺ *m*/*z* 368.0/370. 0 [M-H]⁺ |

### Intermediate 11l

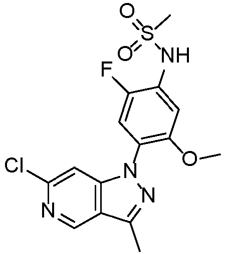

### N-(4-(6-Chloro-3-methyl-1H-pyrazolo[4,3-c]pyridin-1-yl)-2-fluoro-5-methoxyphenyl)methanesulfonamide (Intermediate 111)

A round-bottom-flask, equipped with a magnetic stir bar, a reflux condenser and a drying tube, was charged with intermediate 11a (180 mg, 0.49 mmol) and acetonitrile (10.0 mL). The mixture was heated to 80 °C and Selectfluor^{®} (209 mg, 0.59 mmol) was added. The mixture was left to stir overnight at 80 °C, then a further equivalent of Selectfluor^{®} was added and the mixture stirred at 80 °C for 1h. The RM was evaporated to dryness and the residue purified by flash chromatography on a Si cartridge by eluting with 0-35% EtOAc in cyclohexane to afford the desired product (28.6 mg).

LCMS (Method 1): Rt = 0.92 min, ES⁺ *m*/*z* 385.1/387.1 [M+H]⁺

### Intermediate 11m

### Step 1

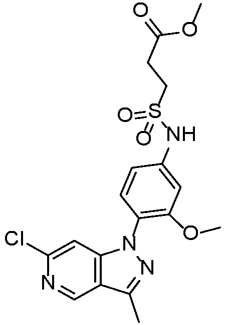

### Methyl 3-(N-(4-(6-chloro-3-methyl-1H-pyrazolo[4,3-c]pyridin-1-yl)-3-methoxyphenyl)sulfamoyl)propanoate (Intermediate 11m-1)

Intermediate 11m-1 was prepared in a similar manner to intermediate 11a starting from intermediate 10a and methyl 3-chlorosulfonylpropanoate.

LCMS (Method 2): Rt = 0.93 min, ES⁺ *m*/*z* 439.1/441.0 [M+H]⁺

### Step 2

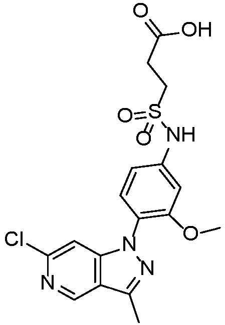

### 3-(N-(4-(6-Chloro-3-methyl-1H-pyrazolo[4,3-c]pyridin-1-yl)-3-methoxyphenyl)sulfamoyl)propanoic acid (Intermediate 11m-2)

Intermediate 11m-1 (320 mg, 0.729 mmol), THF (9 mL) was added of a solution of lithium hydroxide monohydrate (153 mg, 3.65 mmol) in water (4.5 mL) and stirred at RT overnight. The precipitate formed was filtered affording the title product (140 mg).

LCMS (Method 1): Rt = 0.86 min, ES⁺ *m*/*z* 425.1/ 427.1 [M+H]⁺

### Step 3

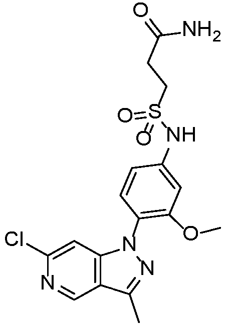

### 3-(N-(4-(6-Chloro-3-methyl-1H-pyrazolo[4,3-c]pyridin-1-yl)-3-methoxyphenyl)sulfamoyl)propanamide (Intermediate 11m)

To a solution of intermediate 11m-2 (50.0 mg, 0.12 mmol), ammonium chloride (31.5 mg, 0.59 mmol) and DIPEA (205 µL, 1.18 mmol) in dry DMF (1.8 mL) was added with HATU (49.2 mg, 0.129 mmol). RM was stirred at 50 °C for 2 hours. A further equivalent of ammonium chloride and HATU (22.4 mg, 0.0588 mmol) were added and RM stirred for further 2h. RM was diluted with water (5 mL) and extracted with EtOAc and then with DCM/isopropanol (3x5 mL). Combined organic layers were evaporated under reduced pressure and the residue purified by flash chromatography on a Si cartridge by eluting with 0-100% DCM/MeOH/NH₄OH (90:9:0.5) in DCM to afford the product (26mg).

LCMS (Method 1): Rt = 0.79 min, ES⁺ *m*/*z* 424.1/426.1 [M+H]⁺

### Intermediate 11n

### Step 1

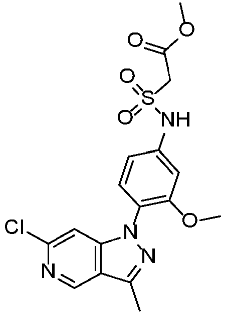

### Methyl 2-(N-(4-(6-chloro-3-methyl-1H-pyrazolo[4,3-c]pyridin-1-yl)-3-methoxyphenyl)sulfamoyl)acetate (Intermediate 11n-1)

Intermediate was prepared in a similar manner to intermediate 11a starting from intermediate 10a and methyl 2-chlorosulfonylacetate.

LCMS (Method 2): Rt = 0.96 min, ES⁺ *m*/*z* 425.1/427.1 [M+H]⁺

### Step 2

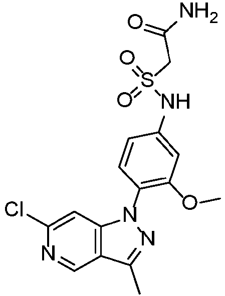

### 2-(N-(4-(6-Chloro-3-methyl-1H-pyrazolo[4,3-c]pyridin-1-yl)-3-methoxyphenyl)sulfamoyl)acetamide (Intermediate 11n-2)

A solution of Intermediate 11n-1 (40 mg, 0.094 mmol) and methanolic ammonia (7.0 M, 2.0 mL, 14 mmol) was heated in a MW reactor at 120 ° C for 20 min. A second equivalent of methanolic ammonia was added and followed by further 20min irradiation at 120° C. RM was evaporated and the residue purified by flash chromatography on a Si cartridge by eluting with 0-100% DCM/MeOH/NH₄OH (90:9:0.5) in DCM to afford the title product (17 mg).

LCMS (Method 2): Rt = 0.44 min, ES⁺ *m*/*z* 410.1/ 412.1 [M+H]⁺

### Intermediate 11o

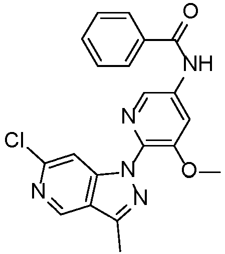

### N-(6-(6-Chloro-3-methyl-1H-pyrazolo[4,3-c]pyridin-1-yl)-5-methoxypyridin-3-yl)benzamide (Intermediate 11o)

Intermediate 10c (95 mg, 0.33 mmol) was dissolved in pyridine (3.7 mL), thenbenzoyl chloride (38 µL, 0.33 mmol) added and the reaction stirred at RT overnight. RM was diluted with DCM (20 mL) and extracted with 2M aqueous citric acid (10 mL), aqueous layer was back-extracted with DCM (3 × 20 mL). Combined organic layers were washed with aqueous saturated NaCl, dried over Na₂SO₄, and solvent evaporated under reduced pressure. The residue was taken with toluene and evaporated under reduced pressure to give a crude material that was purified by flash chromatography on Si cartridge by eluting with 0-50%DCM/MeOH/NH₄OH (90:9:1.5) in DCM to afford the title product (82 mg).

LCMS (Method 2): Rt = 1.01 min, ES⁺ *m*/*z* 394.1/396.1 [M+H]⁺

### Intermediate 11p

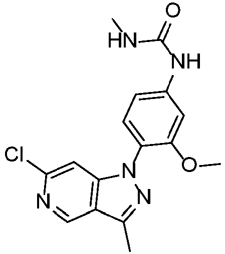

### 1-(4-(6-Chloro-3-methyl-1H-pyrazolo[4,3-c]pyridin-1-yl)-3-methoxyphenyl)-3-methylurea (Intermediate 11p)

Intermediate 10a (150 mg, 0.52 mmol) and TEA (145 µL, 1 mmol) were dissolved in DCM (5 mL), then cooled in an ice bath before addition of methylaminoformyl chloride (53 mg, 0.57 mmol). RM was stirred at RT overnight. Additional amount of TEA (50 µL) and methylaminoformyl chloride (25 mg) were added and stirring continued for further 48 h. RM was diluted with DCM (4 mL) and washed with saturated aqueous NaHCO₃ (8 mL), aqueous 0.1 M HCl (5 mL) and aqueous saturated NaCl (8 mL). Organic layer was evaporated under reduced pressure and the resulting crude was triturated in DCM to afford the title product (38 mg).

LCMS (Method 2): Rt = 0.81 min, ES⁺ *m*/*z* 346.1/348.1 [M+H]⁺

### Intermediate 12a

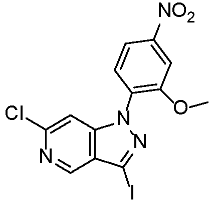

### 6-Chloro-3-iodo-1-(2-methoxy-4-nitrophenyl)-1H-pyrazolo[4,3-c]pyridine

A suspension of 6-chloro-3-iodo-1H-pyrazolo[4,3-c]pyridine (5.38 g, 19.3 mmol), 1-fluoro-2-methoxy-4-nitrobenzene (3.3 g, 19.3 mmol) and K₂CO₃ (8.0 g, 57.8 mmol) in DMF (40 mL) was stirred at 65 °C for 3 h. RM was cooled to RT and diluted with water (300 mL), the formed precipitate filtered, washed with water (4x100 mL) and dried overnight. Trituration in MeOH afforded the title product (7.34 g).

LCMS (Method 2): Rt = 1.24 min, ES⁺ *m*/*z* 430.8/432.8 [M+H]⁺

### Preparation of Intermediates 12b to 12f

The following compounds were prepared in a similar manner to **intermediate 12a** by replacing 1-fluoro-2-methoxy-4-nitrobenzene with indicated starting material. When minor modifications on base, solvent,temperature and/or reaction time were made, they were detailed below in brackets.

| Intermediate | Structure | Starting materials | LC-MC |
|---|---|---|---|
| Intermediate 12b | 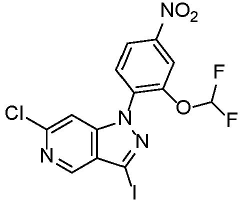 | Intermediate 1 | (Method 1) Rt = 1.27 min, ES⁺ *m*/*z* 466.9/469.0 [M+H]⁺ |
| Intermediate 12c | 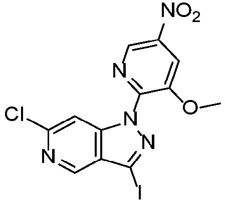 | 2-chloro-3-methoxy-5-nitro-pyridine | (Method 2) Rt = 1.18 min, ES⁺ *m*/*z* 431.9/433.9 [M+H]⁺ |
| Intermediate 12d | 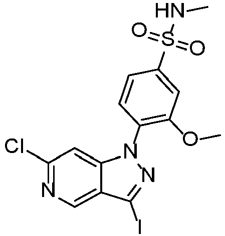 | Intermediate 4 (base: DBU / reaction temperature 140 °C) | (Method 2) Rt = 1.10 min, ES⁺ *m*/*z* 479.0/481.0 [M+H]⁺ |
| Intermediate 12e | 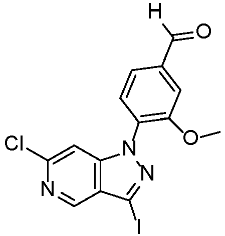 | 4-fluoro-3-methoxybenzaldehyde (reaction temperature 140 °C, overnight) | (Method 2) Rt = 1.17 min, ES⁺ *m*/*z* 413.9/415.9 [M+H]⁺ |
| Intermediate 12f | 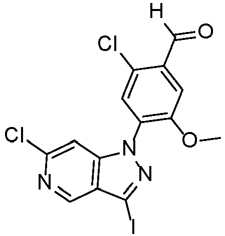 | Intermediate 3 (reaction temperature 90 °C, overnight) | (Method 2) Rt = 1.36 min, ES⁺ *m*/*z* 448.0/450.0[M+H]⁺ |

### Intermediate 13a

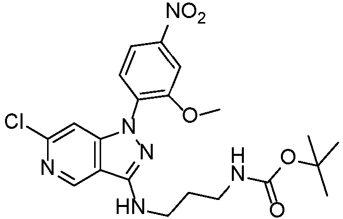

### tert-Butyl (3-((6-chloro-1-(2-methoxy-4-nitrophenyl)-1H-pyrazolo[4,3-c]pyridin-3-yl)amino)propyl)carbamate (Intermediate 13a)

Intermediate 12a (200 mg, 0.464 mmol), proline (16 mg, 0.139 mmol) and K₂CO₃ (385 mg, 2.79 mmol) were dissolved in DMSO (1.8 mL), degassed by nitrogen bubbling, and then added with *tert*-Butyl *N*-(3-aminopropyl)carbamate (0.998 mL,1.93 mmol) and copper (I) iodide (8.85 mg, 0.0464 mmol). RM was heated to 70 °C under argon for 22 h, then cooled to RT, quenched with water (20 mL) and extracted with DCM (3×20 mL). Combined organic layers were washed with aqueous NH₃ (1 M, 20 mL) and aqueous saturated NaCl (20 mL), dried over Na₂SO₄, filtered and evaporated under reduced pressure to afford the product (505 mg) that was used in the next steps without further purifications.

LCMS (Method 2): Rt = 1.24 min, ES⁺ *m*/*z* = 477.17/479.08 [M+H]⁺

### Intermediate 13b

### Step 1

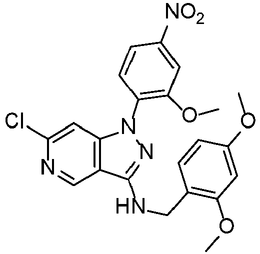

### 6-Chloro-N-(2,4-dimethoxybenzyl)-1-(2-methoxy-4-nitrophenyl)-1H-pyrazolo[4,3-c]pyridin-3-amine (Intermediate 13b-1)

Intermediate 13b-1 was prepared in a similar manner to intermediate 13a starting from intermediate 12a and (2,4-dimethoxyphenyl)methanamine.

LCMS (Method 2): Rt = 1.31 min, ES⁺ *m*/*z* = 470.2/472.1 [M+H]⁺

### Step 2

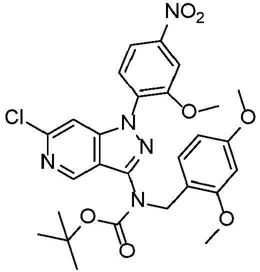

### tert-Butyl (6-chloro-1-(2-methoxy-4-nitrophenyl)-1H-pyrazolo[4,3-c]pyridin-3-yl)(2,4-dimethoxybenzyl)carbamate (Intermediate 13b)

A THF (8.8 mL) solution of intermediate 13b-1 (320 mg, 0.681 mmol) was cooled to 0 °C and purged with argon, then LiHMDS (1.3 M in THF, 1.05 mL, 1.36 mmol) added under argon and RM stirred for 5 minutes before dropwise addition of a solution of Boc₂O (297 mg, 1.36 mmol) in THF (4 mL). RM was stirred at RT for 30 minutes, then quenched with water (50 mL) and extracted with EtOAc (100 mL). Organic layer was separated, washed with aqueous saturated NaCl, dried and concentrated to give the desired compound (400 mg) that was used in the next steps without further purifications.

LCMS (Method 2): Rt = 1.47 min, ES⁺ *m*/*z* 570.2/572.2 [M+H]⁺

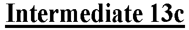

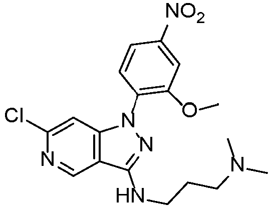

### N1-(6-Chloro-1-(2-methoxy-4-nitrophenyl)-1H-pyrazolo[4,3-c]pyridin-3-yl)-N3,N3-dimethylpropane-1,3-diamine (Intermediate 13c)

The title compound was prepared in a similar manner to intermediate 13a starting from intermediate 12a and *N1*,*N1*-dimethylpropane-1,3-diamine.

LCMS (Method 2): Rt = 1.16, ES⁺ *m*/*z* 405.2/407.2 [M+H]⁺

### Intermediate 13d

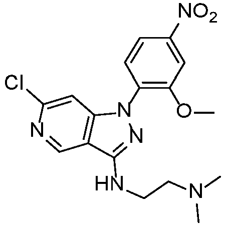

### N1-(6-Chloro-1-(2-methoxy-4-nitrophenyl)-1H-pyrazolo[4,3-c]pyridin-3-yl)-N2,N2-dimethylethane-1,2-diamine (Intermediate 13d)

The title compound was prepared in a similar manner to Intermediate 13a starting from intermediate 12a and *N1*,*N1*-dimethylethane-1,2-diamine.

LCMS (Method 2): Rt = 1.14, ES⁺ *m*/*z* 391.2/393.2 [M+H]⁺

### Intermediate 14a

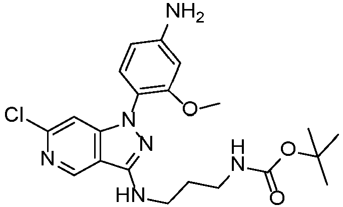

### tert-Butyl (3-((1-(4-amino-2-methoxyphenyl)-6-chloro-1H-pyrazolo[4,3-c]pyridin-3-yl)amino)propyl)carbamate (Intermediate 14a)

Ammonium formate (61 mg, 0.96 mmol) and Pt/C 3% on activated carbon, sulfided (6.3 mg, 0.032 mmol) were added to a mixture of intermediate 13a (153 mg, 0.32 mmol) in EtOH (2 mL), then RM stirred at 65 °C for 16 h. A second equivalent of ammonium formate and Pt/C 3% on activated carbon sulfided were added and RM stirred at 80 °C for further 16 h. RM was cooled to RT, filtered through a pad of Celite^{®}, washed with EtOAc (50 mL) and evaporated to dryness to give the title product (70.1 mg) that was used in the next steps without further purifications.

LCMS (method 2): Rt = 1.00 min, ES⁻ *m*/*z*=447.2/449.2 [M+H]⁺

### Intermediate 14b

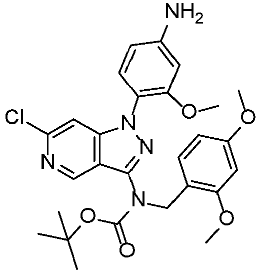

### tert-Butyl (1-(4-amino-2-methoxyphenyl)-6-chloro-1H-pyrazolo[4,3-c]pyridin-3-yl)(2,4-dimethoxybenzyl)carbamate (Intermediate 14b

To a solution of intermediate 13b (340 mg, 0.596 mmol) in a mixture of acetic acid (3 mL)/MeOH (7 mL), Zn (390 mg, 5.96 mmol) was added in one portion and RM stirred at RT for 1 h. RM was concentrated and re-dissolved in DCM (100 mL)/aqueous saturated NaHCO₃ (50 mL), layers were separated, organic layer washed with aqueous saturated NaHCO₃ (2x5 mL), aqueous saturated NaCl (1x10 mL), dried over Na₂SO₄ and concentrated *in vacuo.* The crude was purified by flash chromatography on a Si cartridge by eluting with 0-50% EtOAc in cyclohexane to give the title product (160 mg).

LCMS (Method 2): Rt = 1.29 min, ES⁺ *m*/*z* 540.3/542.2 [M+H]⁺

### Intermediate 14c

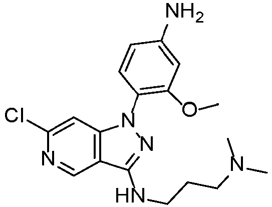

### N1-(1-(4-Amino-2-methoxyphenyl)-6-chloro-1H-pyrazolo[4,3-c]pyridin-3-yl)-N3,N3-dimethylpropane-1,3-diamine (Intermediate 14c)

The title compound was prepared in a similar manner to intermediate 14a starting intermediate 13c.

LCMS (Method 2): Rt = 0.89 min, ES⁺ *m*/*z* 375.2/377.2 [M+H]⁺

### Intermediate 15a

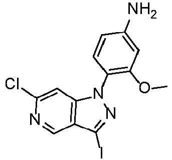

### 4-(6-Chloro-3-iodo-1H-pyrazolo[4,3-c]pyridin-1-yl)-3-methoxyaniline (Intermediate 15a)

Intermediate 12a (2.60 g, 6.04 mmol) in EtOH (30 mL) was heated at 80 °C and added with a solution of ammonium chloride (1.29 g, 24.2 mmol) in water (8 mL) followed by addition of iron (4.05 g, 72.5 mmol). RM was stirred for 2.5 h, cooled to RT, diluted with EtOAc and filtered (filtrate washed thoroughly with EtOAc). The combined filtrate was evaporated, dissolved in EtOAc, washed with water, aqueous saturated NaCl, dried over Na₂SO₄ and solvent evaporated to dryness. The crude material was triturated with DCM/MeOH, then solid filtered and dried to afford the title product (688 mg).

LCMS (Method 2): Rt = 1.07 min, ES⁺ *m*/*z* 401.0/402.9 [M+H]⁺

### Preparation of Intermediates 15b to 15c

The following compounds were prepared using a procedure analogous to that used in the preparation of intermediate 15a starting from indicated starting material.

| Intermediate | Structure, name | Starting materials | LC-MC |
|---|---|---|---|
| Intermediate 15b | 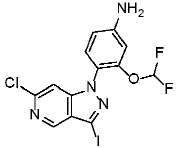 | Intermediate 12b | (Method 1) Rt=1.13, ES⁺ *m*/*z* 437.1/439.0 [M+H]⁺ |
| Intermediate 15c | 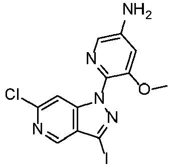 | Intermeidate 12c | (Method 2) Rt = 0.90 min, ES⁺ *m*/*z* 401.9/403.9 [M+H]⁺ |

### Intermediate 16a

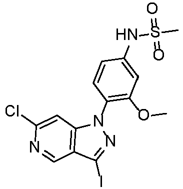

### N-(4-(6-chloro-3-iodo-1H-pyrazolo[4,3-c]pyridin-1-yl)-3-methoxyphenyl)methanesulfonamide (Intermediate 16a)

A mixture of intermediate 15a (700 mg, 1.6 mmol) and DCM (14 mL) was cooled to 0 °C and added with pyridine (145 µL, 1.8 mmol) followed by methanesulfonyl chloride (138 µL, 1.8 mmol). RM was stirred at RT for 16 h, then quenched with water (10 mL) and pH adjusted to 8 before extraction with DCM (4 x 10 mL). Combined organic layers were dried over Na₂SO₄, solvent removed under reduced pressure and the residue purified by flash chromatography on a Si cartridge by eluting with 0-50 % MeOH/DCM (1:20) in DCM to give the title product (420 mg).

LCMS (Method 2): Rt = 0.64 min, ES⁺ *m*/*z* 479.0/481.0 [M+H]⁺

### Preparation of Intermediates 16b to 16f

The following compounds were prepared using a procedure analogous to that used in the preparation of intermediate **16a** starting from indicated starting materials

| Intermediate | Structure, name | Starting materials | LC-MC |
|---|---|---|---|
| Intermediate 16b | 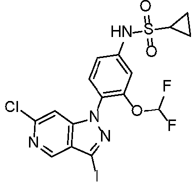 | Intermediate 15b / cyclopropanesulfonyl chloride | (Method 1) Rt = 1.18 min, ES⁺ *m*/*z* 541.0/542.9 [M+H]⁺. |
| Intermediate 16c | 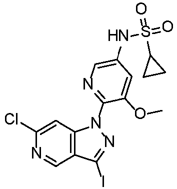 | Intermediate 15c / cyclopropanesulfonyl chloride | (Method 1) Rt = 1.04 min, ES⁺ *m*/*z* 505.6/507.5 [M+H]⁺ |
| Intermediate 16d | 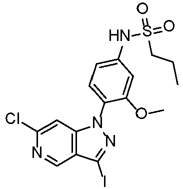 | Intermediate 15a / propanesulfonyl chloride | (Method 2) Rt = 0.72 min, ES⁺ *m*/*z* 506.9/508.8 [M+H]⁺ |
| Intermediate 16e | 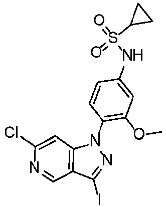 | Intermediate 15a / cyclopropanesulfonyl chloride | (Method 2) Rt = 0.69 min, ES⁺ *m*/*z* 505.0/507.0 [M+H]⁺ |
| Intermediate 16f | 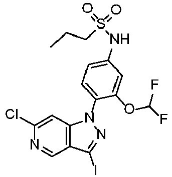 | Intermediate 15b / propanesulfonyl chloride | (Method 1) Rt = 1.22 min, ES⁺ *m*/*z* 543.0/544.9 [M+H]⁺ |

### Preparation of Intermediates 17a to 17c

The following compounds were prepared using a procedure analogous to that used in the preparation of intermediate **11a** starting from the indicated starting materials.

| Intermediate | Structure | Starting materials | LC-MC |
|---|---|---|---|
| Intermediate 17a | 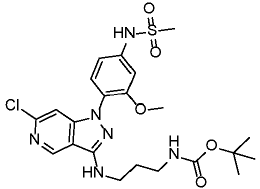 | Intermediate 14a, methanesulfonyl chloride | (Method 2) Rt = 0.66 min, ES⁺ *m*/*z* 525.2/527.2 [M+H]⁺ |
| Intermediate 17b | 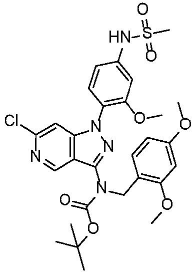 | Intermediate 14b / methanesulfonyl chloride | (Method 2) Rt = 0.88 min, ES⁺ *m*/*z* 618.2/620.2 [M+H]⁺ |
| Intermediate 17c | 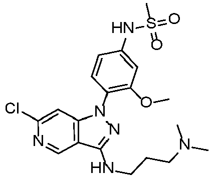 | Intermediate 14c / methanesulfonyl chloride | (Method 2) Rt = 0.55 min, ES⁺ *m*/*z* 453.2/455.1 [M+H]⁺ |

### Preparation of intermediates 17d to 17z

The following intermediates were prepared using a procedure analogous to that used in the preparation of intermediate **13a** from the indicated starting materials. When minor modifications on base, solvent, and/or temperature and/or copper source/ligand were made, they were detailed below in brackets.

| Intermediate | Structure | Starting material | LCMS |
|---|---|---|---|
| Intermediate 17d | 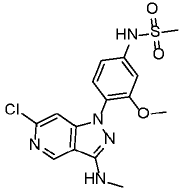 | Intermediate 16a / methylammonim chloride | (Method 1) Rt = 0.81 min, ES⁺ *m*/*z* 382.1/384.1 [M+H]⁺ |
| Intermediate 17e | 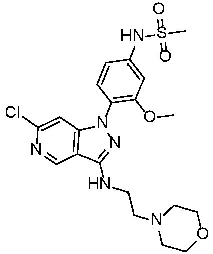 | Intermediate 16a / 2-morpholinoethan-1-amine (Reaction temperature100 °C) | (Method 1) Rt = 0.62 min, ES⁺ *m*/*z* 481.2/483.1 [M+H]⁺ |
| Intermediate 17f | 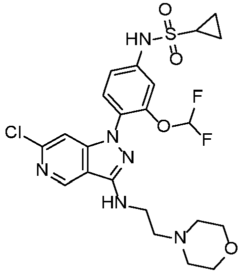 | Intermediate 16b/2-morpholinoethan-1-amine (Reaction temperature100 °C) | (Method 1) Rt = 0.73, ES⁺ *m*/*z* 543.2/545.2 [M+H]⁺ |
| Intermediate 17g | 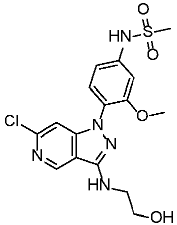 | Intermediate 16a / ethanolamine | (Method 2) Rt = 0.43 min, ES⁺ *m*/*z* 412.2/414.2 [M+H]⁺ |
| Intermediate 17h | 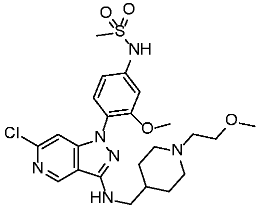 | Intermediate 16a / (1-(2-methoxyethyl)piperidin -4-yl)methanamine | (Method 1) Rt = 0.67 min, ES⁺ *m*/*z* 523.2/525.1 [M+H]⁺ |
| | | (Reaction temperature100 °C) | |
| Intermediate 17i | 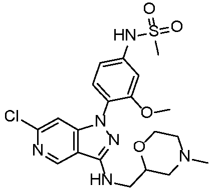 | Intermediate 16a / (4-methylmorpholin-2-yl)methanamine (Reaction temperature100 °C) | (Method 1) Rt = 0.62 min), ES⁺ *m*/*z* 481.1/483.0 [M+H]⁺ |
| Intermediate 17j | 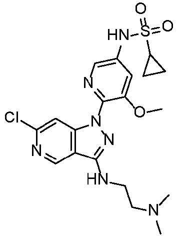 | Intermediate 16c / *N1,N1*-dimethylethane-1,2-diamine (Reaction temperature80 °C) | (Method 1) Rt = 0.64 min, ES⁺ *m*/*z 466.1*/ 468.1 [M+H]⁺ |
| Intermediate 17k | 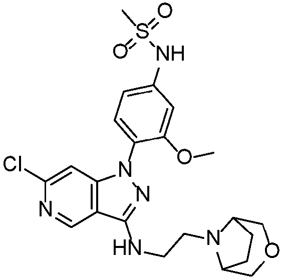 | Intermediate 16a / Intermediate 7 | (Method 2) Rt = 0.55 min, ES⁺ *m*/*z* = 506.9/508.9 [M+H]⁺ |
| | | (Reaction temperature60 °C, proline ligand replaced by 1*H*-pyrrole-2-carboxylic acid) | |
| Intermediate 17l | 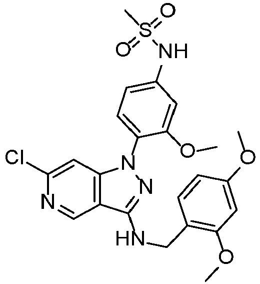 | Intermediate 16a / 2,4-dimethoxybenzylamine (Reaction temperature100 °C) | (Method 2) Rt = 0.71 min, ES⁺ *m*/*z* 518.2/520.1 [M+H]⁺, |
| Intermediate 17m | 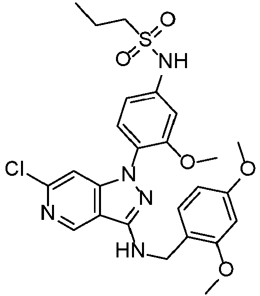 | Intermediate 16d / 2,4-dimethoxybenzylamine (Reaction temperature100 °C) | (Method 2) Rt = 0.85 min, ES⁺ *m*/*z* 546.2/548.1 [M+H]⁺ |
| Intermediate 17n | 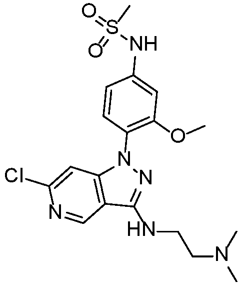 | Intermediate 16a / *N1,N1*-dimethylethane-1,2-diamine | (Method 1) Rt = 0.60 min, ES⁺ *m*/z 439.2/441.1 [M+H]⁺ |
| | | (Reaction temperature100 °C) | |
| Intermediate 17o | 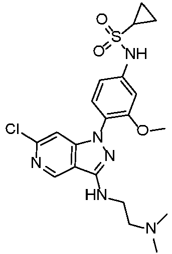 | Intermediate 16e / *N1,N1*-dimethylethane-1,2-diamine | (Method 2) Rt = 0.63 min, ES⁺ *m*/*z* 465.2/467.2 [M+H]⁺ |
| | | (Reaction temperature100 °C) | |
| Intermediate 17p | 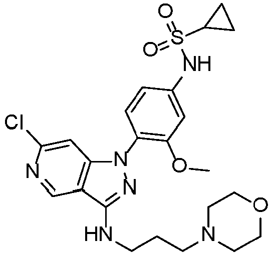 | Intermediate 16e / 3-morpholinopropan-1-amine | (Method 2) Rt = 0.60 min, ES⁺ *m*/*z* 521.2/523.2 [M+H]⁺ |
| | | (Reaction temperature100 °C) | |
| Intermediate 17q | 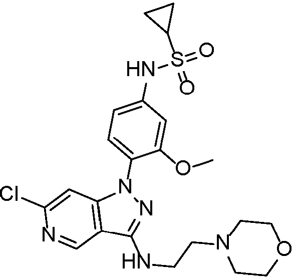 | Intermediate 16e / 2-morpholinoethan-1-amine | (Method 2) Rt = 0.57 min, ES⁺ *m*/*z* 507.2/509.2 MH+) |
| | | (Reaction temperature100 °C) | |
| Intermediate 17r | 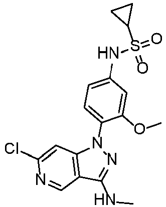 | Intermediate 16e / 2-methylammonium chloride | (Method 2) Rt = 0.53 min, ES⁺ *m*/*z* 408.2/410.2 [M+H]⁺ |
| | | (Reaction temperature100 °C) | |
| Intermediate 17s | 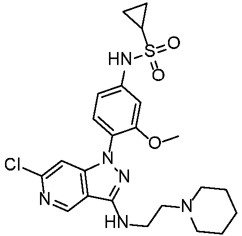 | Intermediate 16e / 2-2-(piperidin-1-yl)ethan-1-amine | (Method 2) Rt = 0.71 min, ES⁺ *m*/*z* 505.3/507.2 [M+H]⁺ |
| | | (Reaction temperature100 °C) | |
| Intermediate 17t | 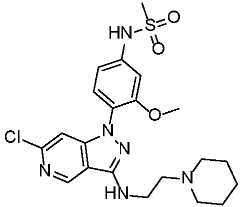 | Intermediate 16a / 2-(piperidin-1-yl)ethan-1-amine | (Method 2) Rt = 0.60 min, ES⁺ *m*/*z* 479.2/481.2 [M+H]⁺ |
| | | (Reaction temperature100 °C) | |
| Intermediate 17u | 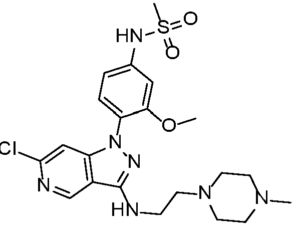 | Intermediate 16a / 2-(4-methylpiperazin-1-yl)ethan-1-amine | (Method 1) Rt = 0.59 min, ES⁺ *m*/*z* 492.2/494.2 [M+H]⁺ |
| | | (Reaction temperature100 °C, DCM reaction solvent replaced by DMF) | |
| Intermediate 17v | 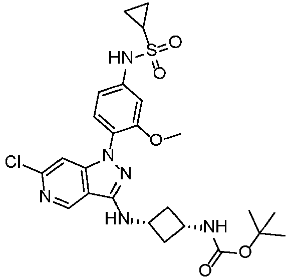 | Intermediate 16e / tert-butyl ((1s,3s)-3-aminocyclobutyl)carba mate | (Method 2) Rt = 1.1 min, ES⁺ *m*/*z* 563.3/565.3 [M+H]⁺. |
| | | (Reaction temperature100 °C, DCM reaction solvent replaced by DMF) | |
| Intermediate 17w | 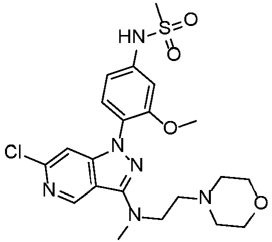 | Intermediate 16a / *N-*methyl-2-morpholinoethan-1-amine | (Method 1) Rt = 0.64 min, ES⁺ *m*/*z* 495.1/497.1 [M+H]⁺. |
| | | (Reaction temperature100 °C) | |
| Intermediate 17x | 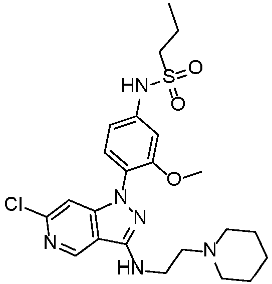 | Intermediate 16d / 2-(piperidin-1-yl)ethan-1-amine | Method 2) Rt = 0.84 min, ES⁺ *m*/*z* 507.3/509.3 [M+H]⁺ |
| | | (Reaction temperature110 °C) | |
| Intermediate 17z | 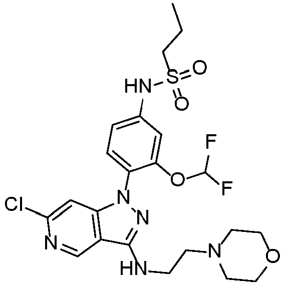 | Intermediate 16f / 2-morpholinoethan-1-amine | (Method 1) Rt = 0.76 min, ES⁺ *m*/*z* 545.2/547.2 [M+H]⁺ |
| | | (Reaction temperature100 °C) | |
| Intermediate 17aa | 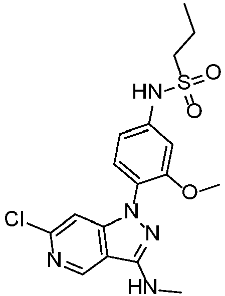 | Intermediate 16d / 2 methylammonium chloride | (Method 2) Rt = 0.58 min, ES⁺ *m*/*z* 410.1/411.9 [M+H]⁺ |
| | | (Reaction temperature110 °C) | |

### Intermediate 17ab

### Step 1

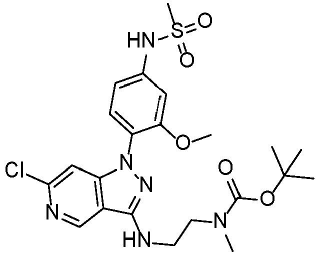

### tert-Butyl (2-((6-chloro-1-(2-methoxy-4-(methylsulfonamido)phenyl)-1H-pyrazolo[4,3-c]pyridin-3-yl)amino)ethyl)(methyl)carbamate (Intermediate 17ab-1)

Intermediate 17ab-1 was prepared using a procedure analogous to that used in the preparation of intermediate 13a starting from intermediate 16a and *tert*-butyl (2-aminoethyl)(methyl)carbamate (Reaction temperature100 °C, DCM reaction solvent replaced by DMF).

LCMS (Method 2): Rt = 0.68 min, ES⁺ *m*/*z* 525.3/527.2 [M+H]⁺

### Step 2

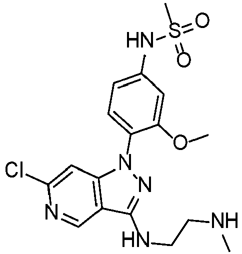

### N-(4-(6-Chloro-3-((2-(methylamino)ethyl)amino)-1H-pyrazolo[4,3-c]pyridin-1-yl)-3-methoxyphenyl)methanesulfonamide (Intermediate 17ab-2)

To a solution of intermediate 17ab-1 (103 mg, 0.20 mmol) in DCM (1.5 mL), TFA (0.437 mL, 5.89 mmol) was added dropwise. RM was stirred at RT for 1 h, then evaporated under reduced pressure to dryness. The residue was dissolved in DCM (10 mL), washed with aqueous saturated NaHCO₃ (3x10 mL), and evaporated to dryness. The crude product was diluted in methanol, added of aqueous 6M HCl and stirred for 2h at RT. RM was partitioned between water (10 mL) / EtOAc (5 mL) with a pH corrected to 8. Aqueous layer was further extracted with EtOAc (4x5 mL), and combined organic layers were washed with aqueous saturated NaCl (2x15 mL) then evaporated to dryness. The residue was chromatographed on a Si by cartridge by eluting with 0-100 % DCM/MeOH/NH₄OH (90:9:0.5) in DCM to afford the title product (35 mg).

LCMS (Method 2): Rt = 0.48 min, ES⁺ *m*/*z* 425.2/427.2 [M+H]⁺

### Step 3

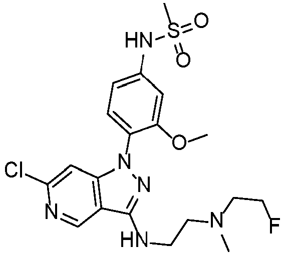

### N-(4-(6-Chloro-3-((2-((2-fluoroethyl)(methyl)amino)ethyl)amino)-1H-pyrazolo[4,3-c]pyridin-1-yl)-3-methoxyphenyl)methanesulfonamide (Intermediate 17ab-3)

Intermediate 17ab-a (32 mg, 0.08 mmol), 1-bromo-2-fluoro-ethane (5.61 µL, 0.08 mmol), DIPEA (78.7 µL, 0.45 mmol) in acetonitrile (1.2 mL) were stirred at RT for 1 h then heated at 50°C for 3 h. An half equivalent of 1-bromo-2-fluoro-ethane was added and stirring continued at 50°C overnight. RM was evaporated and the residue purified by flash chromatography on a Si cartridge by eluting with 0-80 % DCM/MeOH/NH₄OH (90:9:0.5) in DCM to give the title product (29 mg).

LCMS (Method 2): Rt = 0.53 min, ES+ *m*/*z* 471.2/473.2 [M+H]+

### Intermediate 17ac

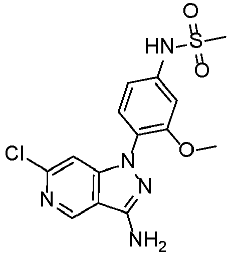

### N-(4-(3-Amino-6-chloro-1H-pyrazolo[4,3-c]pyridin-1-yl)-3-methoxyphenyl)methanesulfonamide (Intermediate 17ac)

Intermediate 17b (129 mg, 0.209 mmol) in DCM (5 mL) was added with TFA (0.975 mL, 13.1 mmol) and stirred at RT for 1h. RM was evaporated under reduced pressure and residue partitioned between DCM / aqueous saturated NaHCO₃. Organic layer was dried over Na₂SO₄ and sevaporated to dryness to give the desired product (77 mg) that was used in the next steps without further purifications.

LCMS (Method 2): Rt = 0.42 min, ES⁺ *m*/*z* 368.0/369.9 [M+H]⁺

### Intermediate 17ad

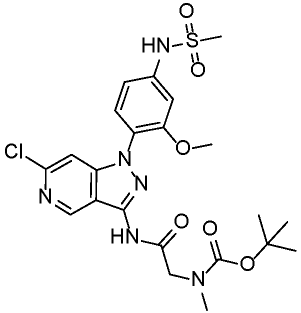

### tert-Butyl (2-((6-chloro-1-(2-methoxy-4-(methylsulfonamido)phenyl)-1H-pyrazolo[4,3-c]pyridin-3-yl)amino)-2-oxoethyl)(methyl)carbamate (Intermediate 17ad)

A suspension of BOC-Sarcosine (37.5 mg, 0.2 mmol) and EEDQ (49.0 mg, 0.2 mmol) in DCE (1 mL) was stirred for 10 minutes at RT, then a solution intermediate 17ac (35.0 mg, 0.1 mmol) in DCE (2 mL) was added and RM stirred at 80°C overnight. After cooling to RT, RM was diluted with DCM and washed with aqueous saturated NaHCO₃ (2x5 mL). Organic layer was evaporated to give a crude product that was purified by flash chromatography on a Si cartridge by eluting with 0-80 % MeOH/DCM (1: 10) in DCM to afford the title product (25 mg).

LCMS (Method 2): Rt 0.62 min, ES⁺ *m*/*z*= 539.1/541.0 [M+H]⁺

### Intermediate 17ae

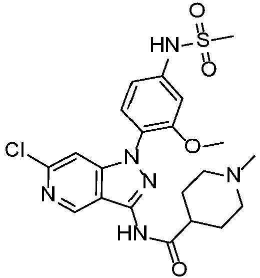

### N-(6-Chloro-1-(2-methoxy-4-(methylsulfonamido)phenyl)-1H-pyrazolo[4,3-c]pyridin-3-yl)-1-methylpiperidine-4-carboxamide (Intermediate 17ae)

Intermediate 17ac (213 mg, 0.58 mmol) was suspended in dry DCM (50 mL), then TEA (0.48 mL, 3.47 mmol) added and RM cooled to 0°C. 1-Methylpiperidine-4-carbonyl chloride hydrochloride (172 mg, 0.87 mmol) was added and RM stirred at RT overnight. A further equivalent of 1-methylpiperidine-4-carbonyl chloride hydrochloride was added and stirring proceeded for further 3 h. Reaction was quenched with aqueous saturated NaHCO₃, extracted with DCM Organic layer was washed with aqueous saturated NaHCO₃, dried over Na₂SO₄ and concentrated. The residue was dissolved in DCM / MeOH / aqueous conc NH₃ 9/1/0.15 (50 mL), added with pyridine and stirred overnight. RM was concentrated and purified by flash chromatography on a Si cartridge by eluting with 0-100 % DCM/MeOH/NH₄OH (90:9:1.5) in DCM to afford the title compound (175 mg).

LCMS (Method 1): Rt = 0.61 min, ES⁺ *m*/*z* 493.2/495.0 [M+H]⁺

### Intermediate 17af

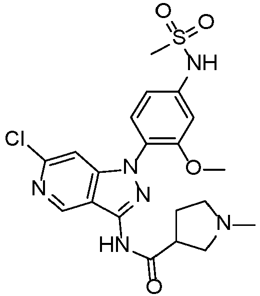

### N-(6-chloro-1-(2-methoxy-4-(methylsulfonamido)phenyl)-1H-pyrazolo[4,3-c]pyridin-3-yl)-1-methylpyrrolidine-3-carboxamide (Intermediate 17af)

1-Methylpyrrolidine-3-carboxylic acid (80 mg, 0.62 mmol) was suspended in SOCl₂ (1 mL) and after stirring at RT for 2h , the RM was evaporated under reduced pressure. The residue was taken in dry THF (0.5 mL), cooled at 0°C, and added with a suspension of intermediate 17ac (120 mg, 0.33 mmol) in THF (2 mL) / TEA (0.16 mL, 1.14 mmol), RM was stirred at RT overnight, quenched with aqueous saturated NaHCO₃ (30 mL) and extracted with DCM (5 x 30 mL). Combined organic layers were washed with aqueous saturated NaCl, dried over Na₂SO₄ and evaporated to dryness. The residue was purified by flash chromatography on a Si cartridge by eluting with 0-100 % DCM/MeOH/NH₄OH (90:9:1.5) in DCM to give the title compound (28 mg).

LCMS (Method 1): Rt = 0.61 min, ES⁺ *m*/*z* 479.1/481.1 [M+H]⁺

### Intermediate 18a

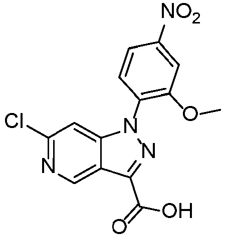

### 6-Chloro-1-(2-methoxy-4-nitrophenyl)-1H-pyrazolo[4,3-c]pyridine-3-carboxylic acid (Intermediate 18a)

To a suspension of 6-chloro-1*H*-pyrazolo[4,3-c]pyridine-3-carboxylic acid (500 mg, 2.53 mmol) in dry DMF (7.5 mL) under argon atmosphere, DBU (1.96 mL, 15.2 mmol) was added, followed by 1-fluoro-2-methoxy-4-nitrobenzene (520 mg, 3.94 mmol). RM was stirred in a MW reactor at 65 °C for 45 minutes. RM of two identical batches obtained as described above were combined, quenched with aqueous 1N HCl (30 mL), and the formed precipitate filtered, washed with water, dried and triturated in diethyl ether to afford the title product (1.02 g).

LCMS (Method 1): Rt = 0.95 min, ES⁺ *m*/*z* 349.0/349.9 [M+H]⁺

### Intermediate 18b

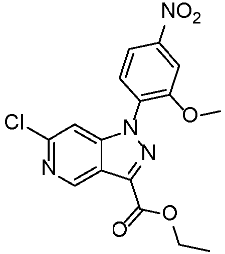

### Ethyl 6-chloro-1-(2-methoxy-4-nitrophenyl)-1H-pyrazolo[4,3-c]pyridine-3-carboxylate (Intermediate 18b)

Intermediate 18a (1.04 g, 2.98 mmol), HBTU (2.82 g, 7.46 mmol), and DMAP (36.4 mg, 0.3 mmol) were dissolved in dry DCM (65 mL), then DIPEA (2.6 mL, 14.9 mmol) and ethanol (556 mL, 8.95 mmol) were added and RM was stirred at RT for 2h. Solvent was removed under reduced pressure and residue was reconstituted in EtOAc (50 mL). Solution was washed with aqueous saturated NaHCO₃ (3 x 50 mL), aqueous saturated NaCl (50 mL) and the organic layer dried over Na₂SO₄. After solvent removal under reduced pressure, the crude was chromatographed on a Si cartridge by eluting with 0-10 % EtOAc in DCM to afford the title product (748 mg).

LCMS (Method 1): Rt = 1.20 min, ES⁺ *m*/*z* 377.0/379.0 [M+H]⁺

### Intermediate 18c

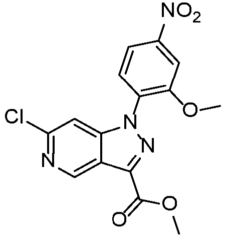

### Methyl 6-chloro-1-(2-methoxy-4-nitrophenyl)-1H-pyrazolo[4,3-c]pyridine-3-carboxylate (Intermediate 18c)

To a suspension of 6-chloro-1*H*-pyrazolo[4,3-c]pyridine-3-carboxylic acid (250 mg, 1.27 mmol) in dry DMF (10 mL) under nitrogen, DBU (568 µL, 3.8 mmol) followed by 1-fluoro-2-methoxy-4-nitrobenzene (260 mg, 1.52 mmol) were added. RM was stirred under MW irradiation 60 °C for 1 h, then a further half equivalent of DBU was added followed by a further irradiation cycle at 60 °C for 30 min .After cooling at RT, RM was treated with methyl iodide (237 µL, 3.80 mmol) overnight at RT a at 60 °C for 30 min. RM was quenched in water and the formed precipitate filtered, washed with water and dried overnight at 45 °C to afford the title product (605 mg).

LCMS (Method 1): Rt = 1.13 min, ES⁺ *m*/*z* 363.0/365.1 [M+H]⁺

### Intermediate 19

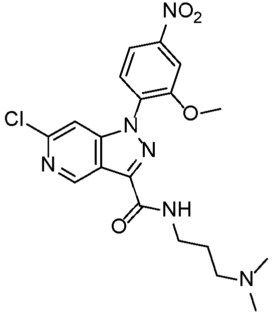

### 6-Chloro-N-(3-(dimethylamino)propyl)-1-(2-methoxy-4-nitrophenyl)-1H-pyrazolo[4,3-c]pyridine-3-carboxamide (Intermediate 19)

Intermediate 18a (100 mg, 0.29 mmol) was dissolved in dry DMF (2 mL), followed by sequential addition of *N*',*N*'-dimethylpropane-1,3-diamine (43 µL, 0.36 mmol), DIPEA (100 µL, 0.57 mmol) and HATU (120 mg, 0.32 mmol). RM was stirred at RT for 1h, diluted with EtOAc (20 mL),washed with aqueous saturated NaHCO₃ (3x15 mL) and aqueous saturated NaCl (15 mL). Organic layer was dried over Na₂SO₄ and solvents removed under reduced pressure to afford the title product (136 mg) that was used in the next steps without further purifications.

LCMS (Method 2): Rt = 1.08 min, ES⁺ *m*/*z* 433.1/435.1 [M+H]⁺

### Intermediate 20

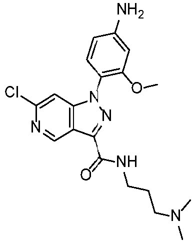

### 1-(4-amino-2-methoxyphenyl)-6-chloro-N-(3-(dimethylamino)propyl)-1H-pyrazolo[4,3-c]pyridine-3-carboxamide (Intermediate 20)

A suspension of intermediate 19 (134 mg, 0.31 mmol) and iron (52 mg, 0.92 mmol) in acetic acid (2 mL) was stirred at 70 °C for 30 min. RM was cooled to RT, diluted with methanol and load on a SCX column. After washings with methanol, the product was eluted with methanolic ammonia. Pooled fractions were evaporated under reduced pressure to give the desired product (96 mg) that was used in the next steps without further purifications.

LCMS (Method 2): Rt = 0.90 min, ES⁺ *m*/*z* 403.2/405.1 [M+H]⁺

### Intermediate 21a

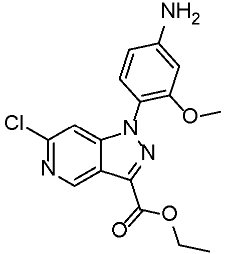

### Ethyl 1-(4-amino-2-methoxyphenyl)-6-chloro-1H-pyrazolo[4,3-c]pyridine-3-carboxylate (Intermediate 21a)

A suspension of intermediate 18b (748 mg, 1.99 mmol) and iron (333 mg, 5.96 mmol) in acetic acid (12 mL) was stirred at 70 °C for 2 hours. RM was cooled to RT, neutralized with cold aqueous saturated NaHCO₃ and extracted with DCM (5x25 mL). Combined organic layers were washed with aqueous saturated NaCl, dried over Na₂SO₄ and solvent removed under reduced pressure to afford the title compound (676 mg) that was used in the next synthetic steps further purifications.

LCMS (Method 1): Rt = 1.02 min, ES⁺ *m*/*z* 347.0/349.0 [M+H]⁺

### Intermediate 21b

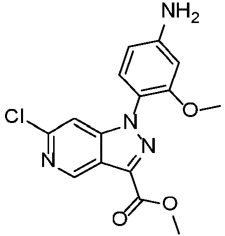

### Methyl 1-(4-amino-2-methoxyphenyl)-6-chloro-1H-pyrazolo[4,3-c]pyridine-3-carboxylate (Intermediate 21b)

Intermediate 18c (605 mg, 1.7 mmol) was heated at 80 °C in a mixture of ethanol (15 mL) / water (5 mL) then ammonium chloride (177 mg, 3.3 mmol) and iron (559 mg, 10 mmol) added. After 1h at 80 °C , RM was cooled to RT, diluted with EtOAc and filtered (filtered solid were thoroughly washed with EtOAc). Combined AcOEt fractions were evaporated to dryness to afford the desired product (375 mg) that was used in the next steps without further purifications.

LCMS (Method 1): Rt = 0.94 min, ES⁺ *m*/*z* 333.1/335.1 [M+H]⁺

### Intermediate 22a

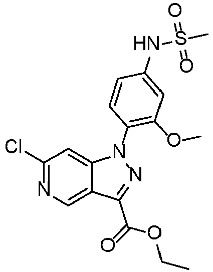

### Ethyl 6-chloro-1-(2-methoxy-4-(methylsulfonamido)phenyl)-1H-pyrazolo[4,3-c]pyridine-3-carboxylate (Intermediate 22a)

Intermediate 22a was prepared in a similar manner to intermediate 11a starting from intermediate 21a and methanesulfonyl chloride.

LCMS (Method 2): Rt = 0.58 min, ES⁺ *m*/*z* 425.2/427.0 [M+H]⁺

### Intermediate 22b

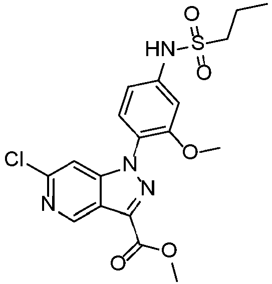

### Methyl 6-chloro-1-(2-methoxy-4-(propylsulfonamido)phenyl)-1H-pyrazolo[4,3-c]pyridine-3-carboxylate (Intermediate 22b)

Intermediate 22b was prepared in a similar manner to intermediate 11a starting from intermediate 21b and propanesulfonyl chloride.

LCMS (Method 1): Rt = 1.07 min, ES⁺ *m*/*z* 439.1/441.0 [M+H]⁺

### Intermediate 23a

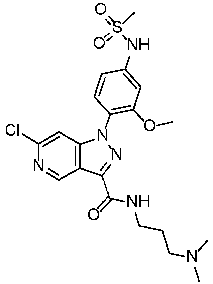

### 6-Chloro-N-(3-(dimethylamino)propyl)-1-(2-methoxy-4-(methylsulfonamido)phenyl)-lH-pyrazolo [4,3-c]pyridine-3-carboxamide (Intermediate 23a)

Intermediate 23a was prepared in a similar manner to intermediate 16a starting from intermediate 20 and methanesulfonyl chloride.

LCMS (Method 2): Rt = 0.55 min, ES⁺ *m*/*z* 481.2/483.1 [M+H]⁺

### Intermediate 23b

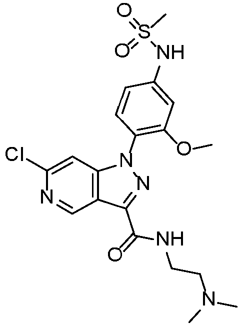

### 6-Chloro-N-(2-(dimethylamino)ethyl)-1-(2-methoxy-4-(methylsulfonamido)phenyl)-1H-pyrazolo[4,3-c]pyridine-3-carboxamide (Intermediate 23b)

A mixture of Intermediate **22a** (100 mg, 0.24 mmol) and DABAL-Me₃ (91 mg, 0.35 mmol) in THF (3 mL), followed by addition of *N',N'*-dimethylethane-1,2-diamine (39 *µ*L; 0.35 mmol) was heated in a MW reactor at 130 °C for 10 min. After cooling to RT, RM was quenched with 4M HCl and neutralized with methanolic ammonia (7M). The mixture was evaporated under reduced pressure and the residue purified by flash chromatography on a Si cartridge by eluting with 0-80 % DCM/MeOH/NH₄OH (90:9:1.5) in DCM To afford the title product (83 mg).

LCMS (Method 2): Rt = 0.50 min, ES⁺ *m*/*z* 467.1/469.1 [M+H]⁺

### Preparation of Intermediates 23c to 23e

The following intermediates were prepared using a procedure analogous to that used in the preparation of intermediate 23b from the indicated starting materials.

| Intermediate | Structure | Starting materials | LCMS |
|---|---|---|---|
| Intermediate 23c | 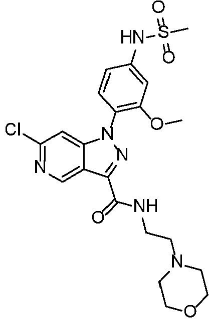 | Intermediate 22a / 2-morpholinoethan-1-amine | (Method 2) Rt = 0.49, ES⁺ *m*/*z* 509.1/511.0 [M+H]⁺ |
| Intermediate 23d | 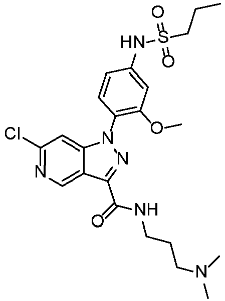 | Intermediate 22b / *N',N'-*dimethylpropane-1,3-diamine | (Method 1) Rt = 0.75, ES⁺ *m*/*z* 509.3/511.2 [M+H]⁺ |
| Intermediate 23e | 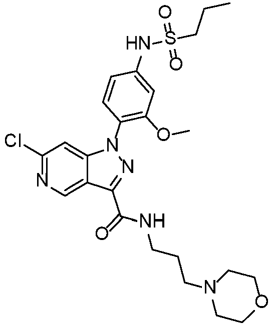 | Intermediate 22b / 3-morpholinopropan-1-amine | (Method 1) Rt = 0.76, ES⁺ *m*/*z* 551.3/553.3 [M+H]⁺ |

### Preparation of intermediates 24a-c

The following intermediates were prepared using a procedure analogous to that used in the preparation of intermediate **13a** from the indicated starting materials. When minor modifications on base, solvent, and/or temperature and/or copper source/ligand were made, they were detailed below in brackets.

| Intermediate | Structure | Starting materials | LCMS |
|---|---|---|---|
| Intermediate 24a | 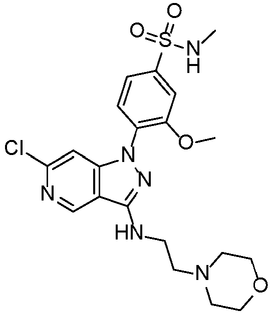 | Intermediate 12d / 2-morpholinoethan-1-amine (Reaction temperature 110 °C) | (Method 2) Rt = 0.87 min, ES⁺ *m*/*z* 481.1/483.1 [M+H]⁺ |
| Intermediate 24b | 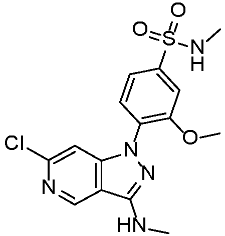 | Intermediate 12d / methylammonim chloride | (Method 2) Rt = 0.87 min, ES⁺ *m*/*z* 382.1/384.1 [M+H]⁺ |
| | | (Reaction temperature 100 °C) | |
| Intermediate 24c | 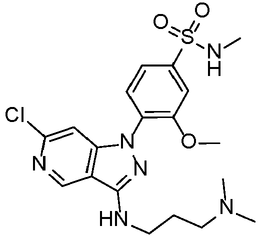 | Intermediate 12d / *N¹,N¹*-dimethylpropane-1,3-diamine (Reaction temperature 100 °C) | (Method 2) Rt = 0.91 min, ES⁺ *m*/*z* 453.2/455.2 [M+H]⁺ |

### Intermediate 25a

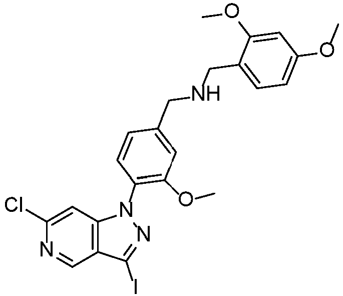

### N-(4-(6-chloro-3-iodo-1H-pyrazolo[4,3-c]pyridin-1-yl)-3-methoxybenzyl)-1-(2,4-dimethoxyphenyl)methanamine (Intermediate 25a)

A mixture of Intermediate 12e (1 g, 2.42 mmol) in DCM (20 mL) followed by the addition of (2,4-Dimethoxyphenyl)methanamine (524 µL, 3.49mmol) and acetic acid (692 µL, 12.1 mmol) was refluxed overnight. RM was cooled to RT, sodium triacetoxyborohydride (2.06 g, 9.74 mmol) added portionwise, and then stirred at RT for 3. RM was partitioned between DCM (20 mL) and aqueous saturated NaHCO₃, aqueous layer further extracted with DCM (2x10 mL) and combined organic layers were concentrated under reduced pressure. The residue chromatographed on a Si cartridge by eluting with 0-100 % EtOAc in DCM to afford the title compound (1.26 g).

LCMS (Method 2): Rt = 1.35 min, ES⁺ *m*/*z* = 565.0/566.9 [M+H]⁺

### Intermediate 25b

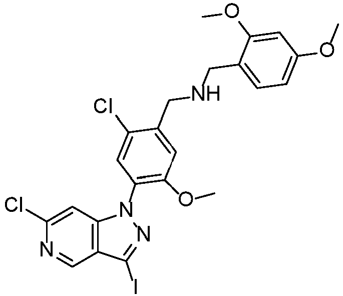

### N-(2-chloro-4-(6-chloro-3-iodo-1H-pyrazolo[4,3-c]pyridin-1-yl)-5-methoxybenzyl)-1-(2,4-dimethoxyphenyl)methanamine (Intermediate 25b)

Intermediate 25b was prepared in a similar manner to intermediate 25a starting from intermediate 12f.

LCMS (Method 2): Rt = 1.16 min, ES⁺ *m*/*z* = 599.2 [M+H]⁺

### Intermediate 26a

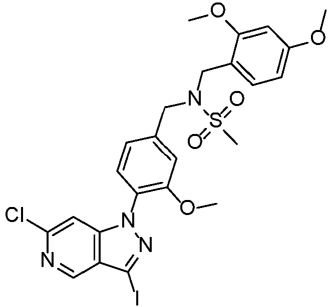

### N-(4-(6-Chloro-3-iodo-1H-pyrazolo[4,3-c]pyridin-1-yl)-3-methoxybenzyl)-N-(2,4-dimethoxybenzyl)methanesulfonamide (Intermediate 26a)

Intermediate 25a (250 mg, 0.44 mmol) was dissolved in dry DCM (4 mL) followed by the addition of methanesulfonyl chloride (41 µL, 0.53 mmol) and TEA (185 µL, 1.3 mmol). RM was stirred at RT for 4 h, then diluted with EtOAc (15 mL), washed with aqueous saturated NaHCO₃ (3x10 mL) and aqueous saturated NaCl (10 ml). Organic layer was dried over Na₂SO₄, filtered and evaporated to dryness to afford the desired product (262 mg) that was used in the next steps without further purifications.

LCMS (Method 2): Rt = 1.35 min, ES⁺ *m*/*z* = 643.0/645.0 [M+H]⁺

### Intermediate 26b

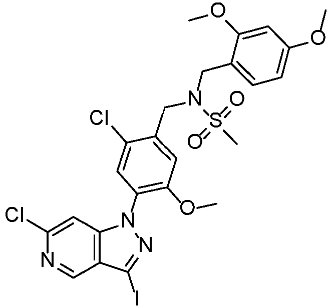

### N-(2-Chloro-4-(6-chloro-3-iodo-1H-pyrazolo[4,3-c]pyridin-1-yl)-5-methoxybenzyl)-N-(2,4-dimethoxybenzyl)methanesulfonamide (Intermediate 26b)

Intermediate 26b was prepared similarly to intermediate 26a starting from intermediate 25b.

LCMS (Method 2): Rt = 1.43 min, ES⁺ *m*/*z* = 677.2/679.2/681.2 [M+H]⁺

### Preparation of intermediates 27a to 27f

The following intermediates were prepared using a procedure analogous to that used in the preparation of intermediate **13a** from the indicated starting materials. When minor modifications on base, solvent, and/or temperature and/or copper source/ligand were made, they were detailed below in brackets

| Intermediate | Structure | Starting materials | LCMS |
|---|---|---|---|
| Intermediate 27a | 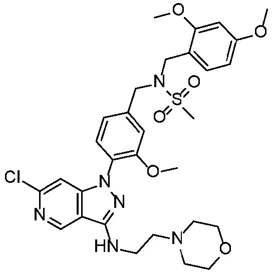 | Intermediate 26a / 2-morpholinoethan-1-amine (Reaction temperature 100 oC) | (Method 1) Rt = 0.92 min, ES⁺ *m*/*z* 645.2/647.2 [M+H]⁺ |
| Intermediate 27b | 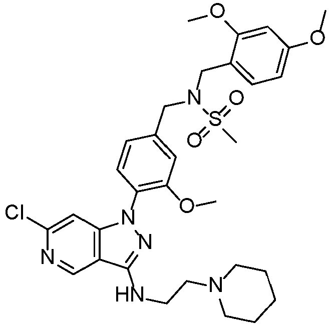 | Intermediate 26a / 2-(1-piperidyl)ethanamine (Reaction temperature 100 oC) | (Method 1) Rt = 0.95 min, ES⁺ *m*/*z* 643.2/645.2[ M+H]⁺ |
| Intermediate 27c | 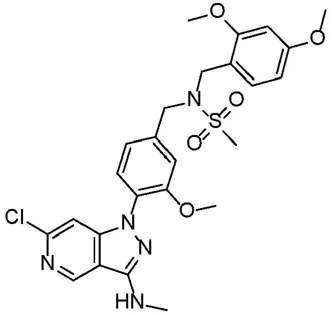 | Intermediate 26a / methylammonium chloride (Reaction temperature 100 oC) | (Method 2) Rt = 1.11 min, ES⁺ *m*/*z* 545.9/547.8 [M+H]⁺ |
| Intermediate 27d | 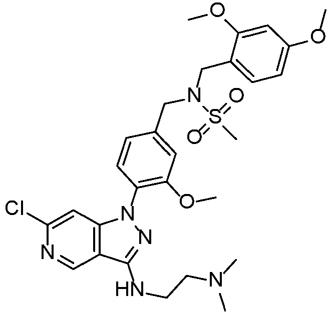 | 26a / *N1,N1-*dimethylethane-1,2-diamine (Reaction temperature 100 oC) | (Method 1) Rt = 0.90 min, ES⁺ *m*/*z* 603.2/605.2[ M+H]⁺ |
| Intermediate 27e | 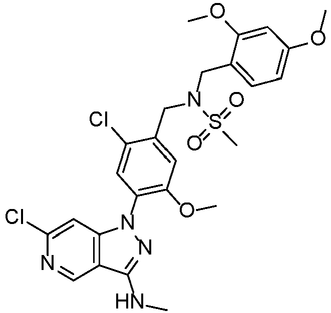 | 26b / methylammonium chloride (Reaction temperature 105 oC) | (Method 1) Rt = 1.24 min, ES⁺ *m*/*z* 579.93/581.9/ 583.9 [M+H]⁺ |
| Intermediate 27f | 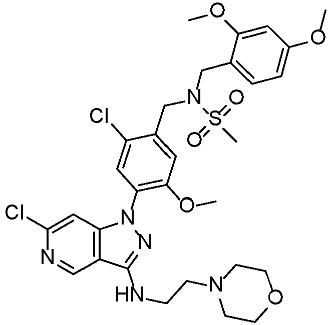 | 26b / 2-morpholinoethan-1-amine (Reaction temperature 100°C) | (Method 2) Rt = 1.20 min, ES⁺ *m*/*z* 679.4/681.4/6 84.3 [M+H]⁺ |

### Intermediate 28

### Step 1

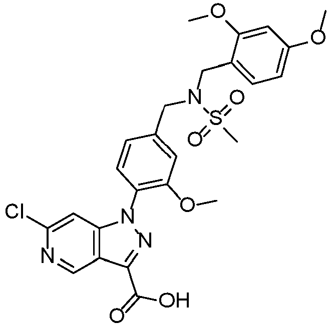

### 6-Chloro-1-(4-((N-(2,4-dimethoxybenzyl)methylsulfonamido)methyl)-2-methoxyphenyl)-1H-pyrazolo[4,3-c]pyridine-3-carboxylic acid (Intermediate 28-1)

An oven-dried tube was charged under argon with Pd(OAc)₂ (1.4 mg, 6 µmol), Xantphos (3.5 mg, 6 µmol) and DMF (1 mL). Formic acid (53.4 µL, 1.42 mmol), and intermediate 26a (130 mg, 0.202 mmol) were added, followed by DCC (83.4 mg, 0.404 mmol) and TEA (56.4 µL, 0.404 mmol). The reaction tube was sealed and RM stirred at 60 °C for 3 h, then cooled to RT and filtered. The filtrate was added with water and pH adjusted to 10 with aqueous 1M NaOH and washed with DCM (3 x 10 mL). Aqueous layer was neutralized with aqueous 1M HCl to pH ~ 3.5 and extracted with EtOAc (2 x 10 mL). Combined organic layers were washed with aqueous saturated NaCl (10 mL), passed through a phase separator and solvent evaporated to give crude product that was purified by flash chromatography on a Si cartridge by eluting with 0-20% DCM/MeOH/formic acid (90:10:0.3) in DCM affording the title product (59 mg).

LCMS (Method 2): Rt = 0.70 min, ES⁺ *m*/*z* 561.1/563.1 [M+H]⁺

### Step 2

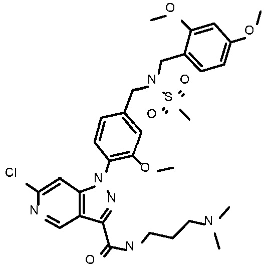

### 6-Chloro-1-(4-((N-(2,4-dimethoxybenzyl)methylsulfonamido)methyl)-2-methoxyphenyl)-N-(3-(dimethylamino)propyl)-1H-pyrazolo[4,3-c]pyridine-3-carboxamide (Intermediate 28)

Intermediate 28-1 (57.0 mg, 0.102 mmol), N',N'-dimethylpropane-1,3-diamine (16.7 µL, 0.132 mmol) and DIPEA (35.4 µL, 0.203 mmol) were dissolved in dry DMF (1 mL), then HATU (42.5 mg, 0.112 mmol) was added and RM stirred at RT for 30 min. RM was diluted with EtOAc (15 mL), washed with aqueous saturated NaHCO₃ (3x10 mL) and aqueous saturated NaCl (10 mL), dried over Na₂SO₄ and concentrated *in vacuo* to provide the title product (63 mg) that was used in the next steps without further purifications.

LCMS (Method 2): Rt = 1.17 min, ES⁺ *m*/*z* 644.9/646.8 [M+H]⁺.

### Intermediate 29

### Step 1

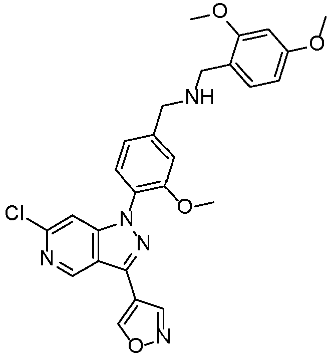

### N-(4-(6-Chloro-3-(isoxazol-4-yl)-1H-pyrazolo[4,3-c]pyridin-1-yl)-3-methoxybenzyl)-1-(2,4-dimethoxyphenyl)methanamine (Intermediate 29-1)

A mixture intermediate 26a (1.2 g, 2.12 mmol), 4-(4,4,5,5-tetramethyl-1,3,2-dioxaborolan-2-yl)isoxazole (622 mg, 3.19 mmol), Pd(dppf)Cl₂.CH₂Cl₂ (260 mg, 0.319 mmol) and Na₂CO₃ (450 mg, 4.25 mmol) in 1,4-dioxane (14 mL)/water (7 mL) was purged with argon. RM was stirred at 60 °C for 1 h, then diluted with EtOAc, washed with water (3x20 mL) and aqueous saturated NaCl (15 mL). Organic layer was passed through phase separator and solvent was evaporated *in vacuo* to give a crude that was chromatographed on a Si cartridge by eluting with 0-20 % DCM/MeOH (9:1) in DCM affording the title compound (680 mg).

LCMS (Method 2): Rt = 1.22 min, ES⁺ *m*/*z* 506.2 [M+H]⁺.

### Step 2

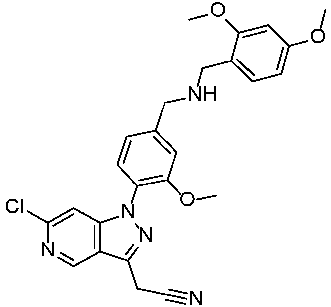

### 2-(6-Chloro-1-(4-(((2,4-dimethoxybenzyl)amino)methyl)-2-methoxyphenyl)-1H-pyrazolo[4,3-c]pyridin-3-yl)acetonitrile (Intermediate 29-2)

Intermediate 29-1 (680 mg, 1.34 mmol) was dissolved in a mixture of DMF/water 2:1 (12 mL), added with KF (234 mg, 4.03 mmol) and RM stirred at 120 °C overnight. RM was diluted with EtOAc (25 mL), then washed with aqueous saturated NaHCO₃ (3x20 mL) and aqueous saturated NaCl (20 mL). The organic extract was dried over Na₂SO₄ and concentrated *in vacuo* to provide a crude product that was purified by flash chromatography on a Si cartridge by eluting with 0-50 % DCM/MeOH (9:1) in DCM affording the title product (276 mg).

LCMS (Method 2): Rt = 1.12 min, ES⁺ *m*/*z* 478.2/480.2 [M+H]⁺.

### Step 3

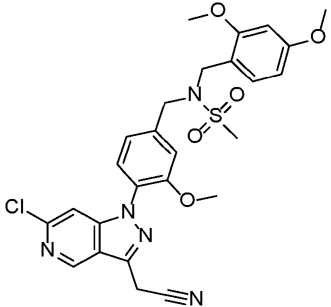

### N-(4-(6-Chloro-3-(cyanomethyl)-1H-pyrazolo[4,3-c]pyridin-1-yl)-3-methoxybenzyl)-N-(2,4-dimethoxybenzyl)methanesulfonamide

Intermediate 29 was prepared similarly to intermediate 11a starting from intermediate 29-2.

LCMS (Method 2): Rt = 1.10 min, ES⁺ *m*/*z* 556.1/558.1 [M+H]⁺.

### Intermediate 30a

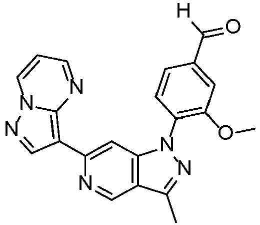

### 3-Methoxy-4-(3-methyl-6-(pyrazolo[1,5-a]pyrimidin-3-yl)-1H-pyrazolo[4,3-c]pyridin-1-yl)benzaldehyde (Intermediate 30a)

A round-bottom-flask was charged (under nitrogen atmosphere) with intermediate 9i (1.4 g, 4.4 mmol), 3-(4,4,5,5-tetramethyl-1,3,2-dioxaborolan-2-yl)pyrazolo[1,5-a]pyrimidine (1.62 g, 6.6 mmol), K₃PO₄(1.88 g, 8.8 mmol) and XPhos Pd G3 (187 mg, 0.22 mmol) and added with previously degassed water (17 mL) and THF (35 mL). RM stirred at 70 °C for 90 min, then water (10 mL) was added and the mixture stirred at 25 °C for 10 min. The RM was filtered and the collected precipitate washed with water (3 x 2 mL) and dried to afford the title product (1.73 g).

LCMS (Method 2): Rt = 0.96 min, ES⁺ *m*/*z* 385.3 [M+H]⁺.

### Intermediate 31

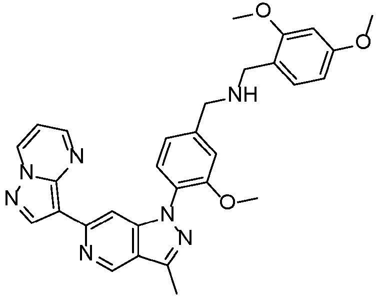

### N-(2,4-Dimethoxybenzyl)-1-(3-methoxy-4-(3-methyl-6-(pyrazolo[1,5-a]pyrimidin-3-yl)-1H-pyrazolo[4,3-c]pyridin-1-yl)phenyl)methanamine (Intermediate 31)

Intermediate 31 was prepared similarly to intermediate 25a starting from intermediate 30a.

LCMS (Method 2): Rt = 1.12 min, ES⁺ *m*/*z* 536.4 [M+H]⁺.

### Intermediate 32a

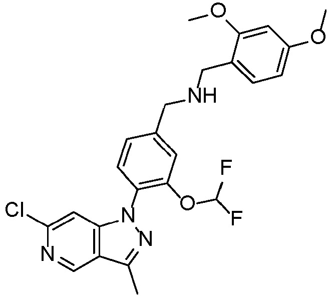

### N-(4-(6-Chloro-3-methyl-1H-pyrazolo[4,3-c]pyridin-1-yl)-3-(difluoromethoxy)benzyl)-1-(2,4-dimethoxyphenyl)methanamine (Intermediate 32a)

Intermediate 32a was prepared similarly to intermediate 25a starting from INT 9g.

LCMS (Method 1): Rt = 0.86 min, ES⁺ *m*/*z* 489.1/491.1 [M+H]⁺

### Intermediate 32b

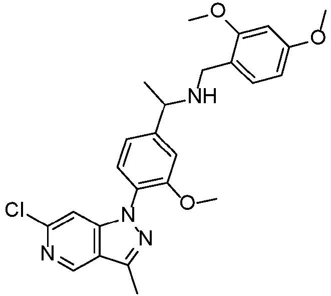

### 1-(4-(6-Chloro-3-methyl-1H-pyrazolo[4,3-c]pyridin-1-yl)-3-methoxyphenyl)-N-(2,4-dimethoxybenzyl)ethan-1-amine (Intermediate 32b)

A mixture of intermediate 9h (230 mg, 0.728 mmol) and 1,2-dichloroethane (5.0 mL) was cooled to 0 °C and added with (2,4-dimethoxyphenyl)methanamine (164 µL, 1.09 mmol), TEA (122 µL, 0.874 mmol) and titanium(IV) isopropoxide (269 mg, 0.947 mmol). RM was heated at 45 °C for 2.5 h, then cooled to 0 °C before addition of sodium triacetoxyborohydride (509 mg, 2.40 mmol). RM was heated at 45 °C for 12 h. After cooling to RT, RM was quenched with aqueous saturated NaCl (10 mL) and DCM (20 mL). The mixture was filtered and the filtrate extracted with DCM (3x20 mL). Combined organic layers were dried over Na₂SO₄, concentrated *in vacuo* and the crude product purified by flash chromatography on a Si cartridge by eluting with 0-100 % DCM/MeOH/NH₄OH (90:9:0.5) in DCM to afford the title product (180 mg).

LCMS (Method 1): Rt = 0.87 min, ES⁺ *m*/*z* 466.9 [M+H]⁺.

### Preparation of intermediates 33a to 33b

The following intermediates were prepared similarly to **intermediate 11a** from the indicated starting materials.

| Intermediate | Structure/name | Starting material | LCMS |
|---|---|---|---|
| Intermediate 33a | 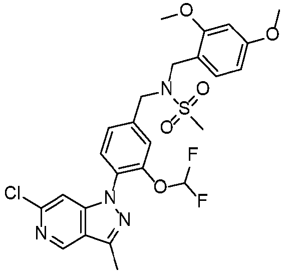 | Intermediate 32a /methanesulfonyl chloride | (Method 1) Rt = 1.21 min, ES⁺ *m*/*z* 566.6/568.6 [M+H]⁺. |
| Intermediate 33b | 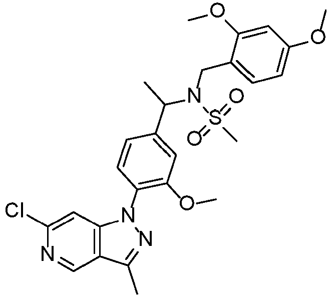 | Intermediate 32b / methanesulfonyl chloride | (Method 1) Rt = 1.27 min, ES⁺ *m*/*z* 545.3/547.2 [M+H]⁺. |

### PREPARATION OF EXAMPLES

### Example 1

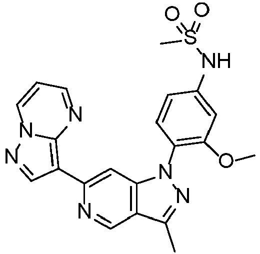

### N-(3-methoxy-4-(3-methyl-6-(pyrazolo[1,5-a]pyrimidin-3-yl)-1H-pyrazolo[4,3-c]pyridin-1-yl)phenyl)methanesulfonamide (Example 1)

A mixture of intermediate 11a (115 mg, 0.31 mmol), 3-(4,4,5,5-tetramethyl-1,3,2-dioxaborolan-2-yl)pyrazolo[1,5-a]pyrimidine (85 mg, 0.34 mmol), K₃PO₄ (133 mg, 0.63 mmol) and XPhos Pd G3 (13 mg) was charged in a sealed reaction tube under argon atmosphere, then added with degassed water (1 mL) and THF (2 mL). RM was stirred at 65 °C for 1.5 h. After cooling to RT, reaction mixture was partitioned between water (1 mL) / DCM (2 mL), and aqueous layer further extracted with DCM (4 x 2 mL). Combined organic layers were dried over Na₂SO₄ and evaporated to dryness. The crude material was purified by flash chromatography on a Si cartridge by eluting with 0-80% DCM/MeOH/NH₄OH (90:9:0.5) in DCM to afford the title product (80 mg).

LCMS (Method 5): Rt = 2.50 min, ES⁺ *m*/*z* 450.2 [M+H]⁺
¹H-NMR (500 MHz, *DMSO*-*d₆*) δ(ppm): 10.12 (s, 1H) 9.22 (dd, J=7.0, 1.5 Hz, 1H) 9.13 (d, J=1.2 Hz, 1H) 8.88 (s, 1H) 8.72 (dd, J=4.1, 1.7 Hz, 1H) 8.21 (d, J=0.9 Hz, 1H) 7.46 (d, J=8.5 Hz, 1H) 7.12 - 7.17 (m, 2H) 6.99 (dd, J=8.5, 2.4 Hz, 1H) 3.78 (s, 3H) 3.15 - 3.17 (m, 3H) 2.64 (s, 3H).

### Preparation of Example 2 to 52

The following Examples were prepared similarly to example 1 from the indicated intermediate.

| Ex. | Structure/Name | Interme diate | ¹H-NMR | LCMS |
|---|---|---|---|---|
| 2 | 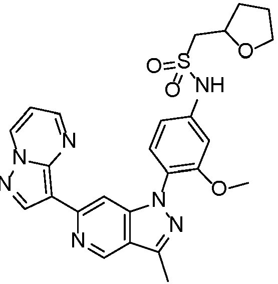 *N*-(3-methoxy-4-(3-methyl-6-(pyrazolo[1,5-a]pyrimidin-3-yl)-1*H-*pyrazolo[4,3-*c*]pyridin-1-yl)phenyl)-1-(tetrahydrofuran-2-yl)methanesulfonamide | 11b | (300 MHz, *DMSO*-*d₆*) δ (ppm): 10.12 (br s, 1H), 9.21 (dd, J=7.0, 1.6 Hz, 1H), 9.12 (d, J=1.0 Hz, 1H), 8.87 (s, 1H), 8.68 (dd, J=4.1, 1.7 Hz, 1H), 8.19 (d, J=1.0 Hz, 1H), 7.43 (d, J=8.5 Hz, 1H), 7.09-7.20 (m, 2H), 6.97 (dd, J=8.5, 2.1 Hz, 1H), 4.24 (quin, J=6.3 Hz, 1H), 3.76 (s, 3H), 3.59-3.73 (m, 2H), 3.43 (d, J=5.9 Hz, 2H), 2.62 (s, 3H), 2.01-2.16 (m, 1H), 1.73-1.92 (m, 2H), 1.57-1.73 (m, 1H) | (Method 5) Rt = 2.90 min, ES⁺ *m*/*z* 520.2 [M+H]⁺ |
| 3 | 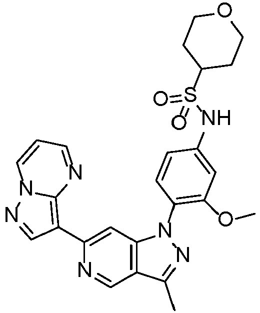 *N*-(3-methoxy-4-(3-methyl-6-(pyrazolo[1,5-a]pyrimidin-3-yl)-1*H-*pyrazolo[4,3*-c*]pyridin-1-yl)phenyl)tetrahydro-2*H-*pyran-4-sulfonamide | 11c | (600 MHz, *DMSO*-*d₆*) δ (ppm) 10.23 (br s, 1H), 9.23 (dd, J=7.1, 1.7 Hz, 1H), 9.14 (d, J=1.3 Hz, 1H), 8.89 (s, 1H), 8.72 (dd, J=4.0, 1.7 Hz, 1H), 8.20 (d, J=1.3 Hz, 1H), 7.45 (d, J=8.4 Hz, 1H), 7.15-7.18 (m, 2H), 7.02 (dd, J=8.4, 2.2 Hz, 1H), 3.95 (dd, J=11.6, 3.3 Hz, 2H), 3.78 (s, 3H), 3.50-3.56 (m, 1H), 3.36-3.39 (m, 2H), 2.64 (s, 3H), 1.95 (br d, J=10.8 Hz, 2H), 1.70-1.77 (m, 2H). | (Method 5) Rt = 2.79 min, ES⁺ *m*/*z* 519.9 [M+H]⁺ |
| 4 | 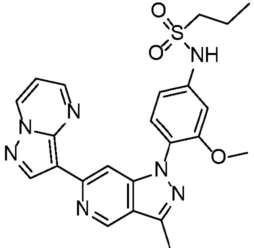 *N*-(3-methoxy-4-(3-methyl-6-(pyrazolo[1,5-*a*]pyrimidin-3-yl)-1*H-*pyrazolo[4,3-*c*]pyridin-1-yl)phenyl)propane-1-sulfonamide | 11d | (500 MHz, DMSO) δ (ppm) 10.15 (s, 1H), 9.21 (dd, J=7.0 Hz, J=1.5 Hz, 1H), 9.13 (s, 1H), 8.89 (s, 1H), 8.70 (dd, J=4.2 Hz, J=1.6 Hz, 1H), 8.20 (s, 1H), 7.43 (d, J=8.9 Hz, 1H), 7.14 (dd, J=6.9 Hz, J=4.2 Hz, 1H), 7.13 (d, J=1.3 Hz, 1H), 6.96 (dd, J=8.3 Hz, J=1.5 Hz, 1H), 3.77 (s, 3H), 3.21 (t, J=8.9 Hz, 2H), 2.63 (s, 3H), 1.72 (sxt, J=6.4 Hz, 2H), 0.98 (t, J=8.2 Hz, 3H). | (Method 6) Rt = 2.40 min, ES⁺ *m*/*z* 478.2 [M+H]⁺ |
| 5 | 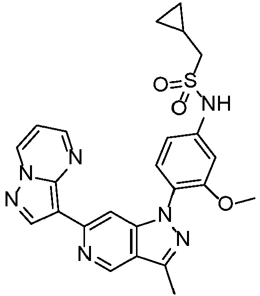 1-cyclopropyl-*N*-(3-methoxy-4-(3-methyl-6-(pyrazolo[1,5-*a*]pyrimidin-3-yl)-1*H*-pyrazolo[4,3-*c*]pyridin-1-yl)phenyl)methanesulfonam ide | 11e | (300 MHz, *DMSO*-*d₆*) δ(ppm) 10.19 (s, 1H), 9.17-9.25 (m, 1H), 9.12 (s, 1H), 8.87 (s, 1H), 8.64-8.73 (m, J=2.6 Hz, 1H), 8.18 (s, 1H), 7.42 (d, J=8.5 Hz, 1H), 7.10-7.20 (m, 2H), 6.93-7.03 (m, 1H), 3.75 (s, 3H), 3.22 (br d, J=7.0 Hz, 2H), 2.62 (s, 3H), 0.98-1.13 (m, 1H), 0.52-0.66 (m, 2H), 0.24-0.37 (m, 2H). | (Method 5) Rt = 3.01 min, ES⁺ *m*/*z* 489.9 [M+H]⁺ |
| 6 | 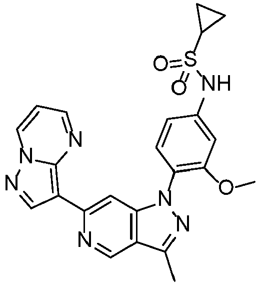 *N*-(3-methoxy-4-(3-methyl-6-(pyrazolo[1,5-*a*]pyrimidin-3-yl)-1*H-*pyrazolo[4,3-*c*]pyridin-1-yl)phenyl)cyclopropanesulf onamide | 11f | (600 MHz, DMSO) δ (ppm): 10.09 (s, 1H), 9.20 (dd, J=7.0 Hz, J=1.8 Hz, 1H), 9.13 (d, J=0.8 Hz, 1H), 8.88 (s, 1H), 8.70 (dd, J=3.9 Hz, J=1.5 Hz. 1H), 8.20 (d, J=1.0 Hz, 1H), 7.44 (d, J=8.7 Hz, 1H), 7.17 (d, J=2 Hz, 1H), 7.14 (dd, J=7.2 Hz, J=4.2 Hz, 1H), 7.00 (dd, J=8,6 Hz, J=2.4 Hz, 1H), 3.78 (s, 3H), 2.81 (kv, J=6.5 Hz, 1H), 2.64 (s, 3H), 1.03 (d, J=6.1 Hz, 4H). | (Method 6) Rt = 2.34 min, ES⁺ *m*/*z* 476.2 [M+H]⁺ |
| 7 | 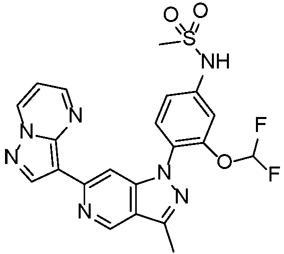 *N*-(3-(difluoromethoxy)-4-(3-methyl-6-(pyrazolo[1,5-*a*]pyrimidin-3-yl)-1*H-*pyrazolo[4,3-*c*]pyridin-1-yl)phenyl)methanesulfonam ide | 11g | (500 MHz, DMSO) δ (ppm) 10.38 (s,1H), 9.22 (dd, J=7.0 Hz, J=1.3 Hz, 1H), 9.17 (s, 1H), 8.89 (d, J=0.3 Hz, 1H), 8.70 (dd, J=3.9 Hz, J=1.3 Hz, 1H), 8.26 (s, 1H), 7.65 (d, J=8.8 Hz, 1H), 7.33 (d, J=1.6 Hz, 1H), 7.29 (dd, J=8.5 Hz, J=2.2 Hz, 1H), 7.18 (t, J=72.9 Hz, 1H), 7.15 (dd, J=7.3 Hz, J=4.2 Hz, 1H), 3.20 (s, 3H), 2.60 (s, 3H). | (Method 5) Rt = 2.78 min, ES⁺ *m*/*z* 486.4 [M+H]⁺ |
| 8 | 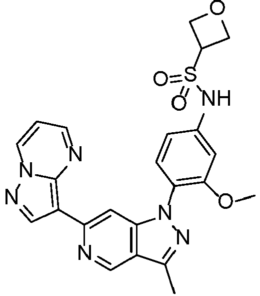 *N*-(3-methoxy-4-(3-methyl-6-(pyrazolo[1,5-*a*]pyrimidin-3-yl)-1*H-*pyrazolo[4,3-*c*]pyridin-1-yl)phenyl)oxetane-3-sulfonamide | 11h | (300 MHz, *DMSO*-*d₆*) δ(ppm) 12.34 (br s, 1H), 10.36 (s, 1H), 9.22 (dd, J=7.0, 1.5 Hz, 1H), 9.13 (s, 1H), 8.88 (s, 1H), 8.70 (dd, J=4.0, 1.5 Hz, 1H), 8.20 (s, 1H), 7.46 (d, J=8.5 Hz, 1H), 7.15 (dd, J=7.0, 4.0 Hz, 1H), 7.12 (d, J=2.1 Hz, 1H), 6.97 (dd, J=8.2, 2.1 Hz, 1H), 4.82-4.90 (m, 3H), 4.70-4.78 (m, 2H), 3.78 (s, 3H), 2.69 (dd, J=49.4, 15.3 Hz, 2H), 2.63 (s, 3H). | (Method 5) Rt = 2.61 min, ES⁺ *m*/*z* 492.1 [M+H]⁺ |
| 9 | 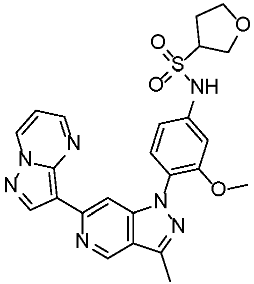 *N*-(3-methoxy-4-(3-methyl-6-(pyrazolo[1,5-*a*]pyrimidin-3-yl)-1*H-*pyrazolo[4,3-*c*]pyridin-1-yl)phenyl)tetrahydrofuran-3-sulfonamide | 11i | ( 500 MHz, DMSO) δ(ppm) 10.28 (s, 1H), 9.22 (dd, J=7.1, 2.0 Hz, 1H), 9.13 (d, J=1.1 Hz, 1H), 8.89 (s, 1H), 8.71 (dd, J=4.1, 1.7 Hz, 1H), 8.21 (d, J=1.2 Hz, 1H), 7.46 (d, J=8.3 Hz, 1H), 7.16 (d, J=2.6 Hz, 1H), 7.15 (d, J=4.0 Hz, 1H), 7.01 (dd, J=8.4, 2.2 Hz, 1H), 4.10-4.15 (m, 1H), 4.05 (dd, J=9.9, 4.7 Hz, 1H), 3.93 (dd, J=10.1, 8.1 Hz, 1H), 3.86 (dd, J=16.0, 6.5 Hz, 1H), 3.79 (s, 3H), 3.69 (dd, J=16.0, 7.1 Hz, 1H), 2.64 (s, 3H), 2.2 (dd, J=15.0, 6.8 Hz, 2H). | (Method 5) Rt = 2.68 min, ES⁺ *m*/*z* 506.2 [M+H]⁺ |
| 10 | 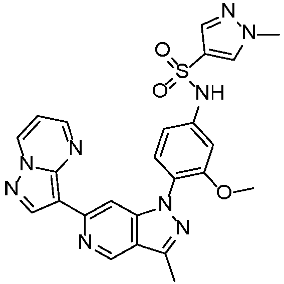 *N*-(3-methoxy-4-(3-methyl-6-(pyrazolo[1,5-a]pyrimidin-3-yl)-1*H-*pyrazolo[4,3-*c*]pyridin-1-yl)phenyl)-1-methyl-1H-pyrazole-4-sulfonamide | 11j | (500 MHz, *DMSO*-*d₆*) δ (ppm): 10.52 (br s, 1H), 9.22 (dd, J=7.02, 1.83 Hz, 1H), 9.12 (d, J=0.92 Hz, 1H), 8.88 (s, 1H), 8.72 (dd, J=4.12, 1.68 Hz, 1H), 8.38 (s, 1H), 8.16 (d, J=0.92 Hz, 1H), 7.83 (s, 1H), 7.38 (d, J=8.54 Hz, 1H), 7.16 (dd, J=7.02, 3.97 Hz, 1H), 7.09 (d, J=2.14 Hz, 1H), 6.90 (dd, J=8.54, 2.14 Hz, 1H), 3.87 (s, 3H), 3.75 (s, 3H), 2.61 (s, 3H). | (Method 7) Rt = 4.05 min, ES⁺ *m*/*z* 516.1 [M+H]⁺ |
| 11 | 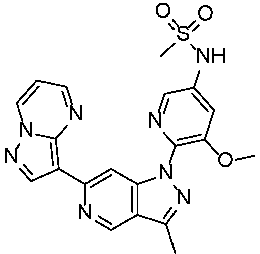 *N*-(5-methoxy-6-(3-methyl-6-(pyrazolo[1,5-*a*]pyrimidin-3-yl)-1*H-*pyrazolo[4,3-*c*]pyridin-1-yl)pyridin-3-yl)methanesulfonamide | 11k | (600 MHz, *DMSO*-*d₆*) δ (ppm): 10.39 (bs, 1H), 9.23 (dd, J=7.0, 1.7 Hz, 1H), 9.16 (d, J=1.0 Hz, 1H), 8.89 (s. 1H), 8.72 (dd, J=4.1, 1.7 Hz, 1H), 8.33 (d, J=1.0 Hz, 1H), 8.06 (d, J=1.9 Hz, 1H), 7.58 (d, J=1.9 Hz, 1H), 7.16 (dd, J=7.1, 4.1 Hz, 1H), 3.87 (s, 3H), 3.27 (s, 3H), 2.65 (s, 3H). | (Method 7) Rt = 3.76 min, ES⁺ *m*/*z* 451.1 [M+H]⁺ |
| 12 | 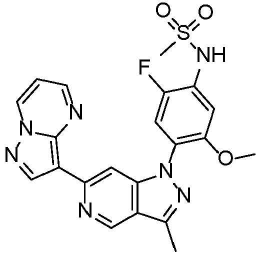 *N*-(2-fluoro-5-methoxy-4-(3-methyl-6-(pyrazolo[1,5-*a*]pyrimidin-3-yl)-1*H-*pyrazolo[4,3-*c*]pyridin-1-yl)phenyl)methanesulfonam ide | 11l | (600 MHz, DMSO) δ (ppm): 9.91 (s, 1H), 9.22 (d, J=7.0 Hz, 1H), 9.15 (s, 1H), 8.89 (s, 1H), 8.74 (d, J=2.2 Hz, 1 Hz), 8.28 (s, 1H), 7.54 (d, J=10.0 Hz, 1H), 7.31 (d, J=7.5 Hz, 1H), 7.15 (dd, J=7.2 Hz, J=4.4 Hz, 1H), 3.84 (s, 3H), 3.19 (s, 3H), 2.65 (s, 3H). | (Method 7) Rt = 4.00 min, ES⁺ *m*/*z* 468.0 [M+H]⁺ |
| 13 | 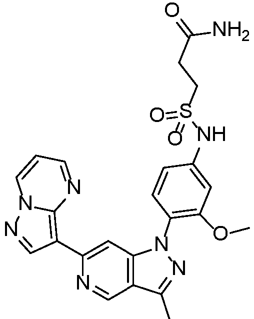 3-(*N*-(3-methoxy-4-(3-methyl-6-(pyrazolo[1,5-*a*]pyrimidin-3-yl)-1*H-*pyrazolo[4,3-*c*]pyridin-1-yl)phenyl)sulfamoyl)propan amide | 11m | (500 MHz, DMSO) δ (ppm): 10.21 (s, 1H), 9.22 (dd, J=6.9, 1.6 Hz, 1H), 9.1 (s, 1H), 8.88 (s, 1H), 8.73 (dd, J=3.9, 1.6 Hz, 1H), 8.25 (s, 1H), 7.53 (s, 1H), 7.45 (d, J=8.5 Hz, 1H), 7.14 (m, 2H), 7.06 (s, 1H), 6.97 (dd, J=8.4, 2.2 Hz, 1H), 3.78 (s, 3H), 3.45 (t, J=7.6 Hz, 2H), 2.64 (s, 3H), 2.58 (t, J=2.6 Hz, 2H). | (Method 6) Rt = 2.37 min, ES⁺ *m*/*z* 507.5 [M+H]⁺ |
| 14 | 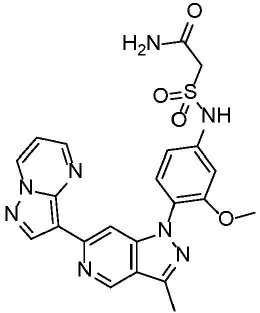 2-(*N*-(3-methoxy-4-(3-methyl-6-(pyrazolo[1,5-*a*]pyrimidin-3-yl)-1*H-*pyrazolo[4,3-*c*]pyridin-1-yl)phenyl)sulfamoyl)acetam ide | 11n | ( 600 MHz, DMSO) δ (ppm) 10.25 (s, 1H), 9.22 (dd, J=7.0, 1.7 Hz, 1H), 9.13 (d, J=1.1 Hz, 1H), 8.89 (s, 1H), 8.72 (dd, J=4.1, 1.9 Hz, 1H), 8.22 (d, J=1.1 Hz), 7.72 (s, 1H), 7.45 (d, J=8.5 Hz, 1H), 7.43 (s, 1H), 7.18 (d, J=2.3 Hz, 1H), 7.15 (dd, J=6.8, 4.2 Hz, 1H), 7.01 (dd, J=8.5, 2.2 Hz, 1H), 4.10 (s, 2H), 3.79 (s, 3H), 2.64 (s, 3H). | (Method 6) Rt = 1.98 min,ES⁺ m/z 493.2 [M+H]⁺ |
| 15 | 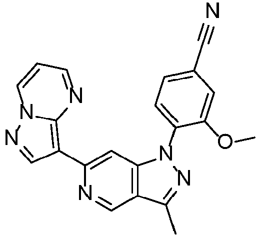 3-methoxy-4-(3-methyl-6-(pyrazolo[1,5*-a*]pyrimidin-3-yl)-1*H*-pyrazolo[4,3-*c*]pyridin-1-yl)benzonitrile | 9e | (500 MHz, *DMSO*-*d₆*) δ (ppm): 9.23 (d, J=7.9 Hz, 1H), 9.17 (s, 1H), 8.89 (s, 1H), 8.72 (d, J=4.4 Hz, 1H), 8.33 (s, 1H), 7.92 (s, 1H), 7.73 (d, J=7.5 Hz, 1H), 7.64 (d, J=7.6 Hz, 1H), 7.17 (dd, J=6.8 Hz, J=4.3 Hz, 1H), 3.97 (s, 3H), 2.66 (s, 3H). | (Method 7) Rt = 4.51 min, ES⁺ *m*/*z* 382.2 [M+H]⁺ |
| 16 | 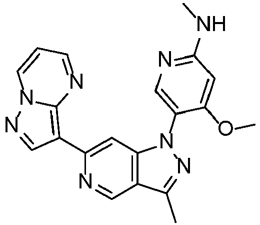 4-methoxy-*N*-methyl-5-(3-methyl-6-(pyrazolo[1,5-*a*]pyrimidin-3-yl)-1*H-*pyrazolo[4,3-*c*]pyridin-1-yl)pyridin-2-amine | 10d | (500 MHz, *DMSO*-*d₆*) δ (ppm): 9.22 (dd, J=7.0, 1.6Hz, 1H), 9.12 (d, J=1.0 Hz, 1H), 8.86 (s. 1H), 8.72 (dd, J=4.1, 1.6Hz, 1H), 8.13 (d, J=1.0 Hz, 1H), 7.98 (s, 1H), 7.15 (dd, J=7.1, 4.1Hz, 1H), 6.88 (q, J=4.9 Hz, 1H), 6.22 (s, 1H), 3.73 (s, 3H), 2.62 (s, 3H), 2.87 (d, J=4.9 Hz, 3H). | (Method 8) Rt = 4.64 min, ES⁺ *m*/*z* 387.2 [M+H]⁺ |
| 17 | 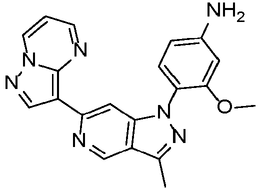 3-methoxy-4-(3-methyl-6-(pyrazolo[1,5-*a*]pyrimidin-3-yl)-1*H*-pyrazolo[4,3-*c*]pyridin-1-yl)aniline | 10a | (500 MHz, *DMSO*-*d₆*) δ (ppm): 9.24 - 9.19 (m, 1H), 9.09 (s, 1H), 8.88 (s, 1H), 8.74 - 8.69 (m, 1H), 8.13 (s, 1H), 7.16 - 7.11 (m, 1H), 7.09 - 7.05 (m, 1H), 6.45 (s, 1H), 6.33 - 6.26 (m, 1H), 5.55 (s, 2H), 3.64 (s, 3H), 2.60 (s, 3H) | (Method 5) Rt = 2.38 min, ES⁺ *m*/*z* 372.33 [M+H]⁺ |
| 18 | 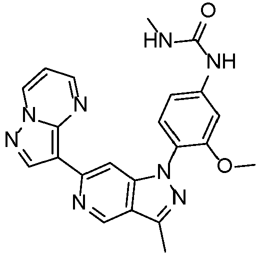 1-(3-methoxy-4-(3-methyl-6-(pyrazolo[1,5-*a*]pyrimidin-3-yl)-1*H-*pyrazolo[4,3-*c*]pyridin-1-yl)phenyl)-3-methylurea | 11p | (500 MHz, *DMSO*-*d₆*) δ (ppm): 9.23 - 9.20 (m, 1H), 9.12 (s, 1H), 8.89 (s, 1H), 8.87 (s, 1H), 8.72 - 8.70 (m, 1H), 8.18 (s, 1H), 7.59 - 7.56 (m, 1H), 7.31 (d, J=8.5Hz, 1H), 7.16 - 7.13 (m, 1H), 7.09 - 7.04 (m, 1H), 6.18 - 6.14 (m, 1H), 3.74 (s, 3H), 2.71 - 2.68 (m, 3H), 2.63 (s, 3H) | (Method 5) Rt = 2.46 min, (ES⁺ *m*/*z* 429.7 [M+H]⁺ |
| 19 | 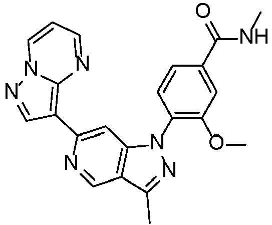 3-methoxy-*N*-methyl-4-(3-methyl-6-(pyrazolo[1,5-*a*]pyrimidin-3-yl)-1*H-*pyrazolo[4,3-*c*]pyridin-1-yl)benzamide | 9f | (500 MHz, *DMSO*-*d₆*) δ (ppm): 9.21 (dd, J=6.9 Hz, J=1.7 Hz, 1H), 9.16 (d, J=0.6Hz, 1H), 8.89 (s, 1H), 8.71 (dd, J=4.1Hz, J=1.5Hz, 1H), 8.65 (m, 1H), 8.29 (d, J=0.6Hz, 1H), 7.78 (s, 1H), 7.59-7.65 (m, 2H), 7.15 (dd, J=7.0 Hz, J=4.0 Hz, 1H), 3.92 (s, 3H), 2.85 (d, J=4.6Hz, 3H), 2.66 (s, 3H). | (Method 6) Rt = 4.63 min, ES⁺ *m*/*z* 414.1 [M+H]⁺ |
| 20 | 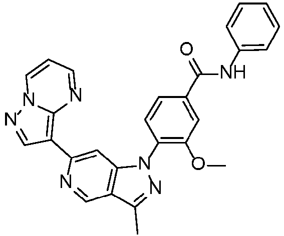 3-methoxy-4-(3-methyl-6-(pyrazolo[1,5*-a*]pyrimidin-3-yl)-1*H*-pyrazolo[4,3-*c*]pyridin-1-yl)-*N-*phenylbenzamide | 9j | (500 MHz, *DMSO*-*d₆*) δ (ppm): 10.42 (s, 1H), 9.23 (dd, J=7.0, 1.5Hz, 1H), 9.17 (d, J=0.9 Hz, 1H), 8.90 (s, 1H), 8.73 (dd, J=4.1, 1.7 Hz, 1H), 8.32 (d, J=0.9 Hz, 1H), 7.88 (d, J=1.5Hz, 1H), 7.76 - 7.83 (m, 3H), 7.69 (d, J=8.2Hz, 1H), 7.43 - 7.36 (m, 2H), 7.13 - 7.18 (m, 2H), 3.98 (s, 3H), 2.68 (s, 3H). | (Method 7) Rt = 4.94 min, ES⁺ *m*/*z* 476.2 [M+H]⁺ |
| 21 | 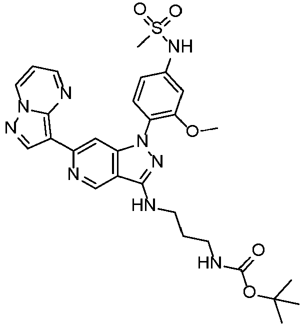 *tert-*butyl (3-((1-(2-methoxy-4-(methylsulfonamido)phenyl) -6-(pyrazolo[1,5-*a*]pyrimidin-3-yl)-1*H-*pyrazolo[4,3-*c*]pyridin-3-yl)amino)propyl)carbamate | 17a | (500 MHz, *DMSO*-*d₆*) δ (ppm): 10.00 (br s, 1H), 9.20 (d, J=6.7 Hz, 1H), 9.04 (s, 1H), 8.85 (s, 1H), 8.71 (dd, J=4.1, 1.7 Hz, 1H), 8.06 (s, 1H), 7.42 (d, J=8.5 Hz, 1H), 7.13 (dd, J=7.0, 4.3 Hz, 1H), 7.10 (s, 1H), 6.95 (br d, J=8.5 Hz, 1H), 6.87 (br s, 1H), 6.70 (t, J=5.6 Hz, 1H), 3.81 (s, 3H), 3.27-3.31 (m, 2H), 3.11 (d, J=1.8 Hz, 3H), 3.03-3.09 (m, 2H), 1.78 (br t, J=6.9 Hz, 2H), 1.37 (s, 9H). | (Method 5) Rt = 3.21 min, ES⁺ *m*/*z* 608.6 [M+H]⁺ |
| 22 | 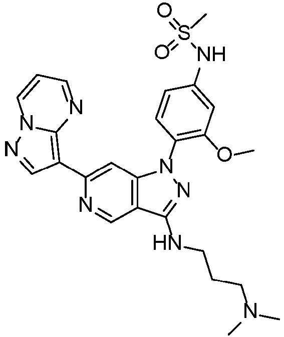 *N*-(4-(3-((3-(dimethylamino)propyl)ami no)-6-(pyrazolo[1,5-*a*]pyrimidin-3-yl)-1*H-*pyrazolo[4,3-*c*]pyridin-1-yl)-3-methoxyphenyl)methanesulf onamide | 17c | (600 MHz, *DMSO*-*d₆*) δ (ppm): 9.83-10.12 (m, 1H), 9.20 (dd, J=7.0, 1.8 Hz, 1H), 9.04 (d, J=1.1Hz, 1H), 8.85 (s, 1H), 8.71 (dd, J=4.1, 1.7 Hz, 1H), 8.06 (d, J=1.1Hz, 1H), 7.42 (d, J=8.4 Hz, 1H), 7.12-7.15 (m, 1H), 7.10 (d, J=2.2Hz, 1H), 6.95 (dd, J=8.4, 2.2 Hz, 1H), 6.72 (t, J=5.5Hz, 1H), 3.81 (s, 3H), 3.26-3.31 (m, 2H), 3.11 (s, 3H), 2.35 (t, J=7.2 Hz, 2H), 2.16 (s, 6H), 1.81 (quin, J=7.2 Hz, 2H). | (Method 3) Rt = 0.82 min, ES⁺ *m*/*z* 536.3 [M+H]⁺ |
| 23 | 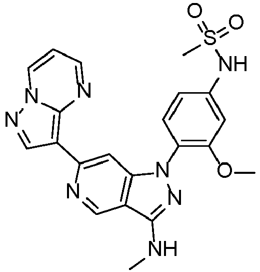 *N*-(3-methoxy-4-(3-(methylamino)-6-(pyrazolo[1,5-*a*]pyrimidin-3-yl)-1H-pyrazolo[4,3-*c*]pyridin-1-yl)phenyl)methanesulfonam ide | 17d | (600 MHz, *DMSO*-*d₆*) δ (ppm): 9.99 (s, 1H), 9.20 (dd, J=7.0, 1.7 Hz, 1H), 9.01 (d, J=1.0 Hz, 1H), 8.84 (s, 1H), 8.71 (dd, J=4.1, 1.7, 1H), 8.05 (d. J=1.0 Hz, 1H), 7.44 (d, J=8.4 Hz, 1H), 7.13 (dd, J=7.1, 4.1 Hz, 1H), 7.10 (d, J=2.3 Hz, 1H), 6.96 (dd, J=8.4, 2.3 Hz, 1H), 6.71 (q, J=5.0 Hz, 1H), 3.81 (s, 3H), 3.12 (s, 3H), 2.90 (d, J=5.0 Hz, 3H). | (Method 3) Rt = 0.95 min, ES⁺ *m*/*z* 465.1 [M+H]⁺ |
| 24 | 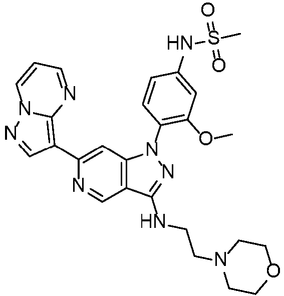 *N*-(3-methoxy-4-(3-((2-morpholinoethyl)amino)-6-(pyrazolo[1,5-*a*]pyrimidin-3-yl)-1*H*-pyrazolo[4,3-*c*]pyridin-1-yl)phenyl)methanesulfonam ide | 17e | (500 MHz, *DMSO*-*d₆*) δ (ppm): 9.98 (br s, 1H), 9.15 - 9.24 (m, 1H), 9.06 (s, 1H), 8.85 (s, 1H), 8.71 (dd, J=4.1, 1.768 Hz, 1H), 8.07 (s, 1H), 7.42 (d, J=8.5 Hz, 1H), 7.13 (dd, J=7.0, 3.97 Hz, 1H), 7.10 (s, 1H), 6.96 (dd, J=8.4, 1.37 Hz, 1H), 6.66 (t, J=5.7 Hz, 1H), 3.81 (s, 3H), 3.59 (t, J=4.6 Hz, 4H), 3.43 (q, J=6.4 Hz, 2H), 3.12 (s, 3H), 2.62 (t, J=6.9 Hz, 2H), 2.45 (br s, 4H). | (Method 3) Rt = 0.73 min, ES⁺ *m*/*z* 564.1 [M+H]⁺ |
| 25 | 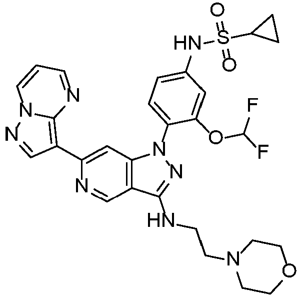 *N*-(3-(difluoromethoxy)-4-(3-((2-morpholinoethyl)amino)-6-(pyrazolo[1,5-*a*]pyrimidin-3-yl)-1H-pyrazolo[4,3-*c*]pyridin-1-yl)phenyl)cyclopropanesulf onamide | 17f | (500 MHz, *DMSO*-*d₆*) δ (ppm): 10.24 (s, 1H), 9.21 (dd, J=7.0, 1.5 Hz, 1H), 9.10 (d, J=0.6 Hz, 1H), 8.86 (s, 1H), 8.69 (dd, J=4.1, 1.7 Hz, 1H), 8.12 (d, J=0.6 Hz, 1H), 7.60 (d, J=8.5 Hz, 1H), 7.34 (d, J=1.8 Hz, 1H), 7.29 (dd, J=8.5, 2.14 Hz, 1H), 7.17 (t, J=73.6 Hz, 1H), 7.14 (dd, J=7.0, 4.3 Hz, 1H), 6.77 (br t, J=5.7 Hz, 1H), 3.60 (t, J=4.6 Hz, 4H), 3.43 (q, J=6.4 Hz, 2H), 2.82 (quin, J=6.3 Hz, 1H), 2.59 - 2.66 (m, 3H), 2.45 (br s, 4H), 1.02 - 1.06 (m, 4H). | (Method 7) Rt = 3.56 min, ES⁺ *m*/*z* 626.2 [M+H]⁺ |
| 26 | 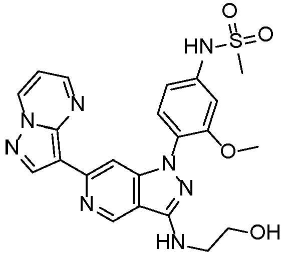 *N*-(4-(3-((2-hydroxyethyl)amino)-6-(pyrazolo[1,5-*a*]pyrimidin-3-yl)-1*H*-pyrazolo[4,3-*c*]pyridin-1-yl)-3-methoxyphenyl)methanesulf onamide | 17g | (500 MHz, DMSO-d3) δ: (ppm): 9.99 (s, 1H), 9.20 (dd, J=7.0, 1.2 Hz, 1H), 9.08 (s, 1H), 8.85 (s, 1H), 8.71 (dd, J=4.0, 1.5 Hz, 1H), 8.06 (s, 1H), 7.42 (d, J=8.5 Hz, 1H), 7.12-7.14 (m, 1H), 7.10 (d, J=2.1 Hz, 1H), 6.96 (dd, J=8.5, 2.1 Hz, 1H), 6.76 (t, J=5.6 Hz, 1H), 4.74 (t, J=5.5 Hz, 1H), 3.81 (s, 3H), 3.67 (q, J=5.9 Hz, 2H), 3.35-3.41 (m, 2H), 3.12 (s, 3H). | (Method 7) Rt = 3.58 min, ES⁺ *m*/*z* 495.2 [M+H]⁺ |
| 27 | 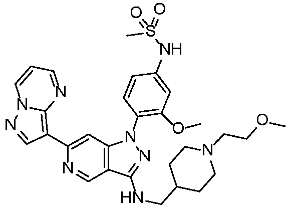 *N*-(3-methoxy-4-(3-(((1-(2-methoxyethyl)piperidin-4-yl)methyl)amino)-6-(pyrazolo[1,5-*a*]pyrimidin-3-yl)-1*H*-pyrazolo[4,3-*c*]pyridin-1-yl)phenyl)methanesulfonam ide | 17h | (500 MHz, *DMSO*-*d₆*) δ (ppm): 10.00 (br s, 1H), 9.20 (dd, J=7.02, 1.53 Hz, 1H), 9.08 (s, 1H), 8.84 (s, 1H), 8.71 (dd, J=3.97, 1.83 Hz, 1H), 8.05 (s, 1H), 7.41 (d, J=8.54 Hz, 1H), 7.13 (dd, J=7.02, 4.27 Hz, 1H), 7.10 (d, J=2.14 Hz, 1H), 6.96 (dd, J=8.54, 2.14 Hz, 1H), 6.72 (t, J=5.65 Hz, 1H), 3.81 (s, 3H), 3.42 (t, J=5.95 Hz, 2H), 3.22 (s, 3H), 3.17 (t, J=6.10Hz, 2H), 3.11 (s, 3H), 2.89 (br d, J=11.29 Hz, 2H), 2.45 (t, J=5.95 Hz, 2H), 1.90 - 2.00 (m, 2H), 1.76 (br d, J=12.21 Hz, 2H), 1.64 - 1.72 (m, 1H), 1.24 (qd, J=11.95, 3.51 Hz, 2H). | (Method 7) Rt = 3.35 min, ES⁺ *m*/*z* 606.3 [M+H]⁺ |
| 28 | 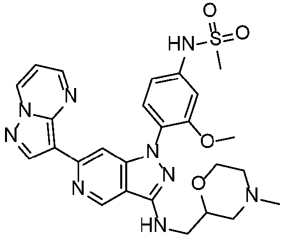 (rac) *N*-(3-methoxy-4-(3-(((4-methylmorpholin-2-yl)methyl)amino)-6-(pyrazolo[1,5-*a*]pyrimidin-3-yl)-1*H*-pyrazolo[4,3-*c*]pyridin-1-yl)phenyl)methanesulfonam ide | 17i | (500 MHz, *DMSO*-*d₆*) δ (ppm): 9.70 (br s, 1H), 9.11 (d, J=7.0 Hz, 1H), 9.09 (s, 1H), 8.82 (s, 1H), 8.66 - 8.68 (m, 1H), 8.06 (s, 1H), 7.41 (d, J=8.5 Hz, 1H), 7.14 (d, J=2.1 Hz, 1H), 7.09 (dd, J=7.0 Hz, J=4.0 Hz, 1H), 6.99 (dd, J=8.4 Hz, J=2.3 Hz, 1H), 6.50 - 6.55 (m, 1H), 3.83 (s, 3H), 3.78-3.85 (m, 2H), 3.57 (td, J=11.1 Hz, J=2.4 Hz, 1H), 3.35 - 3.45 (m, 2H), 3.10 (s, 3H), 2.82 (d, J=11.3 Hz, 1H), 2.59 (d, J=11.3 Hz, 1H), 2.21 (s, 3H), 2.06 (td, J=11.1 Hz, J=3.4 Hz, 1H), 1.87 (t, J=10.5 Hz, 1H). | (Method 7) Rt = 3.16 min, ES⁺ *m*/*z* 564.2; [M+H]⁺ |
| 29 | 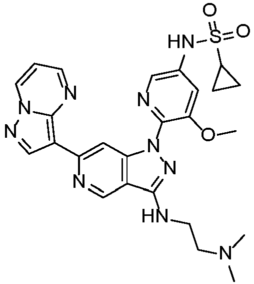 *N*-(6-(3-((2-(dimethylamino)ethyl)amin o)-6-(pyrazolo[1,5-*a*]pyrimidin-3-yl)-1*H-*pyrazolo[4,3*-c*]pyridin-1-yl)-5-methoxypyridin-3-yl)cyclopropanesulfonamide | 17j | (600 MHz, *DMSO*-*d₆*) δ (ppm): 9.21 (dd, J=7.0, 1.7 Hz, 1H), 9.1 (d, J=1.0 Hz, 1H), 8.85 (s. 1H), 8.67 (dd, J=4.1, 1.7 Hz, 1H), 8.18 (d, J=1.0 Hz, 1H), 8.02 (d, J=2.1 Hz, 1H), 7.54 (d, J=2.1 Hz, 1H), 7.14 (dd, J=7.1, 4.1 Hz, 1H), 6.69 (t, J=5.5 Hz, 1H), 3.85 (s, 3H), 3.43-3.39 (m, 2H), 2.87-2.82 (m, 1H), 2.62-2.59 (m, 2H), 2.26 (s, 6H), 1.05-1.01 (m, 4H). | (Method 8) Rt = 3.28 min, ES⁺ *m*/*z* 549.2 [M+H]⁺ |
| 30 | 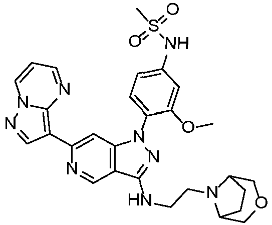 *N*-(4-(3-((2-(3-oxa-8-azabicyclo[3.2.1]octan-8-yl)ethyl)amino)-6-(pyrazolo[1,5-*a*]pyrimidin-3-yl)-1*H*-pyrazolo[4,3-*c*]pyridin-1-yl)-3-methoxyphenyl)methanesulf onamide | 17k | (500 MHz, *DMSO*-*d₆*) δ (ppm): 9.97 (br s, 1H), 9.20 (d, J=7.0 Hz, 1H), 9.05 (s, 1H), 8.84 (s, 1H), 8.71 (d, J=4.0 Hz, 1H), 8.07 (s, 1H), 7.43 (d, J=8.5 Hz, 1H), 7.13 (dd, J=6.7, 4.0 Hz, 1H), 7.10 (s, 1H), 6.96 (br, J=8.5 Hz, 1H), 6.66 (t, J=5.5 Hz, 1H), 3.81 (s, 3H), 3.56 (d, J=9.8 Hz, 2H), 3.36-3.43 (m, 4H), 3.12 (s, 5H), 2.53-2.57 (m, 2H), 1.82-1.90 (m, 2H), 1.72 (br d, J=7.0 Hz, 2H). | (Method 7) Rt = 3.28 min, ES⁺ *m*/*z* 590.2 [M+H]⁺ |
| 31 | 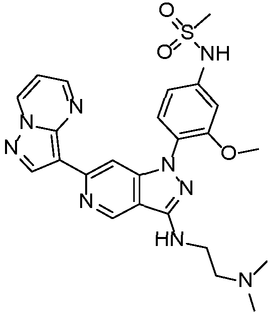 *N*-(4-(3-((2-(dimethylamino)ethyl)amin o)-6-(pyrazolo[1,5-*a*]pyrimidin-3-yl)-1*H-*pyrazolo[4,3-*c*]pyridin-1-yl)-3-methoxyphenyl)methanesulf onamide | 17n | (500 MHz, *DMSO*-*d₆*) δ (ppm): 9.98 (br s, 1H), 9.20 (dd, J=7.02, 1.53Hz, 1H), 9.07 (d, J=0.61Hz, 1H), 8.84 (s, 1H), 8.71 (dd, J=4.12, 1.68 Hz, 1H), 8.06 (d, J=1.22Hz, 1H), 7.41 (d, J=8.54 Hz, 1H), 7.13 (dd, J=7.02, 3.97 Hz, 1H), 7.08 (d, J=1.83Hz, 1H), 6.94 (dd,J=8.24, 1.83Hz, 1H), 6.62 (t, J=5.49 Hz, 1H), 3.81 (s, 3H), 3.38 (m, 2H, overlapped with HDO), 3.10 (s, 3H), 2.55 (t, J=6.71Hz, 2H), 2.22 (s, 6H) | (Method 3) Rt = 0.70 min, ES⁺ *m*/*z* 522.2 [M+H]⁺ |
| 32 | 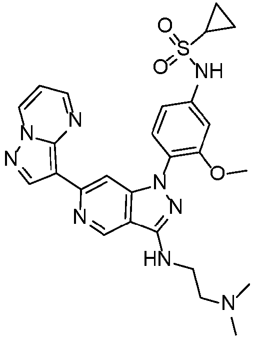 *N*-(4-(3-((2-(dimethylamino)ethyl)amin o)-6-(pyrazolo[1,5-*a*]pyrimidin-3-yl)-1*H-*pyrazolo[4,3-*c*]pyridin-1-yl)-3-methoxyphenyl)cyclopropan esulfonamide | 17o | (500 MHz, *DMSO*-*d₆*) δ (ppm): 9.95 (s, 1H), 9.19 (d, J=7.3Hz, J=2.7 Hz, 1H), 9.07 (d, J=1.0 Hz, 1H), 8.85 (s, 1H), 8.69 (dd, J=4.0 Hz, J=2.0 Hz, 1H), 8.06 (d, J=1.0 Hz, 1H), 7.40 (d, J=8.5Hz, 1H), 7.12-7.16 (m, 2H), 6.99 (dd, J=8.0 Hz, J=2.0 Hz, 1H), 6.66 (t, J=5.5 Hz, 1H), 3.81 (s, 3H), 3.37 (q, J=6.6 Hz, 2H), 2.75 (qv, J=7.4 Hz, 1H), 2.54 (t, J=7.4 Hz, 2H), 2.22 (s, 6H), 1.00-1.04 (m, 4H). | (Method 7) Rt = 3.40 min, ES⁺ *m*/*z* 548.2 [M+H]⁺ |
| 33 | 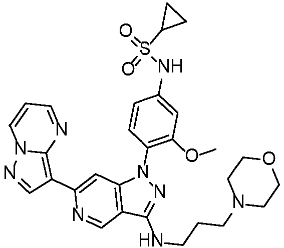 *N*-(3-methoxy-4-(3-((3-morpholinopropyl)amino)-6-(pyrazolo[1,5-*a*]pyrimidin-3-yl)-1*H-*pyrazolo[4,3-*c*]pyridin-1-yl)phenyl)cyclopropanesulf onamide | 17p | (500 MHz, *DMSO*-*d₆*) δ (ppm): 9.98 (br s, 1H), 9.20 (dd, J=1.8, 7.0 Hz, 1H), 9.04 (d, J=0.9 Hz, 1H), 8.85 (s, 1H), 8.70 (dd, J=1.7, 4.1Hz, 1H), 8.06 (d, J=0.9 Hz, 1H), 7.40 (d, J=8.5Hz, 1H), 7.16 - 7.12 (m, 2H), 6.98 (dd, J=2.1, 8.5Hz, 1H), 6.73 (t, J=5.5Hz, 1H), 3.81 (s, 3H), 3.58 (t, J=4.6Hz, 4H), 3.30 (m, 2H, ov with water), 2.81 - 2.75 (m, 1H), 2.44 - 2.33 (m, 6H), 1.83 (quin, J=7.1Hz, 2H), 1.05 - 1.00 (m, 4H) | (Method 5) Rt = 2.14 min, ES⁺ *m*/*z* 604.2 [M+H]⁺ |
| 34 | 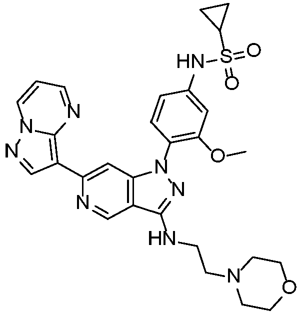 *N*-(3-methoxy-4-(3-((2-morpholinoethyl)amino)-6-(pyrazolo[1,5-*a*]pyrimidin-3-yl)-1*H*-pyrazolo[4,3-*c*]pyridin-1-yl)phenyl)cyclopropanesulf onamide | 17q | (500 MHz, *DMSO*-*d₆*) δ (ppm): 9.99 (s, 1H), 9.20 (dd, J=1.7, 6.9Hz, 1H), 9.06 (s, 1H), 8.85 (s, 1H), 8.70 (dd, J=1.8, 4.0 Hz, 1H), 8.06 (d, J=0.9 Hz, 1H), 7.41 (d, J=8.5 Hz, 1H), 7.17 - 7.11 (m, 2H), 6.99 (dd, J=2.3, 8.4 Hz, 1H), 6.67 (t, J=5.6 Hz, 1H), 3.81 (s, 3H), 3.60 (t, J=4.4 Hz, 4H), 3.43 (q, J=6.4 Hz, 2H), 2.78 (quin, J= 6.3 Hz, 1H), 2.66 - 2.60 (m, 2H), 2.45 (br s, 4H), 1.06 - 1.00 (m, 4H) | (Method 7) Rt = 3.59 min, ES⁺ *m*/*z* 590.2 [M+H]⁺ |
| 35 | 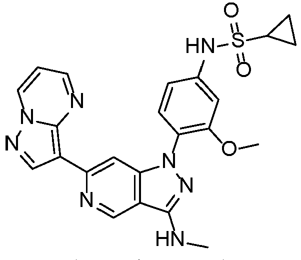 *N*-(3-methoxy-4-(3-(methylamino)-6-(pyrazolo[1,5-*a*]pyrimidin-3-yl)-1*H*-pyrazolo[4,3-*c*]pyridin-1-yl)phenyl)cyclopropanesulf onamide | 17r | (500 MHz, *DMSO*-*d₆*) δ (ppm): 9.98 (s, 1H), 9.19 (dd, J=7.5Hz, J=2.6Hz, 1H), 9.00 (d, J=1.3Hz, 1H), 8.84 (s, 1H), 8.69 (dd, J=4.3Hz, J =1.8 Hz, 1H), 8.05 (d, J=0.9 Hz, 1H), 7.42 (d, J=0.9 Hz, 1H), 7.12-7.15 (m, 2H), 6.97 (dd, J =8.4 Hz, J=2.2Hz, 1H), 6.70 (q, J=4.8 Hz, 1H), 3.80 (s, 3H), 2.90 (d, J=4.9 Hz, 3H), 2.75 (qv, J=6.5Hz, 1H), 1.00-1.04 (m, 4H) | (Method 5) Rt = 2.70 min, ES⁺ *m*/*z* 490.8 [M+H]⁺ |
| 36 | 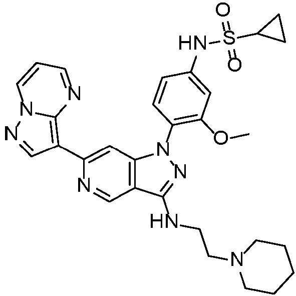 *N*-(3-methoxy-4-(3-((2-(piperidin-1-yl)ethyl)amino)-6-(pyrazolo[1,5-*a*]pyrimidin-3-yl)-1*H*-pyrazolo[4,3-*c*]pyridin-1-yl)phenyl)cyclopropanesulf onamide | 17s | ( 500 MHz, DMSO) δ (ppm): 9.98 (s, 1H), 9.20 (dd, J=6.9, 1.7 Hz, 1H), 9.05 (d, J=0.7 Hz, 1H), 8.84 (s, 1H), 8.70 (dd, J=4.2, 1.8 Hz, 1H), 8.06 (d, J=0.5Hz, 1H), 7.42 (d, J=8.5Hz, 1H), 7.11 - 7.16 (m, 2H), 6.98 (dd, J=8.2, 2.3Hz, 1H), 6.63 (t, J=5.4 Hz,1H), 3.81 (s, 3H), 3.40 (q, J=6.4 Hz, 2H), 2.75 - 2.80 (m, 1H), 2.57 (t, J=6.4 Hz, 2H), 2.42 (s, 4H), 1.47-1.55 (m, 4H), 1.35 - 1.42(m, 2H), 1.00 - 1.05 (m, 4H). | (Method 6) Rt = 2.84 min, ES⁺ *m*/*z* 588.8 [M+H]⁺ |
| 37 | 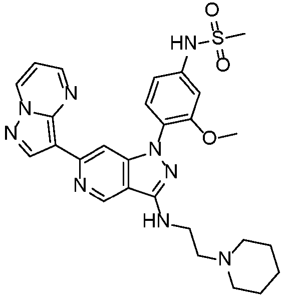 *N*-(3-methoxy-4-(3-((2-(piperidin-1-yl)ethyl)amino)-6-(pyrazolo[1,5-*a*]pyrimidin-3-yl)-1*H*-pyrazolo[4,3-*c*]pyridin-1-yl)phenyl)methanesulfonam ide | 17t | (500 MHz, DMSO) δ (ppm): 9.98 (s, 1H), 9.20 (dd, J=6.9, 1.7 Hz, 1H), 9.05 (d, J=0.8 Hz, 1H), 8.84 (s, 1H), 8.81 (dd, J=3.9, 1.8 Hz, 1H), 8.06 (d, J=0.6Hz, 1H), 7.42 (d, J=8.5Hz, 1H), 7.14 (dd, J=6.9, 3.9 Hz, 1H), 7.10 (d, J=1.9 Hz, 1H), 6.95 (dd, J=8.2, 2.3Hz, 1H), 6.64 (t, J=5.4 Hz,1H), 3.81 (s, 3H), 3.40 (q, J=6.4 Hz, 2H), 3.13 (s, 3H), 2.55 - 2.61 (m, 2H), 2.42 (s, 4H), 1.47-1.55 (m, 4H), 1.35 - 1.43 (m, 2H). | (Method 6) Rt = 2.45 min, ES⁺ *m*/*z* 562.2 [M+H]⁺ |
| 38 | 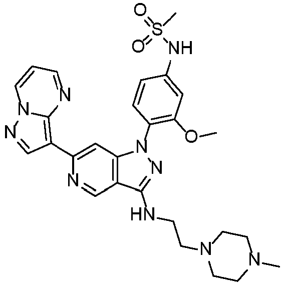 *N*-(3-methoxy-4-(3-((2-(4-methylpiperazin-1-yl)ethyl)amino)-6-(pyrazolo[1,5-*a*]pyrimidin-3-yl)-1*H*-pyrazolo[4,3-*c*]pyridin-1-yl)phenyl)methanesulfonam ide | 17u | (500 MHz, *DMSO*-*d₆*) δ (ppm): 9.98 (s, 1H), 9.21 (dd, J=7.1, 1.4 Hz, 1H), 9.05 (d, J=0.8 Hz, 1H), 8.84 (d, J=0.2Hz, 1H), 8.71 (dd, J=4.1, 1.8 Hz, 1H), 8.06 (d, J=0.7 Hz, 1H), 7.42 (d, J=8.4 Hz, 1H), 7.14 (dd, J=7.2, 3.9 Hz, 1H), 7.10 (d, J=2.1Hz, 1H), 6.96 (dd, J=8.4, 2. 1Hz, 1H), 6.65 (t, J=5.7 Hz, 1H), 3.81 (s, 3H), 3.40 (q, J=6.3Hz, 2H), 3.12 (s, 3H), 2.60 (t, J=6.9 Hz, 2H), 2.25-2.55 (m, 8H), 2.15 (s, 3H). | (Method 6) Rt = 3.09 min, ES⁺ *m*/*z* 577.1 [M+H]⁺ |
| 39 | 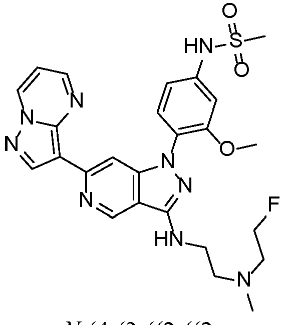 *N*-(4-(3-((2-((2-fluoroethyl)(methyl)amino)e thyl)amino)-6-(pyrazolo[1,5-*a*]pyrimidin-3-yl)-1*H*-pyrazolo[4,3-*c*]pyridin-1-yl)-3-methoxyphenyl)methanesulf onamide | 17ab | (600 MHz, *DMSO*-*d₆*) δ (ppm): 9.99 (s, 1H), 9.21 (dd, J=7.0, 1.9 Hz, 1H), 9.05 (d, J=1.1Hz, 1H), 8.85 (s, 1H), 8.72 (dd, J=4.0, 1.7 Hz, 1H), 8.07 (d, J=1.1Hz, 1H), 7.44 (d, J=8.5Hz, 1H), 7.14 (dd, J=7.1, 4.1Hz, 1H), 7.11 (d, J=2.3Hz, 1H), 6.97 (dd, J=8.5, 2.1Hz, 1H), 6.64 (t, J=5.8 Hz, 1H), 4.45 (dt, J=47.9, 4.9 Hz, 2H), 3.82 (s, 3H), 3.41 (q, J=6.4 Hz, 2H), 3.13 (s, 3H), 2.70-2.78 (m, 4H), 2.32 (s, 3H). | (Method 6) Rt = 3.15 min, ES⁺ *m*/*z* 554.1 [M+H]⁺ |
| 40 | 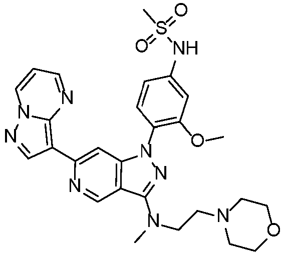 *N*-(3-methoxy-4-(3-(methyl(2-morpholinoethyl)amino)-6-(pyrazolo[1,5-*a*]pyrimidin-3-yl)-1*H*-pyrazolo[4,3-*c*]pyridin-1-yl)phenyl)methanesulfonam ide | 17w | (500 MHz, *DMSO*-*d₆*) δ (ppm): 10.03 (s, 1H), 9.21 (dd, J=7.1, 1.7 Hz, 1H), 9.15 (d, J=0.7 Hz, 1H), 8.84 (s. 1H), 8.71 (dd, J=4.1, 1.7 Hz, 1H), 8.06 (d, J=0.7 Hz, 1H), 7.43 (d, J=8.5Hz, 1H), 7.14 (dd, J=7.1, 4.1 Hz, 1H), 7.11 (d, J=2.2Hz, 1H), 6.97 (dd, J=8.5, 2.2Hz, 1H), 3.80 (s, 3H), 3.69 (t, J=7.0 Hz, 2H), 3.54 (t, J =4.5Hz, 4H), 3.19 (s, 3H), 3.13 (s, 3H), 2.65 - 2.59 (m, 2H, signal partially under DMSO), 2.45 (bs, 4H). | (Method 7) Rt = 3.17 min, ES⁺ *m*/*z* 578.2 [M+H]⁺ |
| 41 | 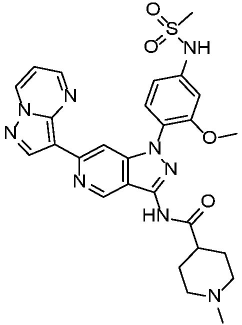 *N*-(1-(2-methoxy-4-(methylsulfonamido)phenyl) -6-(pyrazolo[1,5-*a*]pyrimidin-3-yl)-1*H-*pyrazolo[4,3-*c*]pyridin-3-yl)-1-methylpiperidine-4-carboxamide | 17ae | (600 MHz, *DMSO*-*d₆*) δ(ppm): 10.90 (s, 1H), 10.16 (br s, 1H), 9.32 (d, J=1.1 Hz, 1H), 9.23 (dd, J=7.0, 1.7 Hz, 1H), 8.88 (s, 1H), 8.72 (dd, J=4.1, 1.7 Hz, 1H), 8.15 (d, J=1.1 Hz, 1H), 7.47 (d, J=8.6 Hz, 1H), 7.17 - 7.14 (m, 1H), 7.13 (d, J=2.4 Hz, 1H), 7.01 - 6.97 (m, 1H), 3.79 (s, 3H), 3.17 (s, 3H), 2.88 - 2.84 (m, 2H), 2.49 - 2.46 (m, 1H), 2.19 (s, 3H), 1.96 - 1.89 (m, 2H), 1.88 - 1.83 (m, 2H), 1.79 - 1.71 (m, 2H). | (Method 7) Rt = 3.32 min, ES⁺ *m*/*z* 576.19 [M+H]⁺ |
| 42 | 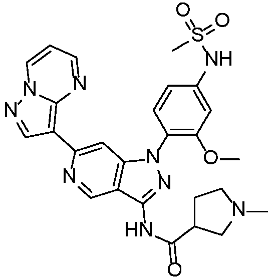 (rac) *N*-(1-(2-methoxy-4-(methylsulfonamido)phenyl) -6-(pyrazolo[1,5-*a*]pyrimidin-3-yl)-1*H-*pyrazolo[4,3-*c*]pyridin-3-yl)-1-methylpyrrolidine-3-carboxamide | 17af | (500 MHz, *DMSO*-*d₆*) δ (ppm): 11.02 (bs, 1H), 9.35 (s, 1H), 9.23 (dd, J=7.0, 1.7 Hz, 1H), 8.88 (s ,1H), 8.72 (dd, J=4.1, 1.7 Hz, 1H), 8.14 (d, J=0.9 Hz, 1H), 7.47 (d, J=8.5 Hz, 1H), 7.18-7.13 (m, 2H), 6.99 (dd, J=8.5, 2.2 Hz, 1H), 3.79 (s, 3H), 3.51 (s, 3H), 3.43-3.39 (m, 1H), 3.17 (s, 3H), 2.74 (bs, 2H), 2.38 (bs, 2H, overlapped with DMSO satellite signal), 2.15-2.09 (m, 2H) | (Method 4) Rt = 0.78 min, ES⁺ *m*/*z* 562.6 [M+H]⁺ |
| 43 | 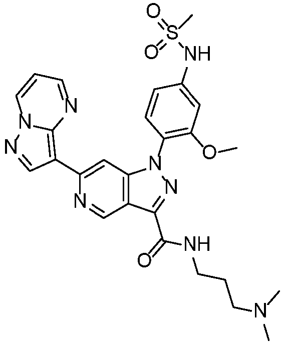 *N-(3-*(dimethylamino)propyl)-1-(2-methoxy-4-(methylsulfonamido)phenyl) -6-(pyrazolo[1,5-*a*]pyrimidin-3-yl)-1*H-*pyrazolo[4,3-*c*]pyridine-3-carboxamide | 23a | (300 MHz, *DMSO*-*d₆*) δ (ppm): 10.18 (br s, 1H), 9.48 (d, J=1.2 Hz, 1H), 9.23 (dd, J=7.1, 1.7 Hz, 1H), 8.91 (s, 1H), 8.68-8.79 (m, 2H), 8.26 (d, J=1.2 Hz, 1H), 7.56 (d, J=8.5 Hz, 1H), 7.09-7.21 (m, 2H), 7.00 (dd, J=8.5, 2.3 Hz, 1H), 3.77 (s, 3H), 3.34-3.41 (m, 2H), 3.15 (s, 3H), 2.24-2.35 (m, 2H), 2.15 (s, 6H), 1.63-1.78 (m, 2H). | (Method 5) Rt = 2.27 min, ES⁺ *m*/*z* 564.2 [M+H]⁺ |
| 44 | 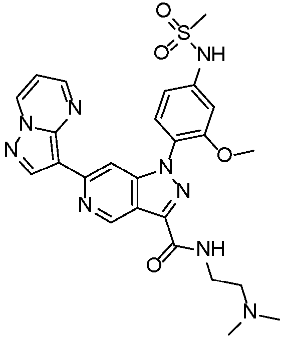 *N-*(2-(dimethylamino)ethyl)-1-(2-methoxy-4-(methylsulfonamido)phenyl) -6-(pyrazolo[1,5-*a*]pyrimidin-3-yl)-1*H-*pyrazolo[4,3*-c*]pyridine-3-carboxamide | 23b | (300 MHz, *DMSO*-*d₆*) δ (ppm): 10.22 (br s, 1H), 9.49 (s, 1H), 9.23 (d, J=7.3 Hz, 1H), 8.91 (s, 1H), 8.72 (d, J=2.8 Hz, 1H), 8.42 (t, J=5.5 Hz, 1H), 8.25 (s, 1H), 7.58 (d, J=8.5 Hz, 1H), 7.09-7.22 (m, 2H), 6.95-7.07 (m, 1H), 3.77 (s, 3H), 3.38-3.49 (m, 2H), 3.16 (s, 3H), 2.48-2.50 (m, 2H, overlapped with DMSO), 2.19 (s, 6H). | (Method 5) Rt = 2.21 min, ES⁺ *m*/*z* 550.1 [M+H]⁺ |
| 45 | 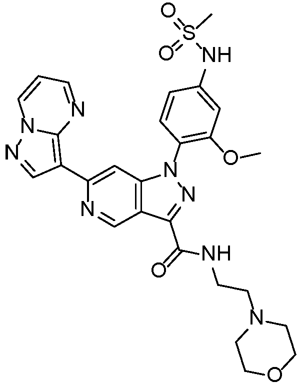 1-(2-methoxy-4-(methylsulfonamido)phenyl) -*N*-(2-morpholinoethyl)-6-(pyrazolo[1,5-*a*]pyrimidin-3-yl)-1*H*-pyrazolo[4,3-*c*]pyridine-3-carboxamide | 23c | (300 MHz, *DMSO*-*d₆*) δ (ppm): 10.22 (br s, 1H), 9.49 (s, 1H), 9.20-9.26 (m, 1H), 8.91 (s, 1H), 8.69-8.75 (m, 1H), 8.53 (t, J=5.7 Hz, 1H), 8.26 (s, 1H), 7.59 (d, J=8.5 Hz, 1H), 7.12-7.19 (m, 2H), 7.02 (dd, J=8.5, 1.7 Hz, 1H), 3.77 (s, 3H), 3.57 (br. t, J=4.2 Hz, 4H), 3.45 (q, J=6.3 Hz, 2H), 3.17 (s, 3H), 2.48-2.50 (m, 2H, overlapped with DMSO), 2.36-2.45 (m, 4H). | (Method 5) Rt = 2.26 min, ES⁺ *m*/*z* 592.1 [M+H]⁺ |
| 46 | 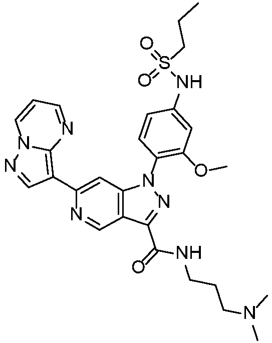 *N-*(3-(dimethylamino)propyl)-1-(2-methoxy-4-(propylsulfonamido)phenyl) -6-(pyrazolo[1,5-*a*]pyrimidin-3-yl)-1*H-*pyrazolo[4,3-*c*]pyridine-3-carboxamide | 23d | (500 MHz, *DMSO*-*d₆*) δ (ppm): 10.22 (br s, 1H), 9.49 (d, J=0.9 Hz, 1H), 9.24 (dd, J=6.9, 1.7 Hz, 1H), 8.92 (s, 1H), 8.69 - 8.77 (m, 2H), 8.27 (d, J=1.2 Hz, 1H), 7.56 (d, J=8.5 Hz, 1H), 7.14 - 7.19 (m, 2H), 7.02 (dd, J=8.5, 2.1 Hz, 1H), 3.77 (s, 3H), 3.33 - 3.39 (m, 2H, overlapped with H2O), 3.21 - 3.27 (m, 2H), 2.29 (t, J=7.0 Hz, 2H), 2.15 (s, 6H), 1.66 - 1.80 (m, 4H) 1.00 (t, J=7.5 Hz, 3H) | (Method 7) Rt = 3.95, ES⁺ m/z 592.2 [M+H]⁺ |
| 47 | 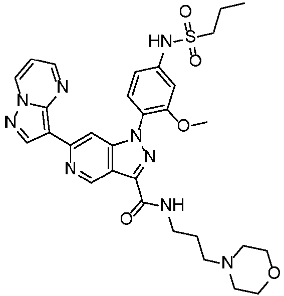 1-(2-methoxy-4-(propylsulfonamido)phenyl) -*N*-(3-morpholinopropyl)-6-(pyrazolo[1,5-*a*]pyrimidin-3-yl)-1*H*-pyrazolo[4,3-*c*]pyridine-3-carboxamide | 23e | (500 MHz, *DMSO*-*d₆*) δ (ppm): 10.26 (br s, 1H), 9.49 (d, J=0.9 Hz, 1H), 9.24 (dd, J=7.0, 1.5 Hz, 1H), 8.88 - 8.94 (m, 2H), 8.71 (dd, J=4.1, 1.7 Hz, 1H), 8.26 (d, J=1.2 Hz, 1H), 7.56 (d, J=8.2 Hz, 1H), 7.14 - 7.19 (m, 2H), 7.02 (dd, J=8.5, 2.1 Hz, 1H), 3.76 (s, 3H), 3.55 (t, J=4.6 Hz, 4H), 3.36 - 3.43 (m, 2H), 3.20 - 3.26 (m, 2H), 2.31 - 2.42 (m, 6H), 1.66 - 1.81 (m, 4H), 0.99 (t, J=7.5 Hz, 3H). | (Method 7) Rt = 3.97, ES⁺ m/z 634.1 [M+H]⁺ |
| 48 | 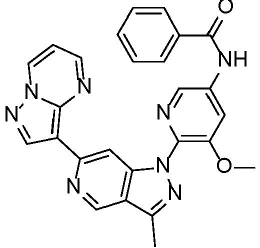 *N*-(5-methoxy-6-(3-methyl-6-(pyrazolo[1,5-*a*]pyrimidin-3-yl)-1*H-*pyrazolo[4,3-*c*]pyridin-1-yl)pyridin-3-yl)benzamide | 11o | (600 MHz, *DMSO*-*d₆*) δ (ppm): 10.76 (bs, 1H), 9.23 (dd, J=7.0, 1.7 Hz, 1H), 9.17 (d, J=1.1Hz, 1H), 8.90 (s. 1H), 8.71 (dd, J=4.1, 1.7 Hz, 1H), 8.64 (d, J=2.0 Hz, 1H), 8.36 (d, J=2.0 Hz, 1H), 8.35 (d, J=1.1 Hz, 1H), 8.08-8.04 (m, 2H), 7.68 - 7.65 (m, 1H), 7.63-7.59 (m, 2H), 7.15 (dd, J=7.0, 4.1Hz, 1H), 3.89 (s, 3H), 2.66 (s, 3H). | (Method 7) Rt = 4.72 min, ES⁺ *m*/*z* 477.2 [M+H]⁺ |
| 49 | 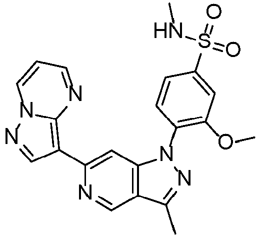 3-methoxy-*N*-methyl-4-(3-methyl-6-(pyrazolo[1,5-*a*]pyrimidin-3-yl)-1H-pyrazolo[4,3-*c*]pyridin-1-yl)benzenesulfonamide | 9d | (500 MHz, DMSO) δ (ppm): 9.22 (dd, J=7.3Hz, J=1.6Hz, 1H), 9.17 (bs, 1H), 8.89 (bs, 1H), 8.74 (dd, J=4.3Hz, J=1.7 Hz, 1H), 8.32 (bs, 1H), 7.77 (d, J=8.2Hz, 1H), 7.68 (d, J=1.6Hz, 1H), 7.66 (bs, 1H), 7.56 (dd, J=8.8 Hz, J=1.7 Hz, 1H), 7.16 (dd, J=7.1HZ, J=3.9 Hz, 1H), 3.96 (s, 3H), 2.67 (s, 3H), 2.54 (s, 3H). | (Method 6) Rt = 3.54 min, ES⁺ *m*/*z* 449.9 [M+H]⁺ |
| 50 | 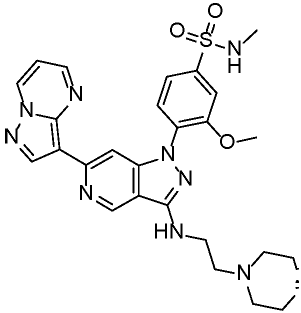 3-methoxy-*N*-methyl-4-(3-((2-morpholinoethyl)amino)-6-(pyrazolo[1,5-*a*]pyrimidin-3-yl)-1*H*-pyrazolo[4,3-*c*]pyridin-1-yl)benzenesulfonamide | 24a | (600 MHz, *DMSO*-*d₆*) δ (ppm): 9.23 (dd, J=7.0, 1.8 Hz, 1H), 9.11 (d, J=0.9 Hz, 1H), 8.87 (s, 1H), 8.76 (dd, J=4.0, 1.8 Hz, 1H), 8.23 (d, J=0.9 Hz, 1H), 7.73 (d, J=8.3Hz, 1H), 7.64 (d, J=2.0 Hz, 1H), 7.59 (br s, 1H), 7.53 (dd, J=8.2, 1.9 Hz, 1H), 7.17 (dd, J=7.0, 4.0 Hz, 1H), 6.89 (t, J=5.7 Hz, 1H), 4.01 (s, 3H), 3.61 (t, J=4.6Hz, 4H), 3.47 (q, J=6.5Hz, 2H), 2.64 (t, J=6.9 Hz, 2H), 2.52-2.53 (m, J=1.0 Hz, 3H), 2.44-2.49 (m, 4H). | (Method 8)Rt = 3.25 min, ES⁺ *m*/*z* 564.1 [M+H]⁺ |
| 51 | 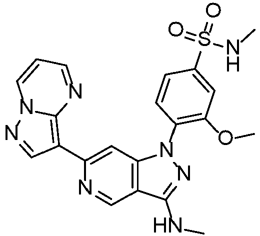 3-methoxy-*N*-methyl-4-(3-(methylamino)-6-(pyrazolo[1,5-*a*]pyrimidin-3-yl)-1*H*-pyrazolo[4,3-*c*]pyridin-1-yl)benzenesulfonamide | 24b | (600 MHz, DMSO-d3) δ (ppm): 9.23 (dd, J=7.0, 1.8 Hz, 1H), 9.06 (d, J=1.1Hz, 1H), 8.86 (s, 1H), 8.75 (dd, J=4.0, 1.7 Hz, 1H), 8.22 (d, J=1.1Hz, 1H), 7.75 (d, J=8.3Hz, 1H), 7.63 (d, J=1.8 Hz, 1H), 7.58 (q, J=5.0 Hz, 1H), 7.53 (dd, J=8.3, 2.0 Hz, 1H), 7.16 (dd, J=7.0, 4.0 Hz, 1H), 6.93 (q, J=5.0 Hz, 1H), 4.01 (s, 3H), 2.94 (d, J=5.0 Hz, 3H), 2.51-2.52 (m, 3H). | (Method 7) Rt = 3.99 min, ES⁺ *m*/*z* 465.1 [M+H]⁺ |
| 52 | 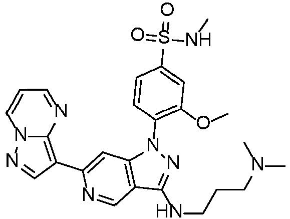 4-(3-((3-(dimethylamino)propyl)ami no)-6-(pyrazolo[1,5-*a*]pyrimidin-3-yl)-1*H-*pyrazolo[4,3-*c*]pyridin-1-yl)-3-methoxy*-N-*methylbenzenesulfonamide | 24c | (500 MHz, *DMSO*-*d₆*) δ (ppm): 9.22 (dd, J=6.9, 0.8 Hz, 1H), 9.08 (s, 1H), 8.86 (s, 1H), 8.75 (dd, J=3.8, 1.4 Hz, 1H), 8.22 (s, 1H), 7.72 (d, J=8.2Hz, 1H), 7.63 (s, 1H), 7.57 (q, J=4.6Hz, 1H), 7.52 (dd, J=7.9, 1.5Hz, 1H), 7.16 (dd, J=7.0, 4.0 Hz, 1H), 6.93 (t, J=5.3Hz, 1H), 4.00 (s, 3H), 3.33-3.40 (m, 2H), 2.51-2.52 (m, 3H), 2.31-2.36 (m, 2H), 2.16 (s, 6H), 1.83 (quin, J=7.2Hz, 2H). | (Method 6) Rt = 3.28 min, ES⁺ *m*/*z* 536.1 [M+H]⁺ |

### Example 53

### Step 1

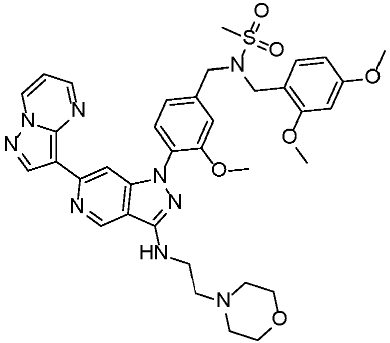

### N-(2,4-Dimethoxybenzyl)-N-(3-methoxy-4-(3-((2-morpholinoethyl)amino)-6-(pyrazolo[1,5-a]pyrimidin-3-yl)-1H-pyrazolo[4,3-c]pyridin-1-yl)benzyl)methanesulfonamide (intermediate step1-example53)

The title product was prepared similarly to example 1 starting from intermediate 27a.

LCMS (Method 2): Rt = 1.04 min, ES⁺ *m*/*z* 728.3 [M+H]⁺

### Step 2

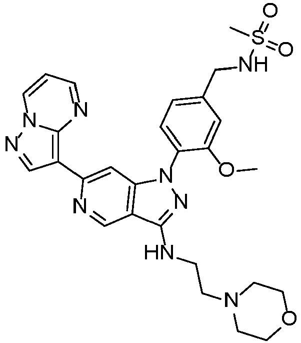

### N-(3-Methoxy-4-(3-((2-morpholinoethyl)amino)-6-(pyrazolo[1,5-a]pyrimidin-3-yl)-1H-pyrazolo[4,3-c]pyridin-1-yl)benzyl)methanesulfonamide

TFA (610 µL, 7.97 mmol) was added to a chilled solution of the intermediate step1-example53 (58.0 mg, 0.08 mmol) in dry DCM (4 mL). RM was stirred at RT for 2h, then loaded on a SCX column, washed with MeOH and eluted with methanolic ammonia (15 mL). Relevant fractions were pooled and evaporated *in vacuo* to afford the title compound (41 mg).

LCMS (Method 7): Rt = 3.13 min, ES⁺ *m*/*z* 578.2 [M+H]⁺
¹H-NMR (300 MHz, *DMSO*-*d₆*) δ (ppm) 9.19 (dd, J=7.1, 1.7 Hz, 1H), 9.06 (d, J=0.9 Hz, 1H), 8.84 (s, 1H), 8.67 (dd, J=4.1, 1.7 Hz, 1H), 8.08 (d, J=0.9 Hz, 1H), 7.66 (t, J=6.3 Hz, 1H), 7.44 (d, J=8.0 Hz, 1H), 7.29 (d, J=1.2 Hz, 1H), 7.05-7.17 (m, 2H), 6.69 (t, J=5.6 Hz, 1H), 4.27 (d, J=6.3 Hz, 2H), 3.86 (s, 3H), 3.59 (br t, J=4.53 Hz, 4H), 3.43 (q, J=6.2 Hz, 2H), 2.93 (s, 3H), 2.62 (t, J=6.8 Hz, 2H), 2.40-2.47 (m, 4H).

### Example 54 to 62

The following examples were prepared with a two step process similarly to example 53 from the indicated starting materials.

| Example | Structure/Name | *Step 1* | | *Step 2* | |
|---|---|---|---|---|---|
| | | Starting Material | Name intermediate after step 1 / LCMS | ¹H-NMR | LCMS |
| 54 | 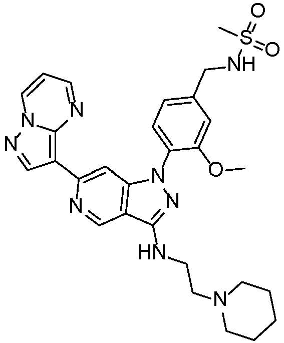 *N*-(3-methoxy-4-(3-((2-(piperidin-1-yl)ethyl)amino)-6-(pyrazolo[1,5-*a*]pyrimidin-3-yl)-1*H*-pyrazolo[4,3-*c*]pyridin-1-yl)benzyl)methanesulfonamide | Intermediate 27b | N-(2,4-dimethoxybenzyl)-N-(3-methoxy-4-(3-((2-(piperidin-1-yl)ethyl)amino)-6-(pyrazolo[1,5-a]pyrimidin-3-yl)-1H-pyrazolo[4,3-c]pyridin-1-yl)benzyl)methanesulfonamide | (300 MHz, *DMSO*-*d₆*) δ(ppm) 9.19 (dd, J=7.1, 1.7 Hz, 1H), 9.05 (d, J=0.9 Hz, 1H), 8.84 (s, 1H), 8.67 (dd, J=4.1, 1.7 Hz, 1H), 8.08 (d, J=0.9 Hz, 1H), 7.67 (t, J=6.3 Hz, 1H), 7.44 (d, J=7.8 Hz, 1H), 7.29 (d, J=1.0 Hz, 1H), 7.05-7.17 (m, 2H), 6.65 (t, J=5.5 Hz, 1H), 4.27 (d, J=6.3 Hz, 2H), 3.86 (s, 3H), 3.41 (q, J=6.4 Hz, 2H), 2.93 (s, 3H), 2.58 (br t, J=7.0 Hz, 2H), 2.33-2.45 (m, 4H), 1.44-1.57 (m, 4H), 1.31-1.44 (m, 2H). | (Method 7) Rt = 3.29 min, ES⁺ *m*/*z* 576.2 [M+H]⁺ |
| | | | (Method 2) Rt = 1.24 min, ES⁺ *m*/*z* 726.3 [M+H]⁺ | | |
| 55 | 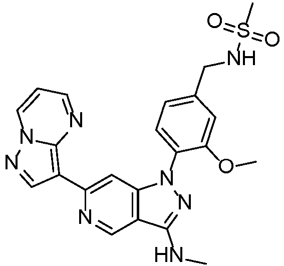 *N*-(3-methoxy-4-(3-(methylamino)-6-(pyrazolo[1,5-*a*]pyrimidin-3-yl)-1*H-*pyrazolo[4,3-*c*]pyridin-1-yl)benzyl)methanesulfonamide | Intermediate 27c | N-(2,4-dimethoxybenzyl)-N-(3-methoxy-4-(3-(methylamino)-6-(pyrazolo[1,5-a]pyrimidin-3-yl)-1H-pyrazolo[4,3-c]pyridin-1-yl)benzyl)methanesulfonamide | (600 MHz, *DMSO*-*d₆*) δ(ppm): 9.20 (dd, J=7.0, 1.7 Hz, 1H), 9.02 (d, J=1.1 Hz, 1H), 8.85 (s, 1H), 8.68 (dd, J=4.1, 1.7 Hz, 1H), 8.08 (d, J=1.1 Hz, 1H), 7.68 (br s, 1H), 7.47 (d, J=7.9 Hz, 1H), 7.30 (d, J=1.7 Hz, 1H), 7.13 (dd, J=7.0, 4.0 Hz,1H), 7.11 (dd, J=8.0, 1.7 Hz, 1H), 6.74 (q, J=5.1 Hz, 1H), 4.28 (br s, 2H), 3.87 (s, 3H), 2.94 (s, 3H), 2.92 (d, J=5.1 Hz, 3H). | (Method 7): Rt = 4.03 min, ES⁺ *m*/*z* 479.2 [M+H]⁺ |
| | | | (Method 2): Rt = 1.04 min, ES⁺ *m*/*z* 629.0 [M+H]⁺ | | |
| 56 | 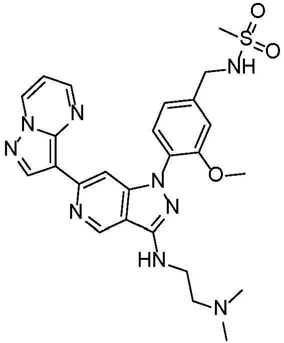 *N*-(4-(3-((2-(dimethylamino)ethyl)amino)-6-(pyrazolo[1,5-*a*]pyrimidin-3-yl)-1*H*-pyrazolo[4,3-*c*]pyridin-1-yl)-3-methoxybenzyl)methanesulfonamide | Intermediate 27d | N-(2,4-dimethoxybenzyl)-N-(4-(3-((2-(dimethylamino)ethyl)am ino)-6-(pyrazolo[1,5-a]pyrimidin-3-yl)-1H-pyrazolo[4,3-c]pyridin-1-yl)-3-methoxybenzyl)methanesulfonamide | (600 MHz, *DMSO-d₆,* 298K) δ(ppm): 9.20 (dd, J=7.0 Hz, J=1.8 Hz, 1H), 9.08 (d, J=1.1 Hz, 1H), 8.85 (s, 1H), 8.69 (dd, J=4.0 Hz, J=1.7 Hz, 1H), 8.09 (d, J=1.1 Hz, 1H), 7.68 (t, J=6.3 Hz, 1H), 7.46 (d, J=8.1 Hz, 1H), 7.30 (d, J=1.7 Hz, 1H), 7.12 - 7.15 (m, 1H), 7.11 (dd, J=8.0 Hz, J=1.7 Hz, 1H), 6.67 (t, J=5.6 Hz, 1H), 4.28 (d, J=6.4 Hz, 2H), 3.88 (s, 3H), 3.41 (q, J=6.5 Hz, 2H), 2.94 (s, 3H), 2.57 (t, J=6.7 Hz, 2H), 2.23 (s, 6H). | (Method 7): Rt = 3.14 min, ES⁺ *m*/*z* 536.2 [M+H]⁺ |
| | | | (Method 1): Rt = 0.66 min, ES⁺ *m*/*z* 686.3 [M+H]⁺ | | |
| 57 | 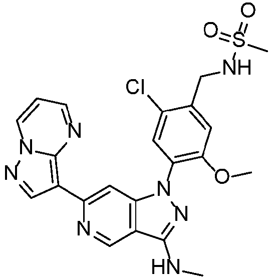 *N*-(2-chloro-5-methoxy-4-(3-(methylamino)-6-(pyrazolo[1,5-*a*]pyrimidin-3-yl)-1*H-*pyrazolo[4,3-*c*]pyridin-1-yl)benzyl)methanesulfonamide | Intermediate 27e | N-(2-chloro-5-methoxy-4-(3-(methylamino)-6-(pyrazolo[1,5-a]pyrimidin-3-yl)-1H-pyrazolo[4,3-c]pyridin-1-yl)benzyl)-N-(2,4-dimethoxybenzyl)methanesulfonamide | (500 MHz, DMSO) δ(ppm): 9.21 (d, J=7.0 Hz, 1H), 9.03 (s, 1H), 8.85 (s, 1H), 8.69-8.71 (m, 1H), 8.15 (s, 1H), 7.72 (t, J=5.9 Hz, 1H), 7.56 (s, 1H), 7.46 (s. 1H), 7.14 (dd, J=7.6 Hz, J=5.0 Hz, 1H), 6.87 (q, J=4.6 Hz, 1H), 4.34 (d, J=6.2 Hz, 2H), 3.92 (s, 3H), 3.02 (s, 3H), 2.93 (d, J=4.7 Hz, 3H). | (Method 7): RT = 4.33 min, ES⁺ *m*/*z* 513.1 [M+H]⁺ |
| | | | (Method 1): Rt = 0.97 min, ES⁺ *m*/*z* 663.4 [M+H]⁺ | | |
| 58 | 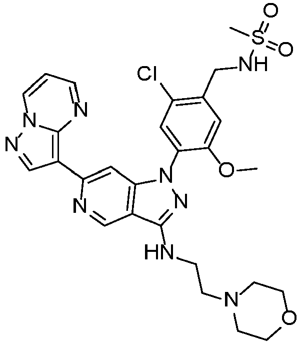 *N*-(2-chloro-5-methoxy-4-(3-((2-morpholinoethyl)amino)-6-(pyrazolo[1,5-*a*]pyrimidin-3-yl)-1H-pyrazolo[4,3-*c*]pyridin-1-yl)benzyl)methanesulfonamide | Intermediate 27f | N-(2-chloro-5-methoxy-4-(3-((2-morpholinoethyl)amino)-6-(pyrazolo[1,5-a]pyrimidin-3-yl)-1H-pyrazolo[4,3-c]pyridin-1-yl)benzyl)-N-(2,4-dimethoxybenzyl)methanesulfonamide | (500 MHz, *DMSO*-*d₆*) δ(ppm): 9.21 (dd, J=1.1, 6.9 Hz, 1H), 9.08 (s, 1H), 8.85 (s, 1H), 8.70 (dd, J=1.2, 4.0 Hz, 1H), 8.16 (s, 1H), 7.72 (t, J=6.3 Hz, 1H), 7.54 (s, 1H), 7.46 (s, 1H), 7.15 (dd, J=4.0, 7.0 Hz, 1H), 6.81 (t, J=5.2 Hz, 1H), 4.34 (d, J=6.1 Hz, 2H), 3.93 (s, 3H), 3.60 (t, J=4.4 Hz, 4H), 3.46 (q, J=6.2 Hz, 2H), 3.02 (s, 3H), 2.63 (br t, J=6.6 Hz, 2H), 2.47 (br s, 4H) | (Method 1): Rt = 3.66 min, ES⁺ *m*/*z* 612.2 [M+H]⁺ |
| | | | (Method 1): Rt = 1.11 min, ES⁺ *m*/*z* 762.5 [M+H]⁺ | | |
| 59 | 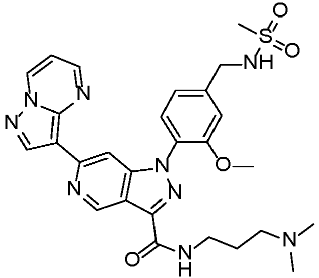 N-(3-(dimethylamino)propyl)-1-(2-methoxy-4-(methylsulfonamidomethyl)phenyl) -6-(pyrazolo[1,5-a]pyrimidin-3-yl)-1H-pyrazolo[4,3-c]pyridine-3-carboxamide | Intermediate 28 | 1-(4-((N-(2,4-dimethoxybenzyl)methyl sulfonamido)methyl)-2-methoxyphenyl)-N-(3-(dimethylamino)propyl)-6-(pyrazolo[1,5-a]pyrimidin-3-yl)-1H-pyrazolo[4,3-c]pyridine-3-carboxamide (Method 2): Rt = 1.08 min, ES⁺ *m*/*z* 728.3 [M+H]⁺ | (300 MHz, *DMSO*-*d₆*) δ(ppm): 9.49 (d, J=1.0 Hz, 1H), 9.23 (dd, J=7.0, 1.6 Hz, 1H), 8.91 (s, 1H), 8.78 (t, J=5.9 Hz, 1H), 8.68 (dd, J=4.2, 1.6 Hz, 1H), 8.27 (d, J=1.0 Hz, 1H), 7.74 (t, J=6.2 Hz, 1H), 7.61 (d, J=8.0 Hz, 1H), 7.34-7.43 (m, 1H), 7.18-7.24 (m, 1H), 7.15 (dd, J=7.1, 4.2 Hz, 1H), 4.33 (d, J=6.3 Hz, 2H), 3.83 (s, 3H), 3.33-3.40 (m, 2H), 2.94 (s, 3H), 2.29 (t, J=7.1 Hz, 2H), 2.14 (s, 6H), 1.70 (quin, J=6.9 Hz, 2H). | (Method 7): Rt = 3.57 min, ES⁺ *m*/*z* 578.1 [M+H]⁺ |
| 60 | 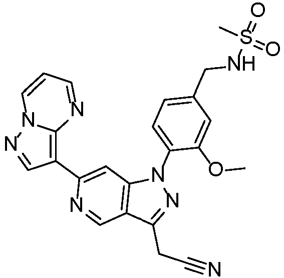 *N*-(4-(3-(cyanomethyl)-6-(pyrazolo[1,5-*a*]pyrimidin-3-yl)-1*H*-pyrazolo[4,3-*c*]pyridin-1-yl)-3-methoxybenzyl)methanesulfonamide | Intermediate 29 | N-(4-(3-(cyanomethyl)-6-(pyrazolo[1,5-a]pyrimidin-3-yl)-1H-pyrazolo[4,3-c]pyridin-1-yl)-3-methoxybenzyl)-N-(2,4-dimethoxybenzyl)methanesulfonamide | (500 MHz, *DMSO*-*d₆*) δ(ppm): 9.20-9.26 (m, 2H), 8.90 (s, 1H), 8.67-8.72 (m, 1H), 8.25 (s, 1H), 7.73 (t, J=6.3 Hz, 1H), 7.53 (d, J=7.9 Hz, 1H), 7.38 (s, 1H), 7.19 (d, J=7.9 Hz, 1H), 7.16 (dd, J=6.6, 4.4 Hz, 1H), 4.62 (s, 2H), 4.33 (d, J=6.1 Hz, 2H), 3.84 (s, 3H), 2.96 (s, 3H). | (Method 7): Rt = 4.10 min, ES⁺ *m*/*z* 489.1[M+H]⁺ |
| | | | (Method 2): Rt = 1.02 min, ES⁺ *m*/*z* 639.9 [M+H]⁺ | | |
| 61 | 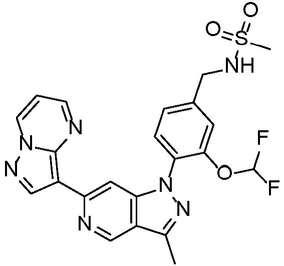 *N*-(3-(difluoromethoxy)-4-(3-methyl-6-(pyrazolo[1,5-*a*]pyrimidin-3-yl)-1*H-*pyrazolo[4,3-*c*]pyridin-1-yl)benzyl)methanesulfonamide | Intermediate 33a | N-(3-(difluoromethoxy)-4-(3-methyl-6-(pyrazolo[1,5-a]pyrimidin-3-yl)-1H-pyrazolo[4,3-c]pyridin-1-yl)benzyl)-N-(2,4-dimethoxybenzyl)methanesulfonamide | (*DMSO*-*d₆*, 400 MHz) δ (ppm): 9.21 (dd, J=7.0, 1.7 Hz, 1H), 9.17 (d, J=1.1 Hz, 1H), 8.89 (s, 1H), 8.67 (dd, J=4.2, 1.7 Hz, 1H), 8.27 (d, J=1.2 Hz, 1H), 7.77 (br s, 1H), 7.68 (d, J=8.2 Hz, 1H), 7.52 (s, 1H), 7.48 (dd, J=8.1, 1.7 Hz, 1H), 7.14 (dd, J=7.1, 4.2 Hz, 1H), 7.17 (t, J=72.5 Hz, 1H), 4.33 (s, 2H), 2.96 (s, 3H), 2.65 (s, 3H). | (Method 7): Rt = 4.32 min, ES⁺ *m*/*z* 500.1 [M+H]⁺ |
| | | | (Method 1): Rt = 0.99 min, ES⁺ *m*/*z* 650.3 [M+H]⁺ | | |
| 62 | 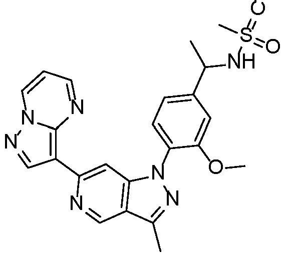 *N-*(1-(3-methoxy-4-(3-methyl-6-(pyrazolo[1,5-*a*]pyrimidin-3-yl)-1*H*-pyrazolo[4,3-*c*]pyridin-1-yl)phenyl)ethyl)methanesulfonamide | Intermediate 33b | N-(2,4-dimethoxybenzyl)-N-(1-(3-methoxy-4-(3-methyl-6-(pyrazolo[1,5-a]pyrimidin-3-yl)-1H-pyrazolo[4,3-c]pyridin-1-yl)phenyl)ethyl)methanesulfonamide | (500 MHz, DMSO) δ(ppm): 9.21 (dd, J=7.1 Hz, J=1.4 Hz, 1H), 9.14 (s, 1H), 8.88 (s, 1H), 8.67 (dd, J=4.3 Hz, J=1.4 Hz, 1H), 8.21 (s, 1H), 7.80 (d, J=8.5 Hz, 1H), 7.47 (d, J=7.9 Hz, 1H), 7.40 (bs, 1H), 7.17-7.20 (m, 1H), 7.13 (dd, J=7.0 Hz, J=4.1 Hz, 1H), 4.60 (quint, J=7.1 Hz, 1H), 3.84 (s, 3H), 2.78 (s, 3H), 2.64 (s, 3H), 1.51 (d, J=6.9 Hz, 3H). | (Method 7): Rt = 4.08 min, ES⁺ *m*/*z* 478.2 [M+H]⁺ |
| | | | (Method 2): Rt = 0.97 min, ES⁺ *m*/*z* 628.4 [M+H]⁺ | | |

### Example 63

### Step 1

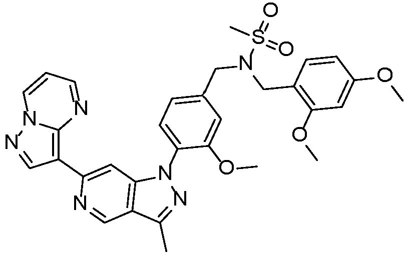

### N-(2,4-Dimethoxybenzyl)-N-(3-methoxy-4-(3-methyl-6-(pyrazolo[1,5-a]pyrimidin-3-yl)-1H-pyrazolo[4,3-c]pyridin-1-yl)benzyl)methanesulfonamide (intermediate step1-example63)

Intermediate step1-example63 was prepared similarly to intermediate 26a starting from intermediate 31 and methanesulfonyl chloride.

LCMS (Method 1): Rt = 0.91 min, ES⁺ *m*/*z* 614.3 [M+H]⁺

### Step 2

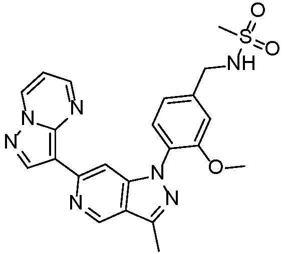

### N-(3-Methoxy-4-(3-methyl-6-(pyrazolo[1,5-alpyrimidin-3-yl)-1H-pyrazolo[4,3-c]pyridin-1-yl)benzyl)methanesulfonamide (Example 63)

Example 63 was prepared similarly to example 53, starting from intermediate step1-example63.

LCMS (Method 7): Rt = 3.87 min, ES⁺ *m*/*z* 464.2 [M+H]⁺
¹H-NMR (500 MHz, *DMSO*-*d₆*) δ (ppm) 9.21 (dd, J=6.87, 1.37 Hz, 1H), 9.14 (s, 1H), 8.89 (s, 1H), 8.69 (dd, J=3.97, 1.22 Hz, 1H), 8.22 (s, 1H), 7.71 (br t, J=6.10 Hz, 1H), 7.49 (d, J=7.93 Hz, 1H), 7.35 (s, 1H), 7.10 - 7.19 (m, 2H), 4.31 (d, J=6.10 Hz, 2H), 3.84 (s, 3H), 2.95 (s, 3H), 2.65 (s, 3H).

### Example 6

### Step 1

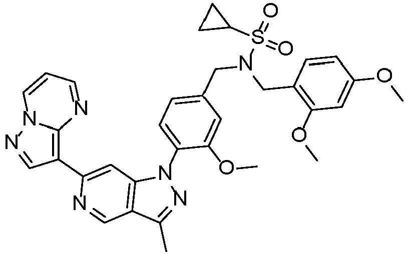

### N-(2,4-dimethoxybenzyl)-N-(3-methoxy-4-(3-methyl-6-(pyrazolo[1,5-a]pyrimidin-3-yl)-1H-pyrazolo[4,3-c]pyridin-1-yl)benzyl)cyclopropanesulfonamide (intermediate step1-example64)

Intermediate step1-example64 was prepared similarly to intermediate 26a starting from intermediate 31 and cyclopropanesulfonyl chloride.

LCMS (Method 1): Rt = 0.97 min, ES⁺ *m*/*z* 640.3 [M+H]⁺

### Step 2

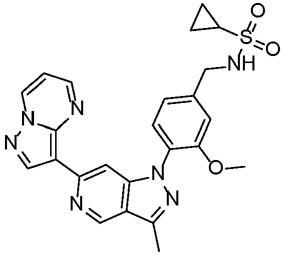

### N-(3-methoxy-4-(3-methyl-6-(pyrazolo[1,5-a]pyrimidin-3-yl)-1H-pyrazolo[4,3-c]pyridin-1-yl)benzyl)cyclopropanesulfonamide (Example 64)

Example 64 was prepared similarly to example 53, starting from intermediate step1-example64.

LCMS (Method 7): Rt = 4.22 min, ES⁺ *m*/*z* 490.2 [M+H]⁺
¹H-NMR (500 MHz, *DMSO-d₆,* 352K) δ (ppm) 9.10 - 9.16 (m, 2H), 8.86 (s, 1H), 8.62 - 8.68 (m, 1H), 8.21 (s, 1H), 7.52-7.58 (m, 1H), 7.47 (d, J=7.7 Hz, 1H), 7.38 (s, 1H), 7.18 (d, J=7.9 Hz, 1H), 7.08 - 7.13 (m, 1H), 4.36 (d, J=6.1 Hz, 2H), 3.85 (s, 3H), 2.66 (s, 3H), 2.53-2.57 (m, 1 H, overlap with DMSO signal), 0.90-1.02 (m, 4H).

### Example 65

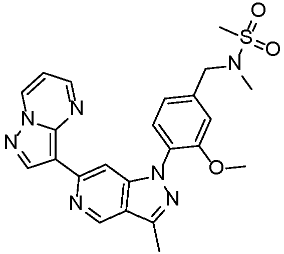

### N-(3-methoxy-4-(3-methyl-6-(pyrazolo[1,5-a]pyrimidin-3-yl)-1H-pyrazolo[4,3-c]pyridin-1-yl)benzyl)-N-methylmethanesulfonamide (Example 65)

Iodomethane (7.45 µL, 0.012 mmol) was added to an ice-cold suspension of example 63 (56.0 mg, 0.11 mmol) and K₂CO₃ (30.1 mg, 0.22 mmol) in DMF (2 mL). RM was stirred at RT for 1.5 h, then partitioned between EtOAc and water. Organic layer was washed with water, aqueous saturated NaCl, dried over Na₂SO₄ and evaporated to dryness. The residue was purified by flash chromatography on a Si cartridge by eluting with 0-100 % DCM/MeOH (20:1) in DCM.

LCMS (Method 7): Rt = 4.24 min, ES⁺ *m*/*z* 478.2 [M+H]⁺
¹H-NMR (500 MHz, *DMSO*-*d₆*) δ (ppm) 9.22 (d, J=7.02 Hz, 1H), 9.14 (s, 1H), 8.89 (s, 1H), 8.70 (br d, J=3.66 Hz, 1H), 8.25 (s, 1H), 7.53 (d, J=8.24 Hz, 1H), 7.29 (s, 1H), 7.11 - 7.17 (m, 2H), 4.39 (s, 2H), 3.85 (s, 3H), 3.03 (s, 3H), 2.79 (s, 3H), 2.65 (s, 3H).

### Example 66

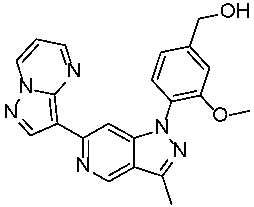

### (3-Methoxy-4-(3-methyl-6-(pyrazolo[1,5-a]pyrimidin-3-yl)-1H-pyrazolo[4,3-c]pyridin-1-yl)phenyl)methanol (Example 66)

Intermediate 30a (40 mg, 0.103 mmol) was suspended in THF (0.5 mL) and cooled in an ice bath before portionwise addition of NaBH₄ (3.90 mg, 0.103 mmol). RM was stirred at RT for 2 hours, then quenched with water (1 mL), stirred for 5 min at RT and precipitate formed filtered, washed with water (2 x 2 mL) and dried to afford the title product (37.4 mg).

LCMS (Method 7): Rt = 3.69 min, ES⁺ *m*/*z* 387.1 [M+H]⁺
¹H-NMR (500 MHz, DMSO-d3) δ/ppm: 9.21 (dd, J=7.0, 1.5 Hz, 1H), 9.14 (s, 1H), 8.89 (s, 1H), 8.70 (dd, J=4.1,1.7 Hz, 1H), 8.22 (s, 1H), 7.45 (d, J=7.9 Hz, 1H), 7.31 (s, 1H), 7.14 (dd, J=7.0, 4.0 Hz, 1H), 7.11 (d, J=7.9 Hz, 1H), 5.41 (t, J=5.8 Hz, 1H), 4.64 (d, J=5.8 Hz, 2H), 3.82 (s, 3H), 2.64 (s, 3H).

### Example 67

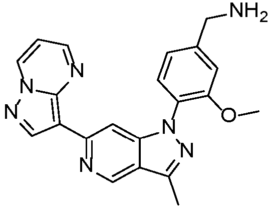

### (3-Methoxy-4-(3-methyl-6-(pyrazolo[1,5-a]pyrimidin-3-yl)-1H-pyrazolo[4,3-c]pyridin-1-yl)phenyl)methanamine (Example 67)

Intermediate 31 (285 mg, 0.30 mmol) was dissolved in TFA (2.9 mL) and heated in a microwave reactor at 120 °C for 30 min. RM was quenched with aqueous saturated NaHCO₃ (5 mL) / DCM (10 mL) and pH adjusted to 11. Organic layer was washed with aqueous saturated NaHCO₃ (2 x 5 mL) and concentrated in vacuo. The residue was purified by flash chromatography on a Si cartridge by eluting with 0-80% DCM/MeOH/NH₄OH (90:9:0.5) in DCM to afford the title compound.

LCMS (Method 8): RT = 4.59 min, ES⁺ *m*/*z* 386.2 [M+H]⁺
¹H-NMR (600 MHz, *DMSO*-*d₆*) δ(ppm) 9.21 (dd, J=6.9, 1.4 Hz, 1H), 9.13 (s, 1H), 8.89 (s, 1H), 8.67-8.70 (m, 1H), 8.21 (s, 1H), 7.41 (d, J=7.9 Hz, 1H), 7.36 (s, 1H), 7.15 (dd, J=6.9, 4.1 Hz, 1H), 7.12 (d, J=7.6 Hz, 1H), 3.86 (s, 2H), 3.82 (s, 3H), 2.64 (s, 3H).

### Example 68

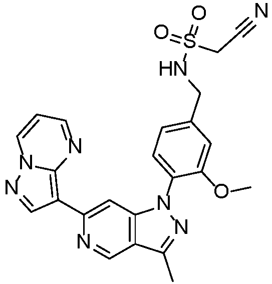

### 1-Cyano-N-(3-methoxy-4-(3-methyl-6-(pyrazolo[1,5-a]pyrimidin-3-yl)-1H-pyrazolo[4,3-c]pyridin-1-yl)benzyl)methanesulfonamide (Example 68)

Example 67 (30 mg, 0.08 mmol) was suspended in DCM (0.5 mL), then cyanomethanesulfonyl chloride (8.0 µL, 0.09 mmol) and pyridine (18 µL, 0.23 mmol) were added. RM was stirred at RT for 1h. Solvent was removed *in vacuo* and residue partitioned in EtOAc (15 mL) and water (10 mL). Organic layer was dried over Na₂SO₄ and concentrated *in vacuo.* The residue was purified by flash chromatography on Si cartridge by eluting with 0-100 % DCM/MeOH/NH₄OH (90:9:0.5) in DCM to afford the title compound (2.4 mg).

LCMS (Method 7): Rt = 4.22 min, ES⁺ *m*/*z* 489.2 [M+H]⁺
¹H-NMR (600 MHz, *DMSO-d₆*) δ(ppm) 9.22 (d, J=7.0 Hz, 1H), 9.14 (s, 1H), 8.89 (s, 1H), 8.78 (t, J=6.1 Hz, 1H), 8.68 (d, J=4.0 Hz, 1H), 8.23 (s, 1H), 7.50 (d, J=7.9 Hz, 1H), 7.36 (s, 1H), 7.12-7.17 (m, 2H), 4.84 (s, 2H), 4.41 (d, J=6.1 Hz, 2H), 3.85 (s, 3H), 2.65 (s, 3H).

### Example 69

### Step 1

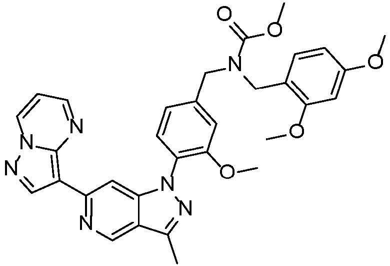

### Methyl (2,4-dimethoxybenzyl)(3-methoxy-4-(3-methyl-6-(pyrazolo[1,5-a]pyrimidin-3-yl)-1H-pyrazolo[4,3-c]pyridin-1-yl)benzyl)carbamate (Intermediate steel-example 69)

Intermediate 31 (100 mg, 0.12 mmol) and TEA (65 µL, 0.46 mL) were dissolved in DCM (0.5 mL), then a cold solution of methyl chloroformate (63 µL, 0.81 mmol) in DCM (0.5 mL) was added dropwise The resulting mixture was stirred at RT for 15 min, the partitioned between EtOAc (10 mL) and water (10 mL). Aqueous layers was extracted with EtOAc (3x10 mL) Combined organic layers were washed with aqueous saturated NaCl (10 mL), dried over Na₂SO₄ and solvent was removed in vacuo. The residue was purified by flash chromatography on a Si cartridge by eluting with 0-100% DCM/MeOH/NH₄OH (90:9:0.5) in DCM to afford the desired product (47.2 mg).

LCMS (Method 2): Rt = 1.19, ES *m*/*z* 594.5 [M+H]⁺

### Step 2

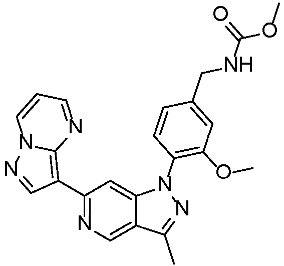

### Methyl (3-methoxy-4-(3-methyl-6-(pyrazolo[1,5-a]pyrimidin-3-yl)-1H-pyrazolo[4,3-c]pyridin-1-yl)benzyl)carbamate (Example 69)

Example 69 was made in a similar way to example 53 step 2 starting from intermediate step1-example 69.

LCMS (Method 7): Rt = 4.07 min, ES⁺ *m*/*z* 444.1 [M+H]⁺
¹H-NMR (500 MHz, *DMSO-d₆*) δ(ppm) 9.22 (dd, J=7.0 Hz, J=1.8 Hz, 1H), 9.14 (d, J=0.9 Hz, 1H), 8.89 (s, 1H), 8.70 (dd, J=4.0 Hz, J=1.5 Hz, 1H), 8.21 (d, J=1.2 Hz, 1H), 7.82 (t, J=5.8 Hz, 1H), 7.46 (d, J=8.2 Hz, 1H), 7.25 (s, 1H), 7.15 (dd, J=7.0 Hz, J=4.0 Hz, 1H), 7.04 (d, J=7.9 Hz, 1H), 4.33 (d, J=6.4 Hz, 2H), 3.82 (s, 3H), 3.60 (s, 3H), 2.64 (s, 3H).

### Example 70

### Step 1

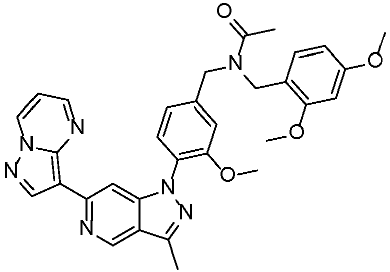

### N-(2,4-Dimethoxybenzyl)-N-(3-methoxy-4-(3-methyl-6-(pyrazolo [1,5-a]pyrimidin-3-yl)-1H-pyrazolo[4,3-c]pyridin-1-yl)benzyl)acetamide (Intermediate steel-example 70)

Intermediate 31 (100 mg, 0.12 mmol) and TEA (65 µL, 0.46 mL) were dissolved in DCM (0.5 mL) and added dropwise to a cold solution of acetic anhydride (77 µL, 0.81 mmol) in DCM (0.5 mL). The resulting mixture was stirred at RT for 15 min. The mixture was diluted with EtOAc (10 mL) and water (10 mL). Layers were separated and aqueous layer extracted with EtOAc (3x10 mL). Combined organic layers were washed with aqueous saturated NaCl (10 mL), dried over Na₂SO₄ and solvent removed under reduced pressure. The residue was purified by flash chromatography on a Si cartridge by eluting with 0-100 % DCM/MeOH/NH₄OH ((90:9:1.5) in DCM to afford the title (61.2 mg).

LCMS (Method 2): Rt = 1.07, ES *m*/*z* 578.5 [M+H]⁺.

### Step 2

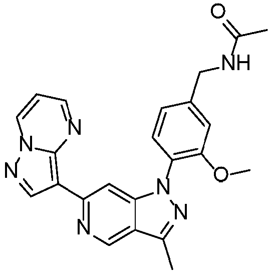

### N-(3-methoxy-4-(3-methyl-6-(pyrazolo[1,5-a]pyrimidin-3-yl)-1H-pyrazolo[4,3-c]pyridin-1-yl)benzyl)acetamide (Example 70)

Example 70 was made in a similar way to example 53 step 2 starting from intermediate step1-example 70

LCMS (Method 7): Rt = 3.62 min, ES⁺ *m*/*z* 428.2 [M+H]⁺
¹H-NMR (600 MHz, *DMSO-d₆*) δ(ppm) 9.25 (dd, J=7.2, 1.4 Hz, 1H), 9.23 (br s, 1H), 8.93 (s, 1H), 8.73 (d, J=2.7 Hz, 1H), 8.48 (br t, J=6.0 Hz, 1H), 8.24 (s, 1H), 7.47 (d, J=7.9 Hz, 1H), 7.26 (s, 1H), 7.19 (dd, J=6.9, 4.1 Hz, 1H), 7.05 (d, J=8.2 Hz, 1H), 4.40 (d, J=5.8 Hz, 2H), 3.82 (s, 3H), 2.64-2.69 (m, 3H), 1.94 (s, 3H).

### Example 71

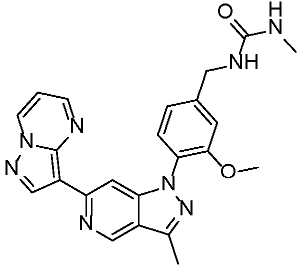

### 1-(3-Methoxy-4-(3-methyl-6-(pyrazolo[1,5-a]pyrimidin-3-yl)-1H-pyrazolo[4,3-c]pyridin-1-yl)benzyl)-3-methylurea (Example 71)

Example 53 (26 mg, 0.062 mmol) and TEA (35 µL, 0.25 mmol) were dissolved in DCM (0.5 mL) and added dropwise to a cold solution of methylaminoformyl chloride (5.8 mg, 0.062 mmol) in DCM (0.5 mL). The resulting mixture was stirred at RT for 15 min. RM was concentrated under reduced pressure and residue was purified by flash chromatography on a Si cartridge by eluting with 0-100 % DCM/MeOH/NH₄OH (90:9:0.5) in DCM to afford the desired product (3.8 mg).

LCMS (Method 7): Rt = 3.62 min, ES⁺ *m*/*z* 443.2 [M+H]⁺
¹H-NMR (600 MHz, *DMSO-d₆*) δ(ppm) 9.21 (d, J=6.7 Hz, 1H), 9.13 (s, 1H), 8.88 (s, 1H), 8.70 (d, J=4.0 Hz, 1H), 8.21 (s, 1H), 7.44 (d, J=7.9 Hz, 1H), 7.24 (s, 1H), 7.14 (dd, J=6.9, 4.1 Hz, 1H), 7.03 (d, J=7.9 Hz, 1H), 6.54 (br t, J=5.8 Hz, 1H), 5.90 (br d, J=4.3 Hz, 1H), 4.34 (d, J=6.1 Hz, 2H), 3.81 (s, 3H), 2.64 (s, 3H), 2.61 (d, J=4.9 Hz, 3H).

### Example 72

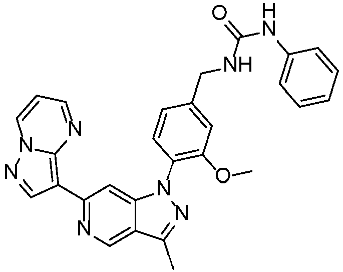

### 1-(3-Methoxy-4-(3-methyl-6-(pyrazolo[1,5-a]pyrimidin-3-yl)-1H-pyrazolo[4,3-c]pyridin-1-yl)benzyl)-3-phenylurea (Example 72)

Example 53 (70.0 mg, 0.11 mmol) was dissolved in DCM/DMF (1 mL) then phenyl isocyanate (16.6 µL, 0.15 mmol) was added. RM was stirred at 50 °C for 1.5 hours. RM was diluted with water (20 mL) and extracted with DCM (3 x 10 mL). Combined organic layers were passed through a phase separator and evaporated to dryness. The residue was purified by flash chromatography on a Si cartridge by eluting with 0-50 % DCM/MeOH/NH₄OH (90:9:0.5) in DCM to afford the title product (21 mg).

LCMS (Method 7): Rt = 4.71 min, ES⁺ *m*/*z* 505.2 [M+H]⁺
¹H-NMR (500 MHz, *DMSO-d₆*) δ(ppm) 9.21 (d, J=7.0 Hz, 1H), 9.14 (s, 1H), 8.88 (s, 1H), 8.65-8.69 (m, 1H), 8.64 (s, 1H), 8.22 (s, 1H), 7.42-7.48 (m, 3H), 7.30 (s, 1H), 7.24 (t, J=7.5 Hz, 2H), 7.14 (dd, J=6.6, 4.4 Hz, 1H), 7.10 (d, J=7.9 Hz, 1H), 6.91 (t, J=7.3 Hz, 1H), 6.76 (t, J=6.0 Hz, 1H), 4.45 (d, J=5.8 Hz, 2H), 3.83 (s, 3H), 2.64 (s, 3H).

### Example 73

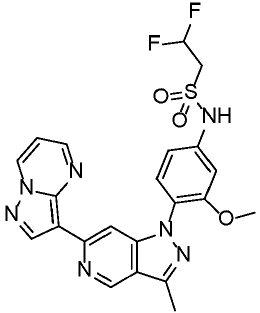

### 2,2-Difluoro-N-(3-methoxy-4-(3-methyl-6-(pyrazolo[1,5-a]pyrimidin-3-yl)-1H-pyrazolo[4,3-c]pyridin-1-yl)phenyl)ethane-1-sulfonamide (Example 73)

To a suspension of example 17 (30 mg, 0.08 mmol) in dry DCM (1 mL), 2,2-difluoroethanesulfonyl chloride (10 µL, 0.10 mmol) and pyridine (20 µL, 0.24 mmol) were added. RM was stirred at RT for 1h. Solvent was evaporated. Residue was dissolved in EtOAc (15 ml) ,washed with water (1x10 mL) and aqueous 2M citric acid (2x 10 mL). The organic extract was dried over Na₂SO₄ and concentrated *in vacuo* to provide crude product that was purified by flash chromatography on a Si cartridge by eluting with 0-50 % DCM/CAN/MeOH (10:10:2) in DCM affording the title product (11 mg).

LCMS (Method 5): Rt = 2.91 min, ES⁺ *m*/*z* 500.1 [M+H]⁺
¹H-NMR (300 MHz, *DMSO-d₆*) δ(ppm): 10.58 (s, 1H), 9.18-9.33 (m, 2H), 8.93 (s, 1H), 8.69-8.77 (m, 1H), 8.24 (s, 1H), 7.49 (d, J=8.5 Hz, 1H), 7.20 (dd, J=7.0, 4.2 Hz, 1H), 7.14 (d, J=1.7 Hz, 1H), 6.99 (dd, J=8.5, 1.7 Hz, 1H), 6.43 (tt, J=54.3, 4.5 Hz, 1H), 4.11 (td, J=14.8, 4.3 Hz, 2H), 3.79 (s, 3H), 2.66 (s, 3H).

### Example 74

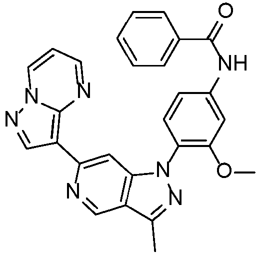

### N-(3-Methoxy-4-(3-methyl-6-(pyrazolo[1,5-a]pyrimidin-3-yl)-1H-pyrazolo[4,3-c]pyridin-1-yl)phenyl)benzamide (Example 74)

Example 17 (33 mg, 0.09 mmol) was dissolved in pyridine (1.0 mL) followed by the addition of benzoyl chloride (10 µL, 0.09 mmol). RM was stirred at RT for 48h. An additional amount of benzoyl chloride (3 µL) was added and stirring continued at RT for 30 min. The formed precipitate was filtered and washed with aqueous 2M citric acid (4x5 mL), water (10 mL) and dried at 45°C for 2h affording the title compound (10.8 mg).

LCMS (Method 5): Rt = 3.27 min, ES⁺ *m*/*z* 476.2 [M+H]⁺
¹H-NMR ( 500 MHz, DMSO) δ(ppm) 10.55 (s, 1H), 9.27 (d, J=6.7 Hz, 2H), 8.98 (s, 1H), 8.77 (d, J=2.8 Hz, 1H), 8.3 (s, 1H), 8.02 (s, 1H), 8.01 (d, J=1.5Hz, 1H), 7.94 (d, J=1.8 Hz, 1H), 7.56-7.66 (m, 4H), 7.52 (d, J=8.3Hz, 1H), 7.21 (dd, J=7.2, 3.5Hz, 1H), 3.83 (s, 3H), 2.69 (s, 3H).

### Example 75

### Step 1

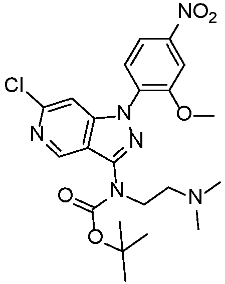

### tert-Butyl (6-chloro-1-(2-methoxy-4-nitrophenyl)-1H-pyrazolo[4,3-c]pyridin-3-yl)(2-(dimethylamino)ethyl)carbamate (intermediate step1-example 75)

Intermediate 13d (1.3 g, 3.33 mmol) in THF (0 mL) was cooled to 0 °C under nitrogen, then LiHMDS (1.3 M in THF, 5.1 mL, 6.65 mmol) was added dropwise. RM was stirred for 15 min then a solution of BoC₂O (1.45 g, 6.65 mmol) in THF (5 mL) was added dropwise. RM was allowed to reach RT, quenched with water (60 ml), and extracted with EtOAc (100 mL). Organic layer was washed with aqueous saturated NaCl (50 mL), dried over Na₂SO₄, and solvent removed under reduced pressure. The residue was purified by to give the title product (1.4 g).

LCMS (Method 2): Rt = 1.37 min, ES⁺ *m*/*z* 491.2/493 [M+H]⁺.

### Step 2

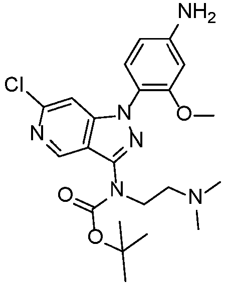

### tert-Butyl (1-(4-amino-2-methoxyphenyl)-6-chloro-1H-pyrazolo[4,3-c]pyridin-3-yl)(2-(dimethylamino)ethyl)carbamate (intermediate step2-example 75)

Intermediate step2-example 75 was prepared similarly to intermediate 10a starting from intermediate step1-example 75 .

LCMS (Method 2): Rt = 1.16, ES⁺ *m*/*z* 461.3/463.3 [M+H]⁺

### Step 3

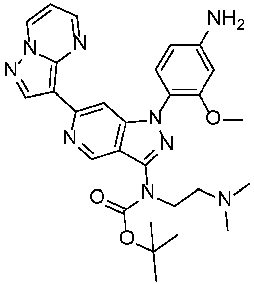

### tert-Butyl (1-(4-amino-2-methoxyphenyl)-6-(pyrazolo[1,5-a]pyrimidin-3-yl)-1H-pyrazolo[4,3-c]pyridin-3-yl)(2-(dimethylamino)ethyl)carbamate (intermediate step3-example 75)

Intermediate step3-example 75 was prepared similarly to example 1 from intermediate step2-example 75.

LCMS (Method 2): Rt = 1.04, ES⁺ *m*/*z* 544.4 [M+H]⁺

### Step 4

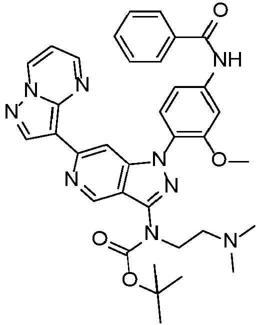

### tert-Butyl (1-(4-benzamido-2-methoxyphenyl)-6-(pyrazolo[1,5-a]pyrimidin-3-yl)-1H-pyrazolo[4,3-c]pyridin-3-yl)(2-(dimethylamino)ethyl)carbamate (intermediate step4-example 75)

Intermediate step3-example 75 was prepared similarly to intermediate 11o from intermediate step3-example 75 and benzoyl chloride.

LCMS (Method 1): Rt = 0.79, ES⁺ *m*/*z* 648.4 [M+H]⁺.

### Step 5

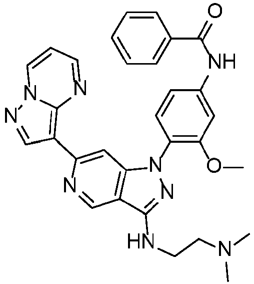

### N-(4-(3-((2-(dimethylamino)ethyl)amino)-6-(pyrazolo[1,5-a]pyrimidin-3-yl)-1H-pyrazolo[4,3-c]pyridin-1-yl)-3-methoxyphenyl)benzamide (Example 75)

Intermediate step4-example 75 (40 mg, 0.06 mmol) was dissolved in DCM (2 mL) then TFA (159 µL, 2.08 mmol) added and RM stirred at RT overnight. RM was diluted with DCM (8 mL) and washed with aqueous saturated NaHCO₃ (2x10 mL) and aqueous saturated NaCl (10 mL). Organic layer was concentrated and the residue purified by flash chromatography on a Si cartridge by eluting with 0-100 % DCM/MeOH/NH₄OH (90:9:0.5) in DCM affording the desired product (20 mg).

LCMS (Method 7): Rt = 3.85 min, ES⁺ *m*/*z* 548.4 [M+H]⁺
¹H-NMR (500 MHz, *DMSO-d₆*) δ(ppm) 10.47 (s, 1H), 9.20 (dd, J=6.6, 1.5 Hz, 1H), 9.08 (s, 1H), 8.86 (s, 1H), 8.70 (dd, J=4.1, 1.3 Hz, 1H), 8.09 (s, 1H), 7.99 - 8.04 (m, 2H), 7.88 (d, J=1.2 Hz, 1H), 7.60 - 7.65 (m, 1H), 7.54-7.60 (m, 3H), 7.45 (d, J=8.2 Hz, 1H), 7.14 (dd, J=6.6, 3.9 Hz, 1H), 6.60- 6.67 (m, 1H), 3.86 (s, 3H), 3.40-3.47 (m, 2H, overlapped with HDO), 2.58 - 2.69 (m, 2H, overlapped with DMSO), 2.28 (s, 6H).

### Example 76

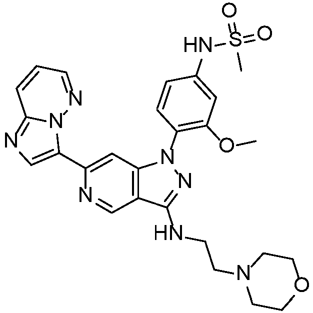

### N-(4-(6-(Imidazo[1,2-b]pyridazin-3-yl)-3-((2-morpholinoethyl)amino)-1H-pyrazolo[4,3-c]pyridin-1-yl)-3-methoxyphenyl)methanesulfonamide (Example 76)

A vial was charged with intermediate 17e (65 mg, 0.14 mmol), Pd(PPh₃)₂Cl₂ (9.9 mg, 0.014 mmol) and DMF (1.5 mL) was degassed under nitrogen for 15 minutes, then intermediate 8 (110 mg, 0.27 mmol) was added. The vial was sealed, evacuated/backfilled with nitrogen for 2 times and stirred at 110 °C overnight. After cooling to RT, RM was partitioned between EtOAc (15 mL)/water (10 mL) and organic layer washed with water (2x10 mL). Combined aqueous layers where neutralized to pH 8 and extracted with EtOAc (3x15 mL). Combined organic layer was evaporated under reduced pressure. Crude product was purified by flash chromatography on Si cartridge by eluting with 0-75% DCM/MeOH/NH₄OH (90:9:0.5) in DCM affording the title compound (41 mg)

LCMS (Method 7): Rt = 3.35 min, ES⁺ *m*/*z* 564.12 [M+H]⁺
¹H-NMR (400 MHz, *DMSO-d₆*) δ(ppm) 9.99 (s, 1H), 9.12 (d, J=1.1Hz, 1H), 8.73 (dd, J=1.7, 4.5Hz, 1H), 8.50 (s, 1H), 8.31 (d, J=1.1Hz, 1H), 8.26 (dd, J=1.7, 9.2Hz, 1H), 7.44 (d, J=8.4 Hz, 1H), 7.35 - 7.29 (m, 1H), 7.11 (d, J=2.3Hz, 1H), 6.95 (dd, J=2.3, 8.5Hz, 1H), 6.74 (t, J=5.6Hz, 1H), 3.83 (s, 3H), 3.64 - 3.55 (m, 4H), 3.49 - 3.38 (m, 2H), 3.11 (s, 3H), 2.62 (t, J=6.8 Hz, 2H), 2.45 (br s, 4H)

### Example 77

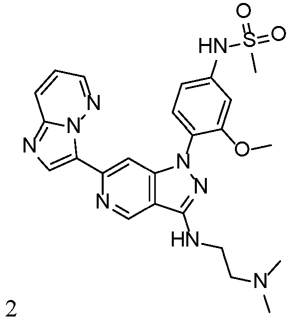

### N-(4-(3-((2-(Dimethylamino)ethyl)amino)-6-(imidazo[1,2-b]pyridazin-3-yl)-1H-pyrazolo[4,3-c]pyridin-1-yl)-3-methoxyphenyl)methanesulfonamide (Example 77)

Example 77 was prepared similarly to example 76 starting from intermediate 17n and intermediate 8.

LCMS (Method 7): Rt = 3.66 min, ES⁺ *m*/*z* 522.2 [M+H]⁺
¹H-NMR (500 MHz, *DMSO-d₆*) δ(ppm): 9.15 (d, J=0.9 Hz, 1H), 8.74 (dd, J=1.4, 4.4 Hz, 1H), 8.51 (s, 1H), 8.32 (s, 1H), 8.29 - 8.22 (m, 2H), 7.45 (d, J=8.5Hz, 1H), 7.33 (dd, J=4.6, 9.2Hz, 1H), 7.12 (d, J=2.1Hz, 1H), 6.96 (dd, J=2.3, 8.4 Hz, 1H), 6.72 (t, J=5.8 Hz, 1H), 3.84 (s, 3H), 3.44 - 3.39 (m, 2H), 3.12 (s, 3H), 2.60 - 2.54 (m, 2H), 2.23 (s, 6H)

### Example 78

### Step 1

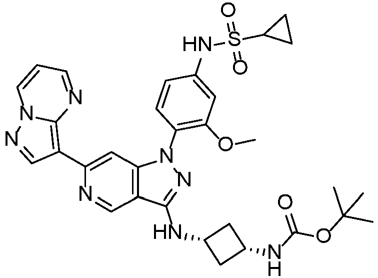

### tert-Butyl ((1s,3s)-3-((1-(4-(cyclopropanesulfonamido)-2-methoxyphenyl)-6-(pyrazolo[1,5-a]pyrimidin-3-yl)-1H-pyrazolo[4,3-c]pyridin-3-yl)amino)cyclobutyl)carbamate (intermediate stepl-example78)

Intermediate step1-example78 was prepared similarly to example 1 starting from intermediate 17v.

LCMS (Method 2): Rt = 0.74, ES⁺ *m*/*z* 646.5 [M+H]⁺

### Step 2

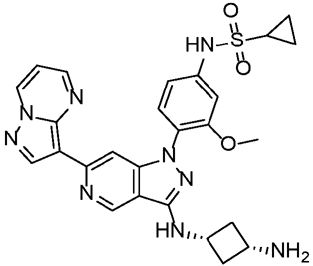

### N-(4-(3-(((1s,3s)-3-Aminocyclobutyl)amino)-6-(pyrazolo[1,5-a]pyrimidin-3-yl)-1H-pyrazolo[4,3-c]pyridin-1-yl)-3 methoxyphenyl)cyclopropanesulfonamide(intermediate step2-example78)

Intermediate step1-example78 (100 mg, 0.15 mmol) in 1,4-dioxane (1 mL) was added with a solution of HCl in dioxane (4.0 M, 194 µL, 0.77 mmol) and stirred at RT overnight. The precipitate formed was filtered, washed with 1,4-dioxane and dried to afford the desired product (55 mg) that was used in the next steps further purifications.

LCMS (Method 2): Rt = 0.54 min, ES⁺ *m*/*z* 546.3 [M+H]⁺.

### Step 3

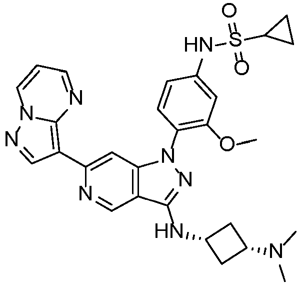

### N-(4-(3-(((1s,3s)-3-(Dimethylamino)cyclobutyl)amino)-6-(pyrazolo[1,5-a]pyrimidin-3-yl)-1H-pyrazolo[4,3-c]pyridin-1-yl)-3-methoxyphenyl)cyclopropanesulfonamide (Example 78)

A vial charged with intermediate step2-example78 (30 mg, 0.06 mmol), formic acid (83 µL, 2.2 mmol) and formaldehyde (37 % solution in water, 164 µL, 2.2 mmol) was stirred at RT overnight. A further amount of formaldehyde (10 eq) was added and stirring proceeded for further 4h. EtOAc (15 mL) was added and resulting solution was washed with aqueous saturated NaHCO₃ (3x15 mL) and aqueous saturated NaCl (1x15 mL). Organic phase was evaporated under reduced pressure and the residue chromatographed on a Si cartridge by eluting with 0-80 % DCM/MeOH/NH₄OH (90:9:0.5) in DCM, affording the title product (8 mg).

LCMS (Method 7): Rt = 3.46 min, ES⁺ *m*/*z* 574.2 [M+H]⁺
¹H-NMR (600 MHz, *DMSO-d₆*) δ(ppm) 9.99 (s, 1H), 9.21 (dd, J=6.8, 1.6Hz, 1H), 9.05 (d, J=0.9 Hz, 1H), 8.85 (s, 1H), 8.72 (dd, J=4.0, 1.9 Hz, 1H), 8.07 (d, J=0.7 Hz, 1H), 7.40 (d, J=8.6Hz, 1H), 7.11 - 7.14 (m, 2H), 6.97 (dd, J=8.5, 2.4 Hz, 1H), 6.91 (d, J=7.6Hz, 1H), 3.83 - 3.92 (m, 1H), 3.81 (s, 3H), 2.75 - 2.81 (m, 1H), 2.51 - 2.56 (m, 2H, overlapped with dmso), 2.33 - 2.39 (m, 1H), 2.06 (s, 6H), 1.74 - 1.83 (m, 2H), 0.99 - 1.05 (m, 4H).

### Example 79

### Step 1

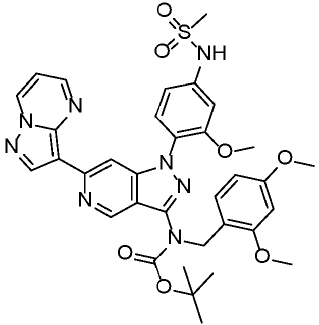

### tert-Butyl (2,4-dimethoxybenzyl)(1-(2-methoxy-4-(methylsulfonamido)phenyl)-6-(pyrazolo[1,5-a]pyrimidin-3-yl)-1H-pyrazolo[4,3-c]pyridin-3-yl)carbamate (intermediate step1-example79)

Intermediate step1-example79 was prepared similarly to example 1 starting from intermediate 17b.

LCMS (Method 2): Rt = 0.80, ES⁺ *m*/*z* 701.3 [M+H]⁺

### Step 2

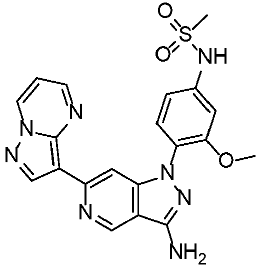

### N-(4-(3-Amino-6-(pyrazolo[1,5-a]pyrimidin-3-yl)-1H-pyrazolo[4,3-c]pyridin-1-yl)-3-methoxyphenyl)methanesulfonamide (Example79

TFA (200 µL, 2.69 mmol) was added to a solution of intermediate step1-example79 (30.0 mg, 0.04 mmol) in DCM (10 mL), and RM stirred at RT for 1h. RM was dried under reduced pressure and the residue partitioned in EtOAc/sat NaHCO₃. Organic layer was washed with aqueous saturated NaHCO₃ (2x5 mL) and aqueous saturated NaCl (5 mL), dried and concentrated *in vacuo* to give a crude that after trituration with DCM/MeOH afforded the title product (8.5 mg).

LCMS (Method 3): Rt = 0.83 min, ES⁺ *m*/*z* 451.27 [M+H]⁺
¹H-NMR (*DMSO-d₆,* 300 MHz): δ (ppm) 9.98 (s, 1H), 9.19 (dd, J=7.0, 1.7 Hz, 1H), 9.02 (d, J=1.0 Hz, 1H), 8.84 (s, 1H), 8.70 (dd, J=4.0, 1.7 Hz, 1H), 8.06 (d, J=1.0 Hz, 1H), 7.39 (d, J=8.5 Hz, 1H), 7.13 (dd, J=7.1, 4.1 Hz, 1H), 7.09 (d, J=2.3 Hz, 1H), 6.93 (dd, J=8.5, 2.3 Hz, 1H), 6.06 (s, 2H), 3.80 (s, 3H), 3.11 (s, 3H)

### Example 80

### Step 1

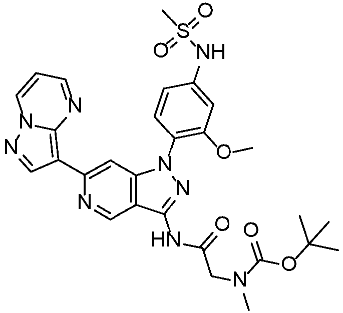

### tert-Butyl (2-((1-(2-methoxy-4-(methylsulfonamido)phenyl)-6-(pyrazolo[1,5-a]pyrimidin-3-yl)-1H-pyrazolo[4,3-c]pyridin-3-yl)amino)-2-oxoethyl)(methyl)carbamate (Intermediate step1-example 80)

Example 80 was prepared similarly to Example 1 starting from intermediate 17ad.

LCMS (Method 1): Rt = 0.80 min, ES⁺ *m*/*z* 622.3 [M+H]⁺

### Step 2

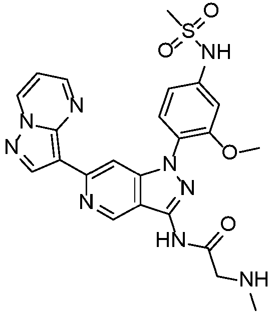

### N-(1-(2-methoxy-4-(methylsulfonamido)phenyl)-6-(pyrazolo[1,5-a]pyrimidin-3-yl)-1H-pyrazolo[4,3-c]pyridin-3-yl)-2-(methylamino)acetamide (Example 80)

Intermediate step1-example 80 (8.00 mg, 0.01 mmol) was dissolved in DCM (3 mL), TFA (60 µL, 0.81 mmol) was added and RM stirred at RT for 1h. RM was partitioned among DCM and aqueous saturated NaHCO₃. Organic phase was washed with aqueous saturated NaHCO₃ (2x5 mL) and aqueous saturated NaCl (5 mL), passed through a phase separator, concentrated and triturated with DCM to afford the title product (6.0 mg).

LCMS (Method 3): Rt = 0.70 min, ES⁺ *m*/*z* 522.2 [M+H]⁺
¹H-NMR (500 MHz, *DMSO-d₆*) δ (ppm) 9.36 (s, 1H), 9.20 (dd, J=7.0, 1.5 Hz, 1H), 8.87 (s, 1H), 8.74 (dd, J=4.0, 1.5 Hz, 1H), 8.11 (d, J=0.9 Hz, 1H), 7.13 (dd, J=7.0, 4.0 Hz, 1H), 7.09 - 7.01 (m, 1H), 6.69 (br s, 1H), 6.63 - 6.57 (m, 1H), 3.64 (s, 3H), 2.70 (br s, 3H), 2.37 (s, 3H) (CH₂ peak overlaps with HDO)

### Example 81

### Step 1

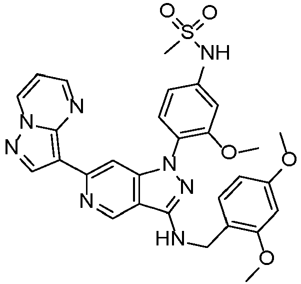

### N-(4-(3-((2,4-Dimethoxybenzyl)amino)-6-(pyrazolo[1,5-a]pyrimidin-3-yl)-1H-pyrazolo[4,3-c]pyridin-1-yl)-3-methoxyphenyl)methanesulfonamide (Intermediate steel-example 81

Intermediate step1-example 81was prepared similarly to example 1 starting from intermediate 171.

LCMS (Method 2): Rt = 0.68 min, ES⁺ *m*/*z* 601.2 [M+H]⁺

### Step 2

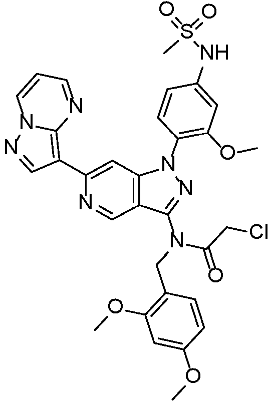

### 2-Chloro-N-(2,4-dimethoxybenzyl)-N-(1-(2-methoxy-4-(methylsulfonamido)phenyl)-6-(pyrazolo[1,5-a]pyrimidin-3-yl)-1H-pyrazolo[4,3-c]pyridin-3-yl)acetamide (Intermediate step2-example 81)

Intermediate step2-example 81 (50 mg, 0.083 mmol) was suspended in THF (1 mL) followed by addition of TEA (17.4 µL, 0.125 mmol) and 2-chloroacetyl chloride (8 µL, 0.1 mmol). RM was stirred for 45 min at RT, then partitioned among EtOAc and water (20 mL each). Aqueous layer was extracted with EtOAc (5×20 mL) and combined organic layers were washed with aqueous saturated NaCl, dried over Na₂SO₄ and evaporated under reduced pressure to give the title product (146 mg) that was used in the next steps without further purifications.

LCMS (Method 2): Rt = 0.65 min, ES⁺ *m*/*z* 677.3 [M+H]⁺

### Step 3

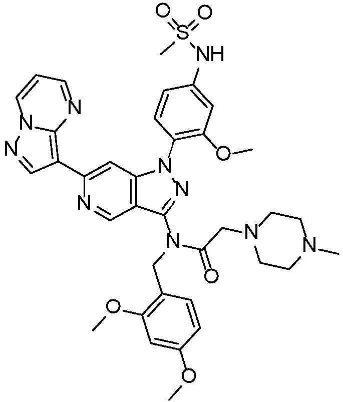

### N-(2,4-Dimethoxybenzyl)-N-(1-(2-methoxy-4-(methylsulfonamido)phenyl)-6-(pyrazolo[1,5-a]pyrimidin-3-yl)-1H-pyrazolo[4,3-c]pyridin-3-yl)-2-(4-methylpiperazin-1-yl)acetamide (Intermediate step3-example 81)

Intermediate step2-example 81 (57mg, 0.08 mmol) was dissolved in DMF (0.6 mL), then1-Methylpiperazine (19 µL, 0.17 mmol) added and RM stirred at 80 °C for 1h. RM was quenched with aqueous saturated NaHCO₃ and extracted with EtOAc (5x25 mL). Combined organic layers were washed with aqueous saturated NaCl, dried over Na₂SO₄ and evaporated under reduced pressure to give the desired product that was used in the next synthetic steps without further purifications.

LCMS (Method 2): Rt = 0.59, ES⁺ *m*/*z* 741.5 [M+H]⁺.

### Step 4

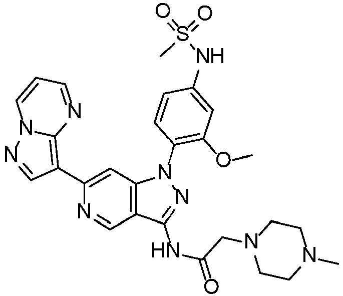

### N-(1-(2-Methoxy-4-(methylsulfonamido)phenyl)-6-(pyrazolo[1,5-a]pyrimidin-3-yl)-1H-pyrazolo[4,3-c]pyridin-3-yl)-2-(4-methylpiperazin-1-yl)acetamide (Example 81)

Intermediate step3-example 81 was dissolved in TFA (2 mL) and stirred at 60 °C overnight. RM was evaporated under reduced pressure and the residue purified by flash chromatography on a Si cartridge by eluting with 0-100 % DCM/MeOH/NH₄OH (90:9:0.5) in DCM to yield the title product (12 mg).

LCMS (Method 3): Rt = 0.76 min, ES⁺ *m*/*z* 591.2 [M+H]⁺
¹H-NMR (500 MHz, *DMSO-d₆,* 353 K) δ(ppm) 10.26 (bs, 1H), 9.38 (d, J=1.1 Hz, 1H), 9.12 (dd, J=7.1, 1.7 Hz, 1H), 8.86 (s, 1H), 8.68 (dd, J=4.0, 1.7 Hz, 1H), 8.15 (d, J=1.1 Hz, 1H), 7.45 (d, J=8.5 Hz), 7.17 (d, J=2.1 Hz, 1H), 7.11 (dd, J=7.1, 4.0 Hz, 1H), 7.03 (dd, J=8.5, 2.2 Hz, 1H), 3.81 (s, 3H), 3.31 (s, 2H), 3.13 (s, 3H), 2.66 (t, J=4.9 Hz, 4H), 2.43 (t, J=4.9 Hz, 4H), 2.21 (s, 3H).

### Example 82

### Step 1

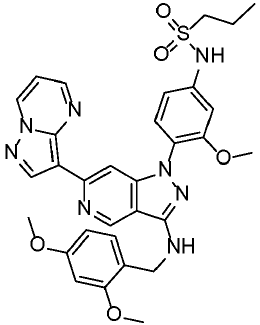

### N-(4-(3-((2,4-Dimethoxybenzyl)amino)-6-(pyrazolo[1,5-a]pyrimidin-3-yl)-1H-pyrazolo[4,3-c]pyridin-1-yl)-3-methoxyphenyl)propane-1-sulfonamide (Intermediate step1-example 82)

Intermediate step1-example 82 was prepared similarly to example 1 starting from intermediate 17m.

LCMS (Method 2): Rt = 0.82 min, ES⁺ *m*/*z* 629.2 [M+H]⁺

### Step 2

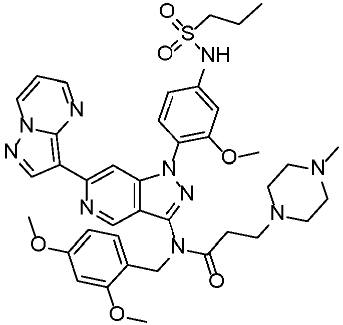

### N-(2,4-Dimethoxybenzyl)-N-(1-(2-methoxy-4-(propylsulfonamido)phenyl)-6-(pyrazolo[1,5-a]pyrimidin-3-yl)-1H-pyrazolo[4,3-c]pyridin-3-yl)-3-(4-methylpiperazin-1-yl)propanamide (Intermediate step2-example 82)

Intermediate step1-example 82 (100 mg, 0.18 mmol) was dissolved in dry DMF (1mL), added with TEA (77 µL, 0.55 mmol) and RM cooled to 0 °C prior the addition of 3-Chloropropanoyl chloride (35 µL, 0.54 mmol). RM was stirred at RT for 3h then quenched with aqueous saturated NaHCO₃ and extracted with EtOAc. Organic phase was evaporated under reduced pressure. The residue was dissolved in DMF and 1-methylpiperazine (60 µL, 0.54 mmol) added. RM was stirred at 80 °C for 3h, then quenched with aqueous saturated NaHCO₃ and extracted with EtOAc. Organic phase was washed with aqueous saturated NaHCO₃, dried over Na₂SO₄ and solvent removed under reduced pressure. The residue was purified by flash chromatography on a Si cartridge by eluting with 0-100% DCM/MeOH/NH₄OH (90:9:1.5) in DCM to afford the title product (50 mg).

LCMS (Method 2): Rt = 0.70 min, ES⁺ *m*/*z* 783.3 [M+H]⁺.

### Step 3

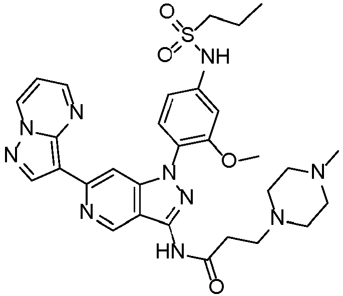

### N-(1-(2-Methoxy-4-(propylsulfonamido)phenyl)-6-(pyrazolo[1,5-a]pyrimidin-3-yl)-1H-pyrazolo[4,3-c]yridin-3-yl)-3-(4-methylpiperazin-1-yl)propanamide

### (Example 82)

Example 82 was synthesized similarly to example 81-step 3 starting from intermediate step2-example 82.

LCMS (Method 7): Rt = 3.72 min, ES⁺ *m*/*z* 633.3 [M+H]⁺
¹H-NMR (*DMSO-d₆,* 300 MHz): δ (ppm) 11.04 (s, 1H), 10.14 (br s, 1H), 9.33 (s, 1H), 9.19-9.24 (m, 1H), 8.87 (s, 1H), 8.69 (dd, J=4.1, 1.7 Hz, 1H), 8.13 (s, 1H), 7.43 (s, 1H), 7.08-7.19 (m, 2H), 6.98 (dd, J=8.4, 2.2 Hz, 1H), 3.77 (s, 3H), 3.18-3.27 (m, 2H), 2.56-2.77 (m, 6H), 2.54 (m, 2H, overlapped with DMSO), 2.22-2.38 (m, 4H), 1.67-1.83 (m, 2H), 0.99 (t, J=7.5 Hz, 3H).

### Example 83

### Step 1

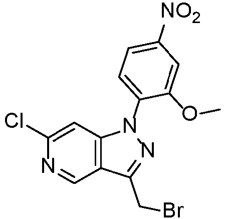

### 3-(Bromomethyl)-6-chloro-1-(2-methoxy-4-nitrophenyl)-1H-pyrazolo[4,3-clpyridine (Intermediate steel-example 83)

A round-bottom-flask was charged under argon with intermediate 9a (2.5 g, 7.84 mmol) and trifluorotoluene (35 mL). NBS (1.67 g, 9.4 mmol) and AIBN (258 mg, 1.57 mmol) were added and RM stirred at 85 °C for 2.5h. A second equivalente of NBS and AIBN was added and RM further stirred at 85 °C for 8 hours After cooling to RT, RM was diluted with EtOAc and washed with aqueous saturated NaHCO₃ (3x) and aqueous saturated NaCl. The organic layer was dried over Na₂SO₄ and concentrated in vacuo. The crude material was purified by flash chromatography on a Si cartridge by eluting with 0-100% DCM in cyclohexane to provide the desired compound (810 mg).

LCMS (Method 1): Rt = 1.20 min, ES⁺ *m*/*z* 396.9/398.9/400.8 [M+H]⁺.

### Step 2

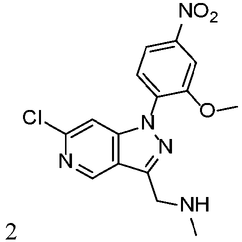

### 1-(6-Chloro-1-(2-methoxy-4-nitrophenyl)-1H-pyrazolo[4,3-c]pyridin-3-yl)-N-methylmethanamine (Intermediate step2-example 83)

A solution of intermediate step1-example 83 (649 mg, 1.63 mmol) in THF (16.2 mL) was added dropwise to a solution of methylamine in THF (2.0 M, 13.1 mL, 26.1 mol). RM was stirred at RT for 1h, then dried under reduced pressure. The residue was partitioned between EtOAc and aqueous saturated NaHCO₃. Organic phase was dried over Na₂SO₄ and evaporated *in vacuo.* The crude product was purified by flash chromatography on silica by eluting with DCM/MeOH 10:1 to afford the title product.

LCMS (Method 2): Rt = 0.97 min, ES⁺ *m*/*z* 348.1/350.1 [M+H]⁺

### Step 3

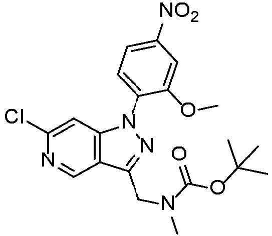

### tert-Butyl ((6-chloro-1-(2-methoxy-4-nitrophenyl)-1H-pyrazolo[4,3-c]pyridin-3-yl)methyl)(methyl)carbamate (Intermediate step3-example 83)

Boc₂O (418 mg, 1.92 mmol) was added to a solution of intermediate step2-example 83 (606 mg, 1.74 mmol) and TEA (680 mg, 4.88 mmol) in DCM (8.2 mL). RM stirred at RT for 1.5 h, then partitioned between DCM and aqueous saturated NaHCO₃. Organic phase was washed with aqueous saturated NaCl, dried over Na₂SO₄ and solvent removed under reduced pressure. The residue was purified by flash chromatography by eluting with DCM/MeOH 30:1 to 20:1 to afford the title product (774 mg).

LCMS (Method 2): Rt = 1.32 min, ES⁺ *m*/*z* 448.1/450.1 [M+H]⁺

### Step 4

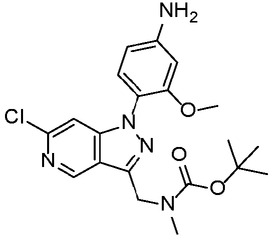

### tert-Butyl ((1-(4-amino-2-methoxyphenyl)-6-chloro-1H-pyrazolo[4,3-c]pyridin-3-yl)methyl)(methyl)carbamate (Intermediate step4-example 83)

Intermediate step4-example 83 was prepared similarly to intermediate 10c starting from intermediate step3-example 83.

LCMS (Method 2): Rt = 1.12 min, ES⁺ *m*/*z* 418.2/420.2 [M+H]⁺

### Step 5

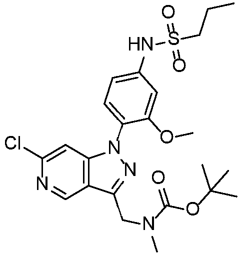

### tert-Butyl ((6-chloro-1-(2-methoxy-4-(propylsulfonamido)phenyl)-1H-pyrazolo[4,3-c]pyridin-3-yl)methyl)(methyl)carbamate (Intermediate step5-example 83)

Intermediate step5-example 83 was prepared similarly to intermediate 16a starting from intermediate step4-example 83 and propanesulfonyl chloride.

LCMS (Method 2): Rt = 0.86 min, ES⁺ *m*/*z* 524.2/526.2 [M+H]⁺

### Step 6

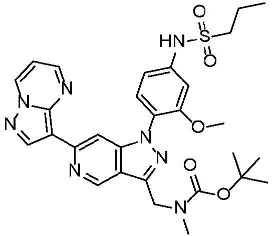

### tert-Butyl ((1-(2-methoxy-4-(propylsulfonamido)phenyl)-6-(pyrazolo[1,5-a]pyrimidin-3-yl)-1H-pyrazolo[4,3-c]pyridin-3-yl)methyl)(methyl)carbamate (Intermediate step6-example 83)

Intermediate step6-example 83 was prepared similarly to example 1 starting from Intermediate step5-example 83.

LCMS (Method 2): Rt = 0.79 min, ES⁺ *m*/*z* 607.3 [M+H]⁺

### Step 7

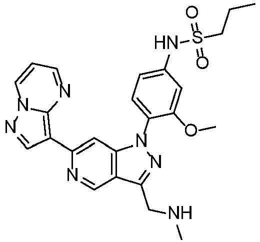

### N-(3-Methoxy-4-(3-((methylamino)methyl)-6-(pyrazolo[1,5-a]pyrimidin-3-yl)-1H-pyrazolo[4,3-c]pyridin-1-yl)phenyl)propane-1-sulfonamide (Example 83)

A solution of intermediate step6-example 83 (83 mg, 0.137, mmol) in DCM (1 mL) was treated with TFA (508 µL, 6.84 mmol). RM was stirred at RT for 40 min. RM was dried under reduced pressure and the residue purified by flash chromatography on silica to afford the title product (59 mg).

LCMS (Method 7): Rt = 3.65 min, ES⁺ *m*/*z* 507.7 [M+H]⁺
¹H-NMR (500 MHz, *DMSO-d₆*) δ (ppm) 9.26 - 9.30 (m, 1H), 9.22 (dd, J=6.87, 1.68 Hz, 1H), 8.88 (s, 1H), 8.68 -8.74 (m, 1H), 8.21 (d, J=1.22 Hz, 1H), 7.44 (d, J=8.54 Hz, 1H), 7.10 - 7.20 (m, 2H), 6.98 (dd, J=8.54, 2.14 Hz, 1H), 4.09 (s, 2H), 3.77 (s, 3H), 3.18 - 3.25 (m, 2H), 2.37 (s, 3H), 1.69 - 1.84 (m, 2H), 1.00 (t, J=7.48 Hz, 3H).

### Example 84

### Step 1

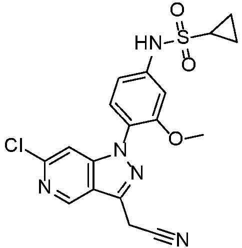

### N-(4-(6-Chloro-3-(cyanomethyl)-1H-pyrazolo[4,3-c]pyridin-1-yl)-3-methoxyphenyl)cyclopropanesulfonamide (Intermediate step1-example84 )

A mixture of intermediate 16e (100 mg, 0.198 mmol), 4-(4,4,5,5-tetramethyl-1,3,2-dioxaborolan-2-yl)isoxazole (38.6 mg, 0.198 mmol), Pd(dppf)Cl₂.CH₂Cl₂ (24.3 mg, 0.0297 mmol) and Na₂CO₃ (42 mg, 0.396 mmol) in 1,4-Dioxane (1.22 mL) / water (610µL) was purged with argon and RM stirred at 50 °C for 1.5 h. A further equivalent of of 4-(4,4,5,5-tetramethyl-1,3,2-dioxaborolan-2-yl)isoxazole and Pd(dppf)Cl₂.CH₂Cl₂ were added and RM heated at 70 °C overnight . KF (34.5 mg, 0.594 mmol) was added to RM and stirred at 100 °C overnight. RM was passed through pad of Celite^{®} and solvent evaporated. The residue was dissolved with EtOAc (20 mL) and washed with aqueous saturated NaHCO₃ (3x15 mL) and aqueous saturated NaCl (15 mL). Organic layer was passed through phase separator and solvent evaporated in vacuo. The crude was purified by flash chromatography on a Si cartridge by eluting with 0-50% EtOAc in DCM to yield the title product (38 mg).

LCMS (Method 2): Rt = 0.57 min, ES⁻ *m*/*z* 416.1/418.1 [M-H]⁻

### Step 2

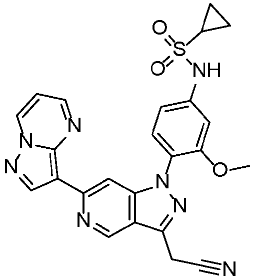

### N-(4-(3-(Cyanomethyl)-6-(pyrazolo[1,5-a]pyrimidin-3-yl)-1H-pyrazolo[4,3-c]pyridin-1-yl)-3-methoxyphenyl)cyclopropanesulfonamide (Example 84)

Example 84 was prepared similarly to example 1 starting from intermediate step1-example 84.

LCMS (Method 7): Rt = 4.47 min, ES⁺ *m*/*z* 501.1 [M+H]⁺
¹H-NMR (300 MHz, *DMSO-d₆*) δ(ppm) 10.16 (s, 1H), 9.16-9.29 (m, 2H), 8.90 (s, 1H), 8.64-8.77 (m, 1H), 8.23 (s, 1H), 7.49 (d, J=8.5 Hz, 1H), 7.11-7.23 (m, 2H), 7.04 (m, 1H), 4.61 (s, 2H), 3.77 (s, 3H), 2.83 (quin, J=6.3 Hz, 1H), 1.04 (d, J=6.3 Hz, 4H).

### Example 85

### Step 1

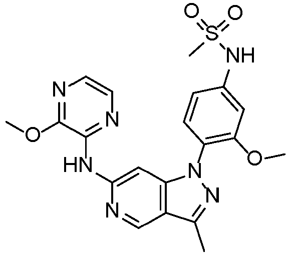

### N-(3-Methoxy-4-(6-((3-methoxypyrazin-2-yl)amino)-3-methyl-1H-pyrazolo[4,3-c]pyridin-1-yl)phenyl)methanesulfonamide (Intermediate step1-example 85)

A vial was charged with intermediate 11a (150 mg, 0.41 mmol), 2-amino-3-methoxypyrazine (67 mg, 0.53 mmol), sodium t-butoxide (59 mg, 0.61 mmol) and XPhos-Pd-G3 (17 mg, 0.02 mmol), then the vessel sealed and backfilled/evacuated with nitrogen (x 3). Previously degassed 1,4-dioxane ( 2 mL) was added via syringe and RM was stirred at 100 °C for 4h. After cooling to RT, the RM was partitioned between EtOAc (10 mL) and water (7 mL). Aqueous layer was extracted with a mixture DCM/i-PrOH (1: 1, 3x10 mL). Combined organics were dried over Na₂SO₄ and solvent removed under reduce pressure. The residue was purified by flash chromatography on a Si cartridge eluting with 0-50 % MeOH/DCM in DCM to afford the title product (180 mg).

LCMS (Method 2): Rt = 0.61, ES⁺ *m*/*z* 456.2 [M+H]⁺

### Step 2

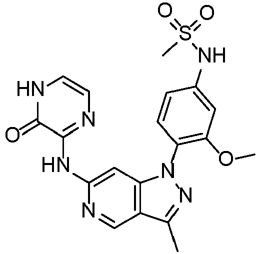

### N-(3-Methoxy-4-(3-methyl-6-((3-oxo-3,4-dihydropyrazin-2-yl)amino)-1H-pyrazolo[4,3-c]pyridin-1-yl)phenyl)methanesulfonamide (Example 85)

Intermediate step1-example 85 (180 mg, 0.39 mmol) was suspended in acetonitrile (6.5 mL), added with TMS-Cl (150 µL, 1.19 mmol) and NaI (178 mg, 1.19 mmol). RM was stirred in a sealed vial at 85 °C for 30 min. RM was allowed to cool to RT and the formed precipitate collected. Solids were taken in DCM (25 mL) and washed with aqueous 10 % Na₂SO₃ (10 mL) and aqueous saturated NaCl (10 mL). Organic phase was dried over MgSO₄ and evaporated under reduced pressure. The residue was purified by flash chromatography on a Si cartridge by eluting with 0-100 % DCM/MeOH/NH₄OH (90:9:0.5) in DCM to afford the title compound (48 mg).

LCMS (Method 5): Rt = 2.55 min, ES⁺ *m*/*z* 442.64 [M+H]⁺
¹H-NMR (500 MHz, *DMSO-d₆*) δ = 12.22 (br s, 1H), 10.10 (s, 1H), 8.89 (s, 1H), 8.91 (brs, 1H), 8.10 (s, 1H), 7.42 (d, J=8.5 Hz, 1H), 7.11 (d, J=2.1 Hz, 1H), 6.98 - 6.94 (m, 2H), 6.91 - 6.88 (m, 1H), 3.77 (s, 3H), 3.14 (s, 3H), 2.59 (s, 3H).

### Example 86 to 92

The following Examples were prepared with a two step process similarly to example 85 from the indicated starting materials.

| Example | Structure/Name | *Step 1* | | *Step 2* | |
|---|---|---|---|---|---|
| | | Starting Material | Name of intermediate Step 1 / LCMS | ¹H-NMR | LCMS |
| 86 | 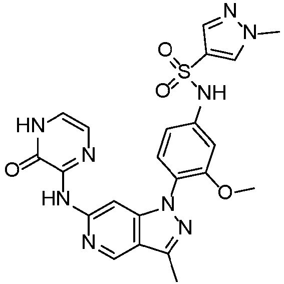 *N*-(3-methoxy-4-(3-methyl-6-((3-oxo-3,4-dihydropyrazin-2-yl)amino)-1*H*-pyrazolo[4,3-*c*]pyridin-1-yl)phenyl)-1-methyl-1H-pyrazole-4-sulfonamide | Intermediate 11j | N-(3-methoxy-4-(6-((3-methoxypyrazin-2-yl)amino)-3-methyl-1H-pyrazolo[4,3-c]pyridin-1-yl)phenyl)-1-methyl-1H-pyrazole-4-sulfonamide | (600 MHz, *DMSO-d₆*) δ(ppm) 12.26 (br d, J=0.55 Hz, 1H), 10.51 (s, 1H), 8.98 - 9.41 (m, 1H), 8.91 (s, 1H), 8.38 (s, 1H), 8.01 (s, 1H), 7.81 (s, 1H), 7.35 (d, J=8.44 Hz, 1H), 7.07 (d, J=2.02 Hz, 1H), 6.91 - 6.99 (m, 2H), 6.89 (dd, J=8.44, 2.20 Hz, 1H), 3.87 (s, 3H), 3.74 (s, 3H), 2.58 (s, 3H) | Method 7), Rt = 3.99 min, ES⁺ *m*/*z* 508.1 [M+H]⁺ |
| | | | (Method 1), Rt = 0.79 min, ES⁺ *m*/*z* 522.2 [M+H]⁺ | | |
| 87 | 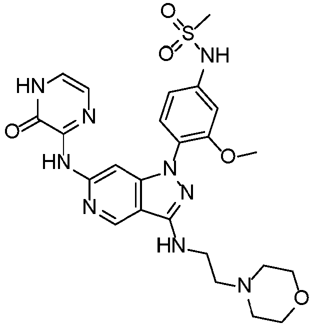 *N*-(3-methoxy-4-(3-((2-morpholinoethyl)amino)-6-((3-oxo-3,4-dihydropyrazin-2-yl)amino)-1*H*-pyrazolo[4,3-*c*]pyridin-1-yl)phenyl)methanesulfonamide | Intermediate 17e | N-(3-methoxy-4-(6-((3-methoxypyrazin-2-yl)amino)-3-((2-morpholinoethyl)amino)-1H-pyrazolo[4,3-c]pyridin-1-yl)phenyl)methanesulfon amide | (600 MHz, *DMSO-d₆*) δ/ppm: 12.16 (bs, 1H), 9.97 (bs, 1H), 8.77 (d, J=1.0 Hz, 1H), 8.65 (s, 1H), 7.98 (d, J=1.0 Hz, 1H), 7.39 (d, J=8.5 Hz, 1H), 7.08 (d, J=2.3 Hz, 1H), 6.95 - 6.92 (m, 2H), 6.87 (bs, 1H), 6.61 (t, J=5.5 Hz, 1H), 3.79 (s, 3H), 3.59 (t, J=4.6 Hz, 4H), 3.39 (q, J=6.5 Hz, 2H), 3.10 (s, 3H), 2.59 (t, J=6.5 Hz, 2H), 2.44 (bs, 4H). | (Method 7): Rt = 3. 10 min, ES⁺ *m*/*z* 556.1 [M+H]⁺ |
| | | | (Method 2): Rt = 0.54 min, ES⁺ *m*/*z* 570.3 [M+H]⁺ | | |
| 88 | 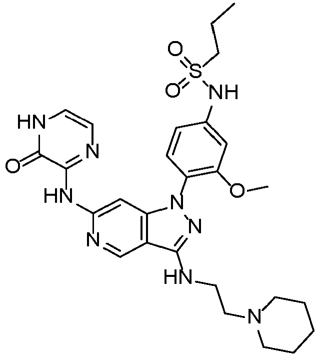 *N*-(3-methoxy-4-(6-((3-oxo-3,4-dihydropyrazin-2-yl)amino)-3-((2-(piperidin-1-yl)ethyl)amino)-1*H*-pyrazolo[4,3-*c*]pyridin-1-yl)phenyl)propane-1-sulfonamide | Intermediate 17x | N-(3-methoxy-4-(6-((3-methoxypyrazin-2-yl)amino)-3-((2-(piperidin-1-yl)ethyl)amino)-1H-pyrazolo[4,3-c]pyridin-1-yl)phenyl)propane-1-sulfonamide | (500 MHz, *DMSO-d₆*) δ(ppm) 12.15 (bs, 1H), 10.0 (bs, 1H), 8.78 (d, J=0.8 Hz, 1H), 8.65 (s, 1H), 7.97 (d, J=0.8 Hz, 1H), 7.37 (d, J=8.5 Hz, 1H), 7.08 (d, J=2.2 Hz, 1H), 6.95 - 6.91 (m, 2H), 6.86 (d, J=4.4 Hz, 1H), 6.56 (t, J=5.5 Hz, 1H), 3.78 (s, 3H), 3.40 - 3.34 (m, 3H, overlapped with water ), 3.21 - 3.16 (m, 2H), 2.56 (bs, 2H, signal partially under DMSO), 2.41 (bs, 3H, overlapped with DMSO), 1.78 - 1.69 (m, 2H), 1.55 - 1.47 (m, 4H), 1.42 - 1.35 (m, 2H), 0.98 (t, J=7.5 Hz, 3H). | (Method 7): Rt = 3.52 min, ES⁺ *m*/*z* 582.2 [M+H]⁺ |
| | | | (Method 2): Rt = 0.87 min, ES⁺ *m*/*z* 596.3 [M+H]⁺ | | |
| 89 | 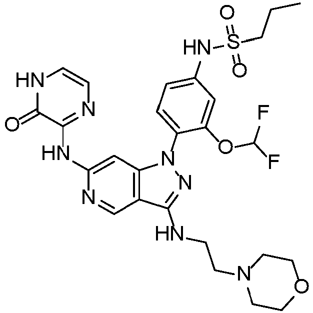 *N*-(3-(difluoromethoxy)-4-(3-((2-morpholinoethyl)amino)-6-((3-oxo-3,4-dihydropyrazin-2-yl)amino)-1*H*-pyrazolo[4,3-*c*]pyridin-1-yl)phenyl)propane-1-sulfonamide | Intermediate 17z | N-(3-(difluoromethoxy)-4-(6-((3-methoxypyrazin-2-yl)amino)-3-((2-morpholinoethyl)amino)-1H-pyrazolo[4,3-c]pyridin-1-yl)phenyl)propane-1-sulfonamide | (500 MHz, *DMSO-d₆*) δ (ppm) 12.17 (brs, 1H), 9.91 - 10.55 (bs, 1H), 8.81 (s, 1H), 8.69 (s, 1H), 8.06 (s, 1H), 7.54 (d, J=8.5 Hz, 1H), 7.28 (s, 1H), 7.22 (dd, J=8.8, 1,9 Hz, 1H), 7.16 (t, J=72.8 Hz, 1H), 6.90 (d, J=4.3, 1H), 6.87 (d, J=4.3 Hz, 1H), 6.69 (br t, J=5.5 Hz, 1H), 3.59 (br t, J=4.3 Hz, 4H), 3.36 - 3.43 (m, 2H), 3.18 - 3.25 (m, 2H), 2.59 (br t, J=6.7 Hz, 2H), 2.44 (br s, 4H), 1.74 (sxt, J=7.5 Hz, 2H), 0.99 (t, J=7.5 Hz, 3H). | (Method 7) Rt = 3.56 min, ES⁺ *m*/*z* 620.2 [M+H]⁺ |
| | | | (Method 1): Rt = 0.69 min, ES⁺ *m*/*z* 634.4 [M+H]⁺ | | |
| 90 | 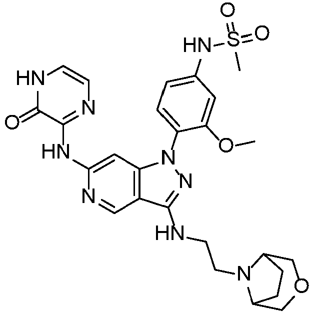 *N*-(4-(3-((2-(3-oxa-8-azabicyclo[3.2.1]octan-8-yl)ethyl)amino)-6-((3-oxo-3,4-dihydropyrazin-2-yl)amino)-1*H-*pyrazolo[4,3-*c*]pyridin-1-yl)-3-methoxyphenyl)methanesulfonam ide | Intermediate 17k | N-(4-(3-((2-(3-oxa-8-azabicyclo[3.2.1]octan-8-yl)ethyl)amino)-6-((3-methoxypyrazin-2-yl)amino)-1H-pyrazolo[4,3-c]pyridin-1-yl)-3-methoxyphenyl)methane sulfonamide | (600 MHz, *DMSO-d₆*) δ(ppm) 8.77 (s, 1H), 8.66 (s, 1H), 7.98 (s, 1H), 7.35 (d, J=1.8 Hz, 1H), 7.04 (d, J=1.8 Hz, 1H), 6.94 (d, J=4.3 Hz, 1H), 6.90 (dd, J=8.8, 1.9 Hz, 1H), 6.87 (d, J=4.3 Hz, 1H), 6.59 (t, J=5.7 Hz, 1H), 3.79 (s, 3H), 3.56 (d, J=9.7 Hz, 2H), 3.36 - 3.43 (m, 4H, overlapped with HDO), 3.11(s, 2H), 3.07 (s, 3H), 2.53 - 2.57 (m, 2H, overlapped with DMSO), 1.80 - 1.88 (m, 2H), 1.69 - 1.74 (m, 2H) | (Method 7): Rt = 3. 10 min, ES⁺ *m*/*z* 582.2 [M+H]⁺ |
| | | | (Method 1): Rt = 0.59 min, ES⁺ *m*/*z* 596.3 [M+H]⁺ | | |
| 91 | 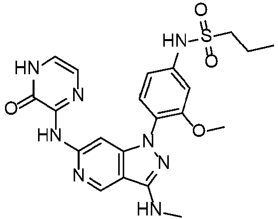 *N*-(3-methoxy-4-(3-(methylamino)-6-((3-oxo-3,4-dihydropyrazin-2-yl)amino)-1*H-*pyrazolo[4,3-*c*]pyridin-1-yl)phenyl)propane-1-sulfonamide | Intermediate 17aa | N-(3-methoxy-4-(6-((3-methoxypyrazin-2-yl)amino)-3-(methylamino)-1H-pyrazolo[4,3-c]pyridin-1-yl)phenyl)propane-1-sulfonamide | (500 MHz, *DMSO-d₆*) δ(ppm) 12.15 (bs, 1H), 10.0 (s, 1H), 8.71 (d, J=1.0 Hz, 1H), 8.64 (s, 1H), 7.95 (d, J=1.1Hz, 1H), 7.38 (d, J=8.5Hz, 1H), 7.08 (d, J=2.4 Hz, 1H), 6.94 - 6.89 (m, 2H), 6.85 (t, J=5.0 Hz, 1H), 6.64 (q, J=5.0 Hz, 1H), 3.77 (s, 3H), 3.2 - 3.5 (m, 2H), 2.86 (d, J=5.0 Hz, 3H), 1.78 - 1.67 (m, 2H),0.97 (t, J=7.5Hz, 3H) | (Method 7) Rt = 4.31 min, ES⁺ *m*/*z* 485.1 [M+H]⁺ |
| | | | (Method 2): Rt = 0.66 min, ES⁺ *m*/*z* 499.2 [M+H]⁺ | | |
| 92 | 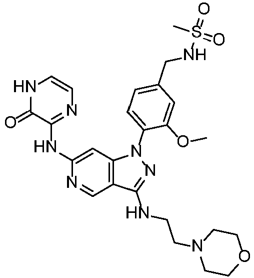 *N*-(3-methoxy-4-(3-((2-morpholinoethyl)amino)-6-((3-oxo-3,4-dihydropyrazin-2-yl)amino)-1*H*-pyrazolo[4,3-*c*]pyridin-1-yl)benzyl)methanesulfonamide | Intermediate 27a, | N-(3-methoxy-4-(3-((2-morpholinoethyl)amino)-6-((3-oxo-3,4-dihydropyrazin-2-yl)amino)-1H-pyrazolo[4,3-c]pyridin-1-yl)benzyl)methanesulfon amide | (300 MHz, *DMSO-d₆*) δ(ppm): 12.12 (br s, 1H), 8.76 (d, J=0.7 Hz, 1H), 8.66 (s, 1H), 8.00 (d, J=0.7 Hz, 1H), 7.64 (br t, J=6.5 Hz, 1H), 7.40 (d, J=8.0 Hz, 1H), 7.26 (d, J=1.2 Hz, 1H), 7.07 (dd, J=8.2, 1.2 Hz, 1H), 6.84-6.89 (m, 2H), 6.61 (t, J=5.5 Hz, 1H), 4.25 (br d, J=5.1 Hz, 2H), 3.84 (s, 3H), 3.58 (br t, J=4.5 Hz, 4H), 3.35-3.45 (m, 2H), 2.91 (s, 3H), 2.59 (br t, J=6.9 Hz, 2H), 2.40-2.46 (m, 4H) | (Method 6): Rt = 2.41 min, ES⁺ *m*/*z* 569.9 [M+H]⁺ |
| | | | (Method 2): Rt = 1.17 min, ES⁺ *m*/*z* 734.3 [M+H]⁺ | | |

### Comparative C31

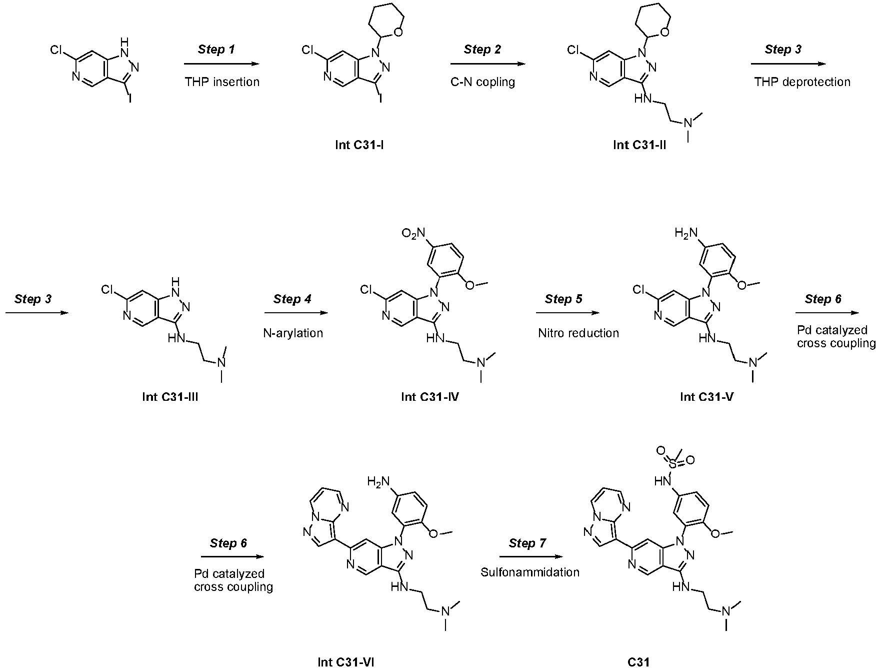

### Step 1

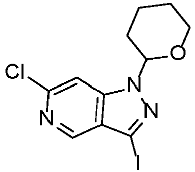

### 6-Chloro-3-iodo-1-(tetrahydro-2H-pyran-2-yl)-1H-pyrazolo[4,3-c]pyridine (Intermediate C31-1)

Dihydropyran (3.4 mL, 37.4 mmol) and methanesulfonic acid (0.16 mL, 2.5 mmol) were added to 6-chloro-3-iodo-1H-pyrazolo[4,3-c]pyridine (3.48 g, 11.1 mmol) in DCM (33 mL) and THF (16.6 mL). RM was stirred at 40 °C for 2 h, then at RT overnight. Solvents were evaporated and the residue purified by flash chromatography on a Si cartridge by eluting with 0-100% of EtOAc in cyclohexane to afford the title product (1.48 g).

LCMS (Method 2): Rt = 1.18 min, ES⁺ *m*/*z* 364.0/366.0 [M+H]⁺

### Step 2

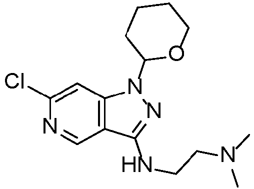

### N1-(6-Chloro-1-(tetrahydro-2H-pyran-2-yl)-1H-pyrazolo[4,3-c]pyridin-3-yl)-N2,N2-dimethylethane-1,2-diamine (Intermediate C31-II)

L-proline (85.5 mg, 0.74 mmol) and copper(I)iodide (94.3 mg, 0.5 mmol) were added to a mixture of intermediate C31-1 (900 mg, 2.48 mmol), *N',N*'-dimethylethane-1,2-diamine (1.23 mL, 11.3 mmol), K₂CO₃ (2.05 g, 14.9 mmol) in DMF (8.0 mL) and RM stirred at 110 °C for 2 h under argon atmosphere. After cooling to RT, RM was diluted with water (80 mL) and extracted with EtOAc (3x10 mL). Combined organic layers were washed with sat. aq. NaHCO₃, passed through phase separator and concentrated *in vacuo.* The residue was purified by flash chromatography on a Si cartridge by eluting with 0-100% DCM / MeOH/ NH₄OH (90:9:0.5) in DCM To give title compound (630 mg).

LCMS (Method 1): Rt = 0.62 min, ES⁺ *m*/*z* 323.9/325.9 [M+H]⁺

### Step 3

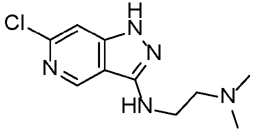

### N1-(6-Chloro-1H-pyrazolo[4,3-c]pyridin-3-yl)-N2,N2-dimethylethane-1,2-diamine (Intermediate C31-III)

Triethylsilane (932 µL, 5.84 mmol) was added dropwise to intermediate C31-II (630 mg, 1.95 mmol) in DCM (9 mL) /TFA (2.29 mL, 29.9 mmol). RM was stirred at RT for 1 h, then diluted with DCM and extracted with sat. aq. NaHCO₃ (10 mL). Aqueous layer (adjusted at pH 9.6) was further extracted with EtOAc (3x), followed by DCM/iPrOH (1:1). Combined organic layers were passed through a phase separator and evaporated under reduced pressure to afford the title product (396 mg) that was used in the next steps without further purification.

LCMS (Method 1): Rt = 0.39 min, ES⁺ *m*/*z* 240.0/242.0 [M+H]⁺

### Step 4

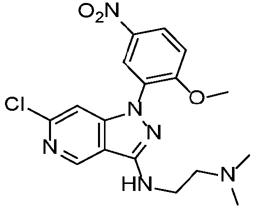

### N1-(6-chloro-1-(2-methoxy-5-nitrophenyl)-1H-pyrazolo[4,3-c]pyridin-3-yl)-N2,N2-dimethylethane-1,2-diamine (Intermediate C31-IV)

Intermediate C31-IV was prepared similarly to intermediate 10d starting from intermediate C31-III and 2-bromo-1-methoxy-4-nitro-benzene.

LCMS(Method 1): Rt = 1.04 min, ES⁺ *m*/*z* 391.1/393.1 [M+H]⁺

### Step 5

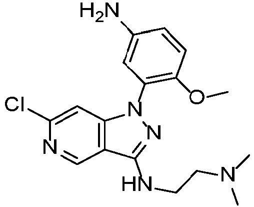

### N1-(1-(5-amino-2-methoxyphenyl)-6-chloro-1H-pyrazolo[4,3-c]pyridin-3-yl)-N2,N2-dimethylethane-1,2-diamine (Intermediate C31-V)

Intermediate C31-V was prepared similarly to intermediate 10c starting from intermediate C31-IV.

LCMS (Method 2): Rt = 0.83, ES+ m/z 361.1/363.1 [M+H]+

### Step 6

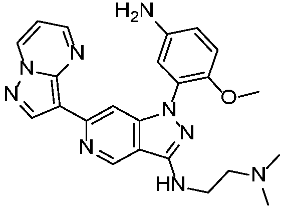

### N1-(1-(5- N1-(1-(5-Amino-2-methoxyphenvyl)-6-(pyrazolo[1,5-a]pyrimidin-3-yl)-1H-pyrazolo[4,3-c]pyridin-3-yl)-N2,N2-dimethylethane-1,2-diamine (Intermediate C31-VI)

Intermediate C31-VI was prepared similarly to Example 1.

LCMS (Method 1): Rt = 0.76, ES+ m/z 443.9 [M+H]+

### Step 7

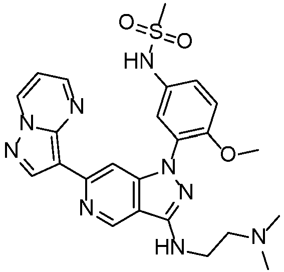

### N-(3-(3-((2-(Dimethylamino)ethyl)amino)-6-(pyrazolo[1,5-a]pyrimidin-3-yl)-1H-pyrazolo[4,3-c]pyridin-1-yl)-4-methoxyphenyl)methanesulfonamide (Comparative C31)

To a cooled (at 0 °C) mixture of intermediate intermediate C31-VI (14 mg, 0.03 pyridine (2.8 mg, 0.03 mmol) followed by methanesulfonyl chloride (2.2 µL, 0.03 mmol) were added. RM was stirred at 0°C for 15 min and at RT overnight. RM was diluted with sat. aq. NaHCO₃ and extracted with DCM (4x15 mL). Combined organic layers were washed with sat. aq. NaCl, dried over Na₂SO₄ and solvent removed under reduced pressure. The residue was purified by flash chromatography on Si cartridge by eluting with 0-100 % DCM/MeOH/NH₄OH (90:9:1.5) in DCM to afford the title product (5 mg).

LCMS (Method 7): Rt = 3.14 min, ES⁺ *m*/*z* 522.2 [M+H]⁺
¹H-NMR (600 MHz, *DMSO-d₆*) δ(ppm): 9.61 (bs, 1H), 9.21 (dd, J=7.1, 1.7 Hz, 1H), 9.09 (d, J=1.0 Hz, 1H), 8.86 (s. 1H), 8.70 (dd, J=4.0, 1.7 Hz, 1H), 8.15 (d, J=1.0 Hz, 1H), 7.37 (d, J=2.6 Hz, 1H), 7.31 (d, J=8.9 Hz, 1H), 7.28 (dd, J=8.9, 2.6 Hz, 1H), 7.15 (dd, J=7.1, 4.1 Hz, 1H), 6.74 (t, J=5.6 Hz, 1H), 3.87 (s, 3H), 3.42 (q, J=6.1 Hz, 2H), 2.98 (s, 3H), 2.60-2.56 (m, 2H), 2.24 (s, 6H).

### PHARMACOLOGICAL ACTIVITY OF THE COMPOUNDS OF THE INVENTION (1-92).

### Biochemical Potency on JAK1, JAK2 , JAK3 and Tyk2

### Assay principle

The objective of this study was to assess the capability of compounds to inhibit all 4 JAK isoforms activity in a cell-free environment. Assay for JAK 1, JAK 2, JAK 3 and TYK2 were performed by Time-resolved fluorescence resonance energy transfer (TR-FRET) technology. It consists in the interaction of two labelled binding partners detected by the energy transfer from an excited donor to an acceptor dye and measurement of light emission by the acceptor dye. LANCE Ultra kinase assay was used. In presence of JAK 1, JAK 2, JAK 3 and TYK2 kinases and ATP (corresponding to Km), the ULight peptide substrate (LANCE Ulight-JAK-1 (Tyr1023) Peptide, Perkin Elmer, TRF0121) is phosphorylated. It is then captured by Eu-anti-phospho-substrate antibody (LANCE Eu-W1024 Anti-phosphotyrosine (PT66), Perkin Elmer, AD0069), which bring the Eu-chelate donor and ULight acceptor dyes into close proximity. Upon excitation at 320 nm, the Eu-chelate transfers its energy to the ULight dye, resulting in a fluorescent light emission at 665 nm.

### Compound testing

Serial dilutions of compounds in pure DMSO are prepared from 10 mM DMSO stock solutions. Compounds were tested in 384-well plate for 11 consecutive 5-fold dilutions starting from 20 µM highest concentration (20 µM - 2 pM). 200 nL of compound were transferred from mother plate to test plate by using Mosquito (TTP labtech). Assay was performed in 384-well Perkin Elmer test plate in 20 µL assay volume (kinase reaction) and 40 µL total volume (stopping reagent and antibody detection reagents). In 10 µL of substrate solution (peptide + ATP) 30/50/20/10nM of peptide and 20/0.7/0.2/12µM of ATP were added for JAK 1, JAK 2, JAK 3 and TYK2 respectively. 10 µL of enzyme solution was added to kinase reaction at these concentrations: 0.15/0.083/0.025/0.144 ng/µL of JAK 1, JAK 2, JAK 3 and TYK2 respectively. After shaking and 1.5h of incubation at r.t., 20 µL of Stop (10 µL EDTA) and Detection mixture (10 µL Europium-anti-phospho antibody, final: 0.5 nM) were added. Reading was performed after 1h of incubation on a EnVision 2104 reader (Perkin Elmer).

Calculation of IC50 data, curves and QC analysis was performed by using Excel tool and GraphPadPrism software, v9. Briefly, individual concentration-effect curves are generated by plotting the logarithm of the tested concentration of tested compounds (X) vs. corresponding percent inhibition values (Y) using least squares (ordinary) fit. Best fit IC50 values are calculated using Log(inhibitor) vs. normalized response - Variable slope equation, where Y=100/(1+10^((LogIC50-X)*HillSlope)). QC criteria parameters (Z', S:B, R2, HillSlope) were checked for every IC50 curve. Calculation of IC50 data, curves and QC analysis were made using Excel tools and GraphPadPrism software. QC criteria parameters: Z' ≥ 0.5, Hill Slope range 0.5 to 5, S:B > 2.

Compounds according to the invention (including exemplified compounds 1a-10a and 1-92) showed values in terms of pIC50 higher than 6 with respect to their inhibitory activity on all JAK isoforms corresponding to ≤ 1 µM in terms of inhibitory concentration. Most compounds preferably showed values higher than 7.3, even more preferably higher than 8.3, at least with respect to their inhibitory activity on JAK1; corresponding to ≤50 nM, even more preferably ≤5 nM, in terms of inhibitory concentration.

Data for compounds 1-92 are reported in the table hereinbelow

| **Example** | **JAK1** | **JAK2** | **JAK3** | **Tyk2** |
|---|---|---|---|---|
| 1 | +++ | +++ | +++ | ++ |
| 2 | ++ | +++ | ++ | ++ |
| 3 | ++ | +++ | ++ | + |
| 4 | +++ | +++ | +++ | ++ |
| 5 | +++ | +++ | +++ | ++ |
| 6 | +++ | +++ | +++ | ++ |
| 7 | +++ | +++ | +++ | ++ |
| 8 | +++ | +++ | +++ | ++ |
| 9 | ++ | +++ | ++ | ++ |
| 10 | +++ | +++ | ++ | ++ |
| 11 | +++ | +++ | +++ | + |
| 12 | ++ | ++ | ++ | + |
| 13 | +++ | +++ | +++ | ++ |
| 14 | +++ | +++ | +++ | ++ |
| 15 | ++ | ++ | ++ | + |
| 16 | ++ | ++ | ++ | + |
| 17 | ++ | ++ | ++ | + |
| 18 | ++ | ++ | ++ | + |
| 19 | + | + | + | + |
| 20 | ++ | ++ | ++ | + |
| 21 | +++ | +++ | +++ | ++ |
| 22 | +++ | +++ | +++ | ++ |
| 23 | +++ | +++ | +++ | ++ |
| 24 | +++ | +++ | +++ | ++ |
| 25 | ++ | ++ | +++ | + |
| 26 | +++ | +++ | +++ | ++ |
| 27 | +++ | +++ | +++ | ++ |
| 28 | +++ | +++ | +++ | ++ |
| 29 | ++ | ++ | ++ | + |
| 30 | +++ | +++ | +++ | ++ |
| 31 | +++ | +++ | +++ | ++ |
| 32 | ++ | +++ | ++ | ++ |
| 33 | ++ | ++ | ++ | ++ |
| 34 | +++ | ++ | ++ | ++ |
| 35 | +++ | +++ | ++ | ++ |
| 36 | ++ | ++ | ++ | ++ |
| 37 | +++ | +++ | +++ | ++ |
| 38 | +++ | +++ | +++ | ++ |
| 39 | +++ | +++ | +++ | ++ |
| 40 | ++ | ++ | ++ | ++ |
| 41 | +++ | +++ | +++ | ++ |
| 42 | ++ | ++ | ++ | ++ |
| 43 | +++ | +++ | ++ | ++ |
| 44 | +++ | +++ | +++ | ++ |
| 45 | +++ | +++ | +++ | ++ |
| 46 | ++ | ++ | ++ | + |
| 47 | ++ | ++ | ++ | + |
| 48 | ++ | +++ | +++ | ++ |
| 49 | ++ | +++ | +++ | + |
| 50 | +++ | +++ | +++ | ++ |
| 51 | +++ | +++ | +++ | ++ |
| 52 | ++ | +++ | +++ | ++ |
| 53 | +++ | +++ | +++ | ++ |
| 54 | ++ | +++ | ++ | ++ |
| 55 | +++ | +++ | +++ | ++ |
| 56 | +++ | +++ | +++ | ++ |
| 57 | +++ | +++ | +++ | ++ |
| 58 | +++ | ++ | ++ | ++ |
| 59 | +++ | ++ | ++ | ++ |
| 60 | +++ | +++ | +++ | ++ |
| 61 | +++ | +++ | +++ | ++ |
| 62 | +++ | +++ | +++ | + |
| 63 | +++ | +++ | +++ | ++ |
| 64 | +++ | +++ | +++ | ++ |
| 65 | +++ | +++ | +++ | ++ |
| 66 | ++ | ++ | ++ | + |
| 67 | + | + | + | + |
| 68 | +++ | +++ | +++ | ++ |
| 69 | + | ++ | ++ | + |
| 70 | + | + | + | + |
| 71 | ++ | ++ | ++ | + |
| 72 | + | ++ | ++ | + |
| 73 | +++ | ++ | ++ | ++ |
| 74 | ++ | ++ | ++ | + |
| 75 | ++ | ++ | ++ | + |
| 76 | +++ | +++ | +++ | ++ |
| 77 | +++ | +++ | +++ | ++ |
| 78 | +++ | +++ | +++ | ++ |
| 79 | +++ | +++ | +++ | ++ |
| 80 | +++ | +++ | +++ | ++ |
| 81 | +++ | +++ | +++ | ++ |
| 82 | ++ | ++ | ++ | ++ |
| 83 | +++ | +++ | +++ | ++ |
| 84 | +++ | +++ | ++ | ++ |
| 85 | +++ | +++ | +++ | +++ |
| 86 | ++ | +++ | +++ | ++ |
| 87 | +++ | +++ | +++ | +++ |
| 88 | +++ | +++ | +++ | ++ |
| 89 | +++ | +++ | +++ | ++ |
| 90 | +++ | +++ | +++ | +++ |
| 91 | +++ | +++ | +++ | ++ |
| 92 | +++ | +++ | +++ | +++ |

The compounds are classified in the table above in term of potency with respect to their inhibitory activity on JAK1, JAK2, JAK3 and Tyk2 isoforms according to the following classification criterion:
+ + + : pIC₅₀ ≥ 8.3
++ : 8.3 > pIC₅₀ ≥ 7.3
+ : pIC₅₀ < 7.3

Where a numerical limit or range is stated herein, the endpoints are included. Also, all values and subranges within a numerical limit or range are specifically included when not explicitly written out.

As used herein the words "a" and "an" and the like carry the meaning of "one or more."

Obviously, numerous modifications and variations of the present invention are possible in light of the above teachings. It is therefore to be understood that, within the scope of the appended claims, the invention may be practiced otherwise than as specifically described herein.

## Claims

1. A compound represented by the formula I 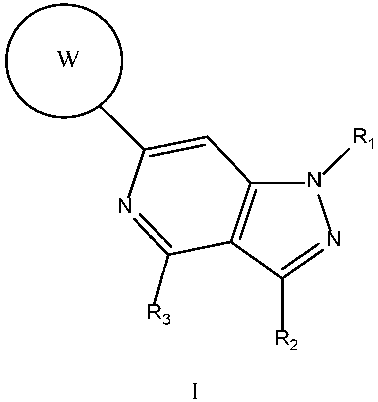 wherein,
**W is** a heteroaryl selected from pyrazolo[1,5-a]pyrimidin-3-yl, imidazo[1,2-b]pyridazin-3-yl and (3-oxo-3,4-dihydropyrazin-2-yl)amino;
**R₁** is selected in the group of pyridinyl or phenyl substituted by one or more group, preferably 2 or 3 groups, independently selected from
halogen,
-OH,
-CN
-NO₂
-(CH₂)ₘNR₄R₅,
(C₁-C₆)alkyl,
(C₁-C₆)hydroxyalkyl,
(C₁-C₆)alkoxy,
(C₁-C₆)alkylthio-
(C₁-C₆)haloalkyl,
(C₁-C₆)haloalkoxy,
and R₁ is substituted by at least one group of formula **K** 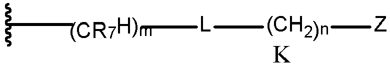 in para position with respect to the point of attachment of R1 with the rest of the molecule,
wherein
**L** is absent or is a divalent group selected from O, S, S(O)₂, (CO), C(O)O, O(O)C, C(O)N(R₆), N(R₆)C(O), NHCONH, N(R₆)S(O)₂, S(O)₂N(R₆);
**Z** is selected from the group consisting of -OH, -CN, -NO₂, (C₁-C₆)alkyl, (C₁-C₆)hydroxyalkyl, (C₁-C₆)haloalkyl, (C₁-C₆)alkoxy, (C₁-C₆)alkoxy (C₁-C₆)alkyl, (C₁-C₆)alkoxycarbonyl, -(CH₂)ₘNR₄R₅, -C(O)NH(R₆), (C₃-C₈)cycloalkyl, aryl, heteroaryl and (C₃-C₆)heterocycloalkyl; wherein said
(C₃-C₈)cycloalkyl, aryl, heteroaryl and (C₃-C₆)heterocycloalkyl are further optionally substituted by one or more substituents selected from the group consisting of
(C₁-C₁₀)alkyl, alkanoyl, (C₁-C₆)alkoxycarbonyl, oxo, -C(O)NH(R₆), (C₁-C₆)alkoxy(C₁-C₆)alkyl;
**R₃** is H, and
**R₂** is selected independently from the group consisting of (C₁-C₆)alkyl, and a group of formula **J** 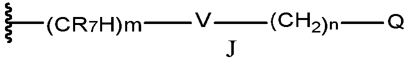
wherein
**V** is absent or is a divalent group selected from O, S, S(O)₂, C(O), C(O)O, O(O)C, C(O)N(R₆), N(R₆)C(O); N(R₆)-(CH₂)ₘ-N(R₆), -N(R₆)-;
**Q** is selected from the group consisting of -CN, -OH, (C₁-C₆)alkyl, (C₁-C₆)hydroxyalkyl, (C₁-C₆)haloalkyl, (C₁-C₆)alkoxy, (C₁-C₆)alkoxycarbonyl, hydroxycarbonyl, -(CH₂)ₘNR₄R₅, -C(O)NR₄R₅, -N(R₆)C(O)R₆, - CH(CN)NR₄R₅, (C₃-C₈)cycloalkyl, aryl, heteroaryl and (C₃-C₆)heterocycloalkyl;
wherein said (C₃-C₈)cycloalkyl, aryl, heteroaryl and (C₃-C₆)heterocycloalkyl are further optionally substituted by one or more substituents selected from the group consisting of
-OH, oxo, (C₁-C₁₀)alkyl, (C₁-C₁₀)alkyl-S(O)₂-O-, alkanoyl, (C₁-C₆)hydroxyalkyl,
(C₁-C₆)alkoxy(C₁-C₆)alkyl, (C₁-C₆)alkoxycarbonyl, (C₁-C₆)alkoxycarbonyl-NH-, -N(R₆)(CH₂)ₘC(O)NR₄R₅, -NR₄R₅, (C₃-C₆)heterocycloalkyl;
**n and m are in each occurrence independently** 0 or an integer selected from 1, 2, 3 and 4;
**R₄ and R₅,** the same or different, are selected from the group consisting of
-H,
(C₁-C₆)alkyl,
(C₁-C₆)haloalkyl,
(C₁-C₆)hydroxyalkyl,
alkanoyl, (C₁-C₆)alkoxycarbonyl, and
(C₃-C₆)heterocycloalkyl;
**R₆** is in each occurrence independently selected from the group consisting of H, (C₁-C₆)alkyl, (C₁-C₆)hydroxyalkyl, and alkanoyl,
**R₇** is in each occurrence independently selected from the group consisting of H, (C₁-C₆)alkyl, -NR₄R₅
single enantiomers, diastereoisomers and mixtures thereof
or a pharmaceutically acceptable salt or solvate thereof.

2. A compound according to claim 1 represented by the formula Ia 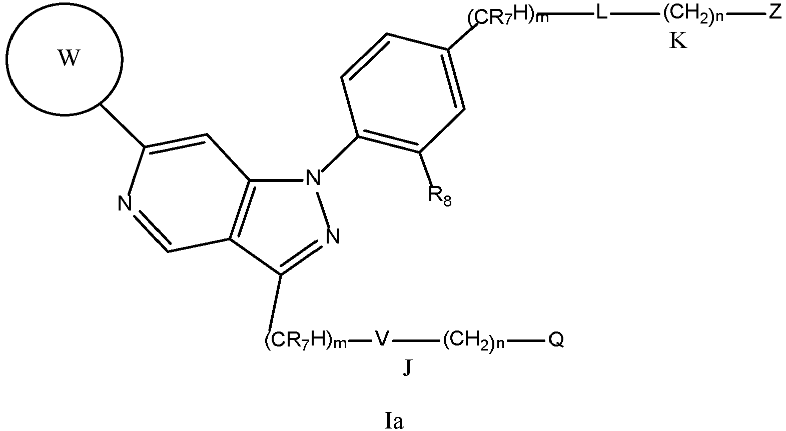 Wherein
**R₈ is** selected in the group consisting of
(C₁-C₆)alkoxy,
(C₁-C₆)haloalkoxy;
**L** is N(R₆)S(O)₂, S(O)₂N(R₆);
**Z** is selected from the group consisting of (C₁-C₆)alkyl, , (C₁-C₆)haloalkyl, (C₁-C₆)alkoxy, (C₁-C₆)alkoxycarbonyl, -(CH₂)ₘNR₄R₅, -C(O)NH(R₆), (C₃-C₈)cycloalkyl, aryl, heteroaryl and (C₃-C₆)heterocycloalkyl; said C₃-C₈)cycloalkyl, aryl, heteroaryl and (C₃-C₆)heterocycloalkyl being optionally substituted by one or more substituents selected from the group consisting of (C₁-C₁₀)alkyl, alkanoyl, (C₁-C₆)alkoxycarbonyl, oxo, -C(O)NH(R₆), (C₁-C₆)alkoxy(C₁-C₆)alkyl;
**V** is absent or is a divalent groups consisting of -N(R₆)-,
**Q** is selected from the group consisting of (C₁-C₆)alkyl, -(CH₂)ₘNR₄R₅ and (C₃-C₆)heterocycloalkyl; said (C₃-C₆)heterocycloalkyl being optionally substituted by one or more (C₁-C₁₀)alkyl;
wherein **n and m are in each occurrence independently** 0 or an integer from 1 to 4;
**R₄ and R₅,** the same or different, are selected from the group consisting of
-H,
(C₁-C₆)alkyl,
**R₆ is** in each occurrence independently selected from the group consisting of H, (C₁-C₆)alkyl, (C₁-C₆)hydroxyalkyl,
**R₇ is** in each occurrence independently selected from the group consisting of H, (C₁-C₆)alkyl,
single enantiomers, diastereoisomers and mixtures thereof
or a pharmaceutically acceptable salt or solvate thereof.

3. A compound according to claim 2, wherein
**R₈ is** (C₁-C₆)alkoxy;
**L** is -N(R₆)S(O)₂-;
**Z** is selected from the group consisting of
(C₁-C₆)alkyl; and
(C₃-C₈)cycloalkyl;
**V** is absent or is -N(R₆)-,
**Q** is selected from the group consisting of
-CN,
(C₁-C₆)alkyl,
-(CH₂)ₘNR₄R₅, and
(C₃-C₆)heterocycloalkyl;
**n is 0 and m is 0 or 1 in group K; and n is 0, 1 or 2 and m is 0 or 1 in group J;**
**R₄ and R₅,** the same or different, are selected from the group consisting of
-H,
(C₁-C₆)alkyl, preferably methyl;
R₆ is H,
R₇ is H,
single enantiomers, diastereoisomers and mixtures thereof
or pharmaceutically acceptable salts and solvates thereof.

4. A compound according to claim 1 selected from:
N-(3-methoxy-4-(3-methyl-6-(pyrazolo[1,5-a]pyrimidin-3-yl)-1H-pyrazolo[4,3-c]pyridin-1-yl)phenyl)methanesulfonamide;
N-(3-methoxy-4-(3-methyl-6-(pyrazolo[1,5-a]pyrimidin-3-yl)-1H-pyrazolo[4,3-c]pyridin-1-yl)phenyl)-1-(tetrahydrofuran-2-yl)methanesulfonamide;
N-(3-methoxy-4-(3-methyl-6-(pyrazolo[1,5-a]pyrimidin-3-yl)-1H-pyrazolo[4,3-c]pyridin-1-yl)phenyl)tetrahydro-2H-pyran-4-sulfonamide;
N-(3-methoxy-4-(3-methyl-6-(pyrazolo[1,5-a]pyrimidin-3-yl)-1H-pyrazolo[4,3-c]pyridin-1-yl)phenyl)propane-1-sulfonamide;
1-cyclopropyl-N-(3-methoxy-4-(3-methyl-6-(pyrazolo[1,5-a]pyrimidin-3-yl)-1H-pyrazolo[4,3-c]pyridin-1-yl)phenyl)methanesulfonamide;
N-(3-methoxy-4-(3-methyl-6-(pyrazolo[1,5-a]pyrimidin-3-yl)-1H-pyrazolo[4,3-c]pyridin-1-yl)phenyl)cy clopropanesulfonamide;
N-(3-(difluoromethoxy)-4-(3-methyl-6-(pyrazolo[1,5-a]pyrimidin-3-yl)-1H-pyrazolo[4,3-c]pyridin-1-yl)phenyl)methanesulfonamide;
N-(3-methoxy-4-(3-methyl-6-(pyrazolo[1,5-a]pyrimidin-3-yl)-1H-pyrazolo[4,3-c]pyridin-1-yl)phenyl)oxetane-3-sulfonamide;
N-(3-methoxy-4-(3-methyl-6-(pyrazolo[1,5-a]pyrimidin-3-yl)-1H-pyrazolo[4,3-c]pyridin-1-yl)phenyl)tetrahydrofuran-3-sulfonamide;
N-(3-methoxy-4-(3-methyl-6-(pyrazolo[1,5-a]pyrimidin-3-yl)-1H-pyrazolo[4,3-c]pyridin-1-yl)phenyl)-1-methyl-1H-pyrazole-4-sulfonamide;
N-(5-methoxy-6-(3-methyl-6-(pyrazolo[1,5-a]pyrimidin-3-yl)-1H-pyrazolo[4,3-c]pyridin-1-yl)pyridin-3-yl)methanesulfonamide;
N-(2-fluoro-5-methoxy-4-(3-methyl-6-(pyrazolo[1,5-a]pyrimidin-3-yl)-1H-pyrazolo[4,3-c]pyridin-1-yl)phenyl)methanesulfonamide;
3-(N-(3-methoxy-4-(3-methyl-6-(pyrazolo[1,5-a]pyrimidin-3-yl)-1H-pyrazolo[4,3-c]pyridin-1-yl)phenyl)sulfamoyl)propanamide;
2-(N-(3-methoxy-4-(3-methyl-6-(pyrazolo[1,5-a]pyrimidin-3-yl)-1H-pyrazolo[4,3-c]pyridin-1-yl)phenyl)sulfamoyl)acetamide;
3-methoxy-4-(3-methyl-6-(pyrazolo[1,5-a]pyrimidin-3-yl)-1H-pyrazolo[4,3-c]pyridin-1-yl)benzonitrile;
4-methoxy-N-methyl-5-(3-methyl-6-(pyrazolo[1,5-a]pyrimidin-3-yl)-1H-pyrazolo[4,3-c]pyridin-1-yl)pyridin-2-amine;
3-methoxy-4-(3-methyl-6-(pyrazolo[1,5-a]pyrimidin-3-yl)-1H-pyrazolo[4,3-c]pyridin-1-yl)aniline;
1-(3-methoxy-4-(3-methyl-6-(pyrazolo[1,5-a]pyrimidin-3-yl)-1H-pyrazolo[4,3-c]pyridin-1-yl)phenyl)-3-methylurea;
3-methoxy-N-methyl-4-(3-methyl-6-(pyrazolo[1,5-a]pyrimidin-3-yl)-1H-pyrazolo[4,3-c]pyridin-1-yl)benzamide;
3-methoxy-4-(3-methyl-6-(pyrazolo[1,5-a]pyrimidin-3-yl)-1H-pyrazolo[4,3-c]pyridin-1-yl)-N-phenylbenzamide;
tert-butyl (3-((1-(2-methoxy-4-(methylsulfonamido)phenyl)-6-(pyrazolo[1,5-a]pyrimidin-3-yl)-1H-pyrazolo[4,3-c]pyridin-3-yl)amino)propyl)carbamate;
N-(4-(3-((3-(dimethylamino)propyl)amino)-6-(pyrazolo[1,5-a]pyrimidin-3-yl)-1H-pyrazolo[4,3-c]pyridin-1-yl)-3-methoxyphenyl)methanesulfonamide;
N-(3-methoxy-4-(3-(methylamino)-6-(pyrazolo[1,5-a]pyrimidin-3-yl)-1H-pyrazolo[4,3-c]pyridin-1-yl)phenyl)methanesulfonamide;
N-(3-methoxy-4-(3-((2-morpholinoethyl)amino)-6-(pyrazolo[1,5-a]pyrimidin-3-yl)-1H-pyrazolo[4,3-c]pyridin-1-yl)phenyl)methanesulfonamide;
N-(3-(difluoromethoxy)-4-(3-((2-morpholinoethyl)amino)-6-(pyrazolo[1,5-a]pyrimidin-3-yl)-1H-pyrazolo[4,3-c]pyridin-1-yl)phenyl)cyclopropanesulfonamide;
N-(4-(3-((2-hydroxyethyl)amino)-6-(pyrazolo[1,5-a]pyrimidin-3-yl)-1H-pyrazolo[4,3-c]pyridin-1-yl)-3-methoxyphenyl)methanesulfonamide;
N-(3-methoxy-4-(3-(((1-(2-methoxyethyl)piperidin-4-yl)methyl)amino)-6-(pyrazolo[1,5-a]pyrimidin-3-yl)-1H-pyrazolo[4,3-c]pyridin-1-yl)phenyl)methanesulfonamide;
N-(3-methoxy-4-(3-(((4-methylmorpholin-2-yl)methyl)amino)-6-(pyrazolo[1,5-a]pyrimidin-3-yl)-1H-pyrazolo[4,3-c]pyridin-1-yl)phenyl)methanesulfonamide;
N-(6-(3-((2-(dimethylamino)ethyl)amino)-6-(pyrazolo[1,5-a]pyrimidin-3-yl)-1H-pyrazolo[4,3-c]pyridin-1-yl)-5-methoxypyridin-3-yl)cyclopropanesulfonamide;
N-(4-(3-((2-(3-oxa-8-azabicyclo[3.2.1]octan-8-yl)ethyl)amino)-6-(pyrazolo[1,5-a]pyrimidin-3-yl)-1H-pyrazolo[4,3-c]pyridin-1-yl)-3-methoxyphenyl)methanesulfonamide;
N-(4-(3-((2-(dimethylamino)ethyl)amino)-6-(pyrazolo[1,5-a]pyrimidin-3-yl)-1H-pyrazolo[4,3-c]pyridin-1-yl)-3-methoxyphenyl)methanesulfonamide;
N-(4-(3-((2-(dimethylamino)ethyl)amino)-6-(pyrazolo[1,5-a]pyrimidin-3-yl)-1H-pyrazolo[4,3-c]pyridin-1-yl)-3-methoxyphenyl)cyclopropanesulfonamide;
N-(3-methoxy-4-(3-((3-morpholinopropyl)amino)-6-(pyrazolo[1,5-a]pyrimidin-3-yl)-1H-pyrazolo[4,3-c]pyridin-1-yl)phenyl)cyclopropanesulfonamide;
N-(3-methoxy-4-(3-((2-morpholinoethyl)amino)-6-(pyrazolo[1,5-a]pyrimidin-3-yl)-1H-pyrazolo[4,3-c]pyridin-1-yl)phenyl)cyclopropanesulfonamide;
N-(3-methoxy-4-(3-(methylamino)-6-(pyrazolo[1,5-a]pyrimidin-3-yl)-1H-pyrazolo[4,3-c]pyridin-1-yl)phenyl)cyclopropanesulfonamide;
N-(3-methoxy-4-(3-((2-(piperidin-1-yl)ethyl)amino)-6-(pyrazolo[1,5-a]pyrimidin-3-yl)-1H-pyrazolo[4,3-c]pyridin-1-yl)phenyl)cyclopropanesulfonamide;
N-(3-methoxy-4-(3-((2-(piperidin-1-yl)ethyl)amino)-6-(pyrazolo[1,5-a]pyrimidin-3-yl)-1H-pyrazolo[4,3-c]pyridin-1-yl)phenyl)methanesulfonamide;
N-(3-methoxy-4-(3-((2-(4-methylpiperazin-1-yl)ethyl)amino)-6-(pyrazolo[1,5-a]pyrimidin-3-yl)-1H-pyrazolo[4,3-c]pyridin-1-yl)phenyl)methanesulfonamide;
N-(4-(3-((2-((2-fluoroethyl)(methyl)amino)ethyl)amino)-6-(pyrazolo[1,5-a]pyrimidin-3-yl)-1H-pyrazolo[4,3-c]pyridin-1-yl)-3-methoxyphenyl)methanesulfonamide;
N-(3-methoxy-4-(3-(methyl(2-morpholinoethyl)amino)-6-(pyrazolo[1,5-a]pyrimidin-3-yl)-1H-pyrazolo[4,3-c]pyridin-1-yl)phenyl)methanesulfonamide;
N-(1-(2-methoxy-4-(methylsulfonamido)phenyl)-6-(pyrazolo[1,5-a]pyrimidin-3-yl)-1H-pyrazolo[4,3-c]pyridin-3-yl)-1-methylpiperidine-4-carboxamide;
N-(1-(2-methoxy-4-(methylsulfonamido)phenyl)-6-(pyrazolo[1,5-a]pyrimidin-3-yl)-1H-pyrazolo[4,3-c]pyridin-3-yl)-1-methylpyrrolidine-3-carboxamide;
N-(3-(dimethylamino)propyl)-1-(2-methoxy-4-(methylsulfonamido)phenyl)-6-(pyrazolo[1,5-a]pyrimidin-3-yl)-1H-pyrazolo[4,3-c]pyridine-3-carboxamide;
N-(2-(dimethylamino)ethyl)-1-(2-methoxy-4-(methylsulfonamido)phenyl)-6-(pyrazolo[1,5-a]pyrimidin-3-yl)-1H-pyrazolo[4,3-c]pyridine-3-carboxamide;
1-(2-methoxy-4-(methylsulfonamido)phenyl)-N-(2-morpholinoethyl)-6-(pyrazolo[1,5-a]pyrimidin-3-yl)-1H-pyrazolo[4,3-c]pyridine-3-carboxamide;
N-(3-(dimethylamino)propyl)-1-(2-methoxy-4-(propylsulfonamido)phenyl)-6-(pyrazolo[1,5-a]pyrimidin-3-yl)-1H-pyrazolo[4,3-c]pyridine-3-carboxamide;
1-(2-methoxy-4-(propylsulfonamido)phenyl)-N-(3-morpholinopropyl)-6-(pyrazolo[1,5-a]pyrimidin-3-yl)-1H-pyrazolo[4,3-c]pyridine-3-carboxamide;
N-(5-methoxy-6-(3-methyl-6-(pyrazolo[1,5-a]pyrimidin-3-yl)-1H-pyrazolo[4,3-c]pyridin-1-yl)pyridin-3-yl)benzamide;
3-methoxy-N-methyl-4-(3-methyl-6-(pyrazolo[1,5-a]pyrimidin-3-yl)-1H-pyrazolo[4,3-c]pyridin-1-yl)benzenesulfonamide;
3-methoxy-N-methyl-4-(3-((2-morpholinoethyl)amino)-6-(pyrazolo[1,5-a]pyrimidin-3-yl)-1H-pyrazolo[4,3-c]pyridin-1-yl)benzenesulfonamide;
3-methoxy-N-methyl-4-(3-(methylamino)-6-(pyrazolo[1,5-a]pyrimidin-3-yl)-1H-pyrazolo[4,3-c]pyridin-1-yl)benzenesulfonamide;
4-(3-((3-(dimethylamino)propyl)amino)-6-(pyrazolo[1,5-a]pyrimidin-3-yl)-1H-pyrazolo[4,3-c]pyridin-1-yl)-3-methoxy-N-methylbenzenesulfonamide;
N-(3-methoxy-4-(3-((2-morpholinoethyl)amino)-6-(pyrazolo[1,5-a]pyrimidin-3-yl)-1H-pyrazolo[4,3-c]pyridin-1-yl)benzyl)methanesulfonamide;
N-(3-methoxy-4-(3-((2-(piperidin-1-yl)ethyl)amino)-6-(pyrazolo[1,5-a]pyrimidin-3-yl)-1H-pyrazolo[4,3-c]pyridin-1-yl)benzyl)methanesulfonamide;
N-(3-methoxy-4-(3-(methylamino)-6-(pyrazolo[1,5-a]pyrimidin-3-yl)-1H-pyrazolo[4,3-c]pyridin-1-yl)benzyl)methanesulfonamide;
N-(4-(3-((2-(dimethylamino)ethyl)amino)-6-(pyrazolo[1,5-a]pyrimidin-3-yl)-1H-pyrazolo[4,3-c]pyridin-1-yl)-3-methoxybenzyl)methanesulfonamide;
N-(2-chloro-5-methoxy-4-(3-(methylamino)-6-(pyrazolo[1,5-a]pyrimidin-3-yl)-1H-pyrazolo[4,3-c]pyridin-1-yl)benzyl)methanesulfonamide;
N-(2-chloro-5-methoxy-4-(3-((2-morpholinoethyl)amino)-6-(pyrazolo[1,5-a]pyrimidin-3-yl)-1H-pyrazolo[4,3-c]pyridin-1-yl)benzyl)methanesulfonamide;
N-(3-(dimethylamino)propyl)-1-(2-methoxy-4-(methylsulfonamidomethyl)phenyl)-6-(pyrazolo[1,5-a]pyrimidin-3-yl)-1H-pyrazolo[4,3-c]pyridine-3-carboxamide;
N-(4-(3-(cyanomethyl)-6-(pyrazolo[1,5-a]pyrimidin-3-yl)-1H-pyrazolo[4,3-c]pyridin-1-yl)-3-methoxybenzyl)methanesulfonamide;
N-(3-(difluoromethoxy)-4-(3-methyl-6-(pyrazolo[1,5-a]pyrimidin-3-yl)-1H-pyrazolo[4,3-c]pyridin-1-yl)benzyl)methanesulfonamide;
N-(1-(3-methoxy-4-(3-methyl-6-(pyrazolo[1,5-a]pyrimidin-3-yl)-1H-pyrazolo[4,3-c]pyridin-1-yl)phenyl)ethyl)methanesulfonamide;
N-(3-methoxy-4-(3-methyl-6-(pyrazolo[1,5-a]pyrimidin-3-yl)-1H-pyrazolo[4,3-c]pyridin-1-yl)benzyl)methanesulfonamide;
N-(3-methoxy-4-(3-methyl-6-(pyrazolo[1,5-a]pyrimidin-3-yl)-1H-pyrazolo[4,3-c]pyridin-1-yl)benzyl)cyclopropanesulfonamide;
N-(3-methoxy-4-(3-methyl-6-(pyrazolo[1,5-a]pyrimidin-3-yl)-1H-pyrazolo[4,3-c]pyridin-1-yl)benzyl)-N-methylmethanesulfonamide;
(3-methoxy-4-(3-methyl-6-(pyrazolo[1,5-a]pyrimidin-3-yl)-1H-pyrazolo[4,3-c]pyridin-1-yl)phenyl)methanol;
(3-methoxy-4-(3-methyl-6-(pyrazolo[1,5-a]pyrimidin-3-yl)-1H-pyrazolo[4,3-c]pyridin-1-yl)phenyl)methanamine;
1-cyano-N-(3-methoxy-4-(3-methyl-6-(pyrazolo[1,5-a]pyrimidin-3-yl)-1H-pyrazolo[4,3-c]pyridin-1-yl)benzyl)methanesulfonamide;
methyl (3-methoxy-4-(3-methyl-6-(pyrazolo[1,5-a]pyrimidin-3-yl)-1H-pyrazolo[4,3-c]pyridin-1-yl)benzyl)carbamate;
N-(3-methoxy-4-(3-methyl-6-(pyrazolo[1,5-a]pyrimidin-3-yl)-1H-pyrazolo[4,3-c]pyridin-1-yl)benzyl)acetamide;
1-(3-methoxy-4-(3-methyl-6-(pyrazolo[1,5-a]pyrimidin-3-yl)-1H-pyrazolo[4,3-c]pyridin-1-yl)benzyl)-3-methylurea;
1-(3-methoxy-4-(3-methyl-6-(pyrazolo[1,5-a]pyrimidin-3-yl)-1H-pyrazolo[4,3-c]pyridin-1-yl)benzyl)-3-phenylurea;
2,2-difluoro-N-(3-methoxy-4-(3-methyl-6-(pyrazolo[1,5-a]pyrimidin-3-yl)-1H-pyrazolo[4,3-c]pyridin-1-yl)phenyl)ethane-1-sulfonamide;
N-(3-methoxy-4-(3-methyl-6-(pyrazolo[1,5-a]pyrimidin-3-yl)-1H-pyrazolo[4,3-c]pyridin-1-yl)phenyl)benzamide;
N-(4-(3-((2-(dimethylamino)ethyl)amino)-6-(pyrazolo[1,5-a]pyrimidin-3-yl)-1H-pyrazolo[4,3-c]pyridin-1-yl)-3-methoxyphenyl)benzamide;
N-(4-(6-(imidazo[1,2-b]pyridazin-3-yl)-3-((2-morpholinoethyl)amino)-1H-pyrazolo[4,3-c]pyridin-1-yl)-3-methoxyphenyl)methanesulfonamide;
N-(4-(3-((2-(dimethylamino)ethyl)amino)-6-(imidazo[1,2-b]pyridazin-3-yl)-1H-pyrazolo[4,3-c]pyridin-1-yl)-3-methoxyphenyl)methanesulfonamide;
N-(4-(3-(((1s,3s)-3-(dimethylamino)cyclobutyl)amino)-6-(pyrazolo[1,5-a]pyrimidin-3-yl)-1H-pyrazolo[4,3-c]pyridin-1-yl)-3-methoxyphenyl)cyclopropanesulfonamide;
N-(4-(3-amino-6-(pyrazolo[1,5-a]pyrimidin-3-yl)-1H-pyrazolo[4,3-c]pyridin-1-yl)-3-methoxyphenyl)methanesulfonamide;
N-(1-(2-methoxy-4-(methylsulfonamido)phenyl)-6-(pyrazolo[1,5-a]pyrimidin-3-yl)-1H-pyrazolo[4,3-c]pyridin-3-yl)-2-(methylamino)acetamide;
N-(1-(2-methoxy-4-(methylsulfonamido)phenyl)-6-(pyrazolo[1,5-a]pyrimidin-3-yl)-1H-pyrazolo[4,3-c]pyridin-3-yl)-2-(4-methylpiperazin-1-yl)acetamide;
N-(1-(2-methoxy-4-(propylsulfonamido)phenyl)-6-(pyrazolo[1,5-a]pyrimidin-3-yl)-1H-pyrazolo[4,3-c]pyridin-3-yl)-3-(4-methylpiperazin-1-yl)propanamide;
N-(3-methoxy-4-(3-((methylamino)methyl)-6-(pyrazolo[1,5-a]pyrimidin-3-yl)-1H-pyrazolo[4,3-c]pyridin-1-yl)phenyl)propane-1-sulfonamide;
N-(4-(3-(cyanomethyl)-6-(pyrazolo[1,5-a]pyrimidin-3-yl)-1H-pyrazolo[4,3-c]pyridin-1-yl)-3-methoxyphenyl)cyclopropanesulfonamide;
N-(3-methoxy-4-(3-methyl-6-((3-oxo-3,4-dihydropyrazin-2-yl)amino)-1H-pyrazolo[4,3-c]pyridin-1-yl)phenyl)methanesulfonamide;
N-(3-methoxy-4-(3-methyl-6-((3-oxo-3,4-dihydropyrazin-2-yl)amino)-1H-pyrazolo[4,3-c]pyridin-1-yl)phenyl)-1-methyl-1H-pyrazole-4-sulfonamide;
N-(3-methoxy-4-(3-((2-morpholinoethyl)amino)-6-((3-oxo-3,4-dihydropyrazin-2-yl)amino)-1H-pyrazolo[4,3-c]pyridin-1-yl)phenyl)methanesulfonamide;
N-(3-methoxy-4-(6-((3-oxo-3,4-dihydropyrazin-2-yl)amino)-3-((2-(piperidin-1-yl)ethyl)amino)-1H-pyrazolo[4,3-c]pyridin-1-yl)phenyl)propane-1-sulfonamide;
N-(3-(difluoromethoxy)-4-(3-((2-morpholinoethyl)amino)-6-((3-oxo-3,4-dihydropyrazin-2-yl)amino)-1H-pyrazolo[4,3-c]pyridin-1-yl)phenyl)propane-1-sulfonamide;
N-(4-(3-((2-(3-oxa-8-azabicyclo[3.2.1]octan-8-yl)ethyl)amino)-6-((3-oxo-3,4-dihydropyrazin-2-yl)amino)-1H-pyrazolo[4,3-c]pyridin-1-yl)-3-methoxyphenyl)methanesulfonamide;
N-(3-methoxy-4-(3-(methylamino)-6-((3-oxo-3,4-dihydropyrazin-2-yl)amino)-1H-pyrazolo[4,3-c]pyridin-1-yl)phenyl)propane-1-sulfonamide;
N-(3-methoxy-4-(3-((2-morpholinoethyl)amino)-6-((3-oxo-3,4-dihydropyrazin-2-yl)amino)-1H-pyrazolo[4,3-c]pyridin-1-yl)benzyl)methanesulfonamide;
single enantiomers, diastereoisomers and mixtures thereof
or a pharmaceutically acceptable salt or solvate thereof.

5. A pharmaceutical composition comprising a compound as defined in any one of claims 1 to 4, or a pharmaceutically acceptable salt thereof, in admixture with one or more pharmaceutically acceptable carrier or excipient.

6. A pharmaceutical composition according to claim 5 suitable to be administered by inhalation, selected from inhalable powders, propellant-containing metering aerosols or propellant-free inhalable formulations.

7. A device comprising the pharmaceutical composition according to claim 6, which may be a single- or multi-dose dry powder inhaler, a metered dose inhaler or a soft mist nebulizer.

8. A compound or a pharmaceutical composition according to any one of claims 1 to 6 for use as a medicament.

9. A compound or a pharmaceutical composition for use according to claim 8 in the prevention and /or treatment of a pulmonary disease selected from the group consisting of asthma, chronic obstructive pulmonary disease COPD, idiopathic pulmonary fibrosis (IPF), acute lung injury and acute respiratory distress syndrome (ARDS).

10. A combination of a compound as defined in any one of the claims 1 to 4 with one or more active ingredients selected from beta2-agonists, antimuscarinic agents, corticosteroids, mitogen-activated kinases (P38 MAP kinases) inhibitors, nuclear factor kappa-B kinase subunit beta inhibitors (IKK2), human neutrophil elastase (HNE) inhibitors, phosphodiesterase 4 (PDE4) inhibitors, leukotriene modulators, non-steroidal anti-inflammatory agents (NSAIDs) and mucus regulators.

## Patentansprüche

1. Verbindung, dargestellt durch die Formel I 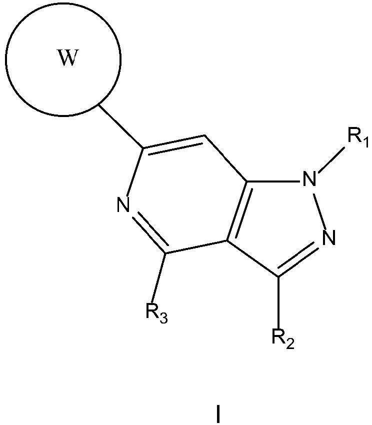 wobei,
**W** ein Heteroaryl ist, ausgewählt aus Pyrazolo[1,5-a]pyrimidin-3-yl, Imidazo[1,2-b]pyridazin-3-yl und (3-Oxo-3,4-dihydropyrazin-2-yl)amino;
**R₁** ausgewählt ist aus der Gruppe von Pyridinyl oder Phenyl, substituiert durch eine oder mehrere Gruppen, vorzugsweise 2 oder 3 Gruppen, unabhängig ausgewählt aus
Halogen,
-OH,
-CN
-NO₂
-(CH₂)ₘNR₄R₅,
(C₁-C₆)-Alkyl,
(C₁-C₆)-Hydroxyalkyl,
(C₁-C₆)-Alkoxy,
(C₁-C₆)-Alkylthio-
(C₁-C₆)-Halogenalkyl,
(C₁-C₆)-Haloalkoxy,
und R₁ durch mindestens eine Gruppe der Formel **K** 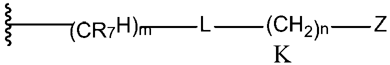 in para-Position in Bezug auf den Verknüpfungspunkt von R1 mit dem Rest des Moleküls substituiert ist,
wobei
**L** fehlt oder eine zweiwertige Gruppe ist, ausgewählt aus O, S, S(O)₂, (CO), C(O)O, O(O)C, C(O)N(R₆), N(R₆)C(O), NHCONH, N(R₆)S(O)₂, S(O)₂N(R₆);
**Z** ausgewählt ist aus der Gruppe, bestehend aus -OH, -CN, -NO₂, (C₁-C₆)-Alkyl, (C₁-C₆)-Hydroxyalkyl, (C₁-C₆)-Halogenalkyl, (C₁-C₆)-Alkoxy, (C₁-C₆)-Alkoxy(C₁-C₆)-Alkyl, (C₁-C₆)-Alkoxycarbonyl, -(CH₂)ₘNR₄R₅, -C(O)NH(R₆), (C₃-C₈)-Cycloalkyl, -Aryl, -Heteroaryl und (C₃-C₆)-Heterocycloalkyl; wobei das
(C₃-C₈)-Cycloalkyl, -Aryl, -Heteroaryl und (C₃-C₆)-Heterocycloalkyl ferner wahlweise durch einen oder mehrere Substituenten, ausgewählt aus der Gruppe, bestehend aus
(C₁-C₁₀)-Alkyl, -Alkanoyl, (C₁-C₆)-Alkoxycarbonyl, -Oxo, -C(O)NH(R₆), (C₁-C₆)-Alkoxy(C₁-C₆)-Alkyl, substituiert ist;
**R₃** H ist, und
**R₂** unabhängig ausgewählt ist aus der Gruppe, bestehend aus (C₁-C₆)-Alkyl und einer Gruppe der Formel **J** 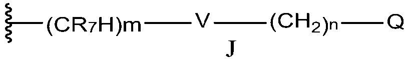
wobei
**V** fehlt oder eine zweiwertige Gruppe ist, ausgewählt aus O, S, S(O)₂, C(O), C(O)O, O(O)C, C(O)N(R₆), N(R₆)C(O); N(R₆)-(CH₂)ₘ-N(R₆), -N(R₆)-;
**Q** ausgewählt ist aus der Gruppe, bestehend aus -CN, -OH, (C₁-C₆)-Alkyl, (C₁-C₆)-Hydroxyalkyl, (C₁-C₆)-Halogenalkyl, (C₁-C₆)-Alkoxy, (C₁-C₆)-Alkoxycarbonyl, -Hydroxycarbonyl, -
(CH₂)ₘNR₄R₅, -C(O)NR₄R₅, -N(R₆)C(O)R₆, -CH(CN)NR₄R₅, (C₃-C₈)-Cycloalkyl, -Aryl, -Heteroaryl und (C₃-C₆)-Heterocycloalkyl;
wobei das (C₃-C₈)-Cycloalkyl, -Aryl, -Heteroaryl und (C₃-C₆)-Heterocycloalkyl ferner wahlweise durch einen oder mehrere Substituenten, ausgewählt aus der Gruppe, bestehend aus
-OH, -Oxo, (C₁-C₁₀)-Alkyl, (C₁-C₁₀)-Alkyl-S(O)₂-O-, Alkanoyl, (C₁-C₆)-Hydroxyalkyl,
(C₁-C₆)-Alkoxy(C₁-C₆)-Alkyl, (C₁-C₆)-Alkoxycarbonyl, (C₁-C₆)-Alkoxycarbonyl-NH-, -N(R₆)(CH₂)ₘC(O)NR₄R₅, -NR₄R₅, (C₃-C₆)-Heterocycloalkyl substituiert sind;
**n und m bei jedem Vorkommen unabhängig** 0 oder eine ganze Zahl, ausgewählt aus 1, 2, 3 oder 4, sind;
**R₄ und R₅,** gleich oder verschieden, ausgewählt sind aus der Gruppe, bestehend aus
-H,
(C₁-C₆)-Alkyl,
(C₁-C₆)-Halogenalkyl,
(C₁-C₆)-Hydroxyalkyl,
-Alkanoyl, (C₁-C₆)-Alkoxycarbonyl und
(C₃-C₆)-Heterocycloalkyl;
**R₆** bei jedem Vorkommen unabhängig ausgewählt ist aus der Gruppe, bestehend aus -H, (C₁-C₆)-Alkyl, (C₁-C₆)-Hydroxyalkyl und -Alkanoyl,
**R₇** bei jedem Vorkommen unabhängig ausgewählt ist aus der Gruppe, bestehend aus H, (C₁-C₆)-Alkyl, -NR₄R₅
einzelne Enantiomere, Diastereoisomere und Mischungen davon
oder ein pharmazeutisch verträgliches Salz oder Solvat davon.

2. Verbindung nach Anspruch 1, dargestellt durch die Formel la 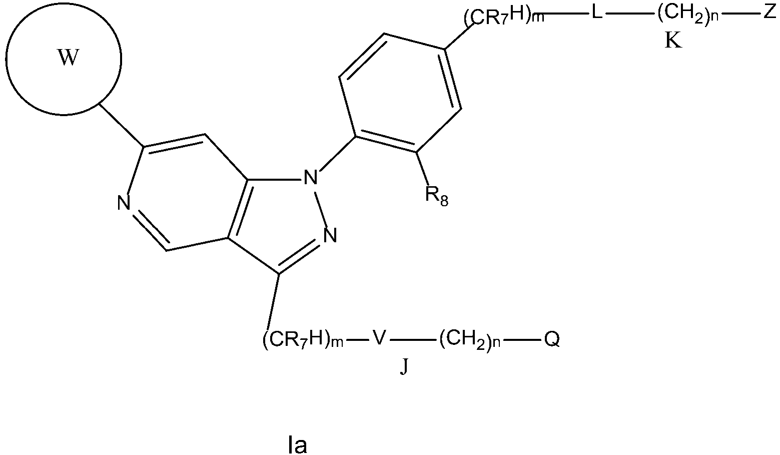 wobei
**R₈** ausgewählt ist aus der Gruppe, bestehend aus
(C₁-C₆)-Alkoxy,
(C₁-C₆)-Haloalkoxy;
**L** N(R₆)S(O)₂, S(O)₂N(R₆) ist;
**Z** ausgewählt ist aus der Gruppe, bestehend aus (C₁-C₆)-Alkyl, (C₁-C₆)-Halogenalkyl, (C₁-C₆)-Alkoxy, (C₁-C₆)-Alkoxycarbonyl, - (CH₂)ₘNR₄R₅, -C(O)NH(R₆), (C₃-C₈)-Cycloalkyl, -Aryl, -Heteroaryl und (C₃-C₆)-Heterocycloalkyl; das (C₃-C₈)-Cycloalkyl, -Aryl, -Heteroaryl und (C₃-C₆)-Heterocycloalkyl wahlweise durch einen oder mehrere Substituenten, ausgewählt aus der Gruppe, bestehend aus
(C₁-C₁₀)-Alkyl, -Alkanoyl, (C₁-C₆)-Alkoxycarbonyl, -Oxo, -C(O)NH(R₆), (C₁-C₆)-Alkoxy(C₁-C₆)-Alkyl, substituiert sind;
**V** fehlt oder eine divalente Gruppe, bestehend aus -N(R₆)-, ist,
**Q** ausgewählt ist aus der Gruppe, bestehend aus (C₁-C₆)-Alkyl, - (CH₂)ₘNR₄R₅ und (C₃-C₆)-Heterocycloalkyl; wobei das (C₃-C₆)-Heterocycloalkyl wahlweise durch ein oder mehrere (C₁-C₁₀)-Alkyl substituiert ist;
wobei **n und m bei jedem Vorkommen unabhängig** 0 oder eine ganze Zahl von 1 bis 4 sind;
**R₄ und R₅,** gleich oder verschieden, ausgewählt sind aus der Gruppe, bestehend aus
-H,
(C₁-C₆)-Alkyl,
**R₆** bei jedem Vorkommen unabhängig ausgewählt ist aus der Gruppe, bestehend aus H, (C₁-C₆)-Alkyl, (C₁-C₆)-Hydroxyalkyl,
**R₇** bei jedem Vorkommen unabhängig ausgewählt ist aus der Gruppe, bestehend aus H, (C₁-C₆)-Alkyl,
einzelne Enantiomere, Diastereoisomere und Mischungen davon
oder ein pharmazeutisch verträgliches Salz oder Solvat davon.

3. Verbindung nach Anspruch 2, wobei
**R₈** (C₁-C₆)-Alkoxy ist;
L -N(R₆)S(O)₂- ist;
**Z** ausgewählt ist aus der Gruppe, bestehend aus
(C₁-C₆)-Alkyl; und
(C₃-C₈)-Cycloalkyl;
**V** fehlt oder -N(R₆)- ist,
**Q** ausgewählt ist aus der Gruppe, bestehend aus
-CN,
(C₁-C₆)-Alkyl,
-(CH₂)ₘNR₄R₅ und
(C₃-C₆)-Heterocycloalkyl;
in Gruppe K n 0 ist und m 0 oder 1 ist; und in Gruppe J n 0, 1 oder 2 ist und m 0 oder 1 ist;
**R₄ und R₅,** gleich oder verschieden, ausgewählt sind aus der Gruppe, bestehend aus
-H,
(C₁-C₆)-Alkyl, vorzugsweise Methyl;
R₆ H ist,
R₇ H ist,
einzelne Enantiomere, Diastereoisomere und Mischungen davon
oder pharmazeutisch verträgliche Salze und Solvate davon.

4. Verbindung nach Anspruch 1, ausgewählt aus:
N-(3-Methoxy-4-(3-methyl-6-(pyrazolo[1,5-a]pyrimidin-3-yl)-1H-pyrazolo[4,3-c]pyridin-1-yl)phenyl)methansulfonamid;
N-(3-Methoxy-4-(3-methyl-6-(pyrazolo[1,5-a]pyrimidin-3-yl)-1H-pyrazolo[4,3-c]pyridin-1-yl)phenyl)-1-(tetrahydrofuran-2-yl)methansulfonamid;
N-(3-Methoxy-4-(3-methyl-6-(pyrazolo[1,5-a]pyrimidin-3-yl)-1H-pyrazolo[4,3-c]pyridin-1-yl)phenyl)tetrahydro-2H-pyran-4-sulfonamid;
N-(3-Methoxy-4-(3-methyl-6-(pyrazolo[1,5-a]pyrimidin-3-yl)-1H-pyrazolo[4,3-c]pyridin-1-yl)phenyl)propan-1-sulfonamid;
1-Cyclopropyl-N-(3-methoxy-4-(3-methyl-6-(pyrazolo[1,5-a]pyrimidin-3-yl)-1H-pyrazolo[4,3-c]pyridin-1-yl)phenyl)methansulfonamid;
N-(3-Methoxy-4-(3-methyl-6-(pyrazolo[1,5-a]pyrimidin-3-yl)-1H-pyrazolo[4,3-c]pyridin-1-yl)phenyl)cyclopropansulfonamid;
N-(3-(Difluormethoxy)-4-(3-methyl-6-(pyrazolo[1,5-a]pyrimidin-3-yl)-1H-pyrazolo[4,3-c]pyridin-1-yl)phenyl)methansulfonamid;
N-(3-Methoxy-4-(3-methyl-6-(pyrazolo[1,5-a]pyrimidin-3-yl)-1H-pyrazolo[4,3-c]pyridin-1-yl)phenyl)oxetan-3-sulfonamid;
N-(3-Methoxy-4-(3-methyl-6-(pyrazolo[1,5-a]pyrimidin-3-yl)-1H-pyrazolo[4,3-c]pyridin-1-yl)phenyl)tetrahydrofuran-3-sulfonamid;
N-(3-Methoxy-4-(3-methyl-6-(pyrazolo[1,5-a]pyrimidin-3-yl)-1H-pyrazolo[4,3-c]pyridin-1-yl)phenyl)-1-methyl-1H-pyrazol-4-sulfonamid;
N-(5-Methoxy-6-(3-methyl-6-(pyrazolo[1,5-a]pyrimidin-3-yl)-1H-pyrazolo[4,3-c]pyridin-1-yl)pyridin-3-yl)methansulfonamid;
N-(2-Fluor-5-methoxy-4-(3-methyl-6-(pyrazolo[1,5-a]pyrimidin-3-yl)-1H-pyrazolo[4,3-c]pyridin-1-yl)phenyl)methansulfonamid;
3-(N-(3-Methoxy-4-(3-methyl-6-(pyrazolo[1,5-a]pyrimidin-3-yl)-1H-pyrazolo[4,3-c]pyridin-1-yl)phenyl)sulfamoyl)propanamid;
2-(N-(3-Methoxy-4-(3-methyl-6-(pyrazolo[1,5-a]pyrimidin-3-yl)-1H-pyrazolo[4,3-c]pyridin-1-yl)phenyl)sulfamoyl)acetamid;
3-Methoxy-4-(3-methyl-6-(pyrazolo[1,5-a]pyrimidin-3-yl)-1H-pyrazolo[4,3-c]pyridin-1-yl)benzonitril;
4-Methoxy-N-methyl-5-(3-methyl-6-(pyrazolo[1,5-a]pyrimidin-3-yl)-1H-pyrazolo[4,3-c]pyridin-1-yl)pyridin-2-amin;
3-Methoxy-4-(3-methyl-6-(pyrazolo[1,5-a]pyrimidin-3-yl)-1H-pyrazolo[4,3-c]pyridin-1-yl)anilin;
1-(3-Methoxy-4-(3-methyl-6-(pyrazolo[1,5-a]pyrimidin-3-yl)-1H-pyrazolo[4,3-c]pyridin-1-yl)phenyl)-3-methylharnstoff;
3-Methoxy-N-methyl-4-(3-methyl-6-(pyrazolo[1,5-a]pyrimidin-3-yl)-1H-pyrazolo[4,3-c]pyridin-1-yl)benzamid;
3-Methoxy-4-(3-methyl-6-(pyrazolo[1,5-a]pyrimidin-3-yl)-1H-pyrazolo[4,3-c]pyridin-1-yl)-N-phenylbenzamid;
tert-Butyl (3-((1-(2-methoxy-4-(methylsulfonamido)phenyl)-6-(pyrazolo[1,5-a]pyrimidin-3-yl)-1H-pyrazolo[4,3-c]pyridin-3-yl)amino)propyl)carbamat;
N-(4-(3-((3-(Dimethylamino)propyl)amino)-6-(pyrazolo[1,5-a]pyrimidin-3-yl)-1H-pyrazolo[4,3-c]pyridin-1-yl)-3-methoxyphenyl)methansulfonamid;
N-(3-Methoxy-4-(3-(methylamino)-6-(pyrazolo[1,5-a]pyrimidin-3-yl)-1H-pyrazolo[4,3-c]pyridin-1-yl)phenyl)methansulfonamid;
N-(3-Methoxy-4-(3-((2-morpholinoethyl)amino)-6-(pyrazolo[1,5-a]pyrimidin-3-yl)-1H-pyrazolo[4,3-c]pyridin-1-yl)phenyl)methansulfonamid;
N-(3-(Difluormethoxy)-4-(3-((2-morpholinoethyl)amino)-6-(pyrazolo[1,5-a]pyrimidin-3-yl)-1H-pyrazolo[4,3-c]pyridin-1-yl)phenyl)cyclopropansulfonamid;
N-(4-(3-((2-Hydroxyethyl)amino)-6-(pyrazolo[1,5-a]pyrimidin-3-yl)-1H-pyrazolo[4,3-c]pyridin-1-yl)-3-methoxyphenyl)methansulfonamid;
N-(3-Methoxy-4-(3-(((1-(2-methoxyethyl)piperidin-4-yl)methyl)amino)-6-(pyrazolo[1,5-a]pyrimidin-3-yl)-1H-pyrazolo[4,3-c]pyridin-1-yl)phenyl)methansulfonamid;
N-(3-Methoxy-4-(3-(((4-methylmorpholin-2-yl)methyl)amino)-6-(pyrazolo[1,5-a]pyrimidin-3-yl)-1H-pyrazolo[4,3-c]pyridin-1-yl)phenyl)methansulfonamid;
N-(6-(3-((2-(Dimethylamino)ethyl)amino)-6-(pyrazolo[1,5-a]pyrimidin-3-yl)-1H-pyrazolo[4,3-c]pyridin-1-yl)-5-methoxypyridin-3-yl)cyclopropansulfonamid;
N-(4-(3-((2-(3-Oxa-8-azabicyclo[3.2.1]octan-8-yl)ethyl)amino)-6-(pyrazolo[1,5-a]pyrimidin-3-yl)-1H-pyrazolo[4,3-c]pyridin-1-yl)-3-methoxyphenyl)methansulfonamid;
N-(4-(3-((2-(Dimethylamino)ethyl)amino)-6-(pyrazolo[1,5-a]pyrimidin-3-yl)-1H-pyrazolo[4,3-c]pyridin-1-yl)-3-methoxyphenyl)methansulfonamid;
N-(4-(3-((2-(Dimethylamino)ethyl)amino)-6-(pyrazolo[1,5-a]pyrimidin-3-yl)-1H-pyrazolo[4,3-c]pyridin-1-yl)-3-methoxyphenyl)cyclopropansulfonamid;
N-(3-Methoxy-4-(3-((3-morpholinopropyl)amino)-6-(pyrazolo[1,5-a]pyrimidin-3-yl)-1H-pyrazolo[4,3-c]pyridin-1-yl)phenyl)cyclopropansulfonamid;
N-(3-Methoxy-4-(3-((2-morpholinoethyl)amino)-6-(pyrazolo[1,5-a]pyrimidin-3-yl)-1H-pyrazolo[4,3-c]pyridin-1-yl)phenyl)cyclopropansulfonamid;
N-(3-Methoxy-4-(3-(methylamino)-6-(pyrazolo[1,5-a]pyrimidin-3-yl)-1H-pyrazolo[4,3-c]pyridin-1-yl)phenyl)cyclopropansulfonamid;
N-(3-Methoxy-4-(3-((2-(piperidin-1-yl)ethyl)amino)-6-(pyrazolo[1,5-a]pyrimidin-3-yl)-1H-pyrazolo[4,3-c]pyridin-1-yl)phenyl)cyclopropansulfonamid;
N-(3-Methoxy-4-(3-((2-(piperidin-1-yl)ethyl)amino)-6-(pyrazolo[1,5-a]pyrimidin-3-yl)-1H-pyrazolo[4,3-c]pyridin-1-yl)phenyl)methansulfonamid;
N-(3-Methoxy-4-(3-((2-(4-methylpiperazin-1-yl)ethyl)amino)-6-(pyrazolo[1,5-a]pyrimidin-3-yl)-1H-pyrazolo[4,3-c]pyridin-1-yl)phenyl)methansulfonamid;
N-(4-(3-((2-((2-Fluorethyl)(methyl)amino)ethyl)amino)-6-(pyrazolo[1,5-a]pyrimidin-3-yl)-1H-pyrazolo[4,3-c]pyridin-1-yl)-3-methoxyphenyl)methansulfonamid;
N-(3-Methoxy-4-(3-(methyl(2-morpholinoethyl)amino)-6-(pyrazolo[1,5-a]pyrimidin-3-yl)-1H-pyrazolo[4,3-c]pyridin-1-yl)phenyl)methansulfonamid;
N-(1-(2-Methoxy-4-(methylsulfonamido)phenyl)-6-(pyrazolo[1,5-a]pyrimidin-3-yl)-1H-pyrazolo[4,3-c]pyridin-3-yl)-1-methylpiperidin-4-carboxamid;
N-(1-(2-Methoxy-4-(methylsulfonamido)phenyl)-6-(pyrazolo[1,5-a]pyrimidin-3-yl)-1H-pyrazolo[4,3-c]pyridin-3-yl)-1-methylpyrrolidin-3-carboxamid;
N-(3-(Dimethylamino)propyl)-1-(2-methoxy-4-(methylsulfonamido)phenyl)-6-(pyrazolo[1,5-a]pyrimidin-3-yl)-1H-pyrazolo[4,3-c]pyridin-3-carboxamid;
N-(2-(Dimethylamino)ethyl)-1-(2-methoxy-4-(methylsulfonamido)phenyl)-6-(pyrazolo[1,5-a]pyrimidin-3-yl)-1H-pyrazolo[4,3-c]pyridin-3-carboxamid;
1-(2-Methoxy-4-(methylsulfonamido)phenyl)-N-(2-morpholinoethyl)-6-(pyrazolo[1,5-a]pyrimidin-3-yl)-1H-pyrazolo[4,3-c]pyridin-3-carboxamid;
N-(3-(Dimethylamino)propyl)-1-(2-methoxy-4-(propylsulfonamido)phenyl)-6-(pyrazolo[1,5-a]pyrimidin-3-yl)-1H-pyrazolo[4,3-c]pyridin-3-carboxamid;
1-(2-Methoxy-4-(propylsulfonamido)phenyl)-N-(3-morpholinopropyl)-6-(pyrazolo[1,5-a]pyrimidin-3-yl)-1H-pyrazolo[4,3-c]pyridin-3-carboxamid;
N-(5-Methoxy-6-(3-methyl-6-(pyrazolo[1,5-a]pyrimidin-3-yl)-1H-pyrazolo[4,3-c]pyridin-1-yl)pyridin-3-yl)benzamid;
3-Methoxy-N-methyl-4-(3-methyl-6-(pyrazolo[1,5-a]pyrimidin-3-yl)-1H-pyrazolo[4,3-c]pyridin-1-yl) benzolsulfonamid;
3-Methoxy-N-methyl-4-(3-((2-morpholinoethyl)amino)-6-(pyrazolo[1,5-a]pyrimidin-3-yl)-1H-pyrazolo[4,3-c]pyridin-1-yl)benzolsulfonamid;
3-Methoxy-N-methyl-4-(3-(methylamino)-6-(pyrazolo[1,5-a]pyrimidin-3-yl)-1H-pyrazolo[4,3-c]pyridin-1-yl)benzolsulfonamid;
4-(3-((3-(Dimethylamino)propyl)amino)-6-(pyrazolo[1,5-a]pyrimidin-3-yl)-1H-pyrazolo[4,3-c]pyridin-1-yl)-3-methoxy-N-methylbenzolsulfonamid;
N-(3-Methoxy-4-(3-((2-morpholinoethyl)amino)-6-(pyrazolo[1,5-a]pyrimidin-3-yl)-1H-pyrazolo[4,3-c]pyridin-1-yl)benzyl)methansulfonamid;
N-(3-Methoxy-4-(3-((2-(piperidin-1-yl)ethyl)amino)-6-(pyrazolo[1,5-a]pyrimidin-3-yl)-1H-pyrazolo[4,3-c]pyridin-1-yl)benzyl)methansulfonamid;
N-(3-Methoxy-4-(3-(methylamino)-6-(pyrazolo[1,5-a]pyrimidin-3-yl)-1H-pyrazolo[4,3-c]pyridin-1-yl)benzyl)methansulfonamid;
N-(4-(3-((2-(Dimethylamino)ethyl)amino)-6-(pyrazolo[1,5-a]pyrimidin-3-yl)-1H-pyrazolo[4,3-c]pyridin-1-yl)-3-methoxybenzyl)methansulfonamid;
N-(2-Chlor-5-methoxy-4-(3-(methylamino)-6-(pyrazolo[1,5-a]pyrimidin-3-yl)-1H-pyrazolo[4,3-c]pyridin-1-yl)benzyl)methansulfonamid;
N-(2-Chlor-5-methoxy-4-(3-((2-morpholinoethyl)amino)-6-(pyrazolo[1,5-a]pyrimidin-3-yl)-1H-pyrazolo[4,3-c]pyridin-1-yl)benzyl)methansulfonamid;
N-(3-(Dimethylamino)propyl)-1-(2-methoxy-4-(methylsulfonamidomethyl)phenyl)-6-(pyrazolo[1,5-a]pyrimidin-3-yl)-1H-pyrazolo[4,3-c]pyridin-3-carboxamid;
N-(4-(3-(Cyanomethyl)-6-(pyrazolo[1,5-a]pyrimidin-3-yl)-1H-pyrazolo[4,3-c]pyridin-1-yl)-3-methoxybenzyl)methansulfonamid;
N-(3-(Difluormethoxy)-4-(3-methyl-6-(pyrazolo[1,5-a]pyrimidin-3-yl)-1H-pyrazolo[4,3-c]pyridin-1-yl)benzyl)methansulfonamid;
N-(1-(3-Methoxy-4-(3-methyl-6-(pyrazolo[1,5-a]pyrimidin-3-yl)-1H-pyrazolo[4,3-c]pyridin-1-yl)phenyl)ethyl)methansulfonamid;
N-(3-Methoxy-4-(3-methyl-6-(pyrazolo[1,5-a]pyrimidin-3-yl)-1H-pyrazolo[4,3-c]pyridin-1-yl)benzyl)methansulfonamid;
N-(3-Methoxy-4-(3-methyl-6-(pyrazolo[1,5-a]pyrimidin-3-yl)-1H-pyrazolo[4,3-c]pyridin-1-yl)benzyl)cyclopropansulfonamid;
N-(3-Methoxy-4-(3-methyl-6-(pyrazolo[1,5-a]pyrimidin-3-yl)-1H-pyrazolo[4,3-c]pyridin-1-yl)benzyl)-N-methylmethansulfonamid;
(3-Methoxy-4-(3-methyl-6-(pyrazolo[1,5-a]pyrimidin-3-yl)-1H-pyrazolo[4,3-c]pyridin-1-yl)phenyl)methanol;
(3-Methoxy-4-(3-methyl-6-(pyrazolo[1,5-a]pyrimidin-3-yl)-1H-pyrazolo[4,3-c]pyridin-1-yl)phenyl)methanamin;
1-Cyano-N-(3-methoxy-4-(3-methyl-6-(pyrazolo[1,5-a]pyrimidin-3-yl)-1H-pyrazolo[4,3-c]pyridin-1-yl)benzyl)methansulfonamid;
Methyl (3-methoxy-4-(3-methyl-6-(pyrazolo[1,5-a]pyrimidin-3-yl)-1H-pyrazolo[4,3-c]pyridin-1-yl)benzyl)carbamat;
N-(3-Methoxy-4-(3-methyl-6-(pyrazolo[1,5-a]pyrimidin-3-yl)-1H-pyrazolo[4,3-c]pyridin-1-yl)benzyl)acetamid;
1-(3-Methoxy-4-(3-methyl-6-(pyrazolo[1,5-a]pyrimidin-3-yl)-1H-pyrazolo[4,3-c]pyridin-1-yl)benzyl)-3-methylharnstoff;
1-(3-Methoxy-4-(3-methyl-6-(pyrazolo[1,5-a]pyrimidin-3-yl)-1H-pyrazolo[4,3-c]pyridin-1-yl)benzyl)-3-phenylharnstoff;
2,2-Difluor-N-(3-methoxy-4-(3-methyl-6-(pyrazolo[1,5-a]pyrimidin-3-yl)-1H-pyrazolo[4,3-c]pyridin-1-yl)phenyl)ethan-1-sulfonamid;
N-(3-Methoxy-4-(3-methyl-6-(pyrazolo[1,5-a]pyrimidin-3-yl)-1H-pyrazolo[4,3-c]pyridin-1-yl)phenyl)benzamid;
N-(4-(3-((2-(Dimethylamino)ethyl)amino)-6-(pyrazolo[1,5-a]pyrimidin-3-yl)-1H-pyrazolo[4,3-c]pyridin-1-yl)-3-methoxyphenyl)benzamid;
N-(4-(6-(Imidazo[1,2-b]pyridazin-3-yl)-3-((2-morpholinoethyl)amino)-1H-pyrazolo[4,3-c]pyridin-1-yl)-3-methoxyphenyl)methansulfonamid;
N-(4-(3-((2-(Dimethylamino)ethyl)amino)-6-(imidazo[1,2-b]pyridazin-3-yl)-1H-pyrazolo[4,3-c]pyridin-1-yl)-3-methoxyphenyl)methansulfonamid;
N-(4-(3-(((1s,3s)-3-(Dimethylamino)cyclobutyl)amino)-6-(pyrazolo[1,5-a]pyrimidin-3-yl)-1H-pyrazolo[4,3-c]pyridin-1-yl)-3-methoxyphenyl)cyclopropansulfonamid;
N-(4-(3-Amino-6-(pyrazolo[1,5-a]pyrimidin-3-yl)-1H-pyrazolo[4,3-c]pyridin-1-yl)-3-methoxyphenyl)methansulfonamid;
N-(1-(2-Methoxy-4-(methylsulfonamido)phenyl)-6-(pyrazolo[1,5-a]pyrimidin-3-yl)-1H-pyrazolo[4,3-c]pyridin-3-yl)-2-(methylamino)acetamid;
N-(1-(2-Methoxy-4-(methylsulfonamido)phenyl)-6-(pyrazolo[1,5-a]pyrimidin-3-yl)-1H-pyrazolo[4,3-c]pyridin-3-yl)-2-(4-methylpiperazin-1-yl)acetamid;
N-(1-(2-Methoxy-4-(propylsulfonamido)phenyl)-6-(pyrazolo[1,5-a]pyrimidin-3-yl)-1H-pyrazolo[4,3-c]pyridin-3-yl)-3-(4-methylpiperazin-1-yl)propanamid;
N-(3-Methoxy-4-(3-((methylamino)methyl)-6-(pyrazolo[1,5-a]pyrimidin-3-yl)-1H-pyrazolo[4,3-c]pyridin-1-yl)phenyl)propan-1-sulfonamid;
N-(4-(3-(Cyanomethyl)-6-(pyrazolo[1,5-a]pyrimidin-3-yl)-1H-pyrazolo[4,3-c]pyridin-1-yl)-3-methoxyphenyl)cyclopropansulfonamid;
N-(3-Methoxy-4-(3-methyl-6-((3-oxo-3,4-dihydropyrazin-2-yl)amino)-1H-pyrazolo[4,3-c]pyridin-1-yl)phenyl)methansulfonamid;
N-(3-Methoxy-4-(3-methyl-6-((3-oxo-3,4-dihydropyrazin-2-yl)amino)-1H-pyrazolo[4,3-c]pyridin-1-yl)phenyl)-1-methyl-1H-pyrazol-4-sulfonamid;
N-(3-Methoxy-4-(3-((2-morpholinoethyl)amino)-6-((3-oxo-3,4-dihydropyrazin-2-yl)amino)-1H-pyrazolo[4,3-c]pyridin-1-yl)phenyl)methansulfonamid;
N-(3-Methoxy-4-(6-((3-oxo-3,4-dihydropyrazin-2-yl)amino)-3-((2-(piperidin-1-yl)ethyl)amino)-1H-pyrazolo[4,3-c]pyridin-1-yl)phenyl)propan-1-sulfonamid;
N-(3-(Difluormethoxy)-4-(3-((2-morpholinoethyl)amino)-6-((3-oxo-3,4-dihydropyrazin-2-yl)amino)-1H-pyrazolo[4,3-c]pyridin-1-yl)phenyl)propan-1-sulfonamid;
N-(4-(3-((2-(3-Oxa-8-azabicyclo[3.2.1]octan-8-yl)ethyl)amino)-6-((3-oxo-3,4-dihydropyrazin-2-yl)amino)-1H-pyrazolo[4,3-c]pyridin-1-yl)-3-methoxyphenyl)methansulfonamid;
N-(3-Methoxy-4-(3-(methylamino)-6-((3-oxo-3,4-dihydropyrazin-2-yl)amino)-1H-pyrazolo[4,3-c]pyridin-1-yl)phenyl)propan-1-sulfonamid;
N-(3-Methoxy-4-(3-((2-morpholinoethyl)amino)-6-((3-oxo-3,4-dihydropyrazin-2-yl)amino)-1H-pyrazolo[4,3-c]pyridin-1-yl)benzyl)methansulfonamid;
einzelne Enantiomere, Diastereoisomere und Mischungen davon
oder ein pharmazeutisch verträgliches Salz oder Solvat davon.

5. Pharmazeutische Zusammensetzung, umfassend eine Verbindung wie in einem der Ansprüche 1 bis 4 definiert oder ein pharmazeutisch verträgliches Salz davon, in Beigabe mit einem oder mehreren pharmazeutisch verträglichen Trägern oder Hilfsstoffen.

6. Zur Verabreichung durch Inhalation geeignete pharmazeutische Zusammensetzung nach Anspruch 5, ausgewählt aus inhalierbaren Pulvern, treibgashaltigen Dosieraerosolen oder treibgasfreien inhalierbaren Formulierungen.

7. Vorrichtung, umfassend die pharmazeutische Zusammensetzung nach Anspruch 6, die ein Trockenpulverinhalator für Einzel- oder Mehrfachdosen, ein Dosierinhalator oder ein Zerstäuber für sanftem Nebel sein kann.

8. Verbindung oder pharmazeutische Zusammensetzung nach einem der Ansprüche 1 bis 6 zur Verwendung als ein Arzneimittel.

9. Verbindung oder pharmazeutische Zusammensetzung zur Verwendung nach Anspruch 8 bei der Vorbeugung und/oder Behandlung einer Lungenkrankheit, ausgewählt aus der Gruppe, bestehend aus Asthma, chronisch obstruktiver Lungenkrankheit COPD, idiopathischer Lungenfibrose (IPF), akuter Lungenverletzung und akutem Atemnotsyndrom (ARDS).

10. Kombination einer Verbindung wie in einem der Ansprüche 1 bis 4 definiert mit einem oder mehreren Wirkstoffen, ausgewählt aus Beta2-Agonisten, Antimuskarinika, Kortikosteroiden, Hemmern der Mitogen-aktivierten-Kinase (P38 MAP-Kinase-Hemmer), Hemmern der Nuclear-Factor-Kappa-B-Kinase-Untereinheit Beta (IKK2-Hemmer), Hemmern der Humanen Neutrophilen Elastase (HNE-Hemmer), Hemmern der Phosphodiesterase 4 (PDE4-Hemmer), Leukotrien-Modulatoren, nicht-steroidalen Antirheumatika (NSAIDs) und Schleim-Regulatoren.

## Revendications

1. Composé représenté par la formule I 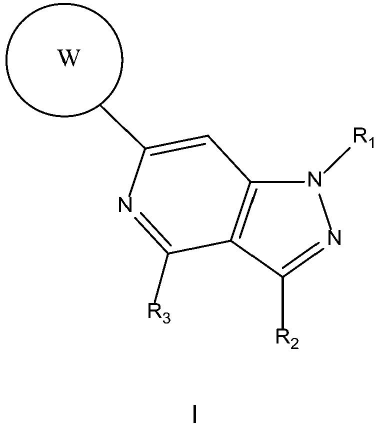 dans lequel,
**W est** un hétéroaryle choisi parmi pyrazolo[1,5-a]pyrimidin-3-yl, imidazo[1,2-b]pyridazin-3-yl et (3-oxo-3,4-dihydropyrazin-2-yl)amino ;
**R₁** est choisi dans le groupe de pyridinyle ou phényle substitué par un groupe ou plus, de préférence 2 ou 3 groupes, indépendamment choisis parmi
halogène,
-OH,
-CN
-NO₂
-(CH₂)ₘNR₄R₅,
alkyle en (C₁-C₆),
hydroxyalkyle en (C₁-C₆),
alcoxy en (C₁-C₆),
alkylthio en (C₁-C₆)-
haloalkyle en (C₁-C₆),
haloalcoxy en (C₁-C₆),
et R₁ est substitué par au moins un groupe de formule **K** 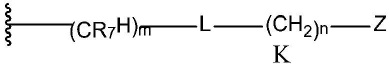 en position para par rapport au point d'attachement de R1 avec le reste de la molécule,
dans lequel
**L** est absent ou est un groupe divalent choisi parmi O, S, S(O)₂, (CO), C(O)O, O(O)C, C(O)N(R₆), N(R₆)C(O), NHCONH, N(R₆)S(O)₂, S(O)₂N(R₆) ;
**Z** est choisi dans le groupe constitué de H, -OH, -CN, -NO₂, alkyle en (C₁-C₆), hydroxyalkyle en (C₁-C₆), haloalkyle en (C₁-C₆), alcoxy en (C₁-C₆), alcoxy en (C₁-C₆)-alkyle en (C₁-C₆), alcoxycarbonyle en (C₁-C₆), -(CH₂)ₘNR₄R₅, - C(O)NH(R₆), cycloalkyle en (C₃-C₈), aryle, hétéroaryle et hétérocycloalkyle en (C₃-C₆) ; dans lequel lesdits
cycloalkyle en (C₃-C₈), aryle, hétéroaryle et hétérocycloalkyle en (C₃-C₆) sont facultativement en outre substitués par un ou plusieurs substituants choisis dans le groupe constitué de
alkyle en (C₁-C₁₀), alcanoyle, alcoxycarbonyle en (C₁-C₆), oxo, - C(O)NH(Rₑ), alcoxy en (C₁-C₆)-alkyle en (C₁-C₆) ;
**R₃** est H, et
**R₂ est** choisi indépendamment dans le groupe constitué d'alkyle en (C₁-C₆), et d'un groupe de formule **J** 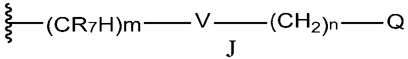
dans lequel
**V** est absent ou est un groupe divalent choisi parmi O, S, S(O)₂, C(O), C(O)O, O(O)C, C(O)N(R₆), N(R₆)C(O) ; N(R₆)-(CH₂)ₘ-N(R₆), -N(R₆)- ;
**Q** est choisi dans le groupe constitué de -CN, -OH, alkyle en (C₁-C₆), hydroxyalkyle en (C₁-C₆), haloalkyle en (C₁-C₆), alcoxy en (C₁-C₆), alcoxycarbonyle en (C₁-C₆), hydroxycarbonyle, -(CH₂)ₘNR₄R₅, -C(O)NR₄R₅, - N(R₆)C(O)R₆, -CH(CN)NR₄R₅, cycloalkyle en (C₃-C₈), aryle, hétéroaryle et hétérocycloalkyle en (C₃-C₆) ;
dans lequel lesdits cycloalkyle en (C₃-C₈), aryle, hétéroaryle et hétérocycloalkyle en (C₃-C₆) sont facultativement en outre substitués par un ou plusieurs substituants choisis dans le groupe constitué de
-OH, oxo, alkyle en (C₁-C₁₀), alkyle en (C₁-C₁₀)-S(O)₂-O-, alcanoyle, hydroxyalkyle en (C₁-C₆),
alcoxy en (C₁-C₆)-alkyle en (C₁-C₆), alcoxycarbonyle en (C₁-C₆), alcoxycarbonyle en (C₁-C₆)-NH-, -N(R₆)(CH₂)ₘC(O)NR₄R₅, -NR₄R₅, hétérocycloalkyle en (C₃-C₆) ;
**n et m valent dans chaque occurrence indépendamment** 0 ou un nombre entier choisi parmi 1, 2, 3 et 4 ;
**R₄ et R₅,** identiques ou différents, sont choisis dans le groupe constitué de
-H,
alkyle en (C₁-C₆),
haloalkyle en (C₁-C₆),
hydroxyalkyle en (C₁-C₆),
alcanoyle, alcoxycarbonyle en (C₁-C₆), et
hétérocycloalkyle en (C₃-C₆) ;
**R₆** est dans chaque occurrence indépendamment choisi dans le groupe constitué de H, alkyle en (C₁-C₆), hydroxyalkyle en (C₁-C₆), et alcanoyle,
**R₇** est dans chaque occurrence indépendamment choisi dans le groupe constitué de H, alkyle en (C₁-C₆), -NR₄R₅
énantiomères simples, diastéréo-isomères et mélanges de ceux-ci
ou sel ou solvate pharmaceutiquement acceptable de ceux-ci.

2. Composé selon la revendication 1 représenté par la formule la 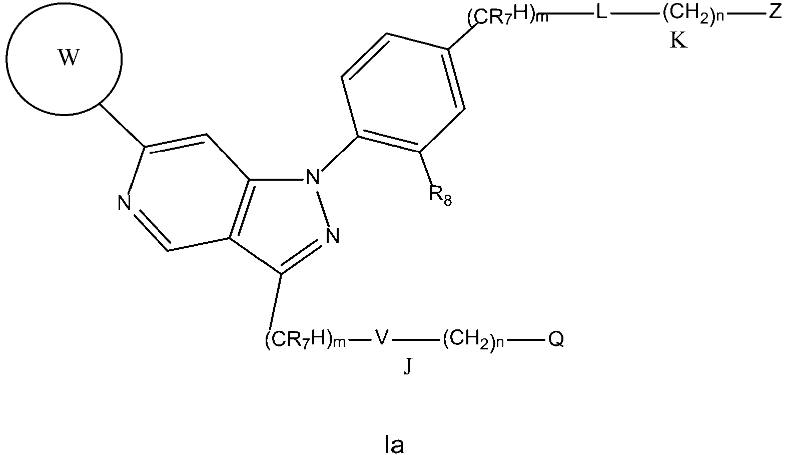 dans lequel
**R₈** est choisi dans le groupe constitué de
alcoxy en (C₁-C₆),
haloalcoxy en (C₁-C₆) ;
**L** est N(R₆)S(O)₂, S(O)₂N(R₆) ;
**Z** est choisi dans le groupe constitué d'alkyle en (C₁-C₆), haloalkyle en (C₁-C₆), alcoxy en (C₁-C₆), alcoxycarbonyle en (C₁-C₆), -(CH₂)ₘNR₄R₅, - C(O)NH(R₆), cycloalkyle en (C₃-C₈), aryle, hétéroaryle et hétérocycloalkyle en (C₃-C₆) ; lesdits cycloalkyle en (C₃-C₈), aryle, hétéroaryle et hétérocycloalkyle en (C₃-C₆) étant facultativement en outre substitués par un ou plusieurs substituants choisis dans le groupe constitué de
alkyle en (C₁-C₁₀), alcanoyle, alcoxycarbonyle en (C₁-C₆), oxo, - C(O)NH(R₆), alcoxy en (C₁-C₆)-alkyle en (C₁-C₆) ;
**V** est absent ou est un groupe divalent constitué de -N(R₆)-,
**Q** est choisi dans le groupe constitué d'alkyle en (C₁-C₆), -(CH₂)ₘNR₄R₅ et hétérocycloalkyle en (C₃-C₆) ; ledit hétérocycloalkyle en (C₃-C₆) étant facultativement substitué par un ou plusieurs alkyles en (C₁-C₁₀) ;
dans lequel **n et m valent dans chaque occurrence indépendamment** 0 ou un nombre entier allant de 1 à 4 ;
**R₄ et R₅,** identiques ou différents, sont choisis dans le groupe constitué de
-H,
alkyle en (C₁-C₆),
**R₆ est** dans chaque occurrence indépendamment choisi dans le groupe constitué de H, alkyle en (C₁-C₆), hydroxyalkyle en (C₁-C₆),
**R₇ est** dans chaque occurrence indépendamment choisi dans le groupe constitué de H, alkyle (C₁-C₆),
énantiomères simples, diastéréo-isomères et mélanges de ceux-ci
ou un sel ou solvate pharmaceutiquement acceptable de ceux-ci.

3. Composé selon la revendication 2, dans lequel
**R₈ est** alcoxy en (C₁-C₆) ;
**L** est -N(R₆)S(O)₂- ;
**Z** est choisi dans le groupe constitué de
alkyle en (C₁-C₆) ; et
cycloalkyle en (C₃-C₈) ;
**V** est absent ou est -N(R₆)-,
**Q** est choisi dans le groupe constitué de
-CN,
alkyle en (C₁-C₆),
-(CH₂)ₘNR₄R₅, et
hétérocycloalkyle en (C₃-C₆) ;
**n vaut 0 et m vaut 0 ou 1 dans le groupe K** ; **et n vaut 0, 1 ou 2 et m vaut 0 ou 1 dans le groupe J** ;
**R₄ et R₅,** identiques ou différents, sont choisis dans le groupe constitué de
-H,
alkyle en (C₁-C₆), de préférence méthyle ;
R₆ est H,
R₇ est H,
énantiomères simples, diastéréo-isomères et mélanges de ceux-ci
ou sels et solvates pharmaceutiquement acceptables de ceux-ci.

4. Composé selon la revendication 1 choisi parmi :
N-(3-méthoxy-4-(3-méthyl-6-(pyrazolo[1,5-a]pyrimidin-3-yl)-1H-pyrazolo[4,3-c]pyridin-1-yl)phényl)méthanesulfonamide ;
N-(3-méthoxy-4-(3-méthyl-6-(pyrazolo[1,5-a]pyrimidin-3-yl)-1H-pyrazolo[4,3-c]pyridin-1-yl)phényl)-1-(tétrahydrofuran-2-yl)méthanesulfonamide ;
N-(3-méthoxy-4-(3-méthyl-6-(pyrazolo[1,5-a]pyrimidin-3-yl)-1H-pyrazolo[4,3-c]pyridin-1-yl)phényl)tétrahydro-2H-pyran-4-sulfonamide ;
N-(3-méthoxy-4-(3-méthyl-6-(pyrazolo[1,5-a]pyrimidin-3-yl)-1H-pyrazolo[4,3-c]pyridin-1-yl)phényl)propane-1-sulfonamide ;
1-cyclopropyl-N-(3-méthoxy-4-(3-méthyl-6-(pyrazolo[1,5-a]pyrimidin-3-yl)-1H-pyrazolo[4,3-c]pyridin-1-yl)phényl)méthanesulfonamide ;
N-(3-méthoxy-4-(3-méthyl-6-(pyrazolo[1,5-a]pyrimidin-3-yl)-1H-pyrazolo[4,3-c]pyridin-1-yl)phényl)cyclopropanesulfonamide ;
N-(3-(difluorométhoxy)-4-(3-méthyl-6-(pyrazolo[1,5-a]pyrimidin-3-yl)-1H-pyrazolo[4,3-c]pyridin-1-yl)phényl)méthanesulfonamide ;
N-(3-méthoxy-4-(3-méthyl-6-(pyrazolo[1,5-a]pyrimidin-3-yl)-1H-pyrazolo[4,3-c]pyridin-1-yl)phényl)oxétane-3-sulfonamide ;
N-(3-méthoxy-4-(3-méthyl-6-(pyrazolo[1,5-a]pyrimidin-3-yl)-1H-pyrazolo[4,3-c]pyridin-1-yl)phényl)tétrahydrofuran-3-sulfonamide ;
N-(3-méthoxy-4-(3-méthyl-6-(pyrazolo[1,5-a]pyrimidin-3-yl)-1H-pyrazolo[4,3-c]pyridin-1-yl)phényl)-1-méthyl-1H-pyrazole-4-sulfonamide ;
N-(5-méthoxy-6-(3-méthyl-6-(pyrazolo[1,5-a]pyrimidin-3-yl)-1H-pyrazolo[4,3-c]pyridin-1-yl)pyridin-3-yl)méthanesulfonamide ;
N-(2-fluoro-5-méthoxy-4-(3-méthyl-6-(pyrazolo[1,5-a]pyrimidin-3-yl)-1H-pyrazolo[4,3-c]pyridin-1-yl)phényl)méthanesulfonamide ;
3-(N-(3-méthoxy-4-(3-méthyl-6-(pyrazolo[1,5-a]pyrimidin-3-yl)-1H-pyrazolo[4,3-c]pyridin-1-yl)phényl)sulfamoyl)propanamide ;
2-(N-(3-méthoxy-4-(3-méthyl-6-(pyrazolo[1,5-a]pyrimidin-3-yl)-1H-pyrazolo[4,3-c]pyridin-1-yl)phényl)sulfamoyl)acétamide ;
3-méthoxy-4-(3-méthyl-6-(pyrazolo[1,5-a]pyrimidin-3-yl)-1H-pyrazolo[4,3-c]pyridin-1-yl)benzonitrile ;
4-méthoxy-N-méthyl-5-(3-méthyl-6-(pyrazolo[1,5-a]pyrimidin-3-yl)-1H-pyrazolo[4,3-c]pyridin-1-yl)pyridin-2-amine ;
3-méthoxy-4-(3-méthyl-6-(pyrazolo[1,5-a]pyrimidin-3-yl)-1H-pyrazolo[4,3-c]pyridin-1-yl)aniline ;
1-(3-méthoxy-4-(3-méthyl-6-(pyrazolo[1,5-a]pyrimidin-3-yl)-1H-pyrazolo[4,3-c]pyridin-1-yl)phényl)-3-méthylurea ;
3-méthoxy-N-méthyl-4-(3-méthyl-6-(pyrazolo[1,5-a]pyrimidin-3-yl)-1H-pyrazolo[4,3-c]pyridin-1-yl)benzamide ;
3-méthoxy-4-(3-méthyl-6-(pyrazolo[1,5-a]pyrimidin-3-yl)-1H-pyrazolo[4,3-c]pyridin-1-yl)-N-phénylbenzamide ;
tert-butyl (3-((1-(2-méthoxy-4-(méthylsulfonamido)phényl)-6-(pyrazolo[1,5-a]pyrimidin-3-yl)-1H-pyrazolo[4,3-c]pyridin-3-yl)amino)propyl)carbamate ;
N-(4-(3-((3-(diméthylamino)propyl)amino)-6-(pyrazolo[1,5-a]pyrimidin-3-yl)-1H-pyrazolo[4,3-c]pyridin-1-yl)-3-méthoxyphényl)méthanesulfonamide ;
N-(3-méthoxy-4-(3-(méthylamino)-6-(pyrazolo[1,5-a]pyrimidin-3-yl)-1H-pyrazolo[4,3-c]pyridin-1-yl)phényl)méthanesulfonamide ;
N-(3-méthoxy-4-(3-((2-morpholinoéthyl)amino)-6-(pyrazolo[1,5-a]pyrimidin-3-yl)-1H-pyrazolo[4,3-c]pyridin-1-yl)phényl)méthanesulfonamide ;
N-(3-(difluorométhoxy)-4-(3-((2-morpholinoéthyl)amino)-6-(pyrazolo[1,5-a]pyrimidin-3-yl)-1H-pyrazolo[4,3-c]pyridin-1-yl)phényl)cyclopropanesulfonamide ;
N-(4-(3-((2-hydroxyéthyl)amino)-6-(pyrazolo[1,5-a]pyrimidin-3-yl)-1H-pyrazolo[4,3-c]pyridin-1-yl)-3-méthoxyphényl)méthanesulfonamide ;
N-(3-méthoxy-4-(3-(((1-(2-méthoxyéthyl)pipéridin-4-yl)méthyl)amino)-6-(pyrazolo[1,5-a]pyrimidin-3-yl)-1H-pyrazolo[4,3-c]pyridin-1-yl)phényl)méthanesulfonamide ;
N-(3-méthoxy-4-(3-(((4-méthylmorpholin-2-yl)méthyl)amino)-6-(pyrazolo[1,5-a]pyrimidin-3-yl)-1H-pyrazolo[4,3-c]pyridin-1-yl)phényl)méthanesulfonamide ;
N-(6-(3-((2-(diméthylamino)éthyl)amino)-6-(pyrazolo[1,5-a]pyrimidin-3-yl)-1H-pyrazolo[4,3-c]pyridin-1-yl)-5-méthoxypyridin-3-yl)cyclopropanesulfonamide ;
N-(4-(3-((2-(3-oxa-8-azabicyclo[3.2.1]octan-8-yl)éthyl)amino)-6-(pyrazolo[1,5-a]pyrimidin-3-yl)-1H-pyrazolo[4,3-c]pyridin-1-yl)-3-méthoxyphényl)méthanesulfonamide ;
N-(4-(3-((2-(diméthylamino)éthyl)amino)-6-(pyrazolo[1,5-a]pyrimidin-3-yl)-1H-pyrazolo[4,3-c]pyridin-1-yl)-3-méthoxyphényl)méthanesulfonamide ;
N-(4-(3-((2-(diméthylamino)éthyl)amino)-6-(pyrazolo[1,5-a]pyrimidin-3-yl)-1H-pyrazolo[4,3-c]pyridin-1-yl)-3-méthoxyphényl)cyclopropanesulfonamide ;
N-(3-méthoxy-4-(3-((3-morpholinopropyl)amino)-6-(pyrazolo[1,5-a]pyrimidin-3-yl)-1H-pyrazolo[4,3-c]pyridin-1-yl)phényl)cyclopropanesulfonamide ;
N-(3-méthoxy-4-(3-((2-morpholinoéthyl)amino)-6-(pyrazolo[1,5-a]pyrimidin-3-yl)-1H-pyrazolo[4,3-c]pyridin-1-yl)phényl)cyclopropanesulfonamide ;
N-(3-méthoxy-4-(3-(méthylamino)-6-(pyrazolo[1,5-a]pyrimidin-3-yl)-1H-pyrazolo[4,3-c]pyridin-1-yl)phényl)cyclopropanesulfonamide ;
N-(3-méthoxy-4-(3-((2-(pipéridin-1-yl)éthyl)amino)-6-(pyrazolo[1,5-a]pyrimidin-3-yl)-1H-pyrazolo[4,3-c]pyridin-1-yl)phényl)cyclopropanesulfonamide ;
N-(3-méthoxy-4-(3-((2-(pipéridin-1-yl)éthyl)amino)-6-(pyrazolo[1,5-a]pyrimidin-3-yl)-1H-pyrazolo[4,3-c]pyridin-1-yl)phényl)méthanesulfonamide ;
N-(3-méthoxy-4-(3-((2-(4-méthylpipérazin-1-yl)éthyl)amino)-6-(pyrazolo[1,5-a]pyrimidin-3-yl)-1H-pyrazolo[4,3-c]pyridin-1-yl)phényl)méthanesulfonamide ;
N-(4-(3-((2-((2-fluoroéthyl)(méthyl)amino)éthyl)amino)-6-(pyrazolo[1,5-a]pyrimidin-3-yl)-1H-pyrazolo[4,3-c]pyridin-1-yl)-3-méthoxyphényl)méthanesulfonamide ;
N-(3-méthoxy-4-(3-(méthyl(2-morpholinoéthyl)amino)-6-(pyrazolo[1,5-a]pyrimidin-3-yl)-1H-pyrazolo[4,3-c]pyridin-1-yl)phényl)méthanesulfonamide ;
N-(1-(2-méthoxy-4-(méthylsulfonamido)phényl)-6-(pyrazolo[1,5-a]pyrimidin-3-yl)-1H-pyrazolo[4,3-c]pyridin-3-yl)-1-méthylpipéridine-4-carboxamide ;
N-(1-(2-méthoxy-4-(méthylsulfonamido)phényl)-6-(pyrazolo[1,5-a]pyrimidin-3-yl)-1H-pyrazolo[4,3-c]pyridin-3-yl)-1-méthylpyrrolidine-3-carboxamide ;
N-(3-(diméthylamino)propyl)-1-(2-méthoxy-4-(méthylsulfonamido)phényl)-6-(pyrazolo[1,5-a]pyrimidin-3-yl)-1H-pyrazolo[4,3-c]pyridine-3-carboxamide ;
N-(2-(diméthylamino)éthyl)-1-(2-méthoxy-4-(méthylsulfonamido)phényl)-6-(pyrazolo[1,5-a]pyrimidin-3-yl)-1H-pyrazolo[4,3-c]pyridine-3-carboxamide ;
1-(2-méthoxy-4-(méthylsulfonamido)phényl)-N-(2-morpholinoéthyl)-6-(pyrazolo[1,5-a]pyrimidin-3-yl)-1H-pyrazolo[4,3-c]pyridine-3-carboxamide ;
N-(3-(diméthylamino)propyl)-1-(2-méthoxy-4-(propylsulfonamido)phényl)-6-(pyrazolo[1,5-a]pyrimidin-3-yl)-1H-pyrazolo[4,3-c]pyridine-3-carboxamide ;
1-(2-méthoxy-4-(propylsulfonamido)phényl)-N-(3-morpholinopropyl)-6-(pyrazolo[1,5-a]pyrimidin-3-yl)-1H-pyrazolo[4,3-c]pyridine-3-carboxamide ;
N-(5-méthoxy-6-(3-méthyl-6-(pyrazolo[1,5-a]pyrimidin-3-yl)-1H-pyrazolo[4,3-c]pyridin-1-yl)pyridin-3-yl)benzamide ;
3-méthoxy-N-méthyl-4-(3-méthyl-6-(pyrazolo[1,5-a]pyrimidin-3-yl)-1H-pyrazolo[4,3-c]pyridin-1-yl)benzènesulfonamide ;
3-méthoxy-N-méthyl-4-(3-((2-morpholinoéthyl)amino)-6-(pyrazolo[1,5-a]pyrimidin-3-yl)-1H-pyrazolo[4,3-c]pyridin-1-yl)benzènesulfonamide ;
3-méthoxy-N-méthyl-4-(3-(méthylamino)-6-(pyrazolo[1,5-a]pyrimidin-3-yl)-1H-pyrazolo[4,3-c]pyridin-1-yl)benzènesulfonamide ;
4-(3-((3-(diméthylamino)propyl)amino)-6-(pyrazolo[1,5-a]pyrimidin-3-yl)-1H-pyrazolo[4,3-c]pyridin-1-yl)-3-méthoxy-N-méthylbenzènesulfonamide ;
N-(3-méthoxy-4-(3-((2-morpholinoéthyl)amino)-6-(pyrazolo[1,5-a]pyrimidin-3-yl)-1H-pyrazolo[4,3-c]pyridin-1-yl)benzyl)méthanesulfonamide ;
N-(3-méthoxy-4-(3-((2-(pipéridin-1-yl)éthyl)amino)-6-(pyrazolo[1,5-a]pyrimidin-3-yl)-1H-pyrazolo[4,3-c]pyridin-1-yl)benzyl)méthanesulfonamide ;
N-(3-méthoxy-4-(3-(méthylamino)-6-(pyrazolo[1,5-a]pyrimidin-3-yl)-1H-pyrazolo[4,3-c]pyridin-1-yl)benzyl)méthanesulfonamide ;
N-(4-(3-((2-(diméthylamino)éthyl)amino)-6-(pyrazolo[1,5-a]pyrimidin-3-yl)-1H-pyrazolo[4,3-c]pyridin-1-yl)-3-méthoxybenzyl)méthanesulfonamide ;
N-(2-chloro-5-méthoxy-4-(3-(méthylamino)-6-(pyrazolo[1,5-a]pyrimidin-3-yl)-1H-pyrazolo[4,3-c]pyridin-1-yl)benzyl)méthanesulfonamide ;
N-(2-chloro-5-méthoxy-4-(3-((2-morpholinoéthyl)amino)-6-(pyrazolo[1,5-a]pyrimidin-3-yl)-1H-pyrazolo[4,3-c]pyridin-1-yl)benzyl)méthanesulfonamide ;
N-(3-(diméthylamino)propyl)-1-(2-méthoxy-4-(méthylsulfonamidométhyl)phényl)-6-(pyrazolo[1,5-a]pyrimidin-3-yl)-1H-pyrazolo[4,3-c]pyridine-3-carboxamide ;
N-(4-(3-(cyanométhyl)-6-(pyrazolo[1,5-a]pyrimidin-3-yl)-1H-pyrazolo[4,3-c]pyridin-1-yl)-3-méthoxybenzyl)méthanesulfonamide ;
N-(3-(difluorométhoxy)-4-(3-méthyl-6-(pyrazolo[1,5-a]pyrimidin-3-yl)-1H-pyrazolo[4,3-c]pyridin-1-yl)benzyl)méthanesulfonamide ;
N-(1-(3-méthoxy-4-(3-méthyl-6-(pyrazolo[1,5-a]pyrimidin-3-yl)-1H-pyrazolo[4,3-c]pyridin-1-yl)phényl)éthyl)méthanesulfonamide ;
N-(3-méthoxy-4-(3-méthyl-6-(pyrazolo[1,5-a]pyrimidin-3-yl)-1H-pyrazolo[4,3-c]pyridin-1-yl)benzyl)méthanesulfonamide ;
N-(3-méthoxy-4-(3-méthyl-6-(pyrazolo[1,5-a]pyrimidin-3-yl)-1H-pyrazolo[4,3-c]pyridin-1-yl)benzyl)cyclopropanesulfonamide ;
N-(3-méthoxy-4-(3-méthyl-6-(pyrazolo[1,5-a]pyrimidin-3-yl)-1H-pyrazolo[4,3-c]pyridin-1-yl)benzyl)-N-méthylméthanesulfonamide ;
(3-méthoxy-4-(3-méthyl-6-(pyrazolo[1,5-a]pyrimidin-3-yl)-1H-pyrazolo[4,3-c]pyridin-1-yl)phényl)méthanol ;
(3-méthoxy-4-(3-méthyl-6-(pyrazolo[1,5-a]pyrimidin-3-yl)-1H-pyrazolo[4,3-c]pyridin-1-yl)phényl)méthanamine ;
1-cyano-N-(3-méthoxy-4-(3-méthyl-6-(pyrazolo[1,5-a]pyrimidin-3-yl)-1H-pyrazolo[4,3-c]pyridin-1-yl)benzyl)méthanesulfonamide ;
(3-méthoxy-4-(3-méthyl-6-(pyrazolo[1,5-a]pyrimidin-3-yl)-1H-pyrazolo[4,3-c]pyridin-1-yl)benzyl)carbamate de méthyle ;
N-(3-méthoxy-4-(3-méthyl-6-(pyrazolo[1,5-a]pyrimidin-3-yl)-1H-pyrazolo[4,3-c]pyridin-1-yl)benzyl)acétamide ;
1-(3-méthoxy-4-(3-méthyl-6-(pyrazolo[1,5-a]pyrimidin-3-yl)-1H-pyrazolo[4,3-c]pyridin-1-yl)benzyl)-3-méthylurea ;
1-(3-méthoxy-4-(3-méthyl-6-(pyrazolo[1,5-a]pyrimidin-3-yl)-1H-pyrazolo[4,3-c]pyridin-1-yl)benzyl)-3-phénylurea ;
2,2-difluoro-N-(3-méthoxy-4-(3-méthyl-6-(pyrazolo[1,5-a]pyrimidin-3-yl)-1H-pyrazolo[4,3-c]pyridin-1-yl)phényl)éthane-1-sulfonamide ;
N-(3-méthoxy-4-(3-méthyl-6-(pyrazolo[1,5-a]pyrimidin-3-yl)-1H-pyrazolo[4,3-c]pyridin-1-yl)phényl)benzamide ;
N-(4-(3-((2-(diméthylamino)éthyl)amino)-6-(pyrazolo[1,5-a]pyrimidin-3-yl)-1H-pyrazolo[4,3-c]pyridin-1-yl)-3-méthoxyphényl)benzamide ;
N-(4-(6-(imidazo[1,2-b]pyridazin-3-yl)-3-((2-morpholinoéthyl)amino)-1H-pyrazolo[4,3-c]pyridin-1-yl)-3-méthoxyphényl)méthanesulfonamide ;
N-(4-(3-((2-(diméthylamino)éthyl)amino)-6-(imidazo[1,2-b]pyridazin-3-yl)-1H-pyrazolo[4,3-c]pyridin-1-yl)-3-méthoxyphényl)méthanesulfonamide ;
N-(4-(3-(((1s,3s)-3-(diméthylamino)cyclobutyl)amino)-6-(pyrazolo[1,5-a]pyrimidin-3-yl)-1H-pyrazolo[4,3-c]pyridin-1-yl)-3-méthoxyphényl)cyclopropanesulfonamide ;
N-(4-(3-amino-6-(pyrazolo[1,5-a]pyrimidin-3-yl)-1H-pyrazolo[4,3-c]pyridin-1-yl)-3-méthoxyphényl)méthanesulfonamide ;
N-(1-(2-méthoxy-4-(méthylsulfonamido)phényl)-6-(pyrazolo[1,5-a]pyrimidin-3-yl)-1H-pyrazolo[4,3-c]pyridin-3-yl)-2-(méthylamino)acétamide ;
N-(1-(2-méthoxy-4-(méthylsulfonamido)phényl)-6-(pyrazolo[1,5-a]pyrimidin-3-yl)-1H-pyrazolo[4,3-c]pyridin-3-yl)-2-(4-méthylpipérazin-1-yl)acétamide ;
N-(1-(2-méthoxy-4-(propylsulfonamido)phényl)-6-(pyrazolo[1,5-a]pyrimidin-3-yl)-1H-pyrazolo[4,3-c]pyridin-3-yl)-3-(4-méthylpipérazin-1-yl)propanamide ;
N-(3-méthoxy-4-(3-((méthylamino)méthyl)-6-(pyrazolo[1,5-a]pyrimidin-3-yl)-1H-pyrazolo[4,3-c]pyridin-1-yl)phényl)propane-1-sulfonamide ;
N-(4-(3-(cyanométhyl)-6-(pyrazolo[1,5-a]pyrimidin-3-yl)-1H-pyrazolo[4,3-c]pyridin-1-yl)-3-méthoxyphényl)cyclopropanesulfonamide ;
N-(3-méthoxy-4-(3-méthyl-6-((3-oxo-3,4-dihydropyrazin-2-yl)amino)-1H-pyrazolo[4,3-c]pyridin-1-yl)phényl)méthanesulfonamide ;
N-(3-méthoxy-4-(3-méthyl-6-((3-oxo-3,4-dihydropyrazin-2-yl)amino)-1H-pyrazolo[4,3-c]pyridin-1-yl)phényl)-1-méthyl-1H-pyrazole-4-sulfonamide ;
N-(3-méthoxy-4-(3-((2-morpholinoéthyl)amino)-6-((3-oxo-3,4-dihydropyrazin-2-yl)amino)-1H-pyrazolo[4,3-c]pyridin-1-yl)phényl)méthanesulfonamide ;
N-(3-méthoxy-4-(6-((3-oxo-3,4-dihydropyrazin-2-yl)amino)-3-((2-(pipéridin-1-yl)éthyl)amino)-1H-pyrazolo[4,3-c]pyridin-1-yl)phényl)propane-1-sulfonamide ;
N-(3-(difluorométhoxy)-4-(3-((2-morpholinoéthyl)amino)-6-((3-oxo-3,4-dihydropyrazin-2-yl)amino)-1H-pyrazolo[4,3-c]pyridin-1-yl)phényl)propane-1-sulfonamide ;
N-(4-(3-((2-(3-oxa-8-azabicyclo[3.2.1]octan-8-yl)éthyl)amino)-6-((3-oxo-3,4-dihydropyrazin-2-yl)amino)-1H-pyrazolo[4,3-c]pyridin-1-yl)-3-méthoxyphényl)méthanesulfonamide ;
N-(3-méthoxy-4-(3-(méthylamino)-6-((3-oxo-3,4-dihydropyrazin-2-yl)amino)-1H-pyrazolo[4,3-c]pyridin-1-yl)phényl)propane-1-sulfonamide ;
N-(3-méthoxy-4-(3-((2-morpholinoéthyl)amino)-6-((3-oxo-3,4-dihydropyrazin-2-yl)amino)-1H-pyrazolo[4,3-c]pyridin-1-yl)benzyl)méthanesulfonamide ;
énantiomères simples, diastéréo-isomères et mélanges de ceux-ci
ou sel ou solvate pharmaceutiquement acceptable de ceux-ci.

5. Composition pharmaceutique comprenant un composé selon l'une quelconque des revendications 1 à 4, ou un sel pharmaceutiquement acceptable de celui-ci, en mélange avec un ou plusieurs supports ou excipients pharmaceutiquement acceptables.

6. Composition pharmaceutique selon la revendication 5, apte à être administrée par inhalation, choisie parmi des poudres inhalables, aérosols-doseurs contenant un agent propulseur ou formulations inhalables sans agent propulseur.

7. Dispositif comprenant la composition pharmaceutique selon la revendication 6, qui peut être un inhalateur de poudre sèche à dose unique ou à doses multiples, un aérosol-doseur ou un nébuliseur à brouillard doux.

8. Composé ou composition pharmaceutique selon l'une quelconque des revendications 1 à 6, pour une utilisation en tant que médicament.

9. Composé ou composition pharmaceutique pour utilisation selon la revendication 8, dans la prévention et/ou le traitement de maladie pulmonaire choisie dans le groupe constitué d'asthme, maladie pulmonaire obstructive chronique MPOC, fibrose pulmonaire idiopathique (IPF), lésion pulmonaire aiguë et syndrome de détresse respiratoire aiguë (ARDS).

10. Combinaison d'un composé tel que défini dans l'une quelconque des revendications 1 à 4 avec un ou plusieurs ingrédients actifs choisis parmi bêta-2-agonistes, agents antimuscariniques, corticostéroïdes, inhibiteurs de kinases activées par mitogène (kinases MAP P38), inhibiteurs de sous-unité bêta de kinase du facteur nucléaire kappa-B (IKK2), inhibiteurs d'élastase neutrophile humaine (HNE), inhibiteurs de phosphodiestérase 4 (PDE4), modulateurs de leucotriènes, anti-inflammatoires non stéroïdiens (AINS) et régulateurs de mucus.
